(19) Europäisches Patentamt
European Patent Office
Office européen des brevets

(11) **EP 1 978 973 B1**

(12) **EUROPEAN PATENT SPECIFICATION**

(45) Date of publication and mention of the grant of the patent:
**16.11.2011 Bulletin 2011/46**

(21) Application number: **06850419.0**

(22) Date of filing: **13.12.2006**

(51) Int Cl.:
***A61K 31/7088*** *(2006.01)* ***A61K 47/48*** *(2006.01)*
***G01N 33/50*** *(2006.01)* ***C12N 5/00*** *(2006.01)*
***A61P 35/00*** *(2006.01)*

(86) International application number:
**PCT/US2006/062052**

(87) International publication number:
**WO 2007/089367 (09.08.2007 Gazette 2007/32)**

(54) **USE HEDGEHOG KINASE ANTAGONISTS TO INHIBIT HEDGEHOG SIGNALING AND TO TREAT CANCER**

VERFAHREN ZUR VERWENDUNG VON HEDGEHOG-KINASE-ANTAGONISTEN ZUR BEHANDLUNG VON HEDGEHOG-VERMITTELTEN KREBS

UTILISATION D'ANTAGONISTES DE HEDGEHOG KINASE POUR LE TRAITEMENT DE CANCERS LIÉS AU MÉCANISME HEDGEHOG

(84) Designated Contracting States:
**AT BE BG CH CY CZ DE DK EE ES FI FR GB GR HU IE IS IT LI LT LU LV MC NL PL PT RO SE SI SK TR**

(30) Priority: **27.12.2005 US 754514 P**
**27.12.2005 US 754464 P**
**27.12.2005 US 754516 P**
**27.12.2005 US 754557 P**
**21.02.2006 US 775525 P**

(43) Date of publication of application:
**15.10.2008 Bulletin 2008/42**

(73) Proprietors:
- **Genentech, Inc.**
**South San Francisco CA 94080-4990 (US)**
- **Curis, Inc.**
**Lexington, MA 02421 (US)**

(72) Inventors:
- **DE SAUVAGE, Frederic, J.**
**Foster City, California 94404 (US)**
- **EVANGELISTA, Marie**
**San Francisco, California 94110 (US)**

(74) Representative: **Dey, Michael et al**
**Weickmann & Weickmann**
**Patentanwälte**
**Richard-Strauss-Strasse 80**
**81679 München (DE)**

(56) References cited:
**WO-A-02/068444** **WO-A-03/000873**
**WO-A-03/070283** **WO-A-2004/054657**
**WO-A-2005/052134**

- **REAGAN-SHAW SHANNON ET AL: "Silencing of polo-like kinase (Plk) 1 via siRNA causes induction of apoptosis and impairment of mitosis machinery in human prostate cancer cells: implications for the treatment of prostate cancer" THE FASEB JOURNAL,, vol. 19, no. 6, 1 April 2005 (2005-04-01), pages 611-613, XP002482340**
- **GRIFFIN K.J. ET AL: "Down-regulation of regulatory subunit type IA of protein kinase A leads to endocrine and other tumors." CANCER RES., vol. 64, 15 December 2004 (2004-12-15), pages 8811-8815, XP002486913**
- **WIEDEMANN M.W. AND CACA K.: "Molecularly targeted therapy for gastrointestinal cancer" CURRENT CANCER DRUG TARGETS, vol. 5, 2005, pages 171-193, XP008068475**
- **BHAVANA D. ET AL.: "CDC2L1 gene expression in non-Hodgkin's lymphoma." PROCEEDINGS OF THE AMERICAN ASSOCIATION FOR CANCER RESEARCH ANNUAL MEETING MARCH 2000, vol. 41, April 2000 (2000-04), pages 776-776, XP001538385**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 978 973 B1

## Description

FIELD OF THE INVENTION

The present invention relates to the novel use of the hedgehog kinases CDC2L1, CSNK1A1, GYK, NEK1, PLK1, PRKAR1A, PRKRA, TTBK2, TTK and antagonists thereagainst to regulate hedgehog signaling and their therapeutic use in various physiological conditions or disorders that are in part mediated by or result therefrom.

BACKGROUND OF THE INVENTION

Members of the *hedgehog* family of signaling molecules mediate many important short-and long-range patterning processes during invertebrate and vertebrate embryonic, fetal, and adult development. In drosophila melanogaster, a single hedgehog gene regulates segmental and imaginal disc patterning. In contrast, in vertebrates, a hedgehog gene family is involved in the control of proliferation, differentiation, migration, and survival of cells and tissues derived from all three germ layers, including, *e.g.*, left-right asymmetry, CNS development, somites and limb patterning, chondrogenesis, skeletogenesis and spermogenesis.

The vertebrate family of hedgehog genes includes at least four members or paralogs of the single *drosophila* hedgehog gene (WO 95/18856 and WO 96/17924). Three of these members, known as Desert hedgehog (*Dhh*), Sonic hedgehog (*Shh*) and Indian hedgehog (*Ihh*), apparently exist in all vertebrates, including fish, birds and mammals. *Dhh* is expressed principally in the testes, both in mouse embryonic development and in the adult rodent and human; *Ihh* is involved in bone development during embryogenesis and in bone formation in the adult; and *Shh* is involved in multiple embryonic and adult cell types derived from all three lineages. Shh is expressed at high levels in the notochoard and floorplate of developing vertebrate embryos, and directs cell fate in the developing limb, somites and neural tube. *In vitro* explant assays as well as ectopic expression of *Shh* in transgenic animals show that Shh plays a key role in neural tube patterning, Echelard et al., (1993), Cell 75: 1417-30 (1993); Ericson et al., Cell 81: 747-56 (1995); Marti et al., Nature 375: 322-25 (1995); Hynes et al., Neuron 19: 15-26 (1997).

Hedgehog signaling also plays a role in the development of limbs (Krauss et al., Cell 75: 1431-44 (1993); Laufer et al., Cell 79: 1165-73 (1994); somites (Fan and Tessier-Lavigne, Cell 79: 1175-86 (1994); Johnson et al., Cell 79: 1165-73 (1994), lungs (Bellusci et al., Devel. 124: 53-63 (1997) and skin (Oro et al., Science 276: 817-21 (1997). Likewise, *Ihh* and *Dhh* are involved in bone, gut and germinal cell development (Apelqvist et al., Curr. Biol. 7: 801-804 (1997); Bellusci et al., Dev. Suppl. 124: 53-63 (1997); Bitgood et al., Curr. Biol. 6: 298-304 (1996); Roberts et al., Development 121: 3163-74 (1995). Specifically, *Ihh* has been implicated in chrondrocyte development (Vortkamp et al., Science 273: 613-22 (1996)), while *Dhh* plays a key role in testes development.

Hedgehog signaling occurs through the interaction of hedgehog protein (e.g., in mammals, *Shh*, *Dhh*, *Ihh*, collectively "*Hh*") with the hedgehog receptor, Patched (*Ptch*), and the co-receptor Smoothened (*Smo*). There are two mammalian homologs of *Ptch*, *Ptch*-1 and *Ptch*-2 ("collectively "*Ptch*"), both of which are 12 transmembrane proteins containing a sterol sensing domain (Motoyama et al., Nature Genetics 18: 104-106 (1998), Carpenter et al., P.N.A.S. (U.S.A.) 95 (23): 13630-40 (1998). The interaction of *Hh* with *Ptch* triggers a signaling cascade that results in the regulation of transcription by zinc-finger transcriptions factors of the Gli family.

The binding of *Hh* to *Ptch* releases Smoothened (Smo), a 7 transmembrane G-coupled protein to then activate an intricate intracellular signal-transduction pathway. The activation of Smo then leads to signaling through a multimolecular complex, including Costa12 (Cos2), Fused (Fu) and suppressor of Fused (Su(Fu)), resulting in nuclear transport of the transcription factor Gli. Ho et al., Curr. Opin. Neurobiol. 12:57-63 (2002); Nybakken et al., Curr. Opin. Genet. Dev. 12: 503-511 (2002); i Altaba et al., Nat. Rev. Neurosci. 3: 24-33 (2002). There are three known *Gli* transcription factors in verebrates: *Gli1*, *Gli2* and *Gli3*. While *Gli1* is a transcriptional activator that is universally induced in *Hh*-responsive cells, Gli2 and Gli3 can act either as activators or repressors of transcription depending on the cellular context. Absent *Hh* signaling, *Gli3* is processed into a smaller, nuclear transcriptional repressor that lacks the carboxy-terminal domain of full-length *Gli3.* Upon activation of *Smo*, *Gli3* protein cleavage is prevented, and the full-length form with transcription-activation function is generated *Gli2* also encodes a repressor function in its carboxy-terminally truncated form, but its formation does not appear to be regulated by *Hh* signaling. Stecca et al., J. Biol. 1(2):9 (2002).

Malignant tumors (cancers) are the second leading cause of death in the United States, after heart disease (Boring et al., CA Cancel J. Clin. 43:7 (1993)). Cancer is characterized by the increase in the number of abnormal, or neoplastic, cells derived from a normal tissue which proliferate to form a tumor mass, the invasion of adjacent tissues by these neoplastic tumor cells, and the generation of malignant cells which eventually spread via the blood or lymphatic system to regional lymph nodes and to distant sites via a process called metastasis. In a cancerous state, a cell proliferates under conditions in which normal cells would not grow. Cancer manifests itself in a wide variety of forms, characterized by different degrees of invasiveness and aggressiveness.

Hedgehog signaling has been implicated in a wide variety of cancers and carcinogenesis. One example of the carcinogenic process is vascularization. Angiogenesis, the process of sprouting new blood vessels from existing vasculature and arteriogenesis, the remodeling of small vessels into larger conduct vessels are both physiologically important aspects of vascular growth in adult tissues (Klagsbrun and D'Amore, Annu. Rev. Physiol. 53: 217-39 (1991); Folkman and Shing, J. Biol. Chem. 267(16): 10931-4 (1992); Beck and D'Amore, FASEB J. 11(5): 365-73 (1997); Yancopoulos et al., Cell 93(5): 661-4 (1998); Buschman and Scaper, J. Pathol. 190(3): 338-42 (2000). These processes ofvascular growth are also required for beneficial processes such as tissue repair, wound healing, recovery from tissue ischemia and menstrual cycling. However, they are also required for the development of pathological conditions such as the growth of neoplasias, diabetic retinopathy, rheumatoid arthritis, psoriasis, certain forms of macular degeneration, and certain inflammatory pathologies (Cherrington et al., Adv. Cancer Res. 79:1-38 (2000). Thus, the inhibition of vascular growth can inhibit cellular proliferation, growth, differentiation and/or survival. As Hh has been shown to promote angiogenesis, Hh antagonists would be expected to possess anti-angiogenic properties.
Kinases are important components of most biological signaling pathways, and the hedgehog signaling pathway is no exception. Applicants have herein identified certain kinases necessary for effective hedgehog signaling. As a result, molecules that modulate these kinases would be expected to modulate hedgehog signaling, as well as to treat various conditions and/or disorders in which hedgehog signaling is a determinative factor.

WO 2004/054657 discloses antisense oligonucleotides that are directed against PLK1.

WO 03/000873 is directed to antisense approaches against lumar serine/theonine protein kinase NEK1.

Summary of the Invention

The present invention is defined by the appended set of claims.

In particular, the present invention relates to the hedge log kinases CSNK1A1, GYK and PRKRA.

All other kinases are presented for illustrative purposes only.

## BRIEF DESCRIPTION OF THE DRAWINGS

**Figures 1 A-I** show the derived amino acid sequence of a murine CDC2L1, CSNK1A1, GYK, NEK1, PLK1, PRKAR1A, PRKRA, TTBK2, TTK native sequence hedgehog kinase polypeptide, SEQ ID NOS: 1-8, respectively

**Figures 2 A-I** show the nucleotide sequence of a cDNA encoding a murine CDC2L1, CSNK1A1, GYK, NEK1, PLK1, PRKAR1A, PRKRA, TTBK2, TTK native sequence hedgehog kinase, respectively. The nucleotide sequences SEQ ID NOS: 9-18, respectively, are clones designated herein as DNA188786, DNA188624, DNA189856, DNA245262, DNA238060, DNA245338, DNA390234, DNA395917, DNA243238, respectively, under the unique gene identification number UNQ7109, UNQ3289, UNQ7640, UNQ10553, UNQ3368, UNQ1101, UNQ14535, UNQ29308, UNQ13762, respectively.

**Figures 3 A-I** show the derived amino acid sequence of a human CDC2L1, CSNK1A1, GYK, NEK1, PLK 1, PRKAR1A, PRKRA, TTBK2, TTK native sequence hedgehog kinase polypeptide, SEQ ID NOS: 19-27, respectively.

**Figures 4 A-I** show the nucleotide sequence of a cDNA encoding a human CDC2L1, CSNK1A1, GYK, NEK1, PLK1, PRKAR1A, PRKRA, TTBK2, TTK native sequence hedgehog kinase, respectively. The nucleotide sequences SEQ ID NO:28-36, respectively, are clones designated herein as DNA363066, DNA254256, DNA370652, DNA364981, DNA227497, DNA357103, DNA354038, DNA380434, DNA269878, under the unique gene identification number UNQ7109, UNQ3289, UNQ7640, UNQ10553, UNQ3368, UNQ1101, UNQ14535, UNQ29308, UNQ13762, respectively.

**Figure 5A** shows a schematic of activation of the hedgehog signaling pathway in the Luciferase reporter assay. In unstimulated cells, Patched (Ptchl) inhibits smoothened (Smo). Upon Hedgehog (Hh) binding, the inhibition is relieved and activated Smo induces a signal transduction pathway leading to the activation of transcription factors Glil and Gli2, and activation of Hedgehog target genes. In the Luciferase Assay, nine Gli1/2 binding sites were fused to the Luciferase Reporter (9XGliBS- Luciferase) and integrated into murine C3H10T1/2 fibroblasts. Thus, the 9XGliBS-Luciferase reporter acts as a faithful measure of Hedgehog pathway induction.

**Figure 5B** shows S12 cells (murine C3H10T1/2 fibroblasts stably expressing the 9XGliBS-Luciferase reporter), transfection of a non-targeting control siRNA does not affect the ability of cells to respond to hedgehog as measured by luciferase induction. In contrast, cells transfected with siRNAs against Smo prevents the ability of cells to respond to hedgehog stimulus. S12 cells were transfected with sRNA and treated with 200 ng/ml of recombinant Octyl-modified Sonic Hedgehog (O-Shh) sixty-six hours post-transfection. Levels of Smo protein (inner panel) were monitored using an antibody (14A5) that detects the COOH-tail of Smo.

**Figure 5C** shows a screen layout of hedgehog siRNA kinome screen. 3248 siRNAs targeting 812 genes (4 siRNAs/target) consisting of all murine kinases and kinase-regulatory proteins. Transfection regent was added to the plates and allowed to incubate for 20 minutes. S12 cells were then seeded at 11,000 cells/well. Sixty-six hours post-transfection, 200ng/ml O-Shh was added to induce the hedgehog pathway. Luciferase production was measured after 24 hours.

**Figure 5D** is a graphical analysis of the kinome siRNA screen. The correlation coefficients (r value) between replicates indicate that the siRNA screen was reproducible. The scores from individual plates were plotted agains replicate plates for both untreated and Hh-treated cells.

**Figure 5E** is a scatter plot from for an individual screen. Raw luciferase values were converted to natural log (LN). To compare between different plates, LN values were normalized to the averaged non-targeting siRNA on each plate. Normalized values were then averaged between plate replicates and a score of "Hh" minus "no Hh" treatment was calculated for reach individual siRNA. Shown is a scatter plot where "Hh" minus "no Hh" treatment scores are plotted on the Y-axis and the corresponding 3248 siRNAs are plotted on the X-axis. siRNAs that scored outside 1.5 standard deviations from the mean of all siRNAs in the library were considered potential hits.

**Figure 6A** is a validation of hits by correlation of Hh-luciferase with mRNA knockdown. To validate the potential his, all four siRNAs for each corresponding gene were retested in the Hh-luciferase assay. A non-targeting siRNA was used as a negative control. Lines with diamond point represent Hh-luciferase values (as a percentage of non-targeting siRNA-treated cells) after stimulation with Shh. Lines with boxed points represent the corresponding mRNA knockdown (as a percentage of non-targeting siRNA-treated cells) as measured by qRT-PCR using gene-specific primers and Rp119 as a reference. Upper panel is the "2 Hit" category where 2 out of 4 siRNAs were found to affect Hh signaling, while the bottom panel is the "≥ 3 Hit" category where at least 3 siRNAs were found to affect Hh signaling. Boxes outlined in light grey are genes where all 4 corresponding siRNAs showed correlation between Hh-luciferase and mRNA knockdown.

**Figure 6B** is a bar graph demonstrating that candidate hits are specific to Hh signaling. Transfection of S12 cells using pooled siRNAs indicates that most candidate genes (with the exception of Pak6 and Scyl1) reduce Hh-luciferase while maintaining the level of mRNA knockdown.

**Figure 6C** is a bar graph showing that pooled siRNAs do not affect SV40-luciferase suggesting candidate genes are specific to Hh signaling.

**Figures 6D** is a bar graph showing that candidate genes affect activation of endogenous Hh-target genes Gli1 and Ptch1 by qRT-PCR analysis.

**Figure 7A** is an epistasis experiment of cells treated with pooled siRNAs targeting the 9 candidate genes, along with SMOM2 (constitutively active Smo mutanat) expression, indicating that 4 of the genes act upstream of Smo while 5 act downstream of Smo. Shown is the mean and standard deviation (SD) for three independent experiments.

**Figure 7B** is an epistasis analysis of cells treated with pooled siRNAs tarteting the 9 candidate genes along with GLI1 expression indicating that all hits act upstream of Glil. Shown is the mean and standard deviation (SD) for three independent experiments.

**Figure 7C** is an epistatis analysis using double diRNA experiments with candidate genes and Gli3 indicating that all 9 candidates act upstream of Gli3. Shown is the mean and standard deviation (SD) for three independent experiments.

**Figure 7D** is a graphical model summarizing where each of the candidate 9 kinases likely act in the mammalian Hh pathway. CSNK1A1, GYK, NEK1 and TTK likely act upstream, while CDC2L1, PLK1, PRKAR1A, PRKRA and TTBK2 likely act downstream of Smo. All candidates act upstream of th Gli transcription factors.

**Figure 7E** are photographs of a pool of stably transfected murine IMCD3 cells expressing control shRNA (lower panel) or NEK1 shRNA (upper panel) were stained with anti-acetylated tubulin antibody to visualize cilia (red) or DAPI to visualize nuclei (blue). 2 to 4 μm of optical sections obtained from the apical surface of cells were flattened and tilted for the presentation.

**Figure 8A** is a representation of a Z-factor calculation of 0.61, indicating that the RNAi assay was robust. To calculate the Z factor for the screen, an average and standard deviation was calculated for cells transfected with either non-targeting or Smo siRNA. A Z factor calculation was measured suing the formula: Z factor = $1-[3 (\sigma_p + \sigma_n)/ | \mu_p - \mu_n | ]$.

**Figure 8B** demonstrates that NEK1 has a role in cilia formation. Stably transfect murine IMCD3 cells expressin control shRNA or NEK1 shRNA were stained with anti-acetylated tubulin antibody to visualize cilia. The graph represents the percentage of cell with a primary cilium in control of NEK1 shRNA-treated cells. Error bars represent the average of three independent counts were n = 300.

**Figure 8C** shows the co-expression of V5-tagged NEK1 expression construct along with pSuper or pSuper-NEK1 in HEK293 cells. pSuper-NEK1 is able to inhibit expression of NEK1-V5 protein as compared to pSuper alone.

## DETAILED DESCRIPTION OF THE INVENTION

### *I. Overview*

The present invention relates to the discovery that signal transduction pathways regulated by hedgehog signaling (*e.g.*, hedgehog, patched, smoothened, fused, suppressor of fused, *etc.*) can be inhibited, at last in partthrough antagonizing the activity of certain kinases that are active in the hedgehog signaling pathway.

Thus, it is specifically contemplated that hedgehog kinase antagonists identified herein will interfere with hedgehog signal transduction activity and will likewise be capable of changing the fate of a cell or tissue that is affected by hedgehog signaling, such as cells undergoing normal development or disease states that are characterized by aberrant hedgehog signaling. More specifically, such hedgehog signaling can occur either (i) as wild-type hedgehog signaling or (ii) result from hyperactivation of hedgehog pathway. Disorders resulting from hyperactivation of the hedgehog pathway can be attributed to mutations arising in hedgehog signaling components or inappropriate activation or stimulation that does not result from a mutation or lesion in a hedgehog signaling component. It is therefore desireable to have a method for identifying those cells in which the hedgehog pathway is hyperactive such that treatment with hedgehog kinase antagonists can be efficiently targeted. One of skill in the art will readily recognize that hedgehog kinase antagonists are suitable for the treatment of conditions or disorders characterized by hyperactive hedgehog signaling as well as modifying the cell fate during development by suppression of hedgehog concentration.

### *II. Definitions*

A hedgehog kinase of the invention is selected from CSNK1A1, GYK and PPKRA. All other kinase are for illustrative purpose only.

A "CDC2L1, CSNK1A1, GYK, NEK1, PLK1, PRKAR1A, PRKRA, TTBK2, TTK hedgehog kinase polypeptide", or "CDC2L1, CSNK1A1, GYK, NEK1, PLK1, PRKAR1A, PRKRA, TTBK2, TTK polypeptide" includes both "CDC2L1, CSNK1A1, GYK, NEK1, PLK1, PRKAR1A, PRKRA, TTBK2, TTK native sequence hedgehog kinase polypeptides" and "CDC2L1, CSNK1A1, GYK, NEK1, PLK1, PRKAR1A, PRKRA, TTBK2, TTK hedgehog kinase polypeptide variants", as described below.

A "native sequence CDC2L1, CSNK1A1, GYK, NEK1, PLK1, PRKAR1A, PRKRA, TTBK2, TTK hedgehog kinase polypeptide" comprises a polypeptide having the same amino acid sequence as the corresponding CDC2L1, CSNK1A1, GYK, NEK1, PLK1, PRKAR1A, PRKRA, TTBK2, TTK hedgehog kinase polypeptide derived from nature. Such native sequence hedgehog kinase polypeptides can be isolated from nature or can be produced by recombinant or synthetic means. The term "native sequence CDC2L1, CSNK1A1, GYK, NEK1, PLK1, PRKAR1A, PRKRA, TTBK2, TTK hedgehog kinase polypeptide" specifically encompasses naturally-occurring truncated, naturally-occurring variant forms (e.g., alternatively spliced forms) and naturally-occurring allelic variants of the polypeptide. In one specific aspect, the native sequence hedgehog kinase polypeptides disclosed herein are mature or full-length native sequence polypeptides corresponding to the sequence recited in Figures 1 and 3. This sequence is specifically referenced herein with the common name CDC2L1, CSNK1A1, GYK, NEK1, PLK1, PRKAR1A, PRKRA, TTBK2, TTK, with the murine sequence referred to as DNA189856, DNA245262, DNA243238, DNA395917, and the human sequence as DNA370652, DNA364981, DNA269878, DNA380434.

"CDC2L1, CSNK1A1, GYK, NEK1, PLK1, PRKAR1A, PRKRA, TTBK2, TTK hedgehog kinase polypeptide variant" means a CDC2L1, CSNK1A1, GYK, NEK1, PLK1, PRKAR1A, PRKRA, TTBK2, TTK hedgehog kinase polypeptide, preferably active forms thereof, as defined herein, having at least about 80% amino acid sequence identity with a full-length native sequence hedgehog kinase polypeptide sequence, as disclosed herein, and variant forms thereof lacking the signal peptide, a fragment sharing the hedgehog kinase activity of the full length native sequence, or any other fragment of a full length native sequence hedgehog kinase polyeptide polypeptide such as those referenced herein. Such variant polypeptides include, for instance, polypeptides wherein one or more amino acid residues are added, or deleted, at the N- or C-terminus of the full-length native amino acid sequence. In a specific aspect, such variant polypeptides will have at least about 80% amino acid sequence identity, alternatively at least about 81%, 82%, 83%, 84%, 85%, 86%, 87%, 88%, 89%, 90%, 91%, 92%, 93%, 94%, 95%, 96%, 97%, 98%, or 99% amino acid sequence identity, to a full-length native sequence hedgehog kinase polypeptide sequence polypeptide, as disclosed herein, and variant forms thereof lacking the signal peptide, a fragment sharing the hedgehog kinase activity of the full length native sequence, or any other fragment of a full length native sequence hedgehog kinase polypeptide polypeptide such as those disclosed herein. In a specific aspect, such variant polypeptides will vary at least about 1, 2, 3, 4, 5, 6, 7, 8, 9, 10, 20, 25, 30, 35, 40, 45, 50, 60, 70, 80, 90, 100, 125, 150, 200, 250, 300 or more amino acid residues in length from the corresponding native sequence polypeptide. Alternatively, such variant polypeptides will have no more than one conservative amino acid substitution as compared to the corresponding native polypeptide sequence, alternatively no more than 2, 3, 4, 5, 6, 7, 8, 9, or 10 conservative amino acid substitution as compared to the native polypeptide sequence.

"Percent (%) amino acid sequence identity" with respect to the CDC2L1, CSNK1A1, GYK, NEK1, PLK1, PRKAR1A, PRKRA, TTBK2, TTK hedgehog kinase polypeptide sequences identified herein is defined as the percentage of amino acid residues in a candidate sequence that are identical with the amino acid residues in the specific CDC2L1, CSNK1A1, GYK, NEK1, PLK1, PRKAR1A, PRKRA, TTBK2, TTK hedgehog kinase polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided was authored by Genentech, Inc. and has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California. The ALIGN-2 program should be compiled for use on a UNIX operating system, preferably digital UNIX V4.0D. All sequence comparison parameters are set by the ALIGN-2 program and do not vary.

"CDC2L1, CSNK1A1, GYK, NEK1, PLK1, PRKAR1A, PRKRA, TTBK2, TTK hedgehog kinase variant polynucleotide" or "CDC2L1, CSNK1A1, GYK, NEK1, PLK1, PRKAR1A, PRKRA, TTBK2, TTK" hedgehog kinase variant nucleic acid sequence" means a nucleic acid molecule which encodes a hedgehog kinase polypeptide, preferably active forms thereof, as defined herein, and which have at least about 80% nucleic acid sequence identity with a nucleotide acid sequence encoding a full-length native sequence hedgehog kinase polypeptide sequence identified herein, or any other fragment of the respective full-length hedgehog kinase polypeptide sequence as identified herein (such as those encoded by a nucleic acid that represents only a portion of the complete coding sequence for a full-length hedgehog kinase polypeptide). Ordinarily, such variant polynucleotides will have at least about 80% nucleic acid sequence identity, alternatively at least about 81%, 82%, 83%, 84%, 85%, 86%, 87%, 88%, 89%, 90%, 91%, 92%, 93%, 94%, 95%, 96%, 97%, 98%, or 99% nucleic acid sequence identity with a nucleic acid sequence encoding the respective full-length native sequence hedgehog kinase polypeptide sequence or any other fragment of the respective full-length hedgehog kinase polypeptide sequence identified herein. Such variant polynucleotides do not encompass the native nucleotide sequence.

Ordinarily, such variant polynucleotides vary at least about 50 nucleotides in length from the native sequence polypeptide, alternatively the variance can be at least about 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleotides in length, wherein in this context the term "about" means the referenced nucleotide sequence length plus or minus 10% of that referenced length.

"Percent (%) nucleic acid sequence identity" with respect to CDC2L1, CSNK1A1, GYK, NEK1, PLK1, PRKAR1A, PRKRA, TTBK2, TTK hedgehog kinase polypeptide-encoding nucleic acid sequences identified herein is defined as the percentage of nucleotides in a candidate sequence that are identical with the nucleotides in the hedgehog kinase nucleic acid sequence of interest, respectively, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, however, % nucleic acid sequence identity values are generated using the sequence comparison computer program ALIGN-2 was authored by Genentech, Inc. has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California. The ALIGN-2 program should be compiled for use on a UNIX operating system, preferably digital UNIX V4.0D. All sequence comparison parameters are set by the ALIGN-2 program and do not vary.

In situations where ALIGN-2 is employed for nucleic acid sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

$$100 \text{ times the fraction } W/Z$$

where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Tables 3 and 4, demonstrate how to calculate the % nucleic acid sequence identity of the nucleic acid sequence designated "Comparison DNA" to the nucleic acid sequence designated "REF-DNA", wherein "REF-DNA" represents a hypothetical hedgehog kinase-encoding nucleic acid sequence of interest, "Comparison DNA" represents the nucleotide sequence of a nucleic acid molecule against which the "REF-DNA" nucleic acid molecule of interest is being compared, and "N", "L" and "V" each represent different hypothetical nucleotides. Unless specifically stated otherwise, all % nucleic acid sequence identity values used herein are obtained as described in the immediately preceding paragraph using the ALIGN-2 computer program.

In other embodiments, hedgehog kinase variant polynucleotides are nucleic acid molecules that encode hedgehog kinase polypeptides, and which are capable of hybridizing, preferably under stringent hybridization and wash conditions, to nucleotide sequences encoding a full-length hedgehog kinase polypeptide, as disclosed herein. Such variant polypeptides may be those that are encoded by such variant polynucleotides.

"Isolated", when used to describe the various hedgehog kinase polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, such polypeptides will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Such isolated polypeptides include the corresponding polypeptides *in situ* within recombinant cells, since at least one component of the hedgehog kinase polypeptide from its natural environment will not be present. Ordinarily, however, such isolated polypeptides will be prepared by at least one purification step.

The term "hedgehog" or "hedgehog polypeptide" (Hh) is used herein to refer generically to any of the mammalian homologs of the *Drosophila* hedgehog, *i.e.*, sonic hedgehog (sHh), desert hedgehog (dHh) or Indian hedgehog (IHh). The term may be used to describe protein or nucleic acid.

The terms "hedgehog signaling pathway", "hedgehog pathway" and "hedgehog signal transduction pathway" as used herein, interchangeably refer to the signaling cascade mediated by hedgehog and its receptors (*e.g., patched, patched-2*) and which results in changes of gene expression and other phenotypic changes typical of hedgehog activity. The hedgehog pathway may be activated in the absence of hedgehog through activation of a downstream component (*e.g.*, overexpression of *Smoothened* or transfections with *Smoothened* or *Patched* mutants to result in constitutive activation of hedgehog signaling in the absence of hedgehog). The transcription factors of the *Gli* family are often used as markers or indicators of hedgehog pathway activation.

The term "Hh signaling component" refers to gene products that participate in the Hh signaling pathway. An Hh signaling component frequently materially or substantially affects the transmission of the Hh signal in cells or tissues, thereby affecting the downstream gene expression levels and/or other phenotypic changes associated with hedgehog pathway activation.

Each Hh signaling component, depending on their biological function and effects on the final outcome of the downstream gene activation or expression, can be classified as either positive or negative regulators. A positive regulator is an Hh signaling component that positively affects the transmission of the Hh signal, *i.e.*, stimulates downstream biological events when Hh is present. A negative regulator is an Hh signaling component that negative affects the transmission of the Hh signal, *i.e.* inhibits downstream biological events when Hh is present.

The term "hedgehog gain-of function" refers to an aberrant modification or mutation of a hedgehog signaling component (e.g., *ptch*, Smo, Fused, Su(fu), Cos-2, *etc.*) or a descrease (or loss) in the level of expression fo such a gene, which results in a phenotype which resembles contacting a cell with a hedgehog protein, e.g., aberrant activation of a hedgehog pathway. The gain-of function may include a loss of the ability of the ptch gene product to regulate the level of expression of the transcription activation factors Gli1, Gli2 and/or Gli3. The term "hedgehog gain-of-function" is also used herein to refer to any similar cellular phenotype (*e.g.*, exhibiting excess proliferation) that occurs due to an alteration anywhere in the hedgehog singal transduction pathway, including, but not limited to, a modification or mutation of hedgehog itself. For example, a tumor cell with an abnormally high proliferation rate to activation of the hedgehog signaling pathway would have a "hedgehog gain-of-function" phenotype, even if hedgehog is not mutated in that cell.

An "isolated" hedgehog kinase polypeptide-encoding nucleic acid is a nucleic acid molecule that is identified and separated from at least one contaminant nucleic acid molecule with which it is ordinarily associated in the natural source of the polypeptide-encoding nucleic acid. Any of the above such isolated nucleic acid molecule is other than in the form or setting in which it is found in nature. Any such nucleic acid molecules therefore are distinguished from the specific polypeptide-encoding nucleic acid molecule as it exists in natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1 % bovine serum albumin/0.1 % Ficoll/0.1 % polyvinylpyrrolidone/50mM sodium phosphate buffer at pH 6.5 with 750 mM sodium chloride, 75 mM sodium citrate at 42°C; or (3) overnight hybridization in a solution that employs 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C; each followed by an initial 10 minute wash at 42°C in 0.2 x SSC (sodium chloride/sodium citrate) followed by a 10 minute high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The ordinarily skilled artisan will recognize how to adjust the temperature, ionic strength, *etc.* as necessary to accommodate factors such as probe length and the like.

The term "epitope tagged" when used herein refers to a chimeric polypeptide comprising a hedgehog kinase polypeptide or hedgehog kinase binding agent fused to a "tag polypeptide". The tag polypeptide has enough residues to provide an epitope against which an antibody can be made, yet is short enough such that it does not interfere with the activity of the polypeptide to which it is fused. The tag polypeptide preferably also is sufficiently unique so that such antibody does not substantially cross-react with other epitopes. Suitable tag polypeptides generally have at least six amino acid residues and usually between about 8 and 50 amino acid residues (preferably, between about 10 and 20 amino acid residues).

"Active" or "activity" for the purposes herein refers to form(s) of a hedgehog kinase polypeptides which retain a biological and/or an immunological activity of native or naturally-occurring hedgehog kinase polypeptide, wherein "biological" activity refers to a biological function (either inhibitory or stimulatory) caused by a native or naturally-occurring hedgehog kinase other than the ability to induce the production of an antibody against an antigenic epitope possessed by a native or naturally-occurring hedgehog kinase polypeptide, and an "immunological" activity refers to the ability to induce the production of an antibody against an antigenic epitope possessed by a native or naturally-occurring hedgehog kinase polypeptide. An active hedgehog kinase polypeptide, as used herein, is an antigen that is differentially expressed, either from a qualitative or quantitative perspective, on a glioma tumor, relative to its expression on similar tissue that is not afflicted with glioma.

"Treating" or "treatment" or "alleviation" refers to both therapeutic treatment and prophylactic or preventative measures, wherein the object is to prevent or slow down (lessen) the progression of a disease. "Diagnosing" refers to the process of identifying or determining the distinguishing characteristics of a disease or tumor. The process of diagnosing is sometimes also expressed as staging or tumor classification based on severity or disease progression.

Subjects in need of treatment or diagnosis include those already with aberrant hedgehog signaling as well as those prone to having or those in whom aberrant hedgehog signaling is to be prevented. A subject or mammal is successfully "treated" for aberrant hedgehog signaling if, according to the method of the present invention, after receiving a therapeutic amount of a hedgehog kinase antagonist, the patient shows observable and/or measurable reduction in or absence of one or more of the following: reduction in the number of tumor cells or absence of such cells; reduction in the tumor size; inhibition (*i.e.*, slow to some extent and preferably stop) of tumor cell infiltration into peripheral organs including the spread of cancer into soft tissue and bone; inhibition (*i.e.*, slow to some extent and preferably stop) of tumor metastasis; inhibition, to some extent, of tumor growth; and/or relief to some extent, one or more of the symptoms associated with the specific cancer; reduced morbidity and mortality, and improvement in quality of life issues. To the extent such hedgehog kinase antagonists may prevent growth and/or kill existing cancer cells, it may be cytostatic and/or cytotoxic. Reduction of these signs or symptoms may also be felt by the patient.

The above parameters for assessing successful treatment and improvement in the disease are readily measurable by routine procedures familiar to a physician. For cancer therapy, efficacy can be measured, for example, by assessing the time to disease progression (TTP) and/or determining the response rate (RR). Metastasis can be determined by staging tests and tests for calcium level and other enzymes to determine the extent of metastasis. CT scans can also be done to look for spread to regions outside of the tumor or cancer. The invention described herein relating to the process of prognosing, diagnosing and/or treating involves the determination and evaluation of hedgehog kinase and hedgehog amplification and expression.

"Mammal" for purposes of the treatment of, alleviating the symptoms of or diagnosis of a cancer refers to any animal classified as a mammal, including humans, domestic and farm animals, and zoo, sports, or pet animals, such as dogs, cats, cattle, horses, sheep, pigs, goats, rabbits, ferrets, *etc.* Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN®, polyethylene glycol (PEG), and PLURONICS®.

By "solid phase" or "solid support" is meant a non-aqueous matrix to which a hedgehog kinase polypeptide or hedgehog kinase polypeptide of the present invention can adhere or attach. Examples of solid phases encompassed herein include those formed partially or entirely of glass (*e.g.*, controlled pore glass), polysaccharides (*e.g.*, agarose), polyacrylamides, polystyrene, polyvinyl alcohol and silicones. In certain embodiments, depending on the context, the solid phase can comprise the well of an assay plate; in others it is a purification column (*e.g.*, an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a hedgehog kinase antagonist) to a mammal. The components of the liposome are commonly arranged in a bilayer formation, similar to the lipid arrangement of biological membranes.

A "small molecule" or "small organic molecule" is defined herein to have a molecular weight below about 500 Daltons.

An "effective amount" of a hedgehog kinase antagonist agent is an amount sufficient to inhibit, partially or entirely, hedgehog signaling in a cell in which hedgehog signaling is active. Alternatively, an effective amount of hedgehog kinase antagonist is an amount sufficient to reduce the rate of proliferation of a cell and/or rate of survival of a cell that is expressing or overexpressing hedgehog. An "effective amount" may be determined empirically and in a routine manner, in relation to this purpose.

The term "therapeutically effective amount" refers to a hedgehog kinase antagonist or other drug effective to "treat" a disease or disorder in a subject or mammal. In the case of hedgehog signaling, the therapeutically effective amount of the drug will reduce or restore aberrant hedgehog signaling to normal physiological levels; reduce the tumor size; inhibit (*i.e.*, slow to some extent and preferably stop) the infiltration of tumor cells into peripheral tissue or organs; inhibit (*i.e.*, slow to some extent and preferably stop) tumor metastasis; inhibit, at least to some extent, tumor growth; and/or relieve to some extent one or more of the symptoms associated with the tumor or cancer. See the definition herein of "treating". To the extent the drug may prevent growth and/or kill existing cancer cells, it may be cytostatic and/or cytotoxic.

A "growth inhibitory amount" of a hedgehog kinase antagonist is an amount capable of inhibiting the growth of a cell, especially tumor, *e.g.*, cancer cell, either *in vitro* or *in vivo.* For purposes of inhibiting neoplastic cell growth, such an amount may be determined empirically and in a routine manner.

A "cytotoxic amount" of a hedgehog kinase antagonist is an amount capable of causing the destruction of a cell, especially a tumor cell, *e.g.*, cancer cell, either *in vitro* or *in vivo.* For purposes of inhibiting neoplastic cell growth may be determined empirically and in a routine manner.

The term "antibody" is used in the broadest sense and specifically covers, for example, anti-hedgehog kinase polypeptide monoclonal antibodies (including antagonist and neutralizing antibodies), anti-hedgehog kinase polypeptide antibody compositions with polyepitopic specificity, polyclonal antibodies, single chain anti-hedghog kinase antibodies, multispecific antibodies (*e.g.*, bispecific) and antigen binding fragments (see below) of all of the above enumerated antibodies as long as they exhibit the desired biological or immunological activity. The term "immunoglobulin" (Ig) is used interchangeably with antibody herein.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

The term "monoclonal antibody" as used herein refers to an antibody from a population of substantially homogeneous antibodies, *i.e.*, the individual antibodies comprising the population are identical and/or bind the same epitope (s), except for possible variants that may arise during production of the monoclonal antibody, such variants generally being present in minor amounts. Such monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. The modifier "monoclonal" indicates the character of the antibody as being obtained from a substantially homogeneous population of antibodies, and is not to be construed as requiring production of the antibody by any particular method.

"Chimeric" antibodies (immunoglobulins) have a portion of the heavy and/or light chain identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)). Humanized antibody as used herein is a subset of chimeric antibodies.

"Humanized" forms of non-human (*e.g.*, murine) antibodies are chimeric antibodies which contain minimal sequence derived from non-human immunoglobulin. For the most part, humanized antibodies are human immunoglobulins (recipient or acceptor antibody) in which hypervariable region residues of the recipient are replaced by hypervariable region residues from a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues which are not found in the recipient antibody or in the donor antibody. These modifications are made to further refine antibody performance such as binding affinity. Generally, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence although the FR regions may include one or more amino acid substitutions that improve binding affinity. The number of these amino acid substitutions in the FR is typically no more than 6 in the H chain, and in the L chain, no more than 3. The humanized antibody optionally also will comprise at least a portion of an immunoglobulin constant region (Fc), typically that of a human immunoglobulin. For further details, see Jones et al., Nature 321:522-525 (1986); Reichmann et al., Nature 332:323-329 (1988); and Presta, Curr. Op. Struct. Biol. 2:593-596 (1992).

"Antibody fragments" comprise a portion of an intact antibody, preferably the antigen binding or variable region of the intact antibody. Examples of antibody fragments include Fab, Fab', $F(ab')_2$, and Fv fragments; diabodies; linear antibodies (see U.S. Patent No. 5,641,870, Example 2; Zapata et al., Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, and a residual "Fc" fragment, a designation reflecting the ability to crystallize readily. The Fab fragment consists of an entire L chain along with the variable region domain of the H chain (VH), and the first constant domain of one heavy chain ($C_H1$). Each Fab fragment is monovalent with respect to antigen binding, *i.e.*, it has a single antigen-binding site. Pepsin treatment of an antibody yields a single large $F(ab')_2$ fragment which roughly corresponds to two disulfide linked Fab fragments having divalent antigen-binding activity and is still capable of cross-linking antigen. Fab' fragments differ from Fab fragments by having additional few residues at the carboxy terminus of the $C_H1$ domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known.

"Fv" is the minimum antibody fragment which contains a complete antigen-recognition and -binding site. This fragment consists of a dimer of one heavy- and one light-chain variable region domain in tight, non-covalent association. "Single-chain Fv" also abbreviated as "sFv" or "scFv" are antibody fragments that comprise the $V_H$ and $V_L$ antibody domains connected into a single polypeptide chain. Preferably, the sFv polypeptide further comprises a polypeptide linker between the $V_H$ and $V_L$ domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994); Borrebaeck 1995, *infra.*

The term "hedgehog kinase antagonist" includes any molecule that partially or fully blocks, inhibits, or neutralizes a biological activity of a CDC2L1, CSNK1A1, GYK, NEK1, PLK1, PRKAR1A, PRKRA, TTBK2, TTK hedgehog kinase polypeptide disclosed herein, including the attenuation of hedgehog signaling. Examples include the blocking the ability of a hedgehog ligand (e.g., Shh, Dhh, Ihh) to induce hedgehog sigaling upon binding to a hedgehog receptor (*e.g.*, *ptch*-1, *ptch*-2, Smo), or Smo from transmitting a signal to a *Gli* transcription factor or other downstream components in the hedgehog signaling pathway. Suitable antagonist molecules specifically include antagonist antibodies or antibody fragments, fragments or amino acid sequence variants of native hedgehog kinase polypeptides, peptides, antisense oligonucleotides, small organic molecules, RNAi molecules, Rnai contruct, *etc.* The "hedgehog kinase antagonist" may be directed to CDC2L1, CSNK1A1, GYK, NEK1, PLK1, PRKAR1A, PRKRA, TTBK2, TTK hedgehog kinase (*e.g.*, anti-CDC2L1, CSNK1A1, GYK, NEK1, PLK1, PRKAR1A, PRKRA, TTBK2, TTK antibody, a CDC2L1, CSNK1A1, GYK, NEK1, PLK1, PRKAR1A, PRKRA, TTBK2, TTK -binding oligopeptide, a CDC2L1, CSNK1A1, GYK, NEK1, PLK1, PRKAR1A, PRKRA, TTBK2, TTK antisense oligonucleotide, a CDC2L1, CSNK1A1, GYK, NEK1, PLK1, PRKAR1A, PRKRA, TTBK2, TTK RNAi molecule and a CDC2L1, CSNK1A1, GYK, NEK1, PLK1, PRKAR1A, PRKRA, TTBK2, TTK small molecule"). According to the invention "hedgehog kinase antagonist" is a CSNK1A1, GYK or PRKRA RNAi molecule.Methods for identifying hedgehog kinase antagonists may comprise contacting such a polypeptide, including a cell expressing it, with a candidate agonist or antagonist molecule and measuring a detectable change in one or more biological activities normally associated with such polypeptide.

An "interfering RNA" or RNAi is RNA of 10 to 50 nucleotides in length which reduces expression of a target gene, wherein portions of the strand are sufficiently complementary (e.g. having at least 80% identity to the target gene). The method of RNA interference refers to the target-specific suppression of gene expression (i.e., "gene silencing"), occurring at a post-transcriptional level (e.g., translation), and includes all posttranscriptional and transcriptional mechanisms of RNA mediated inhibition of gene expression, such as those described in P.D. Zamore, Science 296: 1265 (2002) and Hannan and Rossi, Nature 431: 371-378 (2004). As used herein, RNAi can be in the form of small interfering RNA (siRNA), short hairpin RNA (shRNA), and/or micro RNA (miRNA).

Such RNAi molecules are often a double stranded RNA complexes that may be expressed in the form of separate complementary or partially complementary RNA strands. Methods are well known in the art for designing double-stranded RNA complexes. For example, the design and synthesis of suitable shRNA and siRNA may be found in Sandy et al., BioTechniques 39: 215-224 (2005).

An "RNA coding region" is a nucleic acid that can serve as a template for the synthesis of an RNA molecule, such as a double-stranded RNA complex. Preferabley, the RNA coding region is a DNA sequence.

A "small interfering RNA" or siRNA is a double stranded RNA (dsRNA) duplex of 10 to 50 nucleotides in length which reduces expression of a target gene, wherein portions of the first strand is sufficiently complementary (e.g. having at least 80% identity to the target gene). siRNAs are designed specifically to avoid the anti-viral response characterized by elevated interferon synthesis, nonspecific protein synthesis inhibition and RNA degredation that oftern results in suicide or death of the cell associated with the use of RNAi in mammalian cells. Paddison et al., Proc Natl Acad Sci USA 99(3):1443-8. (2002).

The term "hairpin" refers to a looping RNA structure of 7-20 nucleotides.

A "short hairpin RNA" or shRNA is a single stranded RNA 10 to 50 nucleotides in length charactertized by a hairpin turn which reduces expression of a target gene, wherein portions of the RNA strand are sufficiently complementary (e.g. having at least 80% identity to the target gene).

The term "stem-loop" refers to a pairing between two regions of the same molecule base-pair to form a double helix that ends in a short unpaired loop, giving a lollipop-shaped structure.

A "micro RNA" (previously known as stRNA) is a single stranded RNA of about 10 to 70 nucleotides in length that are initially transcribed as pre-miRNA characterized by a "stem-loop" structure, which are subsequently processed into mature miRNA after further processing through the RNA-induced silencing complex (RISC).

A "hedgehog kinase binding oligopeptide" is an oligopeptide that binds, preferably specifically, to a hedgehog kinase polypeptide, including a receptor, ligand or signaling component, respectively, as described herein. Such oligopeptides may be chemically synthesized using known oligopeptide synthesis methodology or may be prepared and purified using recombinant technology. Such oligopeptides are usually at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more. Such oligopeptides may be identified without undue experimentation using well known techniques. In this regard, it is noted that techniques for screening oligopeptide libraries for oligopeptides that are capable of specifically binding to a polypeptide target are well known in the art (see, *e.g.*, U.S. Patent Nos. 5,556,762, 5,750,373, 4,708,871, 4,833,092, 5,223,409, 5,403,484, 5,571,689, 5,663,143; PCT Publication Nos. WO 84/03506 and WO84/03564; Geysen et al., Proc. Natl. Acad. Sci. U.S.A., 81:3998-4002 (1984); Geysen et al., Proc. Natl. Acad. Sci. U.S.A., 82:178-182 (1985); Geysen et al., in Synthetic Peptides as Antigens, 130-149 (1986); Geysen et al., J. Immunol. Meth., 102:259-274 (1987); Schoofs et al., J. Immunol., 140:611-616 (1988), Cwirla, S. E. et al. Proc. Natl. Acad. Sci. USA, 87:6378 (1990); Lowman, H.B. et al. Biochemistry, 30:10832 (1991); Clackson, T. et al. Nature, 352: 624 (1991); Marks, J. D. et al., J. Mol. Biol., 222:581 (1991); Kang, A.S. et al. Proc. Natl. Acad. Sci. USA, 88:8363 (1991), and Smith, G. P., Current Opin. Biotechnol., 2:668 (1991).

A hedgehog kinase antagonist (*e.g.*, oligopeptide, RNAi molecule, or small molecule) "which binds" a target antigen of interest, *e.g.* a hedgehog kinase, is one that binds the target with sufficient affinity so as to be a useful diagnostic, prognostic and/or therapeutic agent in targeting a cell or tissue expressing the antigen, and does not significantly cross-react with other proteins. The extent of binding to a non desired marker polypeptide will be less than about 10% of the binding to the particular desired target, as determinable by common techniques such as fluorescence activated cell sorting (FACS) analysis or radioimmunoprecipitation (RIA).

Moreover, the term "specific binding" or "specifically binds to" or is "specific for" a particular hedgehog kinase polypeptide or an epitope on a particular hedgehog kinse polypeptide target means binding that is measurably different from a non-specific interaction. Specific binding can be measured, for example, by determining binding of a molecule compared to binding of a control molecule, which generally is a molecule of similar structure that does not have binding activity. For example, specific binding can be determined by competition with a control molecule that is similar to the target, for example, an excess of non-labeled target. In this case, specific binding is indicated if the binding of the labeled target to a probe is competitively inhibited by excess unlabeled target. In one embodiment, such terms refer to binding where a molecule binds to a particular polypeptide or epitope on a particular polypeptide without substantially binding to any other polypeptide or polypeptide epitope. Alternatively, such terms can be described by a molecule having a Kd for the target of at least about $10^{-4}$ M, $10^{-5}$ M, $10^{-6}$ M, $10^{-7}$ M, $10^{-8}$ M, $10^{-9}$ M, $10^{-10}$ M, $10^{-11}$ M, $10^{-12}$ M, or greater.

A hedgehog kinase antagonist that "inhibits the growth of tumor cells expressing a hedgehog kinase polypeptide" or a "growth inhibitory" amount of any such molecule is one which results in measurable growth inhibition of cancer cells expressing a hedgehog kinase polypeptide. Preferred compositions for use in treatment comprise growth inhibitory amounts of at least one type of hedgehog kinase antagonist, so as to inhibit growth of tumor cells by greater than 20%, preferably from about 20% to about 50%, and even more preferably, by greater than 50% (e.g., from about 50% to about 100%) as compared to the appropriate control. In one embodiment, growth inhibition can be measured at a molecule concentration of about 0.1 to 30 μg/ml or about 0.5 nM to 200 nM in cell culture, where the growth inhibition is determined 1-10 days after exposure of the tumor cells to the antibody. Growth inhibition of glioma tumor cells in vivo can be determined in various ways such as is described in the Experimental Examples section below. An amount of any of the above molecules of this paragraph is growth inhibitory *in vivo* if administration of such molecule at about 1 μg/kg to about 100 mg/kg body weight results in reduction in tumor size or tumor cell proliferation within about 5 days to 3 months from the first administration of the antibody, preferably within about 5 to 30 days.

A hedgehog kinase antagonist that "induces apoptosis" is one which induces programmed cell death of a tumor cell as determined by binding of annexin V, fragmentation of DNA, cell shrinkage, dialation of endoplasmic reticulum, cell fragmentation, and/or formation of membrane vesicles (called apoptotic bodies). The cell is usually one which overexpresses a hedgehog polypeptide. Various methods are available for evaluating the cellular events associated with apoptosis. For example, phosphatidyl serine (PS) translocation can be measured by annexin binding; DNA fragmentation can be evaluated through DNA laddering; and nuclear/chromatin condensation along with DNA fragmentation can be evaluated by any increase in hypodiploid cells. Preferably, the antibody, oligopeptide or other organic molecule which induces apoptosis is one which results in about 2 to 50 fold, preferably about 5 to 50 fold, and most preferably about 10 to 50 fold, induction of annexin binding relative to untreated cells in an annexin binding assay.

A hedgehog kinase antagonist that "induces cell death" is one which causes a viable tumor or cancer cell to become nonviable. Such a cell is one which expresses a hedgehog signaling pathway, preferably overexpresses it, as compared to a non-diseased cell. Cell death *in vitro* may be determined in the absence of complement and immune effector cells to distinguish cell death induced by antibody-dependent cell-mediated cytotoxicity (ADCC) or complement dependent cytotoxicity (CDC). Thus, the assay for cell death may be performed using heat inactivated serum (*i.e.*, in the absence of complement) and in the absence of immune effector cells. The ability to induce cell death can be assessed relative to untreated cells by suitable techniques, such as loss of membrane integrity as evaluated by uptake of propidium iodide (PI), trypan blue (see Moore et al. Cytotechnology 17:1-11 (1995)) or 7AAD. In a specific aspect, cell death-inducing hedgehog kinase antagonists are those which induce PI uptake in the PI uptake assay in BT474 cells.

The terms "cell proliferative disorder" and "proliferative disorder" refer to disorders that are associated with some degree of abnormal cell proliferation. In one embodiment, the cell proliferative disorder is cancer.

"Tumor", as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include, but are not limited to, carcinoma, lymphoma, blastoma, sarcoma, and leukemia or lymphoid malignancies.

A tumor that "overexpresses" hedgehog or in which hedgehog signaling is "hyperactive" is one which has significantly higher levels of hedgehog at the cell surface, or one in which hedgehog signaling is activated to a greater extent, compared to a noncancerous cell of the same tissue type. Such overexpression may result from gene amplification or by increased transcription or translation. Various diagnostic or prognostic assays that measure enhanced expression of hedgehog resulting in increased levels at the cell surface or that which is secreted, such as immunohistochemistry assay using anti-hedgehog ligand or anti-hedgehog receptor antibodies, FACS analysis, *etc*. Alternatively, the levels of hedgehog -encoding nucleic acid or mRNA can be measured in the cell, *e.g.*, via fluorescent *in situ* hybridization using a nucleic acid based probe corresponding to a hedgehog-encoding nucleic acid or the complement thereof; (FISH; see WO98/45479 published October, 1998), Southern blotting, Northern blotting, or polymerase chain reaction (PCR) techniques, such as real time quantitative PCR (RT-PCR). Alternatively, hedgehog overexpression is determinable by measuring shed antigen in a biological fluid such as serum, *e.g*, using antibody-based assays (see also, *e.g.*, U.S. Patent No. 4,933,294 issued June 12, 1990; WO91/05264 published April 18, 1991; U.S. Patent 5,401,638 issued March 28, 1995; and Sias et al., J. Immunol. Methods 132:73-80 (1990)). Alternatively, hedgehog activity is determinable by measuring the expression of certain downstream reporters, e.g., the transcription factor Gli. In addition to the above assays, various *in vivo* assays are available to the skilled practitioner. For example, one may expose cells within the body of the patient to an antibody which is optionally labeled with a detectable label, *e.g.*, a radioactive isotope, and binding of the antibody to cells in the patient can be evaluated, *e.g.*, by external scanning for radioactivity or by analyzing a biopsy taken from a patient previously exposed to the therapeutic agent.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (*i.e.*, is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

The word "label" when used herein refers to a detectable compound or composition which is conjugated directly or indirectly to the antibody, oligopeptide or other organic molecule so as to generate a "labeled" antibody, oligopeptide or other organic molecule. The label may be detectable by itself (e.g. radioisotope labels or fluorescent labels) or, in the case of an enzymatic label, may catalyze chemical alteration of a substrate compound or composition which is detectable.

The term "cytotoxic agent" as used herein refers to a substance that inhibits or prevents the function of cells and/or causes destruction of cells. The term is intended to include radioactive isotopes (*e.g.*, $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu), chemotherapeutic agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

A "chemotherapeutic agent" is a chemical compound useful in the treatment of cancer. Examples of chemotherapeutic agents include hydroxyureataxanes (such as paclitaxel and doxetaxel) and/or anthracycline antibiotics; alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gamma 1I and calicheamicin omegaI1 (see, *e.g.*, Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-tricblorotriethylamind; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., TAXOL® paclitaxel (Bristol-Myers Squibb Oncology, Princeton, N.J.), ABRAXANETM Cremophor-free, albumin-engineered nanoparticle formulation of paclitaxel (American Pharmaceutical Partners, Schaumberg, Illinois), and TAXOTERE® doxetaxel (Rhône-Poulenc Rorer, Antony, France); chloranbucil; gemcitabine (GEMZAR®); 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluorometlhylomithine (DMFO); retinoids such as retinoic acid; capecitabine (XELODA®); pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATINTM) combined with 5-FU and leucovovin.

Also included in this definition are anti-hormonal agents that act to regulate, reduce, block, or inhibit the effects of hormones that can promote the growth of cancer, and are often in the form of systemic, or whole-body treatment. They may be hormones themselves.. Examples include anti-estrogens and selective estrogen receptor modulators (SERMs), including, for example, tamoxifen (including NOLVADEX® tamoxifen), EVISTA® raloxifene, droloxifene, 4-hydroxytamoxifen, trioxifene, keoxifene, LY117018, onapristone, and FARESTON® toremifene; anti-progesterones; estrogen receptor down-regulators (ERDs); agents that function to suppress or shut down the ovaries, for example, leutinizing hormone-releasing hormone (LHRH) agonists such as LUPRON® and ELIGARD® leuprolide acetate, goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole. In addition, such definition of chemotherapeutic agents includes bisphosphonates such as clodronate (for example, BONEFOS® or OSTAC®), DIDROCAL® etidronate, NE-58095, ZOMETA® zoledronic acid/zoledronate, FOSAMAX® alendronate, AREDIA® pamidronate, SKELID® tiludronate, or ACTONEL® risedronate; as well as troxacitabine (a 1,3-dioxolane nucleoside cytosine analog); antisense oligonucleotides, particularly those that inhibit expression of genes in signaling pathways implicated in abherant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; LURTOTECAN® topoisomerase 1 inhibitor, ABARELIX® rmRH; lapatinib ditosylate (an ErbB-2 and EGFR dual tyrosine kinase small-molecule inhibitor also known as GW572016); and pharmaceutically acceptable salts, acids or derivatives of any of the above.

A "growth inhibitory agent" when used herein refers to a compound or composition which inhibits growth of a cell, especially a cell with a hyperactive hedgehog pathway, either *in vitro* or *in vivo.* Thus, the growth inhibitory agent may be one which significantly reduces the percentage of hedgehog-expressing cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes or hydroxyureataxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). These molecules promote the assembly of microtubules from tubulin dimers and stabilize microtubules by preventing depolymerization, which results in the inhibition of mitosis in cells.

"Doxorubicin" is an anthracycline antibiotic. The full chemical name of doxorubicin is (8S-cis)-10-[(3-amino-2,3,6-trideoxy-α-L-lyxo-hexapyranosyl)oxy]-7,8,9,10-tetrahydro-6,8,11-trihydroxy-8-(hydroxyacetyl)-1-methoxy-5,12-naphthacenedione.

The term "package insert" is used to refer to instructions customarily included in commercial packages of therapeutic products, that contain information about the indications, usage, dosage, administration, contraindications and/or warnings concerning the use of such therapeutic products.

"Adenocarcinoma" refers to a malignant tumor originaling the glandular epithelium.

"Mesenchymal cells" are cells of mesenchymal origin including fibroblasts, stromal cells, smooth muscle cells, skeletal muscle cells, cells of osteogenic origin such as chondrocytes, cells of hematopoietic origin such as monocytes, macrophages, lymphocytes, granulocytes and cells of adipose origin such as adipocytes.

"Angiogenesis" is the formation of blood vessels, including both the formation of a new vasculature or alteration of an existing vascular system, which benefits tissue perfusion. This includes both the formation of new vessels by sprouting of endothelian cells from existing blood vessels or the remodeling of existing vessels to alter size, maturity, direction of flow properties to improve blood perfusion of tissue. While the latter process is sometimes referred more specifically as "arterogenesis", both processes are enveloped by the definition envisioned herein. Angiogenesis is a multistep process in which endothelial cells focally degrade and invaqde through their own basement membrane, migrate through interstitial stroma toward an angiogenic stimulus, proliferate proximal to the migrating tip, organize into blood vesels, and reattach to newly synthesized basement membrane. Folkman et al., Cancer Res. 43: 175-203 (1985).

"Basal cell carcinoma" refers to a variety fo clinical and histological forms of cancers skin tissues such as nodular-ulcerative, superficial, pigmented, morphealike, fibroepithelioma and nevoid syndrome.

"Burn wounds" are lesions in the skin resulting from exposure to heat or chemical agents.

"Carcinoma" refers to a malignant growth derived from epithelial cells that tends to metastasize to other areas of the body. Examples include "basal cell carcinoma" - an epithelial tumor of the skin that, while seldom metastasizing, can result in local invasion and destruction; "squamous cell carcinoma" - tumors arising from squamous epithelium and having cuboid cells; "carcinosarcoma" - malignant tumors comprising both carcinomatous and sarcomatous tissues; "adenocystic carcinoma"- tumors characterized by large epithelial masses containing round gland-like spaces or cysts, frequently containing mucus, that are bordered by layers of epithelial cells; - "epidermoid carcinoma"- see squamous cell carcinoma; "nasopharyngeal carcinoma" - malignant tumor arising in the epithelial lining of the space behind the nose; "renal cell carcinoma"- tumor in the renal parenchyma composed of tubular cells in varying arrangements. Additional carcinomatous epithelial growth include "papillomas", which are benign tumors derived from the epithelium and having papillomavirus as a causative agent; and "epidermoidomas", which are cerebral of meningeal tumors formed by inclusion of ectodermal elements at the time of closure of the neural groove.

"Corium" or "dermis" refers to the layer of the skin deep to the epidermis, consisting of a dense bed of vascular connective tissue, and containing the nerves and terminal organs of sensations. The hair roots, and sebaceous and sweat glands are structures of the epidermis which are deeply embedded in the dermis.

"Dermal skin ulcers" refer to lesions on the skin cause by superficial loss of tissue, usually with inflammation. Dermal skin ulcers that can be treated by the method of the present invention include decubitus ulcers, diabetic ulcers, venous stasis ulcers and arterial ulcers. Decubitus wounds are chronic ulcers resulting from the application of pressure to the skin for extended periods of time. These type of wounds are also referred to as bedsores or pressure sores. Venous statis ulcers result from the stagnation of blood or other fluids from defective veins. Arterial ulcers refer to necrotic skin in the area around arteries having poor blood flow.

"Epithelia," "epithelial" and "epithelium" refer to the cellular covering of internal and external body surfaces (cutaneous, mucous and serous), including the glands and other structures derived therefrom, *e.g.*, corneal, esophageal, epidermal, and hair follicle epithelial cells. Other exemplary epithelial tissue includes: olfactory epithelium - the pseudostratified epithelium lining the olfactory region of the nasal cavity, and containing the receptors for the sense of smell; glandular epithelium - the epithelium composed of secreting cells squamous epithelium; squamous epithelium - the epithelium comprising one or more cell layers, the most superficial of which is comosed of flat, scalelike or platelike cells. Epithelium can also refer to transitional epithelium, like that which is characteristically found lining hollow organs that are subject to great mechanical change due to contraction and distention, *e.g.*, tissue which represents a transition between stratified squamous and columnar epithelium.

"Epidermal gland" refers to an aggregation of cells associated with the epidermis and specialized to secrete or excrete materials not related to their ordinary metabolic needs. For example, "sebaceous glands" are holocrine glands in the corium that secrete and oily substance and sebum. The term "sweat glands" refers to glands that secret sweat, and situated in the corium or subcutaneous tissue.

"Epidermis" refers to the protective outermost and nonvascular layer of the skin.

"Excisional wounds" include tears, abrasions, cuts, punctures or lacerations in the epithelial layer of the skin and may extend into the dermal layer and deeper, and result from surgical procedures or accidental penetration of the skin.

The "growth state" of a cell refers to the rate of proliferation of the cell and/or the state of differentiation of the cell. An "altered growth state" is a growth state characterized by an abnormal rate of proliferation, e.g., a cell exhibiting an increased or decreased rate of proliferation relative to a normal cell.

The term "hedgehog" or "hedgehog polypeptide" (Hh) is used herein to refer generically to any of the mammalian homologs of the *Drosophilia* hedgehog, *i.e.*, sonic hedgehog (sHh), desert hedgehog (dHh) or Indian hedgehog (IHh). The term may be used to describe protein or nucleic acid.

The terms "hedgehog signaling pathway", "hedgehog pathway" and "hedgehog signal transduction pathway" as used herein, interchangeably refer to the signaling cascade mediated by hedgehog and its receptors (*e.g.*, *patched, patched-2*) and which results in changes of gene expression and other phenotypic changes typical of hedgehog activity. The hedgehog pathway may be activated in the absence of hedgehog through activation of a downstream component (*e.g.*, overexpression of *Smoothened* or transfections with *Smoothened* or *Patched* mutants to result in constitutive activation with activate hedgehog signaling in the absence of hedgehog). The transcription factors of the *Gli* family are often used as markers or indicators of hedgehog pathway activation.

The term "hedgehog (Hh) signaling component" refers to gene products that participate in the Hh signaling pathway. An Hh signaling component frequently materially or substantially affects the transmission of the Hh signal in cells or tissues, thereby affecting the downstream gene expression levels and/or other phenotypic changes associated with hedgehog pathway activation. Each Hh signaling component, depending on their biological function and effects on the final outcome of the downstream gene activation or expression, can be classified as either positive or negative regulators. A positive regulator is an Hh signaling component that positively affects the transmission of the Hh signal, *i.e.*, stimulates downstream biological events when Hh is present. A negative regulator is an Hh signaling component that negative affects the transmission of the Hh signal, i.e. inhibits downstream biological events when Hh is present.

The term "hedgehog gain-of function" refers to an aberrant modification or mutation of a hedgehog signaling component (*e.g., ptch,* Smo, Fused, Su(fu), *etc.)* or a descrease (or loss) in the level of expression fo such a gene, which results in a phenotype which resembles contacting a cell with a hedgehog protein, *e.g.*, aberrant activation of a hedgehog pathway. The gain-of function may include a loss of the ability of the ptch gene product to regulate the level of expression of the transcription activation factors Gli1, Gli2 and/or Gli3. The term "hedgehog gain-of function" is also used herein to refer to any similar cellular phenotype (*e.g.*, exhibiting excess proliferation) that occurs due to an alternation anywhere in the hedgehog singal transduction pathway, including, but not limited to, a modification or mutation of hedgehog itself. For example, a tumor cell with an abnormally high proliferation rate to activation of the hedgehog signaling pathway would have a "hedgehog gain-of function" phenotype, even if hedgehog is not mutated in that cell.

"Internal epithelial tissue" refers to tissue inside the body that has characteristics similar to the epidermal layer of the skin (*e.g.*, the lining of the instestine).

"Keratosis" refers to a proliferative skin disorder characterized by hyperplasis of the horny layer of the epidermis. Example keratotitic disorders include: keratosis follicularis, keratosis palmaris et plantaris, keratosis pharyngea, keratosis pilaris, and actinic keratosis.

"Lamellated bodies" refers to a subcellular structure found in lung cells that are producing surfactants. Lamellated bodies are believed to be the source of lung surfactant biosynthesis.

The term "overexpression" as used herein, refers to cellular gene expression levels of a tissue that is higher than the normal expression levels for that tissue.

The term "patched loss-of-function" refers to an aberrant modification or mutation of a ptch gene, or a decreased expression level of the gene, which results in a phenotype that resembles contacting the cell with a hedgehog protein, *e.g.*, aberrant activation of a hedgehog pathway. The loss-of-function may include a loss of the ability of the ptch gene product to regulate the expression level of the transcription activation factors Gli1, Gli2 and/or Gli3.

The term "smoothened gain-of-function" refers to an aberrant modification or mutation of a Smo gene, or in the ability of a ptch gene product to bind to Smo and thereby suppress hedgehog signaling, which results in a phenotype that resembles activating the hedgehog pathway with hedgehog, e.g., aberrant activation of a hedgehog pathway.

The term "proliferating" and "proliferation" refer to a cellor cells undergoing mitosis.

The term "proliferative skin disorder" refers to any disease/disorder of the skin marked by unwanted or aberrant proliferation of cutaneous tissue. Such conditions are typically characterized by epidermal proliferation or incomplete cell differentiation, and include, for example, X-linked ichthyosis, psoriasis, atopic dermatitis, allergic contact dermatitis, epidermolyitic hyperkeratosis, and seborrheic dermatitis. For example, epidermodysplasia is a form of faulty development of the epidermis. Another example is "epidermolysis", which refers to a loosened state of the epidermis with formation of blebs and bulae either spontenously or at the site of the trauma.

"Psoriasis" refers to a chronic, reoccurring, inflammatory, hyperproliferative skin disorder that alters the skin's regulatory mechanisms. In particular, lesions are formed which involve primary and secondary alternations in epidermal proliferation, inflammatory responses of the skin, and an expression of regulatory molecules such as lymphokines and inflammatory factors. Psoriatic skin is morphologically characterized by an increased turnover of epidermal cells, thickened epidermis, abnormal keratinization, inflammatory cell infiltrates into the dermis layer and polymorophonuclear leukocyte infiltration into the epidermis layer resulting in an increase in the basal cell cycle. There are five types, each with unique signs and symptoms. The most common is plaque psoriasis is the most common type of psoriasis, most commonly characterized as patches of raised, reddish skin covered by silvery-white scale,which appear on the elbows, knees, lower back, and scalp. The other types are (i) guttate psoriasis, characterized by small, red spots on the skin, (ii) pustular psoriasis, characterized by white pustules surrounded by red skin, (iii) inverse psoriasis, characterized by smooth, red lesions form in skin folds, and (iv) erythrodermic psoriasis, characterized by widespread redness, severe itching, and pain.

"Atopic dermatitis" (AD) (eczema) is a pruritic disease of unknown origin that is typified by pruritus, eczematous lesions, xerosis (dry skin), and lichenification on the skin (thickening of the skin and increase in skin markings). AD is associated with other atopic diseases (e.g., asthma, allergic rhinitis, urticaria, acute allergic reactions to foods, increased IgE production) in many patients. AD may also be precipitated in allergic individuals by exposure to allergens such as pollens, foods, dander, insect venoms and plant toxins.

"skin" refers to the outer protective covering of the body, consisting of the corium and the epidermis, including the sweat and sebaceous glands, as well as hair follicle structures.

"small cell carcinoma" refers to malignant neoplasms of the bronchus. Cells of such tumors have endocrine-like characteristics and may secrete one or more of a wide range of hormones, especially regulatory peptides like bombesin.

"Urogenital" refers to the organs and tissues of the urogenital tract, which includes among other tissues, the prostate, ureter, kidney, and bladder. A "urogenital cancer" is a cancer of a urogenital tissue.

**Table 1**

| | | |
|---|---|---|
| Reference | XXXXXXXXXXXXXXX | (Length = 15 amino acids) |
| Comparison Protein | XXXXXYYYYYYY | (Length = 12 amino acids) |
| % amino acid sequence identity | = | |

(the number of identically matching amino acid residues between the two polypeptide sequences as determined by ALIGN-2) divided by (the total number of amino acid residues of the reference polypeptide)

= 5 divided by 15 = 33.3%

**Table 2**

| | | | | |
|---|---|---|---|---|
| Reference | XXXXXXXXXX | (Length | = | 10 amino acids) |
| Comparison Protein | XXXXXYYYYYYZZYZ | (Length = | | 15 amino acids) |
| % amino acid sequence identity | = | | | |

(the number of identically matching amino acid residues between the two polypeptide sequences as determined by ALIGN-2) divided by (the total number of amino acid residues of the reference polypeptide)

= 5 divided by 10 = 50%

**Table 3**

| | | |
|---|---|---|
| Reference-DNA Comparison DNA | NNNNNNNNNNNNNN NNNNNNLLLLLLLLLL | (Length = 14 nucleotides) (Length = 16 nucleotides) |
| % nucleic acid sequence identity | = | |

(the number of identically matching nucleotides between the two nucleic acid sequences as determined by ALIGN-2) divided by (the total number of nucleotides of the reference-DNA nucleic acid sequence)

= 6 divided by 14 = 42.9%

**Table 4**

| | | |
|---|---|---|
| Reference-DNA Comparison DNA | NNNNNNNNNNNN NNNNLLLVV | (Length = 12 nucleotides) (Length = 9 nucleotides) |
| % nucleic acid sequence identity | = | |

(the number of identically matching nucleotides between the two nucleic acid sequences as determined by ALIGN-2) divided by (the total number of nucleotides of the reference-DNA nucleic acid sequence)

= 4 divided by 12 = 33.3%

III. Hedgehog Antagonist Methods

Hedgehog (Hh) proteins are morphogens that act in a long- or short-range fashion governing cell growth and patterning during development. Ogden et al., Biochem Pharmacol. 67: 805 (2004); Huangfu et al., Development 133: 3 (2006); Jia et al., Cell. Mol. Life Sci. 63 (11): 1249-1265 (2006). In mammals, there are three Hh proteins: Sonic hedgehog (Shh), Desert hedgehog (Dhh), and Indian hedgehog (Ihh). In cells that receive the Hh signal (Figure 4a), pathway activation is initiated when Hh ligand binds to Patched (Ptch1), a twelve-transmembrane receptor, relieving its inhibition of Smoothened(Smo), a seven-transmembrane protein. Huangu *et al., supra.,* Jia *et al., supra,* Stone et al., Nature 384: 129-133 (1996). Smo activation leads to downstream signaling events and the activation of the Glioma-associated (Gli) family of zinc-finger transcription factors. Kasper et al., Eur. J. Cancer 42: 437 (2006). In the absence of Hh signaling, protein kinases, such as protein kinase A (Pka), glycogen synthase kinase 3b (Gsk3b), and casein kinase 1 (Ck1), phosphorylate Gli, leading to proteosome-mediated cleavage of Gli into an NH2-terminal truncated form, which acts as a repressor of Hh target gene expression. Chen et al., Proc. Nat. Acad. Sci. USA 95: 2349 (2006); Price et al., Cell 108: 823 (2002); Price et al., Development 126: 4331 (1999); Ji et al., Nature 416: 548 (2002). Suppressor of fused (Sufu) acts as another negative regulator of the pathway by binding to Gli, both in the cytoplasm and in the nucleus, to prevent it from activating Hh target genes. Cheng et al., Proc. Nat. Acad. Sci. USA 99: 5442 (2002); Kogerman et al., Nat. Cell Biol. 1: 312 (1999); Ding et al., Curr. Biol. 9: 1119 (1999); Pearse et al., Dev. Biol. 212: 323 (1999).

In mammalian cells, how the Hh signal is transmitted from Smo to Gli remain unclear (Figure 5A). In *Drosophila,* the kinase Fused (Fu) an the kinesin-like molecule Costal 2 (Cos2) are thought to relay the signal from Smo to the fly Gli homolog Cubitus interruptus (Ci). Lum et al., Mol. Cell 12: 1261 (2003); Jia et al., Genes Dev. 17: 2709 (2003); Ruel et al., Nat. Cell Biol. 5: 907 (2003); Ogden et al., Curr. Biol. 13:1998 (2006). However, in mammals, this mechanism is not clearly conserved since neither mammalian ortholgos of Cos2 or Fu are required for transducing the Hh signal. Merchant et al., Mol. Cell. Biol. 25: 7054 (2005); Chen et al., Mol. Cell Biol. 25: 7042 (2005), Varjosalo et al., Dev. Cell 10: 177 (2006). Furthermore, the carboxy-terminal tail of fly Smo required for signaling and binding Fu and Cos2 [Lum *et al., supra,* Jia *et al., supra*.], is not conserved in mammalian Smo, suggesting mammalian Smo interacts with novel components to transmit the Hh signal.

In contrast, recent studies have shown that mammalian Hh signaling requires the presence of a nonmotile cilium. Huangfu et al., Nature 426: 83 (2006); Huangfu et al., Proc. Nat. Acad. Sci. USA 102: 11325 (2005); May et al., Dev. Biol. 287: 378 (2005); Corbit et al., Nature 437: 1018 (2005); Haycraft et al., PLoS Genet 1: e53 (2005). Genetic studies in mice revealed that disruption of a number of components of anterograde or retrograde intraflagellar transport (IFT) lead to neural tube patterning and limb developmental defects caused by impaired Hh signaling between Smo and Gli. Huangfu *et al.,* (2006), *supra,* Huangfu *et al.,* (2005), *supra,* May *et al., supra.* In addition, a number of components of the pathway including Smo, Sufu, and all three Gli proteins are found at the tip of cilia during Hh signaling. *May et al., supra,* Corbitt *et al., supra,* Haycraft *et al., supra.* Liu et al., Development 132: 1679 (2003). Thus, cilia seem to be an important mediator of Hh signaling in mammals. In Drosophila, cilia are not required for Hh signaling [Avidor-Reiss et al., Cell 117: 527 (2004); Han et al., Curr. Biol. 13: 1679 (2003)], providing further evidence for the divergence in the Hh pathway between flies and mammals.

Because of the relevance of Hh signaling in cancer, it is important to identify additional candidate drug targets in this pathway. To uncover novel kinases that play a role in mammalian Hh signaling, we screened a mouse siRNA kinome library, which included all kinases as well as some kinase-regulatory proteins, in cell-based assays designed to recapitulate Hh signaling (Figure 5A and 5C). We first developed a high-throughput assay (Figure 5C) to monitor induction of the Hh pathway by utilizing the murine C3H10T1/2 mesenchymal cell line that has been used as a model to study Hh signaling. This cell line was engineered to stably express a Hh inducible luciferase reporter, consisting of eight Gli-binding sites driving the expression of a cDNA encoding the firefly luciferase, and is referred to as S 12. Frank-Kamenetsky et al., J. Biol. 1: 10 (2002). In the absence of Shh, S12 cells produce basal amounts of luciferase (Figure 5B). Upon Shh addition, the Hh pathway is activated and luciferase expression increases up to 15 fold (Figure 5B). In contract, cells treated with siRNA against Smo and then stimulated with Sh, are defective in luciferase accumulation (Figure 5B).

*Angiogenesis*

Since hedgehog is known to stimulate angiogenesis, it is expected that hedgehog kinase antagonists, which inhibit hedgehog activity, would be expected to inhibit angiogenesis, particularly when some level of hedgehog signaling is a necessary perquisite for angiogenesis. Angiogenesis is fundamental to many disorders. Persistent, unregulated angiogenesis occurs in a range of disease states, tumor metasteses and abnormal growths by endothelial cells. The vasculature created as a result of angiogenic processes supports the pathological damage seen in these diseases.

Diseases associated with or resulting from angiogenesis include: tumor growth, tumor metastasis or abnormal growths by endothelial cells, including neovascular disease, age-related macular degeneration, diabetic retinopathy, retinopathy of prematurity, corneal graft rejection, neovascular glaucoma, retrolental fibroplasias, epidemic keratoconjuctivitis, Vitamin A deficiency, contact lens overwear, atopic keratitis, superior limbic keratitis, pterygium keratitis sicca, Sjogren's syndrome, acne rosacea, phylctenulosis, syphilis, mycobacteria infections, lipid degeneration, chemical burns, bacterial ulcers, fungal ulcers, Herpes simplex infections, Herpes zoster infections, protozoan infections, Kaposi's sarcoma, Mooren's ulcer, Terrien's marginal degeneration, marginal keratolysis, rheumatoid arthritis, systemic lupus, polyarteritis, trauma, Wegener's granulomatosis, sacroidosis, scleritis, Stevens-Johnson syndrome, pemphigoid radial keratotomy, corneal graph rejection, rheumatoid arthritis, systemic lupus, polyarteritis, trauma, Wegener's granulomatosis, sarcoidosis, scleritis, Stevens-Johnson syndrome, pemphigoid radial keratotomy, corneal graph rejection, rheumatoid arthritis, osteoarthritis chronic inflammation (*e.g.*, ulcerative colitis or Crohn's disease), hemangioma, Osler-Weber Rendu disease, and hereditary hemorrhagic telangiectasis.

Angiogenesis plays a critical role in cancer. A tumor cannot expand without a blood supply to provide nutrients and remove cellular wastes. Tumors in which angiogenesis is important include solid tumors such as rhabdomyosarcomas, retinoblastoma, Ewing sarcoma, neuroblastoma, osteosarcoma, and benign tumors such as acoustic neuroma, neurofibroma, trachoma and pyogenic granulomas. Angiogenic factors have been found associated with several solid tumors, and preventing angiogenesis could halt the growth of these tumors and the resultant damage to the animal due to the presence of the tumor. Angiogenesis is also associated with blood-born tumors such as leukemias, any of various acute or chronic neoplastic diseases of the bone marrow in which unrestrained proliferation of white blood cells occurs, usually accompanied by anemia, impaired blood clotting, and enlargement of the lymph nodes, liver, and spleen. It is believed that angiogenesis plays a role in the abnormalities in the bone marrow that give rise to leukemia-like tumors.

In addition to tumor growth, angiogenesis is important in metastasis. Initially, angiogenesis is important in the vascularization of the tumor which allows cancerous cells to enter the blood stream and to circulate throughout the body. After the tumor cells have left the primary site, and have settled into the secondary, metastatic site, angiogenesis must occur before the new tumor can grow and expand. Therefore, prevention of angiogenesis could lead to the prevention of metastasis of tumors and possibly contain the neoplastic growth at the primary site.

Angiogenesis is also involved in normal physiological processes such as reproduction and wound healing. Angiogenesis is an important step in ovulation and also in implantation of the blastula after fertilization. Prevention of angiogenesis could be used to induce amenorrhea, to block ovulation or to prevent implantation by the blastula.

*Lung function*

The hedgehog kinase antagonists described are useful for the treatment and/or prevention of respiratory distress syndrome or other disorders resulting from inappropriate lung surface tension. Respiratory distress syndrome results from insufficient surfactant in the alveolae of the lungs. The lungs of vertebrates contain surfactant, a complex mixture of lipids and protein that causes surface tension to rise during lung inflation and decrease during lung deflation. During lung deflation, surfactant decreases such that there are no surface forces that would otherwise prevent alveolar collapse. Aerated alveoli that have not collapsed during expiration permit continuous oxygen and carbon dioxide transport between blood and alveolar gas and require much less force to inflate during the subsequent inspiration. During inflation, lung surfactant increases surface tension as the alveolar surface areas increases. A rising surface tension in expanding alveoli opposes over-inflation in those airspaces and tends to divert inspired air to less well-aerated alveoli, thereby facilitating even lung aeration.

Respiration distress syndrome is particularly prevalent among premature infants. Lung surfactant is normally synthesized at a very low rate until the last six weeks of fetal life. Human infants born more than six weeks before the normal term of a pregnancy have a high risk of being born with inadequate amounts of lung surfactant and inadequate rates of surfactant synthesis. The more prematurely an infant is born, the more severe the surfactant deficiency is likely to be. Severe surfactant deficiency can lead to respiratory failure within a few minutes or hours of birth. The surfactant deficiency produces progressive collapse of alveoli (atelectasis) because of the decreasing ability of the lung to expand despite maximum inspiratory effort. As a result, inadequate amounts of oxygen reach the infant's blood. RDS can also in adults, typically as a consequence of failure in surfactant biosynthesis.

Lung tissue of premature infants shows high activity of the hedgehog signaling pathway. Inhibition of this pathway using hedgehog antagonists increases the formation of lamellated bodies and increases the expression of genes involved in surfactant biosynthesis. Lamellar bodies are subsellular structures associated with surfactant biosynthesis. For these reasons, treatment of premature infants with a hedgehog antagonist should stimulate surfactant biosynthesis and ameliorate RDS. In cases where adult RDS is associated with hedgehog pathway activation, treatment with a hedgehog kinase antagonist should also be effective.

*Disorders resulting from hyperactive hedgehog signaling*

The hedgehog kinase antagonists of the invention may be specifically targeted to disorders wherein the affected tissue and/or cells exhibit high hedgehog pathway activation. Expression of *gli* genes activated by the hedgehog signaling pathway, including *gli*-1, *gli*-2 and *gli*-3, most consistently correlate with hedgehog signaling across a wide range or tissues and disorders, while *gli*-3 is somewhat less so. The *gli* genes encode transcription factors that activate expression of many genes needed to elicit the full effects of hedgehog signaling. However, the *Gli*-3 transcription factors can also act as a repressor of hedgehog effector genes, and therefore, expression of *gli*-3 can cause a decreased effect of the hedgehog signaling pathway. Whether *gli*-3 acts as a transcriptional activator or repressor depends on post-translational events, and therefore it is expected that methods for detecting the activating form (versus the repressing form) of *Gli*-3 protein would also be a reliable measure of hedgehog pathway activation. The *gli*-1 gene is strongly expressed in a wide array of cancers, hyperplasias and immature lungs, and serves as a marker for the relative activation of the hedgehog pathway. In addition, tissues such as immature lung, that have high gli gene expression, are strongly affected by hedgehog inhibitors. Accordingly, it is contemplated that the detection of gli gene expression may be used as a powerful predictive tool to identity tissues and disorders that will particularly benefit from treatment with a hedgehog antagonist.

*gli*-1 expression levels may be detected, either by direct detection of the transcript or by detection of protein levels or activity. Transcripts may be detected using any of a wide range of techniques that depend primarily on hybridization or probes to the *gli*-1 transcripts or to cDNAs synthesized therefrom. Well known techniques include Northern blotting, reverse-transcriptase PCR and microarray analysis of transcript levels. Methods for detecting *Gli* protein levels include Western blotting, immunoprecipitation, two-dimensional polyacrylamide gel electrophoresis (2D SDS-PAGE - preferably compared against a standard wherein the position of the *Gli* proteins has been determined), and mass spectroscopy. Mass spectroscopy may be coupled with a series of purification steps to allow high-throughput indentification of many different protein levels in a particular sample. Mass spectroscopy and 2D SDS-PAGE can also be used to identify post-transcriptional modifications to proteins including proteolytic events, ubiquitination, phosphorylation, lipid modification, *etc. Gli* activity may also be assessed by analyzing binding to substrate DNA or in vitro transcriptional activiaton of target promoters. Gel shift assay, DNA footprinting assays and DNA-protein crosslinking assays are all methods that may be used to assess the presence of a protein capable of binding to *Gli* binding sites on DNA. J Mol. Med 77(6):459-68 (1999); Cell 100(4): 423-34 (2000); Development 127(19): 4923-4301 (2000).

*gli* transcript levels may be measured and diseased or disordered tissues showing abnormally high *gli* levels are treated with a hedgehog kinase antagonist. In other embodiments, the condition being treated is known to have a significant correlation with aberrant activation of the hedgehog pathway, even though a measurement of *gli* expression levels is not made in the tissue being treated. Premature lung tissue, lung cancers (*e.g.*, adeno carcinomas, bronco-alveolar adenocarcinoma, small cell carcinomas), breast cancers (*e.g.*, inferior ductal carcinomas, inferior lobular carcinomas, tubular carcinomas), prostate cancers (*e.g.*, adenocarcinomas), and benign prostatic hyperplasias all show strongly elevated *gli*-1 expression levels in certain cases. Accordingly, *gli-1* expression levels are a powerful diagnostic device to determine which of these tissues should be treated with a hedgehog kinase antagonist. In addition, there is substantial correlative evidence that cancers of the urothelial cells (*e.g.*, bladder cancer, other urogenital cancers) wil also have elevated *gli*-1 levels in certain cases. For example, it is known that loss of heterozygosity on chromosome 9q22 is common in bladder cancers. The *ptch*-1 gene is located at this position and *ptch-1* loss of function is probably a partial cause of hyperproliferation, as in mahy other cancer types. Accordingly, such cancers would also show high *gli* expression and would be particularly amenable to treatment with a hedgehog antagonist.

Expression of *ptch-1* and *ptch-2* is also activated by the hedgehog signaling pathway, but not typically to the same extent as *gli* genes, and as a result are inferior to the *gli* genes as markers of hedgehog pathway activation. In certain tissues, only one of *ptch-1* or *ptch-2* is expressed although the hedgehog pathway is highly active. For example, in testicular development, desert hedgehog plays an important role and the hedgehog pathway is activated, but only *ptc-2* is expressed. Accordingly, these genes may be individually unreliable as markers for hedgehog pathway activation, although simultaneous measurement of both genes is contemplated as a more useful indicator for tissues to be treated with a hedgehog antagonist.

Because *gli* is so ubiquitously expressed during hedgehog activation, any degree of *gli* overexpression should be useful in determining that a hedgehog kinase antagonist will be an effective therapeutic. *gli* should be expressed at a level at least twice as high as normal. Expression is four, six, eight or ten times as high as normal.

In light of the broad involvement of hedgehog signaling in the formation of ordered spatial arrangements of differentiated tissues in vertebrates, the hedgehog kinase antagonists of the present invention could be used in a process for generating and/or maintaining an array of different vertebrate tissue both *in vitro* and *in vivo.* The hedgehog kinase antagonist, whether inductive or anti-inductive with respect to proliferation or differentiation of a given tissue type, can be, as appropriate, any of the preparations described above.

*Neuronal cell culture*

The hedgehog kinase antagonists of the present invention are further applicable to cell culture techniques wherein reduction in hedgehog signaling is desirable. *In vitro* neuronal culture systems have proved to be fundamental and indispensable tools for the study of neural development, as well as the identification of neurotrophic factors such as nerve growth factor (NGF), ciliary trophic factors (CNTF), and brain derived neurotrophic factor (BDNF). Once use of the present method may be in culture of neuronal stem cells, such as in the use of such cultures for the generation of new neurons and glia. These cultures can be contacted with hedgehog kinase antagonists in order to alter the rate of proliferation or neuronal stem cells in the culture and/or alter the rate of differentiation, or to maintain the integrity of a culture of certain terminally differentiated neuronal cells. In an exemplary embodiment, the subject method can be used to culture, certain neuron types (*e.g.*, sensory neurons, motor neurons). Such neuronal cultures can be used as convenient assay systems as well as sources of implantable cells for therapeutic treatments.

The hedgehog kinases antagonists of the present invention are further applicable to intracerebral grafting, an emerging treatment for disorders of the central nervous system. For example, one approach to repairing damaged brain tissues involves the transplantation of cells from fetal or neonatal animals into the adult brain. Dunnett et al., J. Exp. Biol. 123: 265-289 (1987). Fetal neurons from a variety of brain regions can be successfully incorporated into the adult brain, and such grafts can alleviate behavioral defects. For example, movement disorder induced by lesions of dopaminergic projections to the basal ganglia can be prevented by grafts of embryonic dopaminergic neurons. Complex cognitive functions that are impaired after lesions of the neocortex can also be partially restored by grafts of embryonic cortical cells. The subject method can be used to regulate the growth state in the culture, or where fetal tissue is used, especially neuronal stem cells, can be used to alter the rate of differentiation of the stem cells.

Stem cells useful in the described methods are generally known. For example, several neural crest cells have been identified, some of which are multipotent and likely represent uncommitted neural crest cells, and others of which can generate only one type of cell, such as sensory neurons, and likely represent committed progenitor cells. The role of hedgehog antagonists employed in the present method to culture such stem cells can be to regulate differentiation of the uncommitted progenitor, or to regulate further restriction of the developmental fate of a committed progenitor, or to regulate further restriction of the developmental fate of a committed progenitor cell towards becoming a terminally differentiated neuronal cell. For example, the present method can be used *in vitro* to regulate the differentiation of neural crest cells into glial cells, schwann cells, chromaffin cells, cholinergic, sympathetic or parasympathetic neurons, as well as peptidergic and serotonergic neurons. The hedgehog kinase antagonist can be used alone, or in combination with other neurotrophic factors that act to more particularly enhance a particular differentiation fate of the neuronal progenitor cell.

*Regulation of neuronal growth and differentiation*

In addition to using the hedgehog kinase antagonists of the present invention in combination with implantation of cell cultures, another aspect of the present invention relates to the therapeutic application of hedgehog kinase antagonists to regulate the growth state of neurons and other neuronal cells in both the central nervous system and the peripheral nervous system. The ability of the hedgehog pathway component (*e.g.*, *ptch,* hedgehog, and smoothened) to regulate neuronal differentiation during development of the nervous sytem and also presumably in the adult state indicates that in certain instances, the subject hedgehog kinase antagonists can be expected to facilitate control of adult neurons with regard to maintenance, functional performance, and aging of normal cells; repair and regeneration processes in chemically or mechanically lesioned cells; and treatment of degeneration in certain pathological conditions. In light of this undertstanding, the present invention specifically contemplated applications of the subject method to the treatment (*e.g.,* prevention, reduction in severity, *etc.)* of neurological conditions deriving from: (i) acute, subacute, or chronic injury to the nervous system, including traumatic injury, chemical injury, vascular injury and deficits (such as the isehemia resulting from stroke), together with infectious/inflammatory and tumor-induced injury; (ii) aging of the nervous system, including Parkinson's disease, Huntington's chorea, amyotrophic lateral sclerosis and the like, as well as spinocerebellar degeneration; and (iv) chronic immunological diseses of the nervous system or affecting the nervous sytem, including multiple sclerosis.

As appropriate, the subject method can also be used in generating nerve prosthesis for the repair of central and peripheral nerve damage. In particular, where a crushed or severed axon is intubulated by the use of a prosthetic device, hedgehog antagonists can be added to the prosthetic device to regulate the rate of growth and regeneration of the dendritic processes. Exemplary nerve guidance channels are described in U.S. Patents 5,092,871 and 4,955,892.

The treatment of neoplastic or hyperplastic transformation such as may occur in the central nervous system is described. For instance, the hedgehog kinase antagonists can be utilitized to cause such transformed cells to become either post-mitotic or apoptotic. The present method may, therefore, be used as part of a treatment for, *e.g.*, malignant gliomas, meningiomas, medulloblastomas, neuroectodermal tumors, and ependymomas.

*Neuronal cancer*

The hedgehog kinase antagonists may be used as part of a treatment regimen for malignant medulloblastoma and other primary CNS malignant neuroectodermal tumors. Medulloblastoma, a primary brain tumor, is the most common brain tumor in children. A medulloblastoma is a primitive neuroectodermal (PNET) tumor arising in the posterior fossa. They account for approximately 25% of all pediatric brain tumors. Histologically, they are small round cell tumors commonly arranged in true rosette, but may display some differentiation to astrocytes, ependymal cells or neurons. PNETs may arise in other areas of the brain including the penial gland (pineoblastoma) and cerebrum. Those arising in the supratentorial region generally have a worsened prognosis.

Medulloblastom/PNETs are known to recur anywhere in the CNS after resection, and can even metastasize to bone. Pretreatment evaluation should therefore include and examination of the spinal cord to exclude the possibility of "dropped metastases". Gadolinium-enhanced MRI has largely replaced myelography for this purpose, and CSF cytology is obtained postoperatively as a routine procedure.

In other embodiment, the subject method is used as part of a treatment program for ependymomas. Ependymomas account for approximately 10% of the pediatric brain tumors in children. Grossly, they are tumors that arise from the ependymal lining of the ventricles and microscopically form rosettes, canals, and perivascular rosettes. In the CHOP series of 51 children reported with epenymomas, ¾ were histologically benign. Approximately 2/3 arose from the region of the 4$^{th}$ ventricule. One third presented in the supratentorial region. Age at presentation peaks between birth and 4 years, as demonstrated by SEER data as well as data from CHOP. The median age is about 5 years. Because so many children with this disease are babies, they often require multimodal therapy.

*Non-neuronal cell culture*

The hedgehog kinase antagonists can be used in cell culture and therapeutic method relating to the generation and maintenance of non-neuronal tissue. Such uses are contemplated as a result of the involvement of hedgehog signaling components *(e.g., ptch,* hedgehog, *smo, etc.)* in morphogenic signals of other vertebrate organogenic pathways, such as endodermal patterning, and mesodermal and endodermal differentiation.

Hedgehog signaling, especially *ptc, hedgehog,* and *smoothened,* are involved in controlling the development of stem cells responsible for formation of the digestive tract, liver, lungs, and other organs derived from the primitive gut.

*Shh* is the inductive signal from the endoderm to the mesoderm, which is critical to gut morphogenesis. Therefore, for example, the hedgehog kinase antagonists of the instant method can be employed for regulating the development and maintenance of an artificial liver that can have multiple metabolic functions of a normal liver. In an exemplary embodiment, the subject method can be used to regulate functions of a normal liver. In an exemplary embodiment, the subject method can be used to regulate the proliferation and differentiation of digestive tube stem cells to form hepatocyte cultures which can be used to populate extracellular matrices, or which can be encapsulated in biocompatible polymers, to form both implantable and extracorporeal artificial livers.

In another embodiment, the subject method can be employed therapeutically to regulate such organs after physical, chemical or pathological insult. For instance, therapeutic comprising comprising hedgehog kinase antagonist can be used in liver repair subsequent to a partial hepactectomy.

In another embodiment, the subject method can be used to control or regulate the proliferation and/or differentiation of pancreatic tissue both *in vivo* and *in vitro.* The generation of the pancreas and small intestine from the embryonic gut depends on intercellular signaling between the endodermal and mesodermal cells of the gut. In particular, the differentiation of intestinal mesoderm into smooth muscle has been suggested to depend on signals from adjacent endodermal cells. One candidate mediator of endodermally derived signals in the embryonic hindgut is Sonic hedgehog *(Shh).* Apelqvist et al., Curr. Biol. 7: 801-4 (1997). The *Shh* gene is expressed throughout the embryonic bud endoderm with the exception of the pancreatic bud endoderm, which instead expressed high levels of the homeodomain protein Ipf1/Pdx1 (insulin promoter factor 1/pancreatic and duodenal homeobox 1), an essential regulator of early pancreatic development. The Ipf1/Pdx1 was used to selectively express *Shh* in the developing pancreatic epithelium. The pancreatic mesoderm of Ipf1/Pdx1-Shh transgenic mice developed into smooth muscle and insterstitial cells of Cajal - cells which are characteristic of the intestine, rather than pancreatic mesenchyme and spleen. Apelqvist *et al., supra.* Also, pancreatic explants exposed to *Shh* underwent as similar expression of endodermally derived *Shh* controls the fate of adjacent mesoderm at different regions of the gut tube.

In another embodiment, hedgehog kinase antagonists are used to modulate the generation of endodermal tissue from non-endodermal stem cells including mesenchymal cells and stem cells derived from mesodermal tissues. Exemplary mesodermal tissues from which stem cells may be isolated include skeletal muscle, cardiac muscle, kidney, cartilage and fat.

*Pancreatic conditions/disorders*

There are a wide variety of pathological cell proliferative pancreatic conditions for which the hedgehog kinase antagonists of the present invention may provide therapeutic benefits. More specifically, such thereapeutic benefits are directed to correcting aberrant insulin expression, or modulation of differentiation of pancreatic cells. More generally, however, the present invention relates to a method of inducing and/or maintaining a differentiated state, enhancing survival and/or affecting proliferation of pancreatic cells, by contacting the cells with the subject hedgehog kinase antagonists. For instance, it is contemplated by the invention that, in light of the apparent involvement of *ptc, hedgehog* and *smoothened* in the formation of ordered spatial arrangements of pancreatic tissues, the subject method could be used as part of a technique to generate and/or maintain such tissue both *in vitro* and *in vivo.* For instance, modulation of hedgehog signaling can be employed in both cell culture and therapeutic methods involving generation and maintenance of β-islet cells and possibly also from non-pancreatic tissue, such as in controlling the development and maintenance of tissue from the digestive tract, spleen, lungs, urogenital organs (*e.g.*, bladder), as well as other organs which derive from the primitive gut.

In a specific embodiment, the hedgehog kinase antagonists of the present invention can be used in the treatment of hyperplastic and neoplastic disorders affecting pancreatic tissue, especially those characterized by aberrant proliferation of pancreatic cells. For instance, pancreatic cancers are marked by abnormal proliferation of pancreatic cells, which can result in alterations of insulin secretory capacity of the pancreas. For instance, certain pancreatic hyperplasias, such as pancreatic carcinomas, can result in hypoinsulinemia due to dysfunction of β-cells or decreased islet cell mass. Moreover, manipulation of hedgehog signaling properties at different points may be useful as part of a strategy for reshaping/repairing pancreatic tissue both *in vivo* and *in vitro.* In one embodiment, the present invention makes use of the apparent involvement of *ptc, hedgehog* and *smoothened* in regulating the development of pancreatic tissue. In another embodment, the subject hedgehog kinase antagonists can be employed therapeutically to regulate the pancreas after physical, chemical or pathological insult. In yet another embodiment, the subject method can be applied to cell culture techniques, and in particular, may be employed to enhance the initial integration of prosthetic pancreatic tissue devices. Manipulation of proliferation and differentiation of pancreatic tissue, such as through using hedgehog kinase antagonists, can provide a means for more carefully controlling the characteristics of a cultured tissue. In an exemplary embodiment, the subject method can be used to augment production of prosthetic devise which require β-islet cells, such as may be used in the encapsulation devices described in, for example, as described in U.S.P. 4,892,538, 5,106,627, 4,391,909 and 4,353,888. Early progenitor cells to the pancreatic islets are multipotential, and apparently coactivate all the islet-specific genes from the time they first appear. As development proceeds, expression of islet-specific hormones, such as insulin, becomes restricted to the pattern of expression characteristic of mature islet cells. The phenotype of mature islet cells, however, is not stable in culture, as reappearance of embryonal traits in mature β-cells can be observed. By utilizing the subject hedgehog kinase antagonists, the differentiaton path or proliferative index of the cells can be regulated.

Furthermore, manipulation of the differentiative state of pancreatic tissue can be utilized in conjunction with transplantation of artificial pancreas. For instance, manipulation of hedgehog function to affect tissue differentiation can be utilized as a means of maintaining graft viability.

The hedgehog kinase antagonists of the present invention may be used to regulate the regeneration of lung tissue, *e.g.,* in the treatment of emphysema. It has been reported that *Shh* regulates lung mesenchymal cell proliferation *in vivo.* Bellusci et al., Development 124: 53 (1997).

*Cell Proliferative disorders, tumors and cancers*

The hedgehog kinase antagonists of the present invention may also be used to treat lung carcinoma and adenocarcinoma, and other proliferative disorders involving the lung epithelia. It is known that *Shh* is expressed in human lung squamous carcinoma and adenocarcinoma cells. Fujita et al., Biochem. Biophys. Res.Commun. 238: 658 (1997). The expression of *Shh* was also detected in the human lung squamous carcinoma tissues, but not in the normal lung tissue of the same patient. It was also observed that *Shh* stimulates the incorporation of BrdU into the carcinoma cells and stimulates their cell growth, while anti-Shh-H inhibited such growth. These results suggest that a *ptc, hedgehog,* and/or *smoothened* is involved in cell growth of such transformed lung tissue and therefore indicates that the hedgehog kinase antagonists can be used to treat lung carcinoma and adenocarcinomas, and other proliferative disorders involving the lung epithelia.

The hedgehog kinase antagonists of the present invention may also be used to treat tumors in which hedgehog signaling is associated with the existence or pathogenesis. Such tumors include, but are not limited to: tumors related to Gorlin's syndrome (*e.g.*, medulloblastoma, meningioma, *etc.),* tumors evidence in *ptc* knock-out mice (e.g., hemangioma, rhabdomyosarcoma, *etc.),* tumors resulting from *gli*-1 amplification (*e.g.*, glioblastoma, sarcoma, *etc.),* tumors resulting from Smo dysfunction *(e.g.,* basal cell carcinoma, etc.), tumors connected with TRC8, a ptc homolog *(e.g.,* renal carcinoma, thyroid carcinoma, *etc.*), *Ext*-1 related tumors (*e.g.*, bone cancer, *etc.), Shh*-induced tumors (*e.g.*, lung cancer, chondrosarcomas, *etc.),* and other tumors (*e.g.*, breast cancer, urogenital cancer (*e.g.*, kidney, bladder, ureter, prostate, *etc.),* adrenal cancer, gastrointestinal cancer (*e.g.*, stomach, intestine, *etc.).*

The hedgehog kinase antagonists of the present invention may also be used to treat several forms of cancer. These cancer include, but are not limited to: prostate cancer, bladder cancer, biliary cancer, lung cancer (including small cell and non-small cell), colon cancer, kidney cancer, liver cancer, breast cancer, cervical cancer, endometrial or other uterine cancer, ovarian cancer, testicular cancer, cancer of the penis, cancer of the vagina, cancer of the urethra, gall bladder cancer, esophageal cancer, or pancreatic cancer. Additional cancer types include cancer of skeletal or smooth muscle, stomach cancer, cancer of the small intestine, cancer of the salivary gland, anal cancer, rectal cancer, thyroid cancer, parathyroid cancer, pituitary cancer, and nasopharyngeal cancer. Further exemplary forms of cancer which can be treated with the hedgehog antagonists of the present invention include cancers comprising hedgehog expressing cells. Still further exemplary forms of cancer which can be treated with the hedgehog antagonists of the present invention include cancers comprising gli expressing cells. In one embodiment, the cancer is not characterized by a mutation in *patched*-1.

*Skeletal and connective tissue:*

Described is the use of hedgelog kinase antagonists in the *in vitro* generation of skeletal tissue, such as from skeletogenic stem cells, as well as the *in vivo* treatment of skeletal tissue deficiencies. More specifically, such hedgehog kinase antagonists may be used to regulate chrondrogenesis and/or osteogenesis. By "skeletal tissue deficiency", it is meant a deficiency in bone or other skeletal connective tissue at any site where it is desired to restore the bone or connective tissue, no matter how the deficiency originated, *e.g.*, whether as a result of surgical intervention, removal of tumor, ulceration, implant, fracture, or other traumatic or degenerative conditions.

The hedgehog kinase antagonists described can be used in a method of restoring cartilage function to connective tissue, for example, the repair of defects or lesions in cartilage tissue resulting from degenerative wear. More specifically, degenerative wear resulting from arthritis (osteo and rheumatoid) and/or trauma - such as a displacement of tom meniscus tissue, meniscectomy, a laxation of a joint by a torn ligament, malignment of joint, bone fracture. Such reparative methods may also be useful for remodeling cartilage matrix, such as in plastic or reconstructive surgery, as well as periodontal surgery. The reparative method may also be applied to augment or correcting a previous procedure, for example, following surgical repair of meniscus, ligament, or cartilage. Furthermore, it may prevent the onset or exacerbation of degenerative disease if applied early enough after trauma.

A reparative method is described which comprises treating the afflicted connective tissue with a therapeutically effective amount of a hedgehog kinase antagonist, in order to regulate a cartilage repair response in the connective tissue by modulating the rate of differentiation and/or proliferation of chondrocytes embedded in the tissue. Such connective tissues as articular artilage, interarticular cartilage (menisci), costal cartilage (connecting the true ribs and the sternum), ligaments, and tendons are particularly amenable to treatment in reconstructive and/or regenerative therapies using the subject method. As used herein, regenerative therapies include treatment wherein the degeneration is manifest, as well as preventive or prophylactic treatments of tissue where degeneration is in its earliest stages or imminent.

The reparative method can be used as part of a therapeutic intervention in the treatment of cartilage of a diarthroidal joint, such as a knee, an ankle, an elbow a hip, a wrist, a knuckle or either a finger or toe, or a tempomandibular joint. The treatment can be directed to the meniscus of the joint, to the articular cartilage of the joint, or both. To further illustrate, the subject method can be used to treat a degenerative disorder or a knee, such as which might be the result of traumatic injury (*e.g.*, a sports injury or excessive wear) or osteoarthritis. The described antagonists may by administered as an injection into the joint with, for instance an arthroscopic needle. In some instances, the injected agent can be in the form of a hydrogel or other slow release vehicle described above in order to permit a more extended and regular contact of the agent with the treated tissue.

The reparative method can be applied to enhancing both the generation of prosthetic cartilage devices and to their implantation. The need for improved treatment has motivated research aimed at creating new cartilage that is based on collagen-glycosaminoglycan templates (Stone et al., Clin. Orthop. Relat. Red. 252: 129 (1990)), isolated chondrocytes (Grande et al., J. Orthop. Res. 7: 208 (1989); Takigawa et al., Bone Miner 2: 449 (1987)), and chondrocytes attached to natural or synthetic polymers (Walitani et al.. J. Bone Jt. Surg. 71B:74 (1989); Vacanti et al., Plast. Resconstr. Surg. 88:753 (1991); von Schroeder et al., J. Biomed Mater. Res. 25: 329 (1991); Freed et al., J. Biomed. Mater. Res. 27: 11 (1993); U.S.P. 5,041,138). For example, chondrocytes can be grown in culture on biodegradable, biocompatible highly porous scaffolds or matrices formed from polymers such as polyglycolic acid, polylactic acid, agarose gel, or other polymers that degrade over time as a function of hydrolysis of the polymer backbone into innocuous monomers. The matrices are designed to allow adequate nutrient and gas exchange to the cells until engraftment occurs. The cells can be cultured in vitro until adequate cell volume and density has developed for the cells to be implanted. One advantage of the matrices is that they can be cast or molded into a desired shape on an individual basis, so that the final product closely resembles the patient's own affected body portion (*e.g.*, ear, nose, *etc.),* or flexible matrices can be used which allow for manipulation at the time of implantation, as in a joint.

Implants may be contacted with the hedgehog kinase antagonist during certain stages of the culturing process in order to manage the rate of differentiation of chondrocytes and the formation of hypertrophic chondrocytes in the culture.

The implanted device is treated with a hedgehog kinase antagonist in order to actively remodel the implanted matrix and to make it more suitable for its intended function. As decribed previously, any artificial transplants suffer from the deficiency of not being derived in a setting which is comparable to the actual mechanical environment in which the matrix is implanted. The ability to regulate the chondrocytes in the matrix by the reparative method can allow the implant to acquire characteristics similar to the tissue for which it is intended to replace.

The hedgehog kinase antagonists can be used in a method for the generation of bone (osteogenesis) at a site in an animal where such skeletal tissue is deficient. As indian hedgehog is particularly associated with the hypertrophic chondrocytes that are ultimately replaced by osteoblasts, the modulation of indian hedgehog function can occur through the use of the present hedgehog kinase anagonists. For instance, administration of a hedgehog kinase antagonist of the present invention can be employed as part of a method for regulating the rate of bone loss in a subject. Preparations comprising hedgehog kinase antagonists can be employed, for example, to control endochondral ossification in the formation of a "model" for ossification.

*Sperinatogenesis and male contraception*

The hedgehog kinase antagonists described can be used to regulate spermatogenesis. The hedgehog proteins, particularly *Dhh*, have been shown to be involved in the differentiation and/or proliferation and maintenance of testicular germ cells. *Dhh* expression is initiated in Sertoli cell precursors shortly after the activation of *Sry* (testicular determining gene) and persists in the testis into the adult. Male mice with *Dhh*-null mutations are viable but infertile, owing to a complete absence of mature sperm. Examination of the developing testis in different genetic backgrounds suggests that *Dhh* regulated both early and late stages of spermatogenesis. Bitgood et al., Cur. Biol 6(3) : 298-304 (1996). In a preferred embodiment, the hedgehog kinase antagonists described can be used as a contraceptive.

*Epithelial tissue*

The hedgehog kinase antagonists described also may be used in the treatment (including prophylaxis) of disorders affecting epithelial tissue. In general, such a treatment comprises administering an amount of a hedgehog kinase antagonist effective to alter the growth state of the treated epithelial tissue. The mode of administration and dosage regimens will vary depending on the epithelial tissue(s) that is to be treated (*e.g.*, dermal, mucosal, glandular, *etc.).* In a specific aspect, the method can be used to regulate the induction of *Shh* induced differentiation and/or inhibit proliferation of epithelially derived tissue. Thus, the hedgehog kinase antagonists described can be used in a method for the treatment of hyperplastic and/or neoplastic conditions involving epithelial tissue.

*(i) Wound healing*

The hedgehog kinase antagonists described may be used to in a method to promote wound healing. Specifically, "promoting wound healing" means a wound healing more efficiently as a result of application of the treatment that a similar wound heals in the absence of the treatment. "Promotion of wound healing" can also mean that the method regulates the proliferation and/or growth of, inter alia, ketatinocytes, or that the wound heals with less scarring, less wound contractions, less collagen deposition and more superficial surface area. In certain instances, "promotion of wound healing" can also mean that certain methods of wound healing have improved success rates, (e.g., the take rates of skin grafts), when used together with the method described.

Despite significant progress in reconstructive surgical techniques, scarring can be an important obstacle in regaining normal function and appearance of healed skin. This is particularly true when pathologic scarring such as keloids or hypertrophic scars of the hands or face causes functional disability or physical deformity. In the severest of circumstances, such scarring may precipitate psychosocial distress and a life of economic deprivation. Wound repair includes the stages of hemostasis, inflammation, proliferation, and remodeling. The proliferative stage involves multiplication of fiproblasts and endothelial and epithelial cells. Application of the hedgehog kinase antagonists described can modulate the proliferation of epithelial cells in and proximal to the wound in order to manage the closure of the wound and/or minimize the formation of scare tissue.

The hedgehog kinase antagonists described can also be used to treat oral, paraoral and mucous membrane ulcers, *e.g.*, such as those resulting from radiation and/or chemotherapy. Such ulcers commonly develop within days after chemotherapy or radiation therapy. In many instances, lack of treatment results in the proliferation of inflammatory tissue around the periphery of the lesion, resulting in loss of continuity of surface epithelium. As a result of the loss of epithelial integrity, the body is disposed to potential secondary infection. If such ulcers proliferate throughout the alimentary canal, the routine ingestion of food and water becomes painful, along with diarrhea and its complicating factors. The hedgehog kinase antagonists of the invention can be a treatment for such ulcers by reducing the abnormal proliferation and differentiation of the affected epithelium, thereby helping to reduce the severity of subsequent inflammatory events.

*(ii) Corneal and lens repair*

The hedgehog kinase antagonists of the invention can be used to inhibit lens epithelial cell proliferation to prevent post-operative complications of extracapsular cataract extraction. Cataracts are an intractable eye disease on which much study has been conducted. However, at present, treatment of this disorder is primarily obtained through surgery. Cataract surgeries have been applied for a long time and various operative methods have been examined. Extracapsular lens extraction has become the method of choice for removing cataracts. The major medical advantages of this technique over the intracapsular extraction are lower incidence of aphakic cystoid macular edema and retinal detachment. Extracapsular extraction is also required for implantation of posterior chamber-type intraocular lenses, which are now considered to be the lenses of choice in most cases.

However, a disadvantage of extracapsular cataract extraction is the high incidence of posterior lens opacification, often called after-cataract, which can occur in up to 50% of cases within three years of surgery. After-cataract is caused by proliferation of equatorial and anterior capsule lens epithelial cells that remain after extracapsular lens extraction. These cells proliferate to cause Sommerling rings, and along with fibroblasts, which also deposit and occur on the posterior capsule, cause opacification of the posterior capsule, which interferes with vision. Prevention of such after-cataracts would be preferable. To inhibit such "after-" or secondary cataract formation, the subject method provides a means for inhibiting proliferation of the remaining lens epithelial cells. For example, such cells can be induced to remain quiescent by instilling a solution continuing a hedgehog kinase antagonist preparation into the anterior chamber of the eye after lens removal. Furthermore, the solution can be osmotically balanced to provide minimal effective dosage when instilled into the anterior chamber of the eye, thereby inhibiting subcapsular epithelial growth with some specificity.

The hedgehog kinase antagonists may also be used in the treatment of corneopathies marked by corneal epithelial cell proliferation, as for example in ocular epithelial disorders such as epithelial downgrowth or squamous cell carcinomas of the ocular surface. Hedgehog proteins have been shown to regulate mitogenesis and photoreceptor differentiation in the vertebrate retina (Levine et al., J. Neurosci. 17: 6277 (1997)), and Ihh is a candidate factor from the pigmented epithelium to promote retinal progenitor proliferation and photoreceptor differentiation. Likewise, Jensen et al., Development 124: 363 (1997), demonstrated that treatment of cultures of perinatal mouse retinal cells with the amino-terminal fragment of *Shh* protein results in an increase in the proportion of cells that incorporate bromodeoxyuridine in rod photoreceptors, amacrine cells and Müller glial cells. This suggests that *Shh* promotes the proliferation of retinal precursor cells, which means that the hedgehog kinase antagonists of the present invention would be expected to modulate such *Shh*-mediated proliferation. Thus, the hedgehog kinase antagonists of the invention can be used in the treatment of proliferative diseases of retinal cells and regulate photoreceptor differentiation.

*(iii) Hair growth*

The hedgeghog kinase antagonists described can also be used to control hair growth. Hair is basically composed of keratin, a tough and insoluble protein. Each individual hair comprises a cylindrical shaft and a root, and is contained in a follicle, a flask-like depression in the skin. The bottom of the follicle contains a finger-like projection termed the papilla, which consists of connective tissue from which hair grows, and through which blood vessels supply the cells with nourishment. The shaft is the part that extends outwards from the skin surface, whilst the root has been described as the buried part of the hair. The base of the root expands into the hair bulb, which rests upon the papilla. Cells from which the hair is produced grow in the bulb of the follicle; they are extruded in the form of fibers as the cells proliferate in the follicle. Hair "growth" refers to the formation and elongation of the hair fiber by the dividing cells.

As is well known in the art, the common hair cycle is divided into three stages: anagen, catagen and telogen. During the active phase (anagen), the epidermal stem cells of the dermal papilla divide rapidly. Daughter cells move upward and differentiate to form the concentric layers of the hair itself. The transitional stage, catagen, is marked by the cessation of mitosis of the stem cells in the follicle. The resting stage is known as telogen, where the hair is retained within the scalp for several weeks before an emerging new hair developing below it dislodges the telogen-phase shaft from its follicle. From this model it has become clear that the larger the pool of dividing stem cells that differentiate into hair cells, the more hair growth occurs. Accordingly, method for increasing or reducing hair growth can be carried out by potentiating or inhibiting, respectively, the proliferation of these stem cells.

The hedgehog kinase antagonists can be used in a method of reducing the growth of human hair, either as a replacement to or in combination with removal by cutting, shaving, or depilation. For instance, the present method can be used in the treatment of trichosis characterized by abnormally rapid or severe growth of hair, *e.g.*, hypertrichosis. In an exemplary embodiment, hedgehog antagonists can be used to manage hirsutism, a disorder marked by abnormal hairiness. The subject method can also provide a process for extending the duration of depilation.

Moreover, because a hedgehog kinase antagonist will often be cytostatic to epithelial cells, rather than cytotoxic, such agents can be used to protect hair follicle cells from cytotoxic agents that require cell progression into S-phase of the cell-cycle for efficacy, *e.g.*, radiation-induced death. Treatment by the hedgehog kinase antagonists can provide protection by causing the hair follicle cells to become quiescent, *e.g.*, by preventing the cells from entering S-phase, and thereby preventing the follicle cells from undergoing mitotic catastrophe or programmed cells death. For example, the hedgehog kinase antagonists can be used in patients undergoing chemo-or radiation-therapies that ordinarily result in hair loss. By inhibiting cell-cycle progression during such therapies, the subject treatment can protect hair follicle cells from death, which might otherwise result from activation of cell death programs in the absence of quiescense. After therapy of the hedgehog kinase antagonists has concluded, the instant method can also be removed with concomitant relief of the inhibition of follicle cell proliferation.

The hedgehog kinase antagonists described can also be used in the treatment of folliculitis, such as folliculitis decalvans, folliculitis ulerythematosis reticulate or keloid folliculitis. For example, a cosmetic preparation of a hedgehog kinase antagonist can be applied topically in the treatment of pseudofolliculitis, a chronic disorder occurring most often in the submandibular region of the neck and associated with shaving, the characteristic lesions of which are erythematous papules and pustules containing buried hairs.

The hedgehog kinase antagonists described can be used in a method of modulating the growth of human hair. Sato et al., J. Clin. Invest. 104: 855-864 (1999) reported that upregulation of *Shh* activity in postnatal skin functions as a biologic switch that induces resting hair follicles to enter anagen with consequent hair growth. Sato *et al.,* used an adenovirus vector, AdShh, to transfer the murine Shh cDNA to skin of postnatal day 19 C57BL/6 mice. The treated skin showed increased mRNA expression of *Shh, Patched,* and *Gli-1.* In mice receiving AdShh, but not in controls, acceleration into anagen was evident, since hair follicle size and melanogenesis increased and the hair-specific keratin ghHb-1 and the melanin synthesis-related tyrosinase mRNAs accumulated. Finally, C57BL/6 mice showed marked acceleration of the onset of new hair growth in the region of AdShh administration to skin weeks after treatment, but not in control vector-treated or untreated areas. After 6 months, Ad*Shh*-treated skin showed normal hair and normal skin morphology. Thus, the hedgehog kinase antagonists described may be useful to regulate or modulate *Shh*-induced hair growth.

*(iv) Excessive epithelial proliferation*

The hedgehog kinase antagonists of the invention can be used in a method for the treatment of hyperplastic conditions (*e.g.*, keratosis) and neoplastic epidermal conditions characterized by a high proliferation rate (*e.g.*, squamous cell carcinoma). The subject method can also be used in the treatment of autoimmune diseases affecting the skin, in particular, or dermatological diseases involving morbid proliferation and/or keratinization of the epidermis, as for example, caused by psoriasis or atopic dermatosis. Common skin disorders that are characterized by localized abnormal proliferation of the skin (*e.g.*, psoriasis, squamous cell carcinoma, keratocanthoma, actinic keratosis) would also be expected to be treatable by application of the hedgehog kinase antagonists of the invention.

Preparations of the hedgehog kinase antagonists are suitable for the treatment of dermatological ailments linked to keratinization disorders causing abnormal proliferation of skin cells, in which such disorders may be marked by either inflammatory or non-inflammatory components. The hedgehog kinase antagonists of the invention, which promote quiescence or differentiation can be used to treat varying forms of psoriasis, *e.g.*, cutaneous, mucosal or lingual. Psoriasis, as described above, is typically characterized by epidermal keratinocytes that display marked proliferative activation and differentiation along a "regenerative" pathway. Treatment with the hedgehog kinase antagonists of the present invention can be used to reverse the pathological epidermal activation and can provide a basis for sustained remission of the disease.

A variety of other disorders characterized by keratotic lesions are also candidates for treatment with the hedgehog kinase antagonists of the invention. Actinic keratoses, for example, are superficial inflammatory premalignant tumors arising on sun-exposed and irradiated skin. Current therapies include excisional and cryosurgery. These treatments are painful, however, and often produce cosmetically unacceptable scarring. Accordingly, treatment of keratosis, such as actinic keratosis, includes applicatoin of a hedgehog antagonist composition in amounts sufficient to inhibit hyperproliferation of epidermal/epidermoid cells of the lesion.

*(v) Acne*

Acne represents yet another dermatologic ailment which may be treated by the hedgheg kinease antagonists described. Acne vulgaris, a multifactor disease most commonly occurring in teenagers and young adults, is characterized by the appearance of inflammatory and noninflammatory lesions on the face and upper trunk. The basic defect which gives rise to acne vulgaris is hypercornification of the duct of a hyperactive sebaceous gland. Hypercomification blocks the normal mobility of skin and follicle microorganisms, and in so doing, stimulates the release of lipases by *Propinobacterium acnes* and *Staphylococcus epidermidis* bacteria and *Pitrosporum ovale,* a yeast. Treatment with hedgehog kinase antagonists, particularly topical preparations, may be useful for preventing the transitional features of the ducts, *e.g.*, hypercornification, which lead to lesion formation. A therapeutic regimen comprising hedgehog kinase antagonists may further include, for example, antibiotics, retinoids and antiandrogens.

*(vi) Dermatitis and other skin ailments*

The hedgehog kinase antagonists described can also be used in a method for treating various forms of dermatitis. Dermatitis is a descriptive term referring to poorly demarcated lesions that are either pruritic, erythematous, scaly, blistered, weeping, fissured or crusted. These lesions arise from any of a wide variety of causes. The most common types of dermatitis are atopic, contact and diaper dermatitis. For example, seborrheic dermatitis is a chronic, usually pruritic, dermatitis with erythema, dry, moist, or greasy scaling, and yellow-crusted patches on various areas, especially the scalp, with exfoliation of an excessive amount of dry scales. The hedgehog kinase antagonists of the subject method may also be used in the treatment of stasis dermatitis, an often chronic, usually eczematous dermatitis. Actinici dermatitis is a dermatitis that due to exposure to actinic radiation such as that from the sun, ultraviolet waves, or x- or gamma-radiation. According to the present invention, the subject method can be used in the treatment and/or prevention of certain symptoms of dermatitis caused by unwanted proliferation of epithelial cells. Such therapies for these various forms of dermatitis can also include topical and systemic corticosteroids, antipruritics, and antibiotics

Additional ailments that may be treated by the subject method are disorders specific to non-humans, such as mange.

*Non-canonical hedgeho signaling*

The hedgehog kinase antagonists of the invention can be used to regulate the activity in a noncanonical *Shh* pathway that is independent of the *Patched-Smoothened* receptor complex and the *Gli* transcription factors. In a recent report, Jarov et al., Dev. Biol. 261(2): 520-536 (2003), describes that, when *Shh* was immobilized to the subsrate (extracellular matrix) or produced by neuroepithelial cells themselves after transfection, neural plate explants failed to disperse and instead formed compact structures. Changes in the adhesive capacities of neuroepithelial cells caused by *Shh* could be accounted for by inactivation of surface β1-integrins combined with an increase in N-cadherin-mediated cell adhesion. This immobilized-*Shh*-mediated adhesion does not contradict or interfere with the previously known (soluble) *Shh*-mediated inductive, mitogenic, and trophic functions, since the immobilized Shh promoted differentiation of neuroepithelial cells into motor neurons and floor plate cells with the same potency as soluble *Shh.* It has also been demonstrated that *Shh*-regulation of adhesion properties during neural tube morphogenesis is rapid and reversible, and it does not involve the classical *Patched-Smoothened-Gli* signaling pathway, and it is independent and discernible from Shh-mediated cell differentiation. Thus, modifications of the adhesive properties of neural epithelial cells induced by *Shh* cannot be attributed to its differentiation-promoting effect, but reveal a novel function of *Shh* in this tissue that has not beeen described previously. Thus, the hedgehog kinase antagonists of the present invention may be used to regulate this non-canonical hedgehog pathway that is independent of *Ptch, Smo, Fu, Su(Fu)* and/or *Gli.* More specifically, such hedgehog kinase antagonists may be used in a method to disrupt this function in neuronal or other applicable tissues, preferably at specific developmental stages.

IV. Compositions and Methods of the Invention

A. Anti-hedgehog kinase Antibodies

Described is use of anti-hedgehog kinase antibodies, which may find use herein as therapeutic, diagnostic and/or prognostic agents in determining the severity of and/or prognosing the disease course of a tumor or cancer. Exemplary antibodies that may be used for such purposes include polyclonal, monoclonal, humanized, bispecific, and heteroconjugate antibodies. The term "antibodies" sometimes also include antigen-binding fragments. Because the hedgehog kinases are intracellular components of the hedgehog signaling pathway, internalizing antibodies, or techniques of intracellular delivery of the antibodies are preferred.

1. Polyclonal Antibodies

Polyclonal antibodies are preferably raised in animals by multiple subcutaneous (sc) or intraperitoneal (ip) injections of the relevant antigen and an adjuvant. It may be useful to conjugate the relevant antigen (especially when synthetic peptides are used) to a protein that is immunogenic in the species to be immunized. For example, the antigen can be conjugated to keyhole limpet hemocyanin (KLH), serum albumin, bovine thyroglobulin, or soybean trypsin inhibitor, using a bifunctional or derivatizing agent, *e.g.*, maleimidobenzoyl sulfosuccinimide ester (conjugation through cysteine residues), N-hydroxysuccinimide (through lysine residues), glutaraldehyde, succinic anhydride, $SOCl_2$, or R'N=C=NR, where R and $R^1$ are different alkyl groups.

Animals are immunized against the antigen, immunogenic conjugates, or derivatives by combining, *e.g.*, 100 μg or 5 μg of the protein or conjugate (for rabbits or mice, respectively) with 3 volumes of Freund's complete adjuvant and injecting the solution intradermally at multiple sites. One month later, the animals are boosted with 1/5 to 1/10 the original amount of peptide or conjugate in Freund's complete adjuvant by subcutaneous injection at multiple sites. Seven to 14 days later, the animals are bled and the serum is assayed for antibody titer. Animals are boosted until the titer plateaus. Conjugates also can be made in recombinant cell culture as protein fusions. Also, aggregating agents such as alum are suitably used to enhance the immune response.

2. Monoclonal Antibodies

Monoclonal antibodies may be made using the hybridoma method first described by Kohler et al., Nature, 256: 495 (1975), or may be made by recombinant DNA methods (U.S. Patent No. 4,816,567).

In the hybridoma method, a mouse or other appropriate host animal, such as a hamster, is immunized as described above to elicit lymphocytes that produce or are capable of producing antibodies that will specifically bind to the protein used for immunization. Alternatively, lymphocytes may be immunized *in vitro.* After immunization, lymphocytes are isolated and then fused with a myeloma cell line using a suitable fusing agent, such as polyethylene glycol, to form a hybridoma cell (Goding, Monoclonal Antibodies: Principles and Practice, pp. 59-103 (Academic Press, 1986)).

The hybridoma cells thus prepared are seeded and grown in a suitable culture medium which medium preferably contains one or more substances that inhibit the growth or survival of the unfused, parental myeloma cells (also referred to as fusion partner). For example, if the parental myeloma cells lack the enzyme hypoxanthine guanine phosphoribosyl transferase (HGPRT or HPRT), the selective culture medium for the hybridomas typically will include hypoxanthine, aminopterin, and thymidine (HAT medium), which substances prevent the growth of HGPRT-deficient cells.

Preferred fusion partner myeloma cells are those that fuse efficiently, support stable high-level production of antibody by the selected antibody-producing cells, and are sensitive to a selective medium that selects against the unfused parental cells. Preferred myeloma cell lines are murine myeloma lines, such as those derived from MOPC-21 and MPC-11 mouse tumors available from the Salk Institute Cell Distribution Center, San Diego, California USA, and SP-2 and derivatives *e.g.*, X63-Ag8-653 cells available from the American Type Culture Collection, Manassas, Virginia, USA. Human myeloma and mouse-human heteromyeloma cell lines also have been described for the production of human monoclonal antibodies (Kozbor, J. Immunol., 133:3001 (1984); and Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunosorbent assay (ELISA).

3. Human and Humanized Antibodies

The anti-hedgehog kinase antibodies useful in the practice of the invention may further comprise humanized antibodies or human antibodies. Humanized forms of non-human (*e.g.*, murine) antibodies are chimeric immunoglobulins, immunoglobulin chains or fragments thereof (such as Fv, Fab, Fab', $F(ab')_2$ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321: 522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity and HAMA response (human anti-mouse antibody) when the antibody is intended for human therapeutic use. According to the so-called "best-fit" method, the sequence of the variable domain of a rodent antibody is screened against the entire library of known human variable domain sequences. The human V domain sequence which is closest to that of the rodent is identified and the human framework region (FR) within it accepted for the humanized antibody (Sims et al., J. Immunol. 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol. 151:2623 (1993)).

Alternatively, phage display technology (McCafferty et al., Nature 348:552-553 [1990]) can be used to produce human antibodies and antibody fragments *in vitro,* from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats, reviewed in, *e.g.*, Johnson, Kevin S. and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). See, also, U.S. Patent Nos. 5,565,332 and 5,573,905.
As discussed above, human antibodies may also be generated by *in vitro* activated B cells (see U.S. Patents 5,567,610 and 5,229,275).

4. Antibody fragments

In certain circumstances there are advantages of using antibody fragments, rather than whole antibodies. The smaller size of the fragments allows for rapid clearance, while retaining similar antigen binding specificity of the corresponding full length molecule, and may lead to improved access to solid tumors.
Various techniques have been developed for the production of antibody fragments. Traditionally, these fragments were derived via proteolytic digestion of intact antibodies (see, *e.g.,* Morimoto et al., J ournal of Biochemical and Biophysical Methods 24:107-117 (1992); and Brennan et al., Science, 229:81 (1985)). However, these fragments can now be produced directly by recombinant host cells. Fab, Fv and scFv antibody fragments can all be expressed in and secreted from *E. coli,* thus allowing the facile production of large amounts of these fragments. Antibody fragments can be isolated from the antibody phage libraries discussed above. Alternatively, Fab'-SH fragments can be directly recovered from *E. coli* and chemically coupled to form $F(ab')_2$ fragments (Carter et al., Bio/Technology 10:163-167 (1992)). According to another approach, $F(ab')_2$ fragments can be isolated directly from recombinant host cell culture. Fab and $F(ab')_2$ fragment with increased in vivo half-life comprising a salvage receptor binding epitope residues are described in U.S. Patent No. 5,869,046. Other techniques for the production of antibody fragments will be apparent to the skilled practitioner. In other embodiments, the antibody of choice is a single chain Fv fragment (scFv). See WO 93/16185; U.S. Patent No. 5,571,894; and U.S. Patent No. 5,587,458. Fv and sFv are the only species with intact combining sites that are devoid of constant regions; thus, they are suitable for reduced nonspecific binding during in vivo use. sFv fusion proteins may be constructed to yield fusion of an effector protein at either the amino or the carboxy terminus of an sFv. See Antibody Engineering, ed. Borrebaeck, supra. The antibody fragment may also be a "linear antibody", *e.g.*, as described in U.S. Patent 5,641,870 for example. Such linear antibody fragments may be monospecific or bispecific.

5. Immunoconjugates

The invention also pertains to immunoconjugates comprising an antibody conjugated to a cytotoxic agent such as a chemotherapeutic agent, a growth inhibitory agent, a toxin (*e.g.*, an enzymatically active toxin of bacterial, fungal, plant, or animal origin, or fragments thereof), or a radioactive isotope (*i.e.*, a radioconjugate).

a. Chemotherapeutic agents

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeniginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include $^{212}Bi$, $^{131}I$, $^{131}In$, $^{90}Y$, and $^{186}Re$. Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.
Conjugates of an antibody and one or more small molecule toxins, such as a calicheamicin, maytansinoids, a trichothene, and CC 1065, and the derivatives of these toxins that have toxin activity, are also contemplated herein.

b. Maytansine and maytansinoids

In anti-hedgehog kinase antibody (full length or fragments thereof) described may be conjugated to one or more maytansinoid molecules.

Maytansinoids are mitototic inhibitors which act by inhibiting tubulin polymerization. Maytansine was first isolated from the east African shrub *Maytenus serrata* (U.S. Patent No. 3,896,111). Subsequently, it was discovered that certain microbes also produce maytansinoids, such as maytansinol and C-3 maytansinol esters (U.S. Patent No. 4,151,042). Synthetic maytansinol and derivatives and analogues thereof are disclosed, for example, in U.S. Patent Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533.

In an attempt to improve their therapeutic index, maytansine and maytansinoids have been conjugated to antibodies specifically binding to tumor cell antigens. Immunoconjugates containing maytansinoids and their therapeutic use are disclosed, for example, in U.S. Patent Nos. 5,208,020, 5,416,064 and European Patent EP 0 425 235 B1. Liu et al., Proc. Natl. Acad. Sci. USA 93:8618-8623 (1996) described immunoconjugates comprising a maytansinoid designated DM1 linked to the monoclonal antibody C242 directed against human colorectal cancer. The conjugate was found to be highly cytotoxic towards cultured colon cancer cells, and showed antitumor activity in an *in vivo* tumor growth assay. Chari et al., Cancer Research 52:127-131 (1992) describe immunoconjugates in which a maytansinoid was conjugated via a disulfide linker to the murine antibody A7 binding to an antigen on human colon cancer cell lines, or to another murine monoclonal antibody TA.1 that binds the HER-2/*neu* oncogene. The cytotoxicity of the TA.1-maytansonoid conjugate was tested *in vitro* on the human breast cancer cell line SK-BR-3, which expresses 3 x $10^5$ HER-2 surface antigens per cell. The drug conjugate achieved a degree of cytotoxicity similar to the free maytansinoid drug, which could be increased by increasing the number of maytansinoid molecules per antibody molecule. The A7-maytansinoid conjugate showed low systemic cytotoxicity in mice.

Hedgehog kinase-maytansinoids, including anti-hedghog kinase antibody-maytansinoid (e.g., anti-GYK antibody-maytansinoid, anti-NEK1 antibody-maytansinoid, anti-TTK antibody-maytansinoid, anti-TTBK1 antibody maytansinoid), antigen binding fragments or oligopeptides of the same, may be prepared by chemically linking the antibody, fragment or oligopeptide to a maytansinoid molecule without significantly diminishing the biological activity of either the antibody, fragment, oligopeptide or the maytansinoid molecule. An average of 3-4 maytansinoid molecules conjugated per antibody molecule has shown efficacy in enhancing cytotoxicity of target cells without negatively affecting the function or solubility of the antibody, although even one molecule of toxin/antibody would be expected to enhance cytotoxicity over the use of naked antibody. Maytansinoids are well known in the art and can be synthesized by known techniques or isolated from natural sources. Suitable maytansinoids are disclosed, for example, in U.S. Patent No. 5,208,020 and in the other patents and nonpatent publications referred to hereinabove. Preferred maytansinoids are maytansinol and maytansinol analogues modified in the aromatic ring or at other positions of the maytansinol molecule, such as various maytansinol esters.

There are many linking groups known in the art for making antibody- or antibody fragment-maytansinoid conjugates, including, for example, those disclosed in U.S. Patent No. 5,208,020 or EP Patent 0 425 235 B1, and Chari et al., Cancer Research 52:127-131 (1992). The linking groups include disufide groups, thioether groups, acid labile groups, photolabile groups, peptidase labile groups, or esterase labile groups, as disclosed in the above-identified patents, disulfide and thioether groups being preferred.

Conjugates of the antibody or antibody fragment and maytansinoid may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Particularly preferred coupling agents include N-succinimidyl-3-(2-pyridyldithio) propionate (SPDP) (Carlsson et al., Biochem. J. 173:723-737 [1978]) and N-succinimidyl-4-(2-pyridylthio)pentanoate (SPP) to provide for a disulfide linkage.

The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hyrdoxymethyl, the C-15 position modified with a hydroxyl group, and the C-20 position having a hydroxyl group. In a preferred embodiment, the linkage is formed at the C-3 position of maytansinol or a maytansinol analogue.

c. Calicheamicin

Another immunoconjugate of interest comprises anti-hedgehog kinase antibody, a hedgehog kinase binding antibody fragment, or hedgehog kinase oligopeptide conjugated to one or more calicheamicin molecules. The calicheamicin family of antibiotics is capable of producing double-stranded DNA breaks at sub-picomolar concentrations. For the preparation of conjugates of the calicheamicin family, see U.S. patents 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, $\gamma_1^I$, $\alpha_2^I$, $\alpha_3^I$, N-acetyl-$\gamma_1^I$, PSAG and $\theta^I_1$ (Hinman et al., Cancer Research 53:3336-3342 (1993), Lode et al., Cancer Research 58:2925-2928 (1998) and the aforementioned U.S. patents to American Cyanamid). Another anti-tumor drug that the antibody can be conjugated is QFA which is an antifolate. Both calicheamicin and QFA have intracellular sites of action and do not readily cross the plasma membrane. Therefore, cellular uptake of these agents through antibody mediated internalization greatly enhances their cytotoxic effects.

d. Other cytotoxic agents

Other antitumor agents that can be conjugated to the anti-hedgehog kinase antagonists (antibodies, antigent binding fragment, oligopeptide) of the invention include BCNU, streptozoicin, vincristine and 5-fluorouracil, the family of agents known collectively LL-E33288 complex described in U.S. patents 5,053,394, 5,770,710, as well as esperamicins (U.S. patent 5,877,296).

Enzymatically active toxins and fragments thereof which can be used include diphtheria A chain, nonbinding active fragments of diphtheria toxin, exotoxin A chain (from *Pseudomonas aruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

The present invention further contemplates an immunoconjugate formed between an antibody and a compound with nucleolytic activity (e.g., a ribonuclease or a DNA endonuclease such as a deoxyribonuclease; DNase).

For selective destruction of the tumor, the antibody may comprise a highly radioactive atom. A variety of radioactive isotopes are available for the production of radioconjugated anti-hedgehog kinase antibodies. Examples include $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu. When the conjugate is used for diagnosis, it may comprise a radioactive atom for scintigraphic studies, for example $tc^{99m}$ or $I^{123}$, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

The radio- or other labels may be incorporated in the conjugate in known ways. For example, the peptide may be biosynthesized or may be synthesized by chemical amino acid synthesis using suitable amino acid precursors involving, for example, fluorine-19 in place of hydrogen. Labels such as $tc^{99m}$ or $I^{123}$, $Re^{186}$, $Re^{188}$ and $In^{111}$ can be attached via a cysteine residue in the peptide. Yttrium-90 can be attached via a lysine residue. The IODOGEN method (Fraker et al (1978) Biochem. Biophys. Res. Commun. 80: 49-57 can be used to incorporate iodine-123. "Monoclonal Antibodies in Immunoscintiography" (Chatal, CRC Press 1989) describes other methods in detail.

Conjugates of the antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al., Cancer Research 52:127-131 (1992); U.S. Patent No. 5,208,020) may be used.

Alternatively, a fusion protein or chimeric molecule comprising the hedgehog kinase antagonist (e.g., antibody, antigen-binding fragment, oligopeptide) and cytotoxic agent may be made, *e.g.*, by recombinant techniques or peptide synthesis. The length of DNA may comprise respective regions encoding the two portions of the conjugate either adjacent one another or separated by a region encoding a linker peptide which does not destroy the desired properties of the conjugate.

The antibody described may be conjugated to a "receptor" (such streptavidin) for utilization in tumor pre-targeting wherein the antibody-receptor conjugate is administered to the patient, followed by removal of unbound conjugate from the circulation using a clearing agent and then administration of a "ligand" (*e.g.*, avidin) which is conjugated to a cytotoxic agent (*e.g.*, a radionucleotide).

B. Hedgehog Kinase Binding Oligopeptides

Hedgehog kinase binding oligopeptides or hedgehog kinase oligopeptides described herein are oligopeptides that bind, preferably specifically, to a hedgehog kinase polypeptide as described herein. Hedgehog kinase binding oligopeptides may be chemically synthesized using known oligopeptide synthesis methodology or may be prepared and purified using recombinant technology. Hedgehog kinase oligopeptides are usually at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more, wherein such oligopeptides that are capable of binding, preferably specifically, to a hedgehog kinase polypeptide as described herein. Hedgehog kinase oligopeptides may be identified without undue experimentation using well known techniques. In this regard, it is noted that techniques for screening oligopeptide libraries for oligopeptides that are capable of specifically binding to a polypeptide target are well known in the art (see, e.g., U.S. Patent Nos. 5,556,762, 5,750,373, 4,708,871, 4,833,092, 5,223,409, 5,403,484, 5,571,689, 5,663,143; PCT Publication Nos. WO 84/03506 and WO84/03564; Geysen et al., Proc. Natl. Acad. Sci. U.S.A., 81:3998-4002 (1984); Geysen et al., Proc. Natl. Acad. Sci. U.S.A., 82:178-182 (1985); Geysen et al., in Synthetic Peptides as Antigens, 130-149 (1986); Geysen et al., J. Immunol. Meth., 102:259-274 (1987); Schoofs et al., J. Immunol., 140:611-616 (1988), Cwirla, S. E. et al. (1990) Proc. Natl. Acad. Sc i. USA 87:6378; Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363, and Smith, G. P. (1991) Current Opin. Biotechnol., 2:668).

In this regard, bacteriophage (phage) display is one well known technique which allows one to screen large oligopeptide libraries to identify member(s) of those libraries which are capable of specifically binding to a polypeptide target. Phage display is a technique by which variant polypeptides are displayed as fusion proteins to the coat protein on the surface of bacteriophage particles (Scott, J.K. and Smith, G. P. (1990) Science 249: 386). The utility of phage display lies in the fact that large libraries of selectively randomized protein variants (or randomly cloned cDNAs) can be rapidly and efficiently sorted for those sequences that bind to a target molecule with high affinity. Display of peptide (Cwirla, S. E. et al. (1990) Proc. Natl. Acad. Sci. USA, 87:6378) or protein (Lowman, H.B. et al. (1991) Biochemistry. 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363) libraries on phage have been used for screening millions of polypeptides or oligopeptides for ones with specific binding properties (Smith, G. P. (1991) Current Opin. Biotechnol., 2:668). Sorting phage libraries of random mutants requires a strategy for constructing and propagating a large number of variants, a procedure for affinity purification using the target receptor, and a means of evaluating the results of binding enrichments. U.S. Patent Nos. 5,223,409, 5,403,484, 5,571,689, and 5,663,143.

Although most phage display methods have used filamentous phage, lambdoid phage display systems (WO 95/34683; U.S. 5,627,024), T4 phage display systems (Ren et al., Gene, 215: 439 (1998); Zhu et al., Cancer Research, 58(15): 3209-3214 (1998); Jiang et al., Infection & Immunity, 65(11): 4770-4777 (1997); Ren et al., Gene, 195(2):303-311 (1997); Ren, Protein Sci., 5: 1833 (1996); Efimov et al., Virus Genes, 10: 173 (1995)) and T7 phage display systems (Smith and Scott, Methods in Enzymology, 217: 228-257 (1993); U.S. 5,766,905) are also known.

Many other improvements and variations of the basic phage display concept have now been developed. These improvements enhance the ability of display systems to screen peptide libraries for binding to selected target molecules and to display functional proteins with the potential of screening these proteins for desired properties. Combinatorial reaction devices for phage display reactions have been developed (WO 98/14277) and phage display libraries have been used to analyze and control bimolecular interactions (WO 98/20169; WO 98/20159) and properties of constrained helical peptides (WO 98/20036). WO 97/35196 describes a method of isolating an affinity ligand in which a phage display library is contacted with one solution in which the ligand will bind to a target molecule and a second solution in which the affinity ligand will not bind to the target molecule, to selectively isolate binding ligands. WO 97/46251 describes a method of biopanning a random phage display library with an affinity purified antibody and then isolating binding phage, followed by a micropanning process using microplate wells to isolate high affinity binding phage. The use of *Staphlylococcus aureus* protein A as an affinity tag has also been reported (Li et al. (1998) Mol Biotech., 9:187). WO 97/47314 describes the use of substrate subtraction libraries to distinguish enzyme specificities using a combinatorial library which may be a phage display library. A method for selecting enzymes suitable for use in detergents using phage display is described in WO 97/09446. Additional methods of selecting specific binding proteins are described in U.S. Patent Nos. 5,498,538, 5,432,018, and WO 98/15833.

Methods of generating peptide libraries and screening these libraries are also disclosed in U.S. Patent Nos. 5,723,286, 5,432,018, 5,580,717, 5,427,908, 5,498,530, 5,770,434, 5,734,018, 5,698,426, 5,763,192, and 5,723,323.

C. Screening for Hedgehog Kinase Antagonists

Techniques for generating the hedgehog kinase antagonists (polypeptides, antibodies, polypeptides, oligopeptides, RNAi molecules, Rnai contructs, ribozymes and organic molecules) for use have been described above. One may further select antibodies (and antigen-binding fragments thereof), oligopeptides or other organic molecules with certain biological characteristics, as desired.

The growth inhibitory effects of the various hedgehog kinase antagonists useable in the invention may be assessed by methods known in the art, e.g., using cells which express a hedgehog kinase polypeptide either endogenously or following transfection with the hedgehog kinase gene. For example, appropriate tumor cell lines and cells transfected with hedgehog kinase -encoding nucleic may be treated with the hedgehog kinase antagonists of the invention at various concentrations for a few days (*e.g.*, 2-7) days and stained with crystal violet or MTT or analyzed by some other colorimetric assay. Another method of measuring proliferation would be by comparing $^3$H-thymidine uptake by the cells treated in the presence or absence of such hedgehog kinase antagonists. After treatment, the cells are harvested and the amount of radioactivity incorporated into the DNA quantitated in a scintillation counter. Appropriate positive controls include treatment of a selected cell line with a growth inhibitory antibody known to inhibit growth of that cell line. Growth inhibition of tumor cells *in vivo* can be determined in various ways known in the art. Preferably, the tumor cell is one that overexpresses a hedgehog polypeptide. Preferably, such hedgehog kinase antagonists will inhibit cell proliferation of a hedgehog-expressing tumor cell *in vitro* or *in vivo* by about 25-100% compared to the untreated tumor cell, more preferably, by about 30-100%, and even more preferably by about 50-100% or 70-100%, in one embodiment, at an antibody concentration of about 0.5 to 30 μg/ml. Growth inhibition can be measured at a hedgehog kinase antagonist concentration of about 0.5 to 30 μg/ml or about 0.5 nM to 200 nM in cell culture, where the growth inhibition is determined 1-10 days after exposure of the tumor cells to the antagonist. The antagonist is growth inhibitory *in vivo* if administration of antagonist and/or agonist at about 1 μg/kg to about 100 mg/kg body weight results in reduction in tumor size or reduction of tumor cell proliferation within about 5 days to 3 months from the first administration of the antibody, preferably within about 5 to 30 days.

To select for hedgehog kinase antagonists which induce cell death, loss of membrane integrity as indicated by, *e.g.*, propidium iodide (PI), trypan blue or 7AAD uptake may be assessed relative to control. A PI uptake assay can be performed in the absence of complement and immune effector cells. Hedgehog kinase polypeptide-expressing tumor cells are incubated with medium alone or medium containing the appropriate hedgehog kinase antagonist. The cells are incubated for a 3 day time period. Following each treatment, cells are washed and aliquoted a into 35 mm strainer-capped 12 x 75 tubes (1ml per tube, 3 tubes per treatment group) for removal of cell clumps. Tubes then receive PI (10μg/ml). Samples may be analyzed using a FACSCAN® flow cytometer and FACSCONVERT® CellQuest software (Becton Dickinson). Those hedgehog kinase antagonists that induce statistically significant levels of cell death as determined by PI uptake may then be selected.

To screen for anti-hedgeghog kinase antibodies which bind to an epitope on a hedgehog kinase polypeptide, a routine cross-blocking assay such as that described in Antibodies: A Laboratory Manual, Cold Spring Harbor Laboratory, Ed Harlow and David Lane (1988), can be performed. This assay can be used to determine if a test antibody, oligopeptide or other organic molecule binds the same site or epitope as a known anti-hedgehog kinase antibody. Alternatively, or additionally, epitope mapping can be performed by methods known in the art. For example, the antibody sequence can be mutagenized such as by alanine scanning, to identify contact residues. The mutant antibody is initially tested for binding with polyclonal antibody to ensure proper folding. In a different method, peptides corresponding to different regions of a hedgehog kinase polypeptide can be used in competition assays with the test antibodies or with a test antibody and an antibody with a characterized or known epitope.

D. Hedgehog Kinase Polypeptide Variants

In addition to the hedgehog kinase polypeptides described herein, it is contemplated that variants of such molecules can be prepared. Such variants can be prepared by introducing appropriate nucleotide changes into the encoding DNA, and/or by synthesis of the desired antibody or polypeptide. Those skilled in the art will appreciate that amino acid changes may alter post-translational processes of these molecules, such as changing the number or position of glycosylation sites or altering the membrane anchoring characteristics.

Variations in amino acid sequence can be made, for example, using any of the techniques and guidelines for conservative and non-conservative mutations set forth, for instance, in U.S. Patent No. 5,364,934. Variations may be a substitution, deletion or insertion of one or more codons encoding the amino acid sequence that results in a change in the amino acid sequence as compared with the native sequence. Optionally the variation is by substitution of at least one amino acid with any other amino acid in one or more of the domains of the amino acid sequence of interest. Guidance in determining which amino acid residue may be inserted, substituted or deleted without adversely affecting the desired activity may be found by comparing the sequence of the amino acid sequence of interest with homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, *i.e.*, conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

Fragments of the various hedgehog kinase polypeptides are provided herein. Such fragments may be truncated at the N-terminus or C-terminus, or may lack internal residues, for example, when compared with a full length native antibody or protein. Such fragments which lack amino acid residues that are not essential for a desired biological activity are also useful with the disclosed methods.

The above polypeptide fragments may be prepared by any of a number of conventional techniques. Desired peptide fragments may be chemically synthesized. An alternative approach involves generating such fragments by enzymatic digestion, *e.g.,* by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding the desired fragment fragment by polymerase chain reaction (PCR). Oligonucleotides that define the desired termini of the DNA fragment are employed at the 5' and 3' primers in the PCR. Preferably, such fragments share at least one biological and/or immunological activity with the corresponding full length molecule.

Conservative substitutions of interest are shown in Table 5 under the heading of preferred substitutions. If such substitutions result in a change in biological activity, then more substantial changes, denominated exemplary substitutions in Table 5, or as further described below in reference to amino acid classes, are introduced and the products screened in order to identify the desired variant.

Table 5

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser, Ala | Ser |
| Gln (Q) | Asn; Glu | Asn |
| Glu (E) | Asp, Gln | Asp |
| Gly (G) | Pro; Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Leu |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Substantial modifications in function or immunological identity of the hedgehog kinase variant polypeptides are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: Norleucine, Met, Ala, Val, Leu, Ile;
(2) neutral hydrophilic: Cys, Ser, Thr; Asn; Gln
(3) acidic: Asp, Glu;
(4) basic: His, Lys, Arg;
(5) residues that influence chain orientation: Gly, Pro; and
(6) aromatic: Trp, Tyr, Phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13: 4331 (1986); Zoller et al., Nucl. Acids Res., 10:6487 (1987)], cassette mutagenesis [Wells et al., Gene, 34:315 (1985)], restriction-selection mutagenesis [Wells et al., Philos. Trans. R. Soc. London SerA, 317:415 (1986)] or other known techniques can be performed on the cloned DNA to produce the hedgehog kinase molecule.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244:1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)). If alanine substitution does not yield adequate amounts of variant, an isoteric amino acid can be used.

Any cysteine residue not involved in maintaining the proper conformation of the hedgehog kinase variant polypeptides also may be substituted, generally with serine, to improve the oxidative stability of the molecule and prevent aberrant crosslinking. Conversely, cysteine bond(s) may be added to such a molecule to improve its stability (particularly where the antibody is an antibody fragment such as an Fv fragment).

A particularly preferred type of substitutional variant involves substituting one or more residues of a binding region. For example, in the case of a hedgehog kinase antagonist that is an anti-hedgehog kinase antibody, a hypervariable region residues of a parent antibody (*e.g.*, a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable region sites (*e.g.*, 6-7 sites) are mutated to generate all possible amino substitutions at each site. The variants thus generated are displayed in a monovalent fashion from filamentous phage particles as fusions to the gene III product of M13 packaged within each particle. The phage-displayed variants are then screened for their biological activity (*e.g.*, binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and target polypeptide. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Nucleic acid molecules encoding amino acid sequence variants of hedgehog kinase variant polypeptides are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of a native sequence or an earlier prepared variant.

E. Preparation of hedgehog polypeptides

The description below relates primarily to production of hedgehog polypeptides by culturing cells transformed or transfected with a vector containing nucleic acid such antibodies, polypeptides, oligopeptides. For purposes of this section only, the term "hedgehog polypeptides" shall include hedgehog signaling components, as well as certain hedgehog kinase antagonists (*i.e.*, antibodies, polypeptides, oligopeptides that may be conveniently prepared by recombinant technology. It is, of course, contemplated that alternative methods, which are well known in the art, may be employed to prepare such antibodies, polypeptides and oligopeptides. For instance, the appropriate amino acid sequence, or portions thereof, may be produced by direct peptide synthesis using solid-phase techniques [see, *e.g.,* Stewart et al., Solid-Phase Peptide Synthesis, W.H. Freeman Co., San Francisco, CA (1969); Merrifield, J. Am. Chem. Soc., 85: 2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of such antibodies, polypeptides or oligopeptides may be chemically synthesized separately and combined using chemical or enzymatic methods to produce the desired product.

1. Isolation of DNA encoding hedgehog polypeptides

DNA encoding a hedgehog polypeptide may be obtained from a cDNA library prepared from tissue believed to possess such antibody, polypeptide or oligopeptide mRNA and to express it at a detectable level. Accordingly, DNA encoding such polypeptides can be conveniently obtained from a cDNA library prepared from human tissue, a genomic library or by known synthetic procedures (*e.g.*, automated nucleic acid synthesis).

Libraries can be screened with probes (such as oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). Alternatively, PCR methodology may be used. [Sambrook *et al.,* supra; Dieffenbach et al., PCR Primer; A Laboratory Manual (Cold Spring Harbor Laboratory Press, 1995)].

Techniques for screening a cDNA library are well known in the art. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like $^{32}P$-labeled ATP, biotinylation or enzyme. labeling. Hybridization conditions, including moderate stringency and high stringency, are provided in Sambrook *et al.,* supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook *et al.,* supra, to detect precursors and processing intermediates of mRNA that may not have been reverse-transcribed into cDNA.

2. Selection and Transformation of Host Cells

Host cells are transfected or transformed with expression or cloning vectors described herein for hedgehog polypeptide production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: A Practical Approach, M. Butler, ed. (IRL Press, 1991) and Sambrook *et al.,* supra.

Methods of eukaryotic cell transfection and prokaryotic cell transformation are known to the ordinarily skilled artisan, for example, $CaCl_2$, $CaPO_4$, liposome-mediated and electroporation. Depending on the host cell used, transformation is performed using standard techniques appropriate to such cells. The calcium treatment employing calcium chloride, as described in Sambrook *et al.,* supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact,, 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, *e.g.*, polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia, e.g., E. coli, Enterobacter, Erwinia, Klebsiella, Proteus, Salmonella, e.g., Salmonella typhimurium, Serratia, e.g., Serratia marcescans,* and *Shigella,* as well as *Bacilli* such as *B. subtilis* and *B. licheniformis* (*e.g., B. licheniformis* 41P disclosed in DD 266,710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA; E. coli* W3110 strain 9E4, which has the complete genotype *tonA ptr3; E. coli* W3110 strain 27C7 (ATCC 55,244), which has the complete genotype *tonA ptr3 phoA E15 (argF-lac)169 degP ompT kan$^r$; E. coli* W3110 strain 37D6, which has the complete genotype *tonA ptr3 phoA E15 (argF-lac)169 degP ompT rbs7 ilvG kan$^r$; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, *e.g.,* PCR or other nucleic acid polymerase reactions, are suitable.

Full length antibody, antibody fragments, and antibody fusion proteins can be produced in bacteria, in particular when glycosylation and Fc effector function are not needed, such as when the therapeutic antibody is conjugated to a cytotoxic agent (*e.g.*, a toxin) and the immunoconjugate by itself shows effectiveness in tumor cell destruction. Full length antibodies have greater half life in circulation. Production in *E. coli* is faster and more cost efficient. For expression of antibody fragments and polypeptides in bacteria, see, *e.g.,* U.S. 5,648,237 (Carter et. al.), U.S. 5,789,199 (Joly et al.*),* and U.S. 5,840,523 (Simmons et al.) which describes translation initiation region (TIR) and signal sequences for optimizing expression and secretion, these patents incorporated herein by reference. After expression, the antibody is isolated from the *E. coli* cell paste in a soluble fraction and can be purified through, *e.g.,* a protein A or G column depending on the isotype. Final purification can be carried out similar to the process for purifying antibody expressed in suitable cells (*e.g.*, CHO cells).

In addition to prokaryotes, eukaryotic microbes such as filamentous fungi or yeast are suitable cloning or expression hosts for vectors encoding hedgehog polypeptides. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, *e.g., K. lactis* (MW98-8C, CBS683, CBS4574; Louvencourt et al., J. Bacteriol., 154 (2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans,* and *K. marxianus; yarrowia* (EP 402,226); *Pichia pastoris* (EP 183,070; Sreekrishna et al., J. Basic Microbiol., 28: 265-278 [1988]); *Candida; Trichoderma reesia* (EP 244,234); *Neurospora crassa* (Case et al., Proc. Natl, Acad. Sci. USA, 76:5259-5263 [1979]); *Schwanniomyces* such as *Schwanniomyces occidentalis* (EP 394,538 published 31 October 1990); and filamentous fungi such as, *e.g., Neurospora, Penicillium, Tolypocladium* (WO 91/00357 published 10 January 1991), and *Aspergillus* hosts such as *A. nidulans* (Ballance et al., Biochem. Biophys. Res. Commun., 112:284-289 [1983]; Tilbum et al., Gene, 26:205-221 [1983]; Yelton et al., Proc. Natl. Acad. Sci. USA, 81: 1470-1474 [1984]) and *A. niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated hedgehog kinase polypeptide production are derived from multicellular organisms. Examples of invertebrate cells include insect cells such as Drosophila S2 and Spodoptera Sf9, as well as plant cells, such as cell cultures of cotton, corn, potato, soybean, petunia, tomato, and tobacco. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* have been identified. A variety of viral strains for transfection are publicly available, *e.g.,* the L-1 variant of *Autographa californica* NPV and the Bm-5 strain of *Bombyx mori* NPV, and such viruses may be used as the virus herein according to the present invention, particularly for transfection of *Spodoptera frugiperda* cells.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CVI ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for hedgehog polypeptide production and cultured in conventional nutrient media modified as appropriate for inducing promoters, selecting transformants, or amplifying the genes encoding the desired sequences.

3. Selection and Use of a Replicable Vector

The nucleic acid (*e.g.*, cDNA or genomic DNA) encoding the respective hedgehog polypeptide may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The hedgehog polypeptide may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the DNA encoding the mature sequence that is inserted into the vector. The signal sequence may be a prokaryotic signal sequence selected, for example, from the group of the alkaline phosphatase, penicillinase, Ipp, or heat-stable enterotoxin II leaders. For yeast secretion the signal sequence may be, *e.g.,* the yeast invertase leader, alpha factor leader (including *Saccharomyces* and *Kluyveromyces* α-factor leaders, the latter described in U.S. Patent No. 5,010,182), or acid phosphatase leader, the C. *albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2μ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, *e.g.,* ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up nucleic acid encoding the desire protein, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the nucleic acid sequence encoding the desired amino acid sequence, in order to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding the desired protein sequence.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

DNA Transcription in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the hedghog polypeptide may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, $\alpha$-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the coding sequence of the preceding amino acid sequences, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding the respective antibody, polypeptide or oligopeptide described in this section.

Still other methods, vectors, and host cells suitable for adaptation to the synthesis of the respective antibody, polypeptide or oligopeptide in recombinant vertebrate cell culture are described in Gething et al., Nature, 293:620-625 (1981); Mantei et al., Nature, 281:40-46 (1979); EP 117,060; and EP 117,058.

4. <u>Culturing the Host Cells</u>

The host cells used to produce the hedgehog polypeptides may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem.102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

5. <u>Detecting Gene Amplification/Expression</u>

Gene amplification and/or expression may be measured in a sample directly, for example, by conventional Southern blotting, Northern blotting to quantitate the transcription of mRNA [Thomas, Proc. Natl. Acad. Sci. USA, 77: 5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies suitable for the present method may be prepared against a native sequence polypeptide or oligopeptide, or against exogenous sequence fused to DNA and encoding a specific antibody epitope of such a polypeptide or oligopeptide.

6. <u>Protein Purification</u>

Hedgehog polypeptides may be recovered from culture medium or from host cell lysates. If membrane-bound, it can be released from the membrane using a suitable detergent solution (e.g. Triton-X 100) or by enzymatic cleavage. Cells employed in expression of the preceding can be disrupted by various physical or chemical means, such as freeze-thaw cycling, sonication, mechanical disruption, or cell lysing agents.

It may be desireable to purify the preceding from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the desired molecules. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular antibody, polypeptide or oligopeptide produced for the claimed methods.

When using recombinant techniques, the hedgehog polypeptide can be produced intracellularly, in the periplasmic space, or directly secreted into the medium. If such molecules are produced intracellularly, as a first step, the particulate debris, either host cells or lysed fragments, are removed, for example, by centrifugation or ultrafiltration. Carter et al., Bio/Technology 10:163-167 (1992) describe a procedure for isolating antibodies which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

Purification can occur using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies that are based on human γ1, γ2 or γ4 heavy chains (Lindmark et al., J. Immunol. Meth. 62:1-13 (1983)). Protein G is recommended for all mouse isotypes and for human γ3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a $C_H3$ domain, the Bakerbond ABX™resin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to low pH hydrophobic interaction chromatography using an elution buffer at a pH between about 2.5-4.5, preferably performed at low salt concentrations (*e.g.*, from about 0-0.25M salt).

## F. Pharmaceutical Formulations

Therapeutic formulations of the hedgehog kinase antagonists ("therapeutic agent") used in accordance with the present invention may be prepared for storage by mixing the therapeutic agent(s) having the desired degree of purity with optional pharmaceutically acceptable carriers, excipients or stabilizers (Remington: The Science of Practice of Pharmacy, 20th edition, Gennaro, A. et al., Ed., Philadelphia College of Pharmacy and Science (2000)), in the form of lyophilized formulations or aqueous solutions. Acceptable carriers, excipients, or stabilizers are nontoxic to recipients at the dosages and concentrations employed, and include buffers such as acetate, Tris, phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; tonicifiers such as trehalose and sodium chloride; sugars such as sucrose, mannitol, trehalose or sorbitol; surfactant such as polysorbate; salt-forming counter-ions such as sodium; metal complexes (*e.g.*, Zn-protein complexes); and/or non-ionic surfactants such as TWEEN®, PLURONICS® or polyethylene glycol (PEG). The antibody preferably comprises the antibody at a concentration of between 5-200 mg/ml, preferably between 10-100 mg/ml.

The formulations herein may also contain more than one active compound as necessary for the particular indication being treated, preferably those with complementary activities that do not adversely affect each other. For example, in addition to the preceding therapeutic agent(s), it may be desirable to include in the formulation, an additional antibody, *e.g.*, a second such therapeutic agent, or an antibody to some other target such as a growth factor that affects the growth of the glioma. Alternatively, or additionally, the composition may further comprise a chemotherapeutic agent, cytotoxic agent, cytokine, growth inhibitory agent, anti-hormonal agent, and/or cardioprotectant. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington: The Science and Practice of Pharmacy, *supra.*

Sustained-release preparations may be prepared. Suitable examples of sustained-release preparations include semi-permeable matrices of solid hydrophobic polymers containing the antibody, which matrices are in the form of shaped articles, *e.g.*, films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT® (injectable microspheres composed of lactic acid-glycolic acid copolymer and leuprolide acetate), and poly-D-(-)-3-hydroxybutyric acid.

The formulations to be used for *in vivo* administration must be sterile. This is readily accomplished by filtration through sterile filtration membranes.

G. Diagnosis and Treatment with hedgehog kinase antagonists

To determine hedgehog kinase expression in tumor or cancer, various diagnostic assays are available. In one embodiment, hedgehog polypeptide overexpresion and/or polypeptide overexpression may be analyzed by immunohistochemistry (IHC). Paraffin embedded tissue sections from a tumor biopsy may be subjected to the IHC assay and accorded a hedgehog kinase polypeptide staining intensity criteria as follows:

Score 0 - no staining is observed or membrane staining is observed in less than 10% of tumor cells.
Score 1+ - a faint/barely perceptible membrane staining is detected in more than 10% of the tumor cells. The cells are only stained in part of their membrane.
Score 2+ - a weak to moderate complete membrane staining is observed in more than 10% of the tumor cells.
Score 3+ - a moderate to strong complete membrane staining is observed in more than 10% of the tumor cells.

Those tumors with 0 or 1+ scores for hedgehog kinase expression may be characterized as not overexpressing hedgehog kinase, respectively, whereas those tumors with 2+ or 3+ scores may be characterized as overexpressing hedgehog kinase, respectively.

Alternatively, or additionally, FISH assays such as the INFORM® (sold by Ventana, Arizona) or PATHVISION® (Vysis, Illinois) may be carried out on formalin-fixed, paraffin-embedded tumor tissue to determine the extent (if any) of hedgehog kinase overexpression in the tumor.

Hedgehog kinase overexpression or amplification may be evaluated using an *in vivo* diagnostic assay, *e.g.*, by administering a molecule (such as an antibody, oligopeptide, organic molecule, RNAi, Rnai construct, antisense oligonucleotide) which binds the molecule to be detected and is tagged with a detectable label (*e.g.*, a radioactive isotope or a fluorescent label) and externally scanning the patient for localization of the label.

Currently, depending on the stage of the cancer, cancer treatment involves one or a combination of the following therapies: surgery to remove the cancerous tissue, radiation therapy, and chemotherapy. Therapy comprising of administering hedgehog kinase antagonists may be especially desirable in elderly patients who do not tolerate the toxicity and side effects of chemotherapy well and in metastatic disease where radiation therapy has limited usefulness. The tumor targeting hedgehog kinase antagonists of the present inventive method may also be used to alleviate hedgehog kinase overexpressing cancers upon initial diagnosis of the disease or during relapse. For therapeutic applications, such hedgehog kinase antagonists can be used in combination with, before or after application of other conventional agents and/or methods for the treatment of glioma, e.g., hormones, antiangiogens, or radiolabelled compounds, or with surgery, cryotherapy, radiotherapy and/or chemotherapy. Chemotherapeutic drugs such as TAXOTERE® (docetaxel), TAXOL® (palictaxel), estramustine and mitoxantrone are used in treating cancer, in particular, in good risk patients.

In particular, combination therapy with palictaxel and modified derivatives (see, e.g., EP0600517) is contemplated. The preceding antibody, polypeptide, oligopeptide or organic molecule will be administered with a therapeutically effective dose of the chemotherapeutic agent. In another embodiment, such antibody, polypeptide, oligopeptide or organic molecule is administered in conjunction with chemotherapy to enhance the activity and efficacy of the chemotherapeutic agent, e.g., paclitaxel. The Physicians' Desk Reference (PDR) discloses dosages of these agents that have been used in treatment of various cancers. The dosing regimen and dosages of these aforementioned chemotherapeutic drugs that are therapeutically effective will depend on the particular cancer being treated, the extent of the disease and other factors familiar to the physician of skill in the art and can be determined by the physician.

In one particular embodiment, an immunoconjugate comprising such a hedgehog kinase antagonist conjugated with a cytotoxic agent is administered to the patient. Preferably, such immunoconjugate is internalized by the cell, resulting in increased therapeutic efficacy of the immunoconjugate in killing the cancer cell to which it binds. In a preferred embodiment, the cytotoxic agent targets or interferes with the nucleic acid in the cancer cell. Examples of such cytotoxic agents are described above and include maytansinoids, calicheamicins, ribonucleases and DNA endonucleases.

The preceding hedgehog kinase antagonists or toxin conjugates thereof are administered to a human patient, in accord with known methods, such as intravenous administration, e.g., as a bolus or by continuous infusion over a period of time, by intracranial, intracerobrospinal, intra-articular, intrathecal, intravenous, intraarterial, subcutaneous, oral, topical, or inhalation routes.

Other therapeutic regimens may be combined with the administration of the foregoing hedgehog kinase antagonists. The combined administration includes co-administration, using separate formulations or a single pharmaceutical formulation, and consecutive administration in either order, wherein preferably there is a time period while both (or all) active agents simultaneously exert their biological activities. Preferably such combined therapy results in a synergistic therapeutic effect.

In another embodiment, the use of a medicament according to the present invention involves the combined administration of one or more of the above hedgehog kinase antagonist and one or more chemotherapeutic agents or growth inhibitory agents, including co-administration of cocktails of different chemotherapeutic agents. Example chemotherapeutic agents have been provided previously. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992).

For the prevention or treatment of disease, the dosage and mode of administration will be chosen by the physician according to known criteria. The appropriate dosage of hedgehog kinase antagonists will depend on the type of disease to be treated, the severity and course of the disease, whether administration is for preventive or therapeutic purposes, previous therapy (including) the patient's clinical history and response, and the discretion of the attending physician. The preceding hedgehog kinase antagonists may be suitably administered to the patient at one time or over a series of treatments. Administration may occur by intravenous infusion or by subcutaneous injections. Depending on the type and severity of the disease, about 1 μg/kg to about 50 mg/kg body weight (e.g., about 0.1-15mg/kg/dose) of hedgehog kinase antagonist can be an initial candidate dosage for administration to the patient, whether, for example, by one or more separate administrations, or by continuous infusion. A dosing regimen can comprise administering an initial loading dose of about 4 mg/kg, followed by a weekly maintenance dose of about 2 mg/kg of such a hedgehog kinase antagonist. However, other dosage regimens may be useful. A typical daily dosage might range from about 1 μg/kg to 100 mg/kg or more, depending on the factors mentioned above. For repeated administrations over several days or longer, depending on the condition, the treatment is sustained until a desired suppression of disease symptoms occurs. The progress of this therapy can be readily monitored by conventional methods and assays and based on criteria known to the physician or other persons of skill in the art.

Aside from administration of the antibody protein to the patient, the present application describes administration of the antibody by gene therapy. Such administration of nucleic acid encoding the hedgehog kinase polypeptide antagonists is encompassed by the expression "administering a therapeutically effective amount of an antibody". See, for example, WO96/07321 published March 14, 1996 concerning the use of gene therapy to generate intracellular antibodies.

There are two major approaches to getting such nucleic acid (optionally contained in a vector) into the patient's cells; *in vivo* and *ex vivo.* For *in vivo* delivery the nucleic acid is injected directly into the patient, usually at the site where the antibody is required. For *ex vivo* treatment, the patient's cells are removed, the nucleic acid is introduced into these isolated cells and the modified cells are administered to the patient either directly or, for example, encapsulated within porous membranes which are implanted into the patient (see, *e.g.*, U.S. Patent Nos. 4,892,538 and 5,283,187). There are a variety of techniques available for introducing nucleic acids into viable cells. The techniques vary depending upon whether the nucleic acid is transferred into cultured cells *in vitro,* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, *etc.* A commonly used vector for *ex vivo* delivery of the gene is a retroviral vector.

The currently preferred *in vivo* nucleic acid transfer techniques include transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). For review of the currently known gene marking and gene therapy protocols see Anderson et al., Science 256:808-813 (1992). See also WO 93/25673 and the references cited therein.

H. Articles of Manufacture and Kits

For therapeutic applications, the article of manufacture comprises a container and a label or package insert on or associated with the container indicating a use for the inhibition in whole or in part of hedgehog signaling, or alternatively for the treatment of a disorder or condition resulting from activation of the hedgehog signaling pathway. Suitable containers include, for example, bottles, vials, syringes, etc. The containers may be formed from a variety of materials such as glass or plastic. The container holds a composition which is effective for treating the cancer condition and may have a sterile access port (for example the container may be an intravenous solution bag or a vial having a stopper pierceable by a hypodermic injection needle). At least one active agent in the composition is a hedgehog kinase antagonist. The label or package insert indicates that the composition is used for treating glioma. The label or package insert will further comprise instructions for administering the hedgehog kinase antagonist. Additionally, the article of manufacture may further comprise a second container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Kits may also be provided that are useful for various other purposes, *e.g.*, for hedgehog kinase-expressing cell killing assays, for purification or immunoprecipitation of hedgehog kinase from cells. For isolation and purification of hedgehog kinase polypeptide, the kit can contain the respective hedgehog kinase-binding reagent coupled to beads (e.g., sepharose beads). Kits can be provided which contain such molecules for detection and quantitation of hedgehog kinase polypeptide *in vitro, e.g.,* in an ELISA or a Western blot. As with the article of manufacture, the kit comprises a container and a label or package insert on or associated with the container. The container holds a composition comprising at least one such hedgehog kinase binding- antibody, oligopeptide, RNAi, Rnai construct or organic molecule useable with the invention. Additional containers may be included that contain, e.g., diluents and buffers, control antibodies. The label or package insert may provide a description of the composition as well as instructions for the intended in vitro or diagnostic use.

I. Sense and Anti-Sense Hedgehog Kinase-Encoding Nucleic Acids

Molecules that would be expected to inhibit hedgehog kinase, and therefore activate or amplify hedgehog signaling include fragments of the hedgehog kinase-encoding nucleic acids such as antisense or oligonucleotides, which comprise a single-stranded nucleic acid sequence (either RNA or DNA) capable of binding to target hedgehog kinase mRNA (sense) or target hedgehog kinase DNA (antisense) sequences. Antisense or sense oligonucleotides described herein, comprise a fragment of the coding region of the respective hedgehog kinase DNA. The ability to derive an antisense or a sense oligonucleotide, based upon a cDNA sequence encoding a given protein is described in, for example, Stein and Cohen (Cancer Res. 48:2659, 1988) and van der Krol et al. (BioTechniques 6:958, 1988).

Binding of antisense or sense oligonucleotides to target nucleic acid sequences results in the formation of duplexes that block transcription or translation of the target sequence by one of several means, including enhanced degradation of the duplexes, premature termination of transcription or translation, or by other means. Such methods are encompassed by the present invention. The antisense oligonucleotides thus may be used to block expression of hedgehog kianse proteins, wherein those hedgehog kinase proteins may play a role in the innhibiton or attenuation of hedgehog signaling. Antisense or sense oligonucleotides further comprise oligonucleotides having modified sugar-phosphodiester backbones (or other sugar linkages, such as those described in WO 91/06629) and wherein such sugar linkages are resistant to endogenous nucleases. Such oligonucleotides with resistant sugar linkages are stable *in vivo* (*i.e.,* capable of resisting enzymatic degradation) but retain sequence specificity to be able to bind to target nucleotide sequences.

The antisense compounds used as described herein may be conveniently and routinely made through the well-known technique of solid phase synthesis. Equipment for such synthesis is sold by several vendors including, for example, Applied Biosystems (Foster City, Calif.). Any other means for such synthesis known in the art may additionally or alternatively be employed. It is well known to use similar techniques to prepare oligonucleotides such as the phosphorothioates and alkylated derivatives. The compounds of the invention may also be admixed, encapsulated, conjugated or otherwise associated with other molecules, molecule structures or mixtures of compounds, as for example, liposomes, receptor targeted molecules, oral, rectal, topical or other formulations, for assisting in uptake, distribution and/or absorption. Patents that teach the preparation of such uptake, distribution and/or absorption assisting formulations include, but are not limited to, U.S. Pat. Nos. 5,108,921; 5,354,844; 5,416,016; 5,459,127; 5,521,291; 5,543,158; 5,547,932; 5,583,020; 5,591,721; 4,426,330; 4,534,899; 5,013,556; 5,108,921; 5,213,804; 5,227,170; 5,264,221; 5,356,633; 5,395,619; 5,416,016; 5,417,978; 5,462,854; 5,469,854; 5,512,295; 5,527,528; 5,534,259; 5,543,152; 5,556,948; 5,580,575; and 5,595,756.

Other examples of sense or antisense oligonucleotides include those oligonucleotides which are covalently linked to organic moieties, such as those described in WO 90/10048, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid sequence by any gene transfer method, including, for example, $CaPO_4$-mediated DNA transfection, electroporation, or by using gene transfer vectors such as Epstein-Barr virus. In a preferred procedure, an antisense or sense oligonucleotide is inserted into a suitable retroviral vector. A cell containing the target nucleic acid sequence is contacted with the recombinant retroviral vector, either *in vivo* or *ex vivo.* Suitable retroviral vectors include, but are not limited to, those derived from the murine retrovirus M-MuLV, N2 (a retrovirus derived from M-MuLV), or the double copy vectors designated DCT5A, DCT5B and DCT5C (see WO 90/13641).

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

Antisense or sense RNA or DNA molecules are generally at least about 5 nucleotides in length, alternatively at least about 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleotides in length, wherein in this context the term "about" means the referenced nucleotide sequence length plus or minus 10% of that referenced length.

J. <u>Other Hedeghog Kinase Antagonists</u>

A hedgehog kinase antagonists described herein may be a ribozyme. Ribozymes are a form an antisense oligonucleotides in which catalytic RNA molecules with ribonuclease activity which are capable of cleaving a single-stranded nucleic acid, such as an mRNA, to which they have a complementary region (for reviews on ribozymes see e.g., Ohkawa. J. et al. (1995) J. Biochem 118:251-258; Sigurdsson, S. T. and Eckstein. F. (1995) Trends Biotechnol 13: 286-289; Rossi, J. J. (1995) Trends Biotechnol. 13:301-306; Kiehntopf, M. et al. (1995) J. Mol. Med. 73:65-71). A ribozyme having specificity for human hedgehog kinase mRNA can be designed based upon the nucleotide sequence of the human hedgehog kinase cDNA. For example, a derivative of a *Tetrahymena* L-19 IVS RNA can be constructed in which the base sequence of the active site is complementary to the base sequence to be cleaved in a human hedgehog kinase mRNA. See for example U.S. Pat. Nos. 4,987,071 and 5,116,742. both by Cech et al. Alternatively, human hedgehog kinase mRNA can be used to select a catalytic RNA having a specific ribonuclease activity from a pool of RNA molecules. See for example Bartel, D. and Szostak. J. W. (1993) Science 261: 1411-1418.

Another type of inhibitory agent that can be used to inhibit the expression and/or activity of human hedgehog kinase in a cell is an intracellular antibody specific for the human hedgehog kinase protein. The use of intracellular antibodies to inhibit protein function in a cell is known in the art (see e.g., Carlson, J. R. (1988) Mol. Cell. Biol. 8:2638-2646; Biocca, S. et al. (1990) EMBO J. 9:101-108; Werge, T. M. et al. (1990) FEBS Letters 274:193-198; Carlson, J. R. (1993) Proc. Natl. Acad. Sci. USA 90:7427-7428; Marasco, W. A. et al. (1993) Proc. Natl. Acad. Sci. USA 90:7889-7893; Biocca, S. et al. (1994) Bio/Technology 12:396-399; Chen, S-Y. et al. (1994) Human Gene Therapy 5:595-601; Duan, L et al. (1994) Proc. Natl. Acad. Sci. USA 91:5075-5079; Chen, S-Y. et al. (1994) Proc. Natl. Acad. Sci. USA 91:5932-5936; Beerli, R. R. et al. (1994) J. Biol Chem. 269:23931-23936; Beerli. R. R. et al. (1994) Biochem. Biophys. Res. Commun. 204:666-672. Mhashilkar, A. M. et al. (1995) EMBO J. 14:1542-1551; Richardson, J. H. et al. (1995) Proc. Natl. Acad Sci. USA 92:3137-3141; PCT Publication No. WO 94/02610 by Marasco et al.; and PCT Publication No. WO 95/03832 by Duan et al.).

To inhibit protein activity using an intracellular antibody, a recombinant expression vector is prepared which encodes the antibody chains in a form such that upon introduction of the vector into a cell, the antibody chains are expressed as a functional antibody in an intracellular compartment of the cell. For inhibition of human hedgehog kinase activity according to the inhibitory methods of the invention, an intracellular antibody that specifically binds the human hedgehog kianse protein is expressed in the cytoplasm of the cell. To prepare an intracellular antibody expression vector, antibody light and heavy chain cDNAs encoding antibody chains specific for the target protein of interest. e.g., human hedgehog kinase, are isolated, typically from a hybridoma that secretes a monoclonal antibody specific for the human hedgehog kinase protein. Hybridomas secreting anti-human hedgehog kinase monoclonal antibodies, or recombinant anti-human hedgehog kinase monoclonal antibodies, can be prepared as described above. Once a monoclonal antibody specific for human hedgehog kinase protein has been identified (e.g., either a hybridoma-derived monoclonal antibody or a recombinant antibody from a combinatorial library), DNAs encoding the light and heavy chains of the monoclonal antibody are isolated by standard molecular biology techniques. For hybridoma derived antibodies, light and heavy chain cDNAs can be obtained, for example, by PCR amplification or cDNA library screening. For recombinant antibodies, such as from a phage display library, cDNA encoding the light and heavy chains can be recovered from the display package (e.g., phage) isolated during the library screening process. Nucleotide sequences of antibody light and heavy chain genes from which PCR primers or CDNA library probes can be prepared are known in the art. For example, many such sequences are disclosed in Kabat, E. A., et al (1991) Sequences of Proteins of Immunological Interest, Fifth Edition, U.S. Department of Health and Human Services, NIH Publication No. 91-3242 and in the "Vbase" human germline sequence database.

Once obtained, the antibody light and heavy chain sequences are cloned into a recombinant expression vector using standard methods. To allow for cytoplasmic expression of the light and heavy chains, the nucleotide sequences encoding the hydrophobic leaders of the light and heavy chains are removed. An intracellular antibody expression vector can encode an intracellular antibody in one of several different forms. For example, in one embodiment, the vector encodes full-length antibody light and heavy chains such that a full-length antibody is expressed intracellularly. In another embodiment, the vector encodes a full-length light chain but only the VH/CH1 region of the heavy chain such that a Fab fragment is expressed intracellularly. In the most preferred embodiment, the vector encodes a single chain antibody (scFv) wherein the variable regions of the light and heavy chains are linked by a flexible peptide linker (e.g., $(Gly_4Ser)_3$) and expressed as a single chain molecule. To inhibit human hedgehog kinase activity in a cell, the expression sector encoding the anti-human hedgehog kinase intracellular antibody is introduced into the cell by standard transfection methods, as discussed hereinbefore.

Yet another form of a hedgehog kinase antagonist described is an inhibitory form of human hedgehog kinase, also referred to herein as a dominant negative inhibitor. The hedgehog kinase proteins are known to modulate the activity of hedgehog signaling component target molecules, particularly by modulating the phosphorylation state of the hedgehog signaling component target molecule. One means to inhibit the activity of molecule that has an enzymatic activity is through the use of a dominant negative inhibitor that has the ability to interact with the target molecule but that lacks enzymatic activity. By interacting with the target molecule, such dominant negative inhibitors can inhibit the activation of the target molecule. This process may occur naturally as a means to regulate enzymatic activity of a cellular signal transduction molecule.

Accordingly, an inhibitory agent described herein can be a form of a human hedgehog kinase protein that has the ability to interact with other proteins but that lacks enzymatic activity. This dominant negative form of a human hedgehog kinase protein may be, for example, a mutated form of human hedgehog kinase in which a *kinase* consensus sequence has been altered. Such dominant negative human hedgehog kinase proteins can be expressed in cells using a recombinant expression vector encoding the human hedgehog kinase protein, which is introduced into the cell by standard transfection methods. The mutated DNA is inserted into a recombinant expression vector, which is then introduced into a cell to allow for expression of the mutated human hedgehog kinase, lacking enzymatic activity.

Other inhibitory agents that can be used to inhibit the activity of a human hedgehog kinase protein are chemical compounds that directly inhibit human hedgehog kinase activity or inhibit the interaction between human hedgehog kinase and target molecules. Such compounds can be identified using screening assays that select for such compounds, as described in detail above.

K. Screening Assays for Use in Identification of Hedgehog Kinase Antagonists:

The assays can be performed in a variety of formats, biochemical screening assays, cell-based assays, and kinase assays which are well characterized in the art.

All assays for antagonists are common in that they call for contacting the drug candidate with a hedgehog kinase polypeptide (hereinafter "target molecule"), herein under conditions and for a time sufficient to allow these two components to interact.

In binding assays, the interaction is binding between the candidate compound and target molecule and the complex formed can be isolated or detected in the reaction mixture. In a particular embodiment, the target molecule encoded by the gene identified herein or the drug candidate is immobilized on a solid phase, e.g., on a microtiter plate, by covalent or non-covalent attachments. Non-covalent attachment generally is accomplished by coating the solid surface with a solution of the target molecule and drying. Alternatively, an immobilized antibody, e.g., a monoclonal antibody, specific for the target molecule to be immobilized can be used to anchor it to a solid surface. The assay is performed by adding the non-immobilized component, which may be labeled by a detectable label, to the immobilized component, *e.g.*, the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a particular target molecule encoded by a gene identified herein, its interaction with that polypeptide can be assayed by methods well known for detecting protein-protein interactions. Such assays include traditional approaches, such as, *e.g.*, cross-linking, co-immunoprecipitation, and co-purification through gradients or chromatographic columns. In addition, protein-protein interactions can be monitored by using a yeast-based genetic system described by Fields and co-workers (Fields and Song, Nature (London), 340:245-246 (1989); Chien et al., Proc. Natl. Acad. Sci. USA, 88:9578-9582 (1991)) as disclosed by Chevray and Nathans, Proc. Natl. Acad. Sci. USA, 89: 5789-5793 (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lacZ* reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions. Compounds that interfere with the interaction of a gene encoding a target molecule identified herein and other intra- or extracellular components can be tested as follows: usually a reaction mixture is prepared containing the product of the gene and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner. To assay for suitable drug candidates, the target molecule may be added to a cell along with the compound to be screened for a particular activity (e.g, hedgehog signaling activation or inhibition) and the ability of the compound to inhibit the activity of interest in the presence of the target molecule indicates that the test compound is an antagonist to the target molecule. Alternatively, antagonists may be detected by combining the target molecule and a potential antagonist with membrane-bound target molecule or recombinant receptors under appropriate conditions for a competitive inhibition assay. The target molecule can be labeled, such as by radioactivity, such that the number of target molecules bound to the receptor can be used to determine the effectiveness of the potential antagonist. The gene encoding the receptor can be identified by numerous methods known to those of skill in the art, for example, ligand panning and FACS sorting. Coligan et al., Current Protocols in Immun., 1(2): Chapter 5 (1991). Preferably, expression cloning is employed wherein polyadenylated RNA is prepared from a cell responsive to the target molecule and a cDNA library created from this RNA is divided into pools and used to transfect COS cells or other cells that are not responsive to the target molecule. Transfected cells that are grown on glass slides are exposed to labeled target molecule. The target molecule can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor. As an alternative approach for receptor identification, labeled target molecule can be photoaffinity-linked with cell membrane or extract preparations that express the receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro- sequencing would be used to design a set of degenerate oligonucleotide probes to screen a cDNA library to identify the gene encoding the putative receptor. In another assay for antagonists, mammalian cells expressing active hedgehog signaling can be incubated with labeled target molecule in the presence of the candidate compound. The ability of the compound to enhance or block such hedgehog signaling could then be measured (*e.g.,* by measuring the level of downstream *Gli* activation). More specific examples of potential antagonists include an oligonucleotide that binds to the fusions of immunoglobulin with target molecule, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the target molecule that recognizes the receptor but imparts no effect, thereby competitively inhibiting the action of the target molecule. Another potential hedgehog kinase antagonist is an antisense RNA or DNA construct prepared using antisense technology, where, e.g., an antisense RNA or DNA molecule acts to block directly the translation of mRNA by hybridizing to targeted mRNA and preventing protein translation. Additional potential hedgehog kinase antagonists include small molecules that bind to the active site, the receptor binding site, or growth factor or other relevant binding site of the hedgehog kinase polypeptide, thereby blocking the normal biological activity of the hedgehog kinase polypeptide. Examples of small molecules include, but are not limited to, small peptides or peptide-like molecules, preferably soluble peptides, and synthetic non-peptidyl organic or inorganic compounds. Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, *e.g.*, Rossi, Current Biology, 4:469-471 (1994), and PCT publication No. WO 97/33551 (published September 18, 1997). Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, *e.g.,* PCT publication No. WO 97/33551, *supra.* These small molecules can be identified by any one or more of the screening assays discussed hereinabove and/or by any other screening techniques well known for those skilled in the art. When the screening methods are carried out as an *ex vivo* assay, the target molecule (*e.g.,* hedgehog kinase) can be a substantially purified polypeptide. The kinase substrate (e.g., MBP, *Gli*) is a substantially purified substrate, in which the assay is phosphorylated in a reaction with a substantially purified phosphate source that is catalyzed by the kinase. The extent of phosphorylation is determined by measuring the amount of substrate phosphorylated in the reaction. A variety of possible substrates may be used, including the kinase itself in which instance the phosphorylation reaction measured in the assay is autophosphorylation. Exogenous substrates may also be used, including standard protein substrates such as myelin basic protein (MBP); yeast protein substrates; synthetic peptide substrates, and polymer substrates. Of these, MBP and other standard protein substrates may be regarded as preferred (see Example 10). Other substrates may be identified, however, which are superior by way of affinity for the kinase, minimal perturbation of reaction kinetics, possession of single or homogenous reaction sites, ease of handling and post-reaction recover, potential for strong signal generation, and resistance or inertness to test compounds. Measurement of the amount of substrate phosphorylated in the preceding *ex vivo* assay of the invention may be carried out by means of immunoassay, radioassay or other well-known methods. In an immunoassay measurement, an antibody (such as a goat or mouse anti-phosphoserine/threonine antibody) may be used which is specific for phosphorylated moieties formed during the reaction. Using well-known ELISA techniques, the phosphoserine/threonine antibody complex would itself be detected by a further antibody linked to a label capable of developing a measurable signal (as for example a fluorescent or radioactive label). Additionally, ELISA-type assays in microtitre plates may be used to test purified substrates. Peraldi et al., J. Biochem. 285: 71-78 (1992); Schraag et al., Anal. Biochem. 211: 233-239 (1993); Cleavland, Anal. Biochem. 190: 249-253 (1990); Farley, Anal. Biochem. 203: 151-157 (1992) and Lozaro, Anal. Biochem. 192: 257-261 (1991). For example, detection schemes can measure substrate depletion during the kinase reaction. Initially, the phosphate source may be radiolabeled with an isotope such as $^{32}P$ or $^{33}P$, and the amount of substrate phosphorylation may be measured by determining the amount of radiolabel incorporated into the substrate during the reaction. Detection may be accomplished by:

(a) commercially available scintillant-containing plates and beads using a beta-counter, after adsorption to a filter or a microtitre well surface, or (b) photometric means after binding to a scintillation proximity assay bead or scintillant plate. Weernink and Kijken, J. Biochem. Biophs. Methods 31: 49, 1996; Braunwalder et al., Anal. Biochem. 234: 23 (1996); Kentrup et al., J. Biol. Chem. 271: 3488 (1996) and Rusken et al., Meth. Enzymol. 200: 98 (1991).

Preferably, the substrate is attached to a solid support surface by means of non-specific or, preferably, specific binding. Such attachment permits separation of the phosphorylated substrate from unincorporated, labeled phosphate source (such as adenosine triphosphate prior to signal detection. In one embodiment, the substrate may be physically immobilized prior to reaction, as through the use of Nunc™ high protein binding plate (Hanke et al., J. Biol. Chem. 271: 695 (1996)) or Wallac ScintiStrip™ plates (Braunwalder et al., Anal. Biochem. 234: 23 (1996). Substrate may also be immobilized after reaction by capture on, for example, P81 phophocellulose (for basic peptides), PEI/acidic molybdate resin or DEAE, or TCA precipitation onto Whatman™ 3M paper, Tiganis et al., Arch. Biochem. Biophys. 325: 289 (1996); Morawetz et al.. Mol. Gen. Genet. 250; 17 (1996); Budde et al, Int J. Pharmacognosy 33: 27 (1995) and Casnellie, Meth. Enz. 200: 115 (1991). Yet another possibility is the attachment of the substrate to the support surface, as by conjugation with binding partners such as glutathione and streptavidin (in the case of GST and biotin), respectively) which have been attached to the support, or via antibodies specific for the tags which are likewise attached to the support.
Further detection methods may be developed which are preferred to those described above. Especially for use in connection with high-throughput screening, it is expected that such methods would exhibit good sensitivity and specificity, extended linear range, low background signal, minimal fluctuation, compatibility with other reagents, and compatibility with automated handling systems.

The *in vivo* efficacy of the treatment of the present invention can be studied against chemically induced tumors in various rodent models. Tumor cell lines propagated in *in vitro* cell cultures can be introduced in experimental rodents, e.g. mice by injection, for example by the subcutaneous route. Techniques for chemical inducement of tumors in experimental animals are well known in the art.

L. Preparation of RNAi

An "RNA coding region" is a nucleic acid that can serve as a template for the synthesis of an RNA molecule, such as a double-stranded RNA complex. Preferably, the RNA coding region is a DNA sequence.

The RNA coding region preferably encodes a double-stranded RNA complex (e.g., siRNA, miRNA, shRNA) that is capable of down-regulating the expression of a particular gene or genes. In some embodiments, a double-stranded RNA complex is expressed in the form of an RNA molecule having a stem-loop or a so- called "hairpin" structure. As used herein, "hairpin" structure encompasses shRNAs and miRNAs. In some embodiments, a double-stranded RNA complex is expressed in the form of separate complementary or partially complementary RNA strands.

Methods are well-known in the art for designing double-stranded RNA complexes, eg, siRNA, miRNA, and shRNAs. For example, resources and citations describing the design of effective shRNA and siRNA are found in Sandy *et al, BioTechniques* 39:215-224 (2005). It is understood that the sequences of a double-stranded RNA complex may be of natural origin or may be synthetic. For example, Example 13 discloses a hybrid miRNA comprising a synthetic double stranded portion embedded in the backbone of a naturally occurring microRNA.

The RNA complex comprises a double-stranded region corresponding to a region of a gene to be down-regulated is expressed in the cell. One strand of the RNA double-stranded region is substantially identical (typically at least about 80%, 85%, 90%, 91%, 92%, 93%, 94%, 95%, 96%, 97%, 98%, 99% identical) in sequence to the sequence of the coding region targeted for down regulation. The other strand of the double-stranded region (interchangeably termed "RNA double-stranded region) is complementary to the sequence of the coding region targeted for down regulation, or partially complementary to the coding region targeted for down regulation (typically at least about 80%, 85%, 90%, 91%, 92%, 93%, 94%, 95%, 96%, 97%, 98%, 99% identical to the complement of the coding region targeted). It is understood that the double-stranded region can be formed by two separate RNA stranded, or by the self-complementary portions of a single RNA having a hairpin structure. The double-stranded region is generally at least about 15 nucleotides in length and, in some embodiments, is about 15 to about 30 nucleotides in length. However, a significantly longer double-stranded region can be used effectively in some organisms. In a more preferred embodiment, the double-stranded region is between about 19 and 22 nucleotides in length. The double-stranded region is preferably identical to the target nucleotide sequence over this region.

When the coding region to be down regulated is in a family of highly conserved genes, the sequence of the RNA double-stranded region can be chosen with the aid of sequence comparison to target only the desired gene. On the other hand, if there is sufficient identity among a family of homologous genes within an organism, a double-stranded molecule can be designed that would down regulate a plurality of genes simultaneously.

In some embodiments, a single RNA coding region in the construct serves as a template for the expression of a self- complementary hairpin RNA, comprising a sense region, a loop region and an antisense region. The sense and antisense regions are each preferably about 15 to about 30 nucleotides in length. The loop region preferably is about 2 to about 15 nucleotides in length, more preferably from about 4 to about 9 nucleotides in length. Following expression the sense and antisense regions form a duplex.

In another embodiment, the vector comprises two RNA coding regions. The first coding region is a template for the expression of a first RNA and the second coding region is a template for the expression of a second RNA. Following expression, the first and second RNAs form a duplex. The retroviral construct preferably also comprises a first Pol III promoter operably linked to the first RNA coding region and a second Pol III promoter operably linked to the second RNA coding region.

It is understood that, in certain embodiments, a vector of the invention can encompass nucleic acid sequences sufficient to form more than RNA coding region that inhibit expression of distinct target genes. In this embodiment, simultaneous inhibition of distinct target genes can be accomplished with a single vector of the invention. The number of different RNA complex transcripts that can be expressed simultaneously is limited only by the packaging capacity of the vector (if a viral vector is used) and adjacent promoters, including any of the promoters described below, can be selected to eliminate or minimize interference and allow for efficient simultaneous inhibition of multiple target genes. The inhibition of multiple RNA construct transcripts of adjacent promoters, for example, 2 or more, 3 or more, 4 or more, 5 or more, 6 or more, 7 or more, 8 or more, 9 or more, or 10 or more adjacent promoters allows the user to generate a desire phenotype that develops only when several coding regions (e.g., genes) are targeted simultaneously and enables manipulation and elucidation of complex genetic systems.

The following examples are offered for illustrative purposes only, and are not intended to limit the scope of the present invention in any way.

EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA. The present invention relates to CSNK1A1, GYK and PRKRA. All other kinases one presented for illustrative purposes only.

EXAMPLE 1: A Kinase siRNA Screen Identifies Regulators of Hedgehog Signaling

| Validated Gene List | Ref Seq Murine | Ref Seq Human | UNQ ID | Murine PRO ID | Murine DNA ID | Human PRO ID | Human DNA ID |
|---|---|---|---|---|---|---|---|
| CDC2L1 * | NM_ 007661 | NM_ 024011 | UNQ7109 | PRO22118 | DNA188786 | PRO111470 | DNA363066 |
| CSNK1A1 inventive | NM_ 146087 | NM_ 001892 | UNQ3289 | PRO22429 | DNA188624 | PRO100386 | DNA254256 |
| GYK inventive | NM_ 008194 | NM_ 000167 | UNQ7640 | PRO22649 | DNA189856 | PRO119056 | DNA370652 |
| NEK1 * | XM_ 620663 | NM_ 012224 | UNQ10553 | PRO44282 | DNA245262 | PRO113385 | DNA364981 |
| PLK1* | NM_ 011121 | NM_ 005030 | UNQ3368 | PRO39145 | DNA238060 | PRO37960 | DNA227497 |
| PRKAR1A * | NM_ 021880 | NM_ 002734 | UNQ11001 | PRO45644 | DNA245338 | PRO105508 | DNA357103 |
| PRKRA inventive | NM_ 011871 | NM_ 003690 | UNQ14535 | PRO137956 | DNA390234 | PRO102445 | DNA354038 |
| TTBK2* | NM_ 080788 | NM_ 173500 | UNQ29308 | PRO143411 | DNA395917 | PRO128708 | DNA380434 |
| TTK* | NM_ 009445 | NM_ 003318 | UNQ13762 | PRO42503 | DNA243238 | PRO58276 | DNA269878 |
| * for comparison only | | | | | | | |

**Methods:**

Cell lines

S12 cells used in the RNAi screen are drived from C3H10T1/2 cells (ATCC) stably integrated with a Hh-luciferase reporter as described in Frank-Kamenetsky et al., J Biol. 1: 10 (2002) and maintained according to ATCC guidelines. SV40 cells, used to determine the specificity of Hh candidates, are derived from C3H10T1/2 cells stably integrated with the SV40 promotor driving the expression of luciferase.

siRNA library and controls

A murine siRNA kinase library containing 3248 siRNAs was purchased from Dharmacon. The library contained four unique siRNAs per gene with a total of 812 genes consisting of all known murine kinases, and kinase-regulatory proteins. Dharmacon non-targeting siRNA (Dharmacon D001210-01) was used as a negative control, whereas Smo siRNA (Dharmacon M-041026) was used as a positive control.

High-throughout siRNA screening of Hedgehog regulators

Dharmacon libraries were dissolved in Dharmacon siRNA buffer and aliquoted into daughter plates at a concentration of 0.5 uM and stored at -80°C. Daughter plates were subsequently thawed the day of the transfection and 7 μl of each siRNA was transferred into a new 96-well plate with 7 μl of Optimem® (Gibco). A mixture consisting of 0.3 μl Dharmafect reagent #3 with 13.7 μl of Optimen® was then added to the siRNA and incubated for 20 mins. S12 cells were plated at a density of 11 000 cells/well. Sixty-four hours post transfection, 200 ng/ml Octyl modified Sonic Hedgehog (O-Shh, Frank-Kamenetsky et al., *supra*) was added to cells. Luciferase substrate was added 24 hours later using Steady Lite® according to the manufacturer's protocol and counts were measured using the Top Count® Luminometer. The assay was done in triplicate for each condition (-/+ 200 ng/ml O-Shh) with a final concentration of 25 nM siRNA. A separate set of siRNA-transfected plates was reserved and used to measure cell viability (Promega CellTiter-Glo) according to the manufacter's instructions. Before conducting the siRNA screen, we optimized several parameters for use in 96-well formate, including the type of transfection reagent used, number of cells seeded, and concentration of siRNA. For instance, S12 cells maximally respond to Hh when confluent and we found that seeding out cells at about 80% confluency at the time of siRNA transfection gave maximal Hh response. After optimization, Z-score calculation of 0.61 indicated our assay was robust Figure 8A.

Data Analysis

Luciferase measurements were converted to natural log (LN) values and normalized against a plate reference (Dharmacon non-targeting control, D001210-01). The triplicate normalized values were averaged and a score of "Hedgehog treatment" minus "no Hedgehog treatment" was calculated for each siRNA. Genes were considered potential hits if two or more siRNAs against the corresponding gene fell outside 1.5 standard deviations from the mean of all siRNAs in the library.

qRT-PCR

RNA was collected from S12 cells 88 hours post siRNA transfection to determine transcript knockdown (same conditions as the screen) or from S12 cells transfected with siRNA for 64 hours and then stimulated with Hh for 24 hours to determine the effects on Hh target genes (Gli1 and Ptch1) using the RNeasy Mini kit (Qiagen). Samples were then subjected to Dnase I (Invitrogen) treatment during purification to remove genomic DNA contamination. For real-time qRT-PCR assays, 100 ng/well of total RNA was used as a template using the One-Step qRT-PCR kit (Qiagen). Probes consisted of a 5'-FAM reporter and 3'-Tamra quencher. Duplicate wells were assayed for expression of the gene of interest (*Gli-1, Ptch1,* and *Rpl19* (control)) using SDS7700 (Applied Biosystems, Foster City, CA). The relative abundance of transcript was normalized to RPL19 levels using the 2-Ct method. In correlating phenotype with mRNA knockdown, we found that one gene was not expressed in our cell line since the CT value was 40. This was not due to the qRT-probe set since the same set gave a CT value of 23 uing testis mRNA.

Analysis was done using SDS software (version 1.7; Applied Biosystems).

| Oligo Name | qRT-PCR primer sequence |
|---|---|
| mCDC2L1_Forward | GCTGAGCCATGCTGGCA |
| mCDC2L1_Reverse | GTGAACCATACTCCCGAGCC |
| mCDC2L1_Probe | TCTCAAGGTGGGCGACTTTGGGC |
| mCSNKIA1_Forward | GGC TCC AAG GCC GAA TTT AT |
| mCSNK1A1_Reverse | CGA AAG AGC CAG ATC CGA TC |
| mCSNK1A1_Probe | TCG GTG GAA AAT ACA AAC TGG TGC GG |
| mGYK_Forward | CAC GTA TGG AAC AGG GTG CTT |
| mGYK_Reverse | CAG GAG GCC ATG TTC AGA AAA |
| mGYK_Probe | TTG TGC AAC ACG GGC CAT AAG TGT G |

| | |
|---|---|
| mNEK1_Forward | GAG GGT AAA AAG CTC AGA TGT TCC |
| mNEK1_Reverse | AAT GAC AGG CTT CAC CTG CTG |
| mNEK1_Probe | TGC CTT TGG AAC TTC TTG AAA CAG GTG GTT |
| mPLK1_Forward | GGGAGAAAGAGGAACCGGTG |
| mPLK1_Reverse | AGGTGGCACTCAATGGCC |
| mPLK1_Probe | TCCGGGAGACAAATG |
| mPRKAR1A_Forward | TTTGAACGCGTCCTTGGC |
| mPRKAR1A_Reverse | TGCTGGATGTTCCGCTTGA |
| mPRKAR1A_Probe | CGTGCTCAGACATC |
| mPRKRA_Forward | CAC CAG TCC TAT CAC CGT GTG T |
| mPRKRA_Reverse | AGC AGC GTC ACT CTG TGC AT |
| mPRKRA_Probe | ACG GCT CAG GCA TCT CCT GTG GC |
| mTTBK2_Forward | AGT TCA TCA CAG TGA CTC CCA CA |
| mTTBK2_Reverse | GGA ATT GTA ATC GCA GTC AGGG |
| mTTBK2_Probe | TCC CAT GGA GGC ACA AGC AGA AGG |
| mTTK_Forward | TACCCTTGCCGTACGTAACCTG |
| mTTK_Reverse | CCCTTTGCCAAGCCTGC |
| mTTK_Probe | CCTGCACTTACAGCTGCTGGCGC |
| Gli1_Forward | GCA GTG GGT AAC ATG AGT GTCT |
| Gli1_Reverse | AGG CAC TAG AGT TGA GGA ATT GT |
| Gli1_Probe | CTC TCC AGG CAG AGA CCC CAGC |
| Ptch1_Forward | CAG CTC TGT GCC CAG CTA |
| Ptch1_Reverse | GCA ACA GTC ACC GAA GCA |
| Ptch1_Probe | CCC ATC ACC ACT GTG ACG GCTT |
| Smo_Forward | CCC GGG CCA GGA GCT |
| Smo_Reverse | AAC CCG CAA CAG GTC CATC |
| Smo_Probe | TGG GAG ACA GTG TGC ATG CTG AAGGA |
| RPL19_Forward | AGA AGG TGA CCT GGA TGA GAA |
| RPL19_Reverse | TGA TAC ATA TGG CGG TCA ATCT |

RPL19_Probe CTT CTC AGG AGA TAC CGG GAA TCC AAG

Epistasis Experiments

GLI1 and SMO2 overexpression experiments were carried out in triplicate in 6-well format using 100 nM pooled siRNA and Dharmafect #3 transfection reagent (Dharmacon). After 24 hours, cells were trypsinized and re-plated onto 12-well plates. The following day (about 48 hours post siRNA transfection), cells were transfected with 400 ng vector control, SMOM2, or GLI1, 200 ng Hh-luciferase reporter, and 100 ng TK-Renilla using Gene Juice (Stratagene). Media was replaced after 6 hours. After an additional 48 hours, luciferase readings were measured using Dual-Glo Luciferase (Promega) according to the manufacturer's protocol. Renilla counts were also measured to normalize for plasmid transfection efficiency. Experiments were done in triplicate.

For double siRNA experiments, cells were transfected in 96-well format with 50 nM Gli3 siRNA along with 50 nM candidate siRNA (100 nM final concentration total siRNA) and Dharmafect #4 transfection reagent. We found that although Dharmafect #4 transfection reagent was slightly more toxic to cells than Dharmafect #3, transfection efficiencies were much higher as indicated by the level of Hh activation for Gli3 siRNA-treated cells (2 fold Gli3 HH pathway inducation for Dharmafect #3 as compared to 5 fold for Dharmafect #4). After 88 hours, luciferase assays were done using Steady Lite ® as described previously. Experiments were done in triplicate.

Cilia Experiments

Murine IMCD3 (ATCC) cells were transfected using lipfectamine 2000 (Invitrogen) with pSuper (Oligoengine) or pSuper-shNek1 and selected in media containing 2 μg/ml puromycin. After 3 weeks, the remaining pool of stably transfected cells were harvested and plated on LabTek 2-well chamber slides and grown for 7 days. Cells were fixed in 4% formaldehyde for 10 minutes at room temperature and blocked with Tris-buffered saline with 10% Donkey serum, 0.5% Triton X-100. Anti-acetylated tubulin antibody (1:5000) was incubated for 50 minutes and washed out with TBS with 0.1% Tween-20. Cy3-conjugated anti-mouse secondary antibody was used to visualize acetylated tubulin. For counter staining of nuclei, cells were mounted with Vectorshield mounting medium with DAPI (Vector Laboratories). For cilia labeled IMCD3 cells, Zeiss upright laster scanning confocal microscope 9LSM 510 Meta/NLO) equipped with a 63X, 0.9-NA water-dipping objective, and Chameleon multi-photon Excitation (650-950 nM) and Ar (488-nm) and two He/Ne (543- and 633-nm) lasers were used to make optical sections 0.5 μm thick, representing about 15-18 μm in z-height. For the counting of cells with cilia, three independent experiments were done with n=300 for each experiment.

**Results:**

To conduct the RNAi screen, we used a kinome siRNA library targeting 812 genes and consisting of 4 siRNAs per gene totaling 3248 unique siRNAs (Figure 5C). 64 hours post siRNA transfection, bacterially expressed and purified Sonic hedgehog (Shh) was added to the media to induce the Hh pathway. Assays for luciferase activity were conducted after another 24 hours. The screen was performed in triplicate for each treatment (with and without Shh), and a parallel screen to measure cell viability by more than 20% were eliminated from the screen.

To analyze the data, raw values were transformed to natural log (LN) and then normalized against the averaged non-targeting control siRNAs within the same plate (in triplicate on each experimental plate). Analysis of the normalized data showed correlation coefficients between replicates ranging from r= 0.71-0.82 indicating the assay was highly reproducible (Figure 5D). To determine which siRNAs affected induction of the Hh pathway, the difference between "Hh treatment" and "no Hh treatment" from the averaged triplicate values was determined for each siRNA (Figure 5E). Only genes that contained 2 or more independent siRNAs that scored outside 1.5 standard deviations from the mean of all siRNAs in the library were considered potential hits. Using this criteria, 30 kinases were chosen for further characterization.

To validate the 30 hits, we repeated the Hh-luciferase assay with each of the 4 siRNAs targeting the 30 hits and found that the same siRNAs against 29 of the 30 genes were able to reproducibly reduce Hh signaling. (Data not shown). Second, by quantitative RT-PCR, we determined whether Hh-luciferase reporter knockdown correlated with mRNA knockdown for each of the 4 siRNAs targeting the 29 genes (Figure 6A). Of the 29 genes, we could not detect expression of 1 gene in the S12 cells and could therefore only correlate Hh-luciferase reporter and mRNA knockdown for the remaining 28 genes. Surprisingly, many of the genes that reduced Hh signaling with only 2 out of 4 siRNAs (Figure 6A, "2 Hit" category), lacked correlation between Hh-luciferase reporter and mRNA knockdown, suggesting most of the hits in this category were false postives. Indeed, only 2 of the 19 genes (about 11%) in the "2 Hit" category showed correlation between Hh-luciferase reporter and transcript knockdown. Conversely, genes that reduced Hh signaling withat least 3 siRNAs (Figure 6A, "≥3 Hit" category) had high correlation between Hh-luciferase reporter and mRNA knockdown. In this category, 9 out of 9 genes (100%) showed correlation between Hh-luciferase reporter activity and transcript knockdown (Figure 6A, "≥3 Hit" category). Thus, a significant false-positive rate (89%) is still observed with 2 siRNAs/gene in contrast to 3 or 4 siRNAs/gene.

Increasing numbers of reports showing that siRNAs have significant off-target effects have generated skepticism about data produced by high-throughput RNAi screens, emphasizing the need for secondary assays to validate any hits. Although we felt that correlating the phenotype with the transcript knockdown filtered out the majority of false-positives, we wanted to further assess whether the effects on Hh signaling were due to gene-specific knockdown or to off-target effects. Off-target genes resulting from sense strand incorporation into the RISC complex and translational inhibition or "micro RNA effect" resulting from similarity of the anti-sense strand with non-specific targets. Lin et al., Nucleic Acid Res. 33: 4527 (2005); Jackson et al., RNA 12: 1197 (2006); Birmingham et al., Nat. Methods 3: 199 (2006); Fedorov et al., RNA 12: 1188 (2006).

Pooling of siRNAs has been shown to reduce significant off-target effects while maintaining on-target silencing. Fedorov *et al., supra.* To distinguish between phenotypes caused by on-target from off-target gene silencing, we re-synthesized and pooled siRNAs corresponding to each of the four original siRNA or each hit and repeated the Hh-luciferase assay. As shown in Figure 6B, 9 of the 11 hits were required for Hh signaling. The 2 hits that did not repeat (Pak6 and Scyll) in this assay are likely false-positives since the transcript knockdown using pooled siRNAs was equal if not better than the individual siRNAs despite no significant decrease in Hh-luciferase signaling (compare Figures 6A and 6B).

The table in Example 1 shows our 9 validated hits from the kinase screen. We next sought to determine whether the 9 genes were specifically required for Hh signaling, but perhaps did not affect general cell viability or transcription, by testing the corresponding pooled siRNAs for their ability to reduce luciferase expressed from a constitutive promoter SV40 (SV40-luciferase). As shown in Figure 2C, none of the hits had any significant effect on the SV40-luciferase reporter suggesting all 9 hits are specifically although maybe not exclusively, required for Hh signaling (Figure 6C). We also reasoned that if the candidate genes were essential for Hh signaling, they would be required for activation of endogenous Hh-target genes, Gli1 and Ptch1. When stimulated with Hh ligand, Smo siRNA-treated cells can only induce Gli1 and Ptch1 to about 20% of the level of cells transfected with control siRNA (Figure 6D). Similar to Smo siRNA treated cells, siRNA targeting all 9 candidates led to drastic reduction of Hh-induced activation of Gli1 and Ptch1 (Figure 6D). Thus, the candidates are required for Hh signaling and activation of endogenous Hh-target genes.

To place the candidates in the Hh pathway, we conducted epistasis experiments using contructs that either overexpress human SMO or GLI1, known positive regulators of the pathway, or siRNA against Gli3, a known negative regulator of the Hh pathway. SMO is required to relay the signal from Hh to the GLI transcription factors and mutations in human SMO, encoding SMOM2 (W535L), lead to constitutive activation of the Hh pathway *[*Xie et al., Nature 391:90 (1998)]. In C3H10T1/2 cells, transfection of SMOM2 can activate the pathway in a ligand independent manner. Murone et al., Curr. Biol. 9: 76 (1999). We reasoned that if the candidate genes acted upstream of Smo, silencing the target gene would not affect SMOM2-mediated Hh pathway activation. In contrast, if the hits acted downstream of Smo, silencing their expression would prevent SMOM2-mediated pathway activation. To assess whether the hits acted upstream or downstream of Smo, C3H10T1/2 cells were transformed with our candidate siRNAs and 48 hours post siRNA transfection, cells were transfected with control vector of human SMOM2, the Hh-luciferase reporter, and a renilla reporter to normalize for plasmid transfection efficiency. As a positive control, cells were transfected with a non-targeting siRNA control and Smo siRNA, both of which should not affect SMOM2's ability to activate the pathway (Figure 7A). Overexpression of SMOM2 was able to activate the pathway in cells treated with siRNA targeting Csnkla1, Gyk, Hek1 and Ttk, suggesting these genes likely act upstream of or in parallel to Smo (Figure 7A and 7D). In contract, overexpreession of SMOM2 was not able to or could only partially activate the pathway in cells treated with siRNA targeting CDC2L1, Plk1, Prkarla, Prkra and Ttbk2, suggesting these genes likely act downstream of Smo (Figure 7A and 7B).

Gli1 overexpression is also sufficient to activate the Hh pathway in a ligand independent manner but acts downstream of Smo. To place the hits relative to Gli1, we repeated the epistasis assay described above, but replaced human SMOM2 with GLI1. None of the siRNAs was able to reduce Hh pathway activation of GLI1 indicating that Gli1 likely acts downstream of our 9 hits (Figures 7B and 7D).

In contrast to Gli1, Gli3 acts as mostly a repressor of the Hh pathway and reporter cells treated with Gli3 siRNA show increased Hh-luciferase activity in a ligand-independent manner (Figure 5A). To place the candidates relative to Gli3, we transfected cells with siRNAs targeting Gli3 and each of the candidate genes. In comparison to control cells, cells treated with Gli3 siRNA show a five-fold increase in Hh activation as measured in the Hh-luciferase assay. Cells treated with both Gli3 and Smo siRNA also activate the pathway similar to Gli3, consistent with Smo acting upstream of Gli3 (Figure 7C). When Gli3 was combined with each of the siRNAs corresponding to the 9 hits, no reduction was observed in Gli3-dependent activation of the Hh pathway (Figure 7C). Thus, all of the nine candidates appear to act upstream of Gli3 (Figure 7D). A summary of where the hits act in the Hh pathway is shown in Figure 7D.

In the last few years, intraflagellar transport (IFT) within cilia has been shown to be critical for Hh signaling. Huangfu *et al.,* (2003), *supra;* Huangfu *et al.,* (2005), *supra*; May *et al. supra,* Corbit *et al*., *supra,* Haycraft *et al., supra.* Nek1 belongs to the NIMA family of kinases and in *Tetrahymena* and *Chlamydomomas,* NIMA-realted kinases that are required for ciliary function. Mahjoub et al., Mol. Biol. Cell 15: 5172 (2004), Wloga et al., Mol. Biol. Cell 17: 2799 (2006). Human NEK1 has been implicated in polycystic kidney disease (PKD), a syndrome thought to arise from cilia defects within the kidney. Upadhya et al., Proc. Natl. Acad. Sci. USA 97: 217 (2000). Nek1 has also been localized to the centrosome [Mahjoub et al., J Am. Soc. Nephrol. 16: 3485 (2005)], and has been shown to interact with the IFT anterograde motor protein, Kif3a [Surpili et al., Biochemistry 42: 15369 (2003). Because we isolated Nek1 in our RNAi screen, we wondered if it might have a primary role in cilia transport and/or structure. To address this question, we engineered murine IMCD3 cells, which have been shown to form cilia *in vitro,* to stably express small-hairpin RNAi (shRNA) against Nek1. In contrast to control cells in which about 40% of cell are ciliated, IMCD3 cells expressing Nek1 shRNA show a drastic reduction in cilia formationk, with only about 1% of cells being ciliated. Figures 7E and 8B. Thus, we propose that mammalian Nek1 impacts Hh signaling through its role in ciliogenesis.
To our knowledge this is the first RNAi screen that attempts to correlate phenotype with transcript knockdown of all the primary candidates isolated from an RNAi screen. Given the recent number of papers that raise concern over the high false-positive rates in RNAi screens [Echeverri et al., Nat. Methods 3: 777 (2006), Ma et al., Nature 443: 359 (2006)], we felt this validation step was necessary and likely eliminated most false-positives. Surprisingly, identification of 2 independent siRNAs, which has been suggested as a standard for RNAi screens, still produced a great number of false-positives. In our experience, identification of 3 or more independent siRNA produced a higher percentage of validated hits and should be used as the criteria for selecting hits for follow-up studies. However, we cannot conclude that a lack of correlation between phenotype and mRNA knockdown for a given gene hit should always be considered a false-positive. From the 9 hits validated in our screen, CDC2L1, Csnkla1 and Prkarla are conserved in flies. Prkarl a is thought to negatively regulate Pka, a known inhibitor of Hh signaling in flies and vertebrates. Chen et al., (1998) *supra.,* Price et al., (1999), supra; Wang et al., Genes Dev. 13: 2838 (1999); Huang et al., J. Biol. Chem 277: 19889 (2002). Epistasis experiments placed Prkarla downstream of Smo consistent with a role for Prkar1a and Pka in the phosphorylation and regulation of Gli proteins in the absence of Hh signaling. Our screen also identified Csnk1a1 (CK1$\alpha$) as having a positive role in Hh signaling. In flies, Csnk1a1 has also been previously shown to play both a negative regulatory role through phosphorylation of Ci (the fly homolog of Gli) in the absence of Hh [Price *et al.,* 2002, *supra;* Jia et al., Dev Cell. 9: 819 (2005); Lum et al., Science 299: 2039 (2003)] and a positive role in the presence of Hh signaling through phosphorylation of Smo. Jia et al., Nature 432: 1045 (2004); Zhang et al., Proc. Natl. Acad. Sci. USA 101: 17900 (2004); Apionishev et al., Nat. Cell Biol. 7: 86 (2005). Identification of Csnk1a1 in our siRNA screen suggests that Csnk1a1 may also play a positive role in mammalian Hh signaling. Since mammalian Smo lacks any putative Csnkla phosphorylation sites, Csnkla1 likely acts on other components necessary for Hh activation. In support of a positive role for Csnklal in mammalian Hh signaling, our epistasis experiments placed Csnk1a1 upstream of, or at the same level as Smo. Finally, CDC2L1 is the Drosophila ortholog of Pitslre, recently isolated in a genome-wide RNAi screen to identify Hh regulators in flies. Nybakken et al., Nat. Genet. 37: 1323 (2005). Because we isolated the mammalian homolog of Pitslre (Cdc2L1), this protein likely plays a conserved although unknown role in Hh signaling.
Our screen also isolated 6 potentially novel kinases in the Hh pathway including Gyk1, Nek1, Plk1, Prkra, Ttbk2 and Ttk. Interestingly, a number of them including Nek1 Plk1 and Ttk localize to the centrosome and are thought to regulate the cell cycle. Several models may explain how these specific centrosomal genes might be affecting Hh signaling. The first possibility is that siRNA against these centrosomal components may impact cell proliferation of viability and therefore indirectly affect Hh signaling. However, this is likely not the case since none of thr hits had a drastic effect on cell viability as judged in our ATP assays, nor did they have an effect on the SV40-Luciferase reporter. In addition, by FACs analysis, cells treated with Nek1 and TtK siRNA did not show a cell cycle defect and cells treated with Plk1 siRNA only showed a slight cell cycle defect (data not shown). Furthermore, these cell cycle regulators are thought to be required for mitosis but since our assays were done suing cells at 80% confluency, where most cells are in G1 arrest, gene which function only in mitosis would not have been isolated in our screen. In support of this, we did not isolate the vast number of cell cycle regulators present in our library.
Another possibility may be that these specific centrosomal genes are affecting a distinct function of the centrosome such as cilia formation. Indeed, such awas the case for Nek1. It is possible that Plk1 and Ttk also have a specific role in ciliogenesis. Interestingly, Plk1 has been linked to ciliogenesis through its interaction with Cenexin (Odf2) since Cenexin has been recently shown to be required for targeting Plk1 to centrosomes. Soung et al., Mol. Cell Biol. 26: 8316-8335 (2006). Furthermore, analysis of mice that lack Cenexin show that cenexin is indispensable for the generation of primary cilia, but not for other cell cycle related centriolar functions. Ishikawa et al., Nat. Cell Biol. 7: 517 (2005). Thus, Cenexin may exert its effects on cilia formation via interaction witih Plk1 and a specific role for Plk1 in cilia formation may exist that is distinct from its role in regulating mitosis.
A third possibility is that these kinases directly phosphorylate components of the Hh pathway. Indeed, sequence analysis of Smo, Sufu, and Gli-2, -2 and -3 identify multiple candidate phosphorylation sites with notable consequences for Plk1 suggesting at least this kinase may be directly phosphorylating Hh components.

Based on analysis of our validated hits, we have surmised that function identified below for the hedgehog kinased identified in our screen:

| Gene | Function |
|---|---|
| Cdc2l1 * | Cell cycle |
| Csnkla1 inventive | Signaling |
| Gyk inventive | Osmolarity |
| Nek1 * | Cilia |
| Plk1 * | Cell cycle |
| Prkarla * | Signaling |
| Prkra inventive | RNAi silencing |
| Ttbk2 * | Microtubule-binding |
| Ttk * | Cell cycle |
| * for comparison | |

EXAMPLE 2: Microarray Analysis to Detect Upregulation of Hedgehog Kinase Polypeptides in

Cancer or Tumors

Nucleic acid microarrays, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (disease tissue) sample is greater than hybridization signal of a probe from a control (normal tissue) sample, the gene or genes overexpressed in the disease tissue are identified. The implication of this result is that an overexpressed protein in a diseased tissue is useful not only as a diagnostic marker for the presence of the disease condition, but also as a therapeutic target for treatment of the disease condition.

The methodology of hybridization of nucleic acids and microarray technology is well known in the art. In the present example, the specific preparation of nucleic acids for hybridization and probes, slides, and hybridization conditions are all detailed in PCT Patent Application Serial No. PCT/US01/10482, filed on March 30, 2001.

EXAMPLE 3: Quantitative Analysis of Hedgehog Kinase mRNA Expression

In this assay, a 5' nuclease assay (for example, TaqMan®) and real-time quantitative PCR (for example, ABI Prizm 7700 Sequence Detection System® (Perkin Elmer, Applied Biosystems Division, Foster City, CA)), is used to find genes that are significantly overexpressed in a cancerous glioma tumor or tumors as compared to other cancerous tumors or normal noncancerous tissue. The 5' nuclease assay reaction is a fluorescent PCR-based technique which makes use of the 5' exonuclease activity of Taq DNA polymerase enzyme to monitor gene expression in real time. Two oligonucleotide primers (whose sequences are based upon the gene or EST sequence of interest) are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the PCR amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative and quantitative interpretation of the data. This assay is well known and routinely used in the art to quantitatively identify gene expression differences between two different human tissue samples, see, *e.g.*, Higuchi et al., Biotechnology 10:413-417 (1992); Livak et al., PCR Methods Appl., 4:357-362 (1995); Heid et al., Genome Res. 6:986-994 (1996); Pennica et al., Proc. Natl. Acad. Sci. USA 95(25): 14717-14722 (1998); Pitti et al., Nature 396(6712):699-703 (1998) and Bieche et al., Int. J. Cancer 78:661-666 (1998).

The 5' nuclease procedure is run on a real-time quantitative PCR device such as the ABI Prism 7700TM Sequence Detection. The system consists of a thermocycler, laser, charge-coupled device (CCD) camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

The starting material for the screen is mRNA isolated from a variety of different cancerous tissues. The mRNA is quantitated precisely, e.g., fluorometrically. As a negative control, RNA is isolated from various normal tissues of the same tissue type as the cancerous tissues being tested. Frequently, tumor sample('s) are directly compared to "matched" normal sample(s) of the same tissue type, meaning that the tumor and normal sample(s) are obtained from the same individual.

5' nuclease assay data are initially expressed as Ct, or the threshold cycle. This is defined as the cycle at which the reporter signal accumulates above the background level of fluorescence. The ΔCt values are used as quantitative measurement of the relative number of starting copies of a particular target sequence in a nucleic acid sample when comparing cancer mRNA results to normal human mRNA results. As one Ct unit corresponds to 1 PCR cycle or approximately a 2-fold relative increase relative to normal, two units corresponds to a 4-fold relative increase, 3 units corresponds to an 8-fold relative increase and so on, one can quantitatively and quantitatively measure the relative fold increase in mRNA expression between two or more different tissues. In this regard, it is well accepted in the art that this assay is sufficiently technically sensitive to reproducibly detect an at least 2-fold increase in mRNA expression in a human tumor sample relative to a normal control.

EXAMPLE 4: *In situ* Hybridization

*In situ* hybridization is a powerful and versatile technique for the detection and localization of nucleic acid sequences within cell or tissue preparations. It may be useful, for example, to identify sites of gene expression, analyze the tissue distribution of transcription, identify and localize viral infection, follow changes in specific mRNA synthesis and aid in chromosome mapping.

*In situ* hybridization is performed following an optimized version of the protocol by Lu and Gillett, Cell Vision 1:169-176 (1994), using PCR-generated $^{33}$P-labeled riboprobes. Briefly, formalin-fixed, paraffin-embedded human tissues are sectioned, deparaffinized, deproteinated in proteinase K (20 g/ml) for 15 minutes at 37°C, and further processed for *in situ* hybridization as described by Lu and Gillett, *supra*. A [$^{33}$-P] UTP-labeled antisense riboprobe are generated from a PCR product and hybridized at 55°C overnight. The slides are dipped in Kodak NTB2 nuclear track emulsion and exposed for 4 weeks.

$^{33}$P-Riboprobe synthesis

6.0 μl (125 mCi) of $^{33}$P-UTP (Amersham BF 1002, SA<2000 Ci/mmol) were speed vac dried. To each tube containing dried $^{33}$P-UTP, the following ingredients were added:

2.0 μl 5x transcription buffer
1.0 μl DTT (100 mM)
2.0 μl NTP mix (2.5 mM : 10 μ; each of 10 mM GTP, CTP & ATP + 10 μl $H_2O$)
1.0 μl UTP (50 μM)
1.0 μl Rnasin
1.0 μl DNA template (1 μg)
1.0 μl $H_2O$
1.0 μl RNA polymerase (for PCR products T3 = AS, T7 = S, usually)

The tubes are incubated at 37°C for one hour. 1.0 μl RQ1 DNase is added, followed by incubation at 37°C for 15 minutes. 90 μl TE (10 mM Tris pH 7.6/1 mM EDTA pH 8.0) are added, and the mixture was pipetted onto DE81 paper. The remaining solution is loaded in a Microcon-50 ultrafiltration unit, and spun using program 10 (6 minutes). The filtration unit is inverted over a second tube and spun using program 2 (3 minutes). After the final recovery spin, 100 μl TE is added. 1 μl of the final product is pipetted on DE81 paper and counted in 6 ml of Biofluor II.

The probe is run on a TBE/urea gel. 1-3 μl of the probe or 5 μl of RNA Mrk III is added to 3 μl of loading buffer. After heating on a 95°C heat block for three minutes, the probe is immediately placed on ice. The wells of gel are flushed, the sample loaded, and run at 180-250 volts for 45 minutes. The gel is wrapped in saran wrap and exposed to XAR film with an intensifying screen in -70°C freezer one hour to overnight.

$^{33}P$-Hybridization

A. Pretreatment of frozen sections

The slides are removed from the freezer, placed on aluminium trays and thawed at room temperature for 5 minutes. The trays are placed in 55°C incubator for five minutes to reduce condensation. The slides are fixed for 10 minutes in 4% paraformaldehyde on ice in the fume hood, and washed in 0.5 x SSC for 5 minutes, at room temperature (25 ml 20 x SSC + 975 ml SQ $H_2O$). After deproteination in 0.5 μg/ml proteinase K for 10 minutes at 37°C (12.5 μl of 10 mg/ml stock in 250 ml prewarmed RNase-free RNAse buffer), the sections are washed in 0.5 x SSC for 10 minutes at room temperature. The sections are dehydrated in 70%, 95%, 100% ethanol, 2 minutes each.

B. Pretreatment of paraffin-embedded sections

The slides are deparaffinized, placed in SQ $H_2O$, and rinsed twice in 2 x SSC at room temperature, for 5 minutes each time. The sections are deproteinated in 20 μg/ml proteinase K (500 μl of 10 mg/ml in 250 ml RNase-free RNase buffer; 37°C, 15 minutes) - human embryo, or 8 x proteinase K (100 μl in 250 ml Rnase buffer, 37°C, 30 minutes) - formalin tissues. Subsequent rinsing in 0.5 x SSC and dehydration are performed as described above.

C. Prehybridization

The slides are laid out in a plastic box lined with Box buffer (4 x SSC, 50% formamide) - saturated filter paper.

D. Hybridization

1.0 x $10^6$ cpm probe and 1.0 μl tRNA (50 mg/ml stock) per slide are heated at 95 °C for 3 minutes. The slides are cooled on ice, and 48 μl hybridization buffer are added per slide. After vortexing, 50 μl $^{33}P$ mix are added to 50 μl prehybridization on slide. The slides are incubated overnight at 55°C.

E. Washes

Washing is done 2 x 10 minutes with 2xSSC, EDTA at room temperature (400 ml 20 x SSC + 16 ml 0.25M EDTA, $V_f$=4L), followed by RNaseA treatment at 37°C for 30 minutes (500 μl of 10 mg/ml in 250 ml Rnase buffer = 20 μg/ml). The slides are washed 2 x 10 minutes with 2 x SSC, EDTA at room temperature. The stringency wash conditions can be as follows: 2 hours at 55°C, 0.1 x SSC, EDTA (20 ml 20 x SSC + 16 ml EDTA, $V_f$=4L).

F. Oligonucleotides

*In situ* analysis is perfonned on a variety of DNA sequences disclosed herein. The oligonucleotides employed for these analyses is obtained so as to be complementary to the nucleic acids (or the complements thereof) as shown in the accompanying figures.

EXAMPLE 5: Preparation of Antibodies that Bind Hedgehog Kinase

Techniques for producing monoclonal antibodies are known in the art and are described, for instance, in Coding, supra. Immunogens that may be employed include purified hedgehog kinase polypeptides, fusion proteins containing hedgehog kinase polypeptides and cell expressing recombinant hedgehog kinase polypeptides on the cell surface. Selection of the immunogen can be made by the skilled artisan without undue experimentation.

Mice, such as Balb/c, are immunized with the above immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-hedgehog kinase antibodies.

After a suitable antibody titer has been detected, the animals "positive" for antibodies can be injected with a final intravenous injection of hedgehog kinase polypeptide. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells are screened in an ELISA for reactivity against hedgehog kinase. Determination of "positive" hybridoma cells secreting the desired monoclonal antibodies against hedgehog kinase is within the skill in the art.

The positive hybridoma cells can be injected intraperitoneally into syngeneic Balb/c mice to produce ascites containing the anti-hedgehog kinase monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

EXAMPLE 6: Preparation of Toxin-Coniugated Antibodies that Bind hedgehog kinase

The use of antibody-drug conjugates (ADC), *i.e*. immunoconjugates, for the local delivery of cytotoxic or cytostatic agents, *i.e.* drugs to kill or inhibit tumor cells in the treatment of cancer (Payne (2003) Cancer Cell 3:207-212; Syrigos and Epenetos (1999) Anticancer Research 19:605-614; Niculescu-Duvaz and Springer (1997) Adv. Drug Del. Rev. 26:151-172; US 4,975,278) allows targeted delivery of the drug moiety to tumors, and intracellular accumulation therein, where systemic administration of these unconjugated drug agents may result in unacceptable levels of toxicity to normal cells as well as the tumor cells sought to be eliminated (Baldwin et al., (1986) Lancet (Mar. 15, 1986) pp. 603-05; Thorpe, (1985) "Antibody Carriers Of Cytotoxic Agents In Cancer Therapy: A Review," in Monoclonal Antibodies '84: Biological And Clinical Applications, Pinchera et al. (eds.), pp. 475-506). Maximal efficacy with minimal toxicity is sought thereby. Efforts to design and refine ADC have focused on the selectivity of monoclonal antibodies (mAbs) as well as drug-linking and drug-releasing properties. Both polyclonal antibodies and monoclonal antibodies have been reported as useful in these strategies (Rowland et al., (1986) Cancer Immunol. Immunother., 21:183-87). Drugs used in these methods include daunomycin, doxorubicin, methotrexate, and vindesine (Rowland *et al.*, (1986) *supra*). Toxins used in antibody-toxin conjugates include bacterial toxins such as diphtheria toxin, plant toxins such as ricin, small molecule toxins such as geldanamycin (Mandler et al. (2000) J. of the Nat. Cancer Inst. 92(19):1573-1581; Mandler et al. (2000) Bioorganic & Med. Chem. Letters 10:1025-1028; Mandler et al. (2002) Bioconjugate Chem. 13:786-791), maytansinoids (EP 1391213; Liu et al., (1996) Proc. Natl. Acad. Sci. USA 93:8618-8623), and calicheamicin (Lode et al. (1998) Cancer Res. 58:2928; Hinman et al. (1993) Cancer Res. 53:3336-3342).

Techniques for producing antibody-drug conjugates by linking toxins to purified antibodies are well known and routinely employed in the art. For example, conjugation of a purified monoclonal antibody to the toxin DM1 may be accomplished as follows. Purified antibody is derivatized with N-succinimidyl-4-(2-pyridylthio)-pentanoate to introduce dithiopyridyl groups. Antibody (376.0 mg, 8 mg/mL) in 44.7 ml of 50 mM potassium phosphate buffer (pH 6.5) containing NaCl (50 mM) and EDTA (1 mM) is treated with SPP (5.3 molar equivalents in 2.3 ml ethanol). After incubation for 90 minutes under argon at ambient temperature, the reaction mixture is gel filtered through a Sephadex G25 column equilibrated with 35 mM sodium citrate, 154 mM NaCl and 2 mM EDTA. Antibody containing fractions are then pooled and assayed. Antibody-SPP-Py (337.0 mg with releasable 2-thiopyridine groups) is diluted with the above 35 mM sodium citrate buffer, pH 6.5, to a final concentration of 2.5 mg/ml. DM1 (1.7 equivalents, 16.1 mols) in 3.0 mM dimethylacetamide (DMA, 3% v/v in the final reaction mixture) is then added to the antibody solution. The reaction is allowed to proceed at ambient temperature under argon for 20 hours. The reaction is loaded on a Sephacryl S300 gel filtration column (5.0 cm x 90.0 cm, 1.77 L) equilibrated with 35 mM sodium citrate, 154 mM NaCl, pH 6.5. The flow rate is 5.0 ml/min and 65 fractions (20.0 ml each) are collected. Fractions are pooled and assayed, wherein the number of DM1 drug molecules linked per antibody molecule (p') is determined by measuring the absorbance at 252 nm and 280 nm.

For illustrative purposes, conjugation of a purified monoclonal antibody to the toxin DM1 I may also be accomplished as follows. Purified antibody is derivatized with (Succinimidyl 4-(N-manaleimidomethyl) cyclohexane-1-carboxylate (SMCC, Pierce Biotechnology, Inc) to introduce the SMCC linker. The antibody is treated at 20 mg/ml in 50mM potassium phosphate/ 50 mM sodium chloride/ 2 mM EDTA, pH 6.5 with 7.5 molar equivalents of SMCC (20 mM in DMSO, 6.7 mg/ml). After stirring for 2 hours under argon at ambient temperature, the reaction mixture is filtered through a Sephadex G25 column equilibrated with 50mM potassium phosphate/ 50 mM sodium chloride/ 2 mM EDTA, pH 6.5. Antibody containing fractions are pooled and assayed. Antibody-SMCC is then diluted with 50mM potassium phosphate/ 50 mM sodium chloride/ 2 mM EDTA, pH 6.5, to a final concentration of 10 mg/ml, and reacted with a 10 mM solution of DM1 (1.7 equivalents assuming 5 SMCC/antibody, 7.37 mg/ml) in dimethylacetamide. The reaction is stirred at ambient temperature under argon 16.5 hours. The conjugation reaction mixture is then filtered through a Sephadex G25 gel filtration column (1.5 x 4.9 cm) with 1 x PBS at pH 6.5. The DM1/antibody ratio (p) is then measured by the absorbance at 252 nm and at 280 nm.

Cytotoxic drugs have typically been conjugated to antibodies through the often numerous lysine residues of the antibody. Conjugation through thiol groups present, or engineered into, the antibody of interest has also been accomplished. For example, cysteine residues have been introduced into proteins by genetic engineering techniques to form covalent attachment sites for ligands (Better et al. (1994) J. Biol. Chem. 13:9644-9650; Bernhard et al. (1994) Bioconjugate Chem. 5:126-132; Greenwood et al. (1994) Therapeutic Immunology 1:247-255; Tu et al. (1999) Proc. Natl. Acad. Sci USA 96:4862-4867; Kanno et al. (2000) J. of Biotechnology. 76:207-214; Chmura et al. (2001) Proc. Nat. Acad. Sci. USA 98(15):8480-8484; U.S. Patent No. 6,248,564). Once a free cysteine residue exists in the antibody of interest, toxins can be linked to that site. As an example, the drug linker reagents, maleimidocaproyl-monomethyl auristatin E (MMAE), *i.e.* MC-MMAE, maleimidocaproyl-monomethyl auristatin F (MMAF), *i.e.* MC-MMAF, MC-val-cit-PAB-MMAE or MC-val-cit-PAB-MMAF, dissolved in DMSO, is diluted in acetonitrile and water at known concentration, and added to chilled cysteine-derivatized antibody in phosphate buffered saline (PBS). After about one hour, an excess of maleimide is added to quench the reaction and cap any unreacted antibody thiol groups. The reaction mixture is concentrated by centrifugal ultrafiltration and the toxin conjugated antibody is purified and desalted by elution through G25 resin in PBS, filtered through 0.2m filters under sterile conditions, and frozen for storage.

Moreover, a free cysteine on an antibody of choice may be modified by the bis-naleimido reagent BM(PEO)4 (Pierce Chemical), leaving an unreacted maleimido group on the surface of the antibody. This may be accomplished by dissolving BM(PEO)4 in a 50% ethanol/water mixture to a concentration of 10 mM and adding a tenfold molar excess to a solution containing the antibody in phosphate buffered saline at a concentration of approximately 1.6 mg/ml (10 micromolar) and allowing it to react for 1 hour. Excess BM(PEO)4 is removed by gel filtration in 30 mM citrate, pH 6 with 150 mM NaCl buffer. An approximate 1 fold molar excess DM1 is dissolved in dimethyl acetamide (DMA) and added to the antibody-BMPEO intermediate. Dimethyl formamide (DMF) may also be employed to dissolve the drug moiety reagent. The reaction mixture is allowed to react overnight before gel filtration or dialysis into PBS to remove unreacted drug. Gel filtration on S200 columns in PBS is used to remove high molecular weight aggregates and furnish purified antibody-BMPEO-DM1 conjugate.

EXAMPLE 7 - *In Vitro* Cell Killing Assays

Mammalian cells expressing an active hedgehog signaling pathway may be obtained using standard expression vector and cloning techniques. Alternatively, many tumor cell lines actively expressing a hedgehog signaling pathway are publicly available, for example, through the ATCC and can be routinely identified using standard ELISA or FACS analysis. Anti-hedgehog pathway polypeptide monoclonal antibodies (and toxin conjugated derivatives thereof) may then be employed in assays to determine the ability of the antibody to kill *in vitro* cell expressing an active hedgehog signaling pathway.

With specific regard to the present invention, a PC3-derived cell line that stably expresses hedgehog kinase polypeptide on its cells surface (herein called PC3-gD-MDP) may be engineered using standard techniques and expression of the hedgehog kinase polypeptide by the PC3-gD-MDP cells can be confirmed using standard FACS cell sorting, ELISA and immunohistochemistry analyses. The ability of an MMAE-conjugated anti-hedgehog kinase monoclonal antibody to cause the death of the respective hedgehog kinase-expressing cells may be determined using an *in vitro* cell killing assay employing the following protocol (Promega Corp. Technical Bulletin TB288; Mendoza et al (2002) Cancer Res. 62:5485-5488):

1. An aliquot of 50 μl of cell culture containing about $10^4$ cells (either PC3-gD-MDP cells or untransfected PC3 cells which do not express hedgehog kinase) in growth medium is deposited in each well of a 96-well, opaque-walled plate. Additional control wells are set up which contain 50 μl of growth medium without cells.
2. The hedgehog kinase-MMAE conjugated antibody, or an MMAE-conjugated control monoclonal antibody that does not bind to hedgehog kinase, is added to each well in a volume of 50 μl and at various concentrations ranging from 0.0001 to 100 μg/ml and the plates are incubated at 37"C and 5% $CO_2$ for 3-5 days.
3. The plates are equilibrated to room temperature for approximately 30 minutes.
4. A volume of the CellTiter-Glo Luminescent Cell Viability Reagent from Promega Corp. equal to the volume of cell culture medium present in each well is added and the plates are shaken for 2 minutes on an orbital shaker to induce cell lysis.
5. The plates are incubated at room temperature for 10 minutes to stabilize the luminescence signal.
6. Luminescence is recorded on a luminometer with the Tropix Winglow Program and reported as RLU = relative luminescence units.

The results obtained from the above described assay can demonstrate that the hedgehog kinase-MMAE antibody is capable of inducing the death of cells that express the corresponding hedgehog kinase polypeptide in an antibody-dependent fashion. That is, neither hedgehog kinase-MMAE nor MMAE-conjugated control can induce significant death of untransfected PC3 cells at an antibody concentration of 1 μg/ml and below. At antibody concentrations above I μg/ml, the amount of untransfected PC3 cell death may increase linearly with antibody concentration in an antibody-independent manner. Therefore, it will appear that the death of untransfected PC3 cells at antibody concen-

trations above 1 μg/ml is a non-specific result of the increasing levels of the MMAE toxin present in the reaction mixture and is not a function of the binding specificity of the antibody employed.

With regard to the PC3-gD-MDP cells that stably express the hedgehog kinase polypeptide, however, while the MMAE-conjugated control induces cell death with a pattern that is identical to that antibody's ability to kill untransfected PC3 cells, the hedgehog kinase-MMAE will induce significant cell killing at antibody concentrations significantly below this level (*e.g.*, as low as 0.001 μg/ml). In fact, at an antibody concentration of 1 μg/ml (where the non-hedgehog kinase specific MMAE-conjugated control antibody exhibits no significant cell killing), virtually all of the PC3-gD-MDP cells will be killed by hedgehog kinase-MMAE. As such, such data will demonstrate that hedgehog kinase-specific monoclonal antibody binds to the hedgehog kinase polypeptide as it is expressed on the surface of cells and is capable of inducing the death of those cells to which it binds.

EXAMPLE 8: *In Vivo* Tumor Cell Killing Assay

To test the efficacy of toxin-conjugated or unconjugated anti-hedgehog kinase polypeptide monoclonal antibodies for the ability to induce the death of tumor cells *in vivo,* the following protocol may be employed.

Inoculate a group of athymic nude-mice with 5 x $10^6$ of the hedgehog kinase polypeptide-expressing tumor promoting cells subcutaneously in the flank. When the tumors reach a mean tumor volume of between 100-200 $mm^3$, the mice are grouped equally into 5 groups and are treated as follows:

Group I - PBS control vehicle administered once per week for 4 weeks;
Group 2 - non-specific control antibody administered at 1 mg/kg, once per week for 4 weeks;
Group 3 - non-specific control antibody administered at 3 mg/kg, once per week for 4 weeks;
Group 4 - specific anti-hedgehog kinase polypeptide antibody administered at 1 mg/kg, once per week for 4 weeks;
Group 5 - specific anti-hedgehog kinase polypeptide antibody administered at 3 mg/kg, once per week for 4 weeks.

Mean tumor volume may then be determined in the mice of each treatment group at periodic intervals and the efficacy of the antibodies determined.

EXAMPLE 9: Use of hedgehog kinase as a hybridization probe

The following method describes use of a nucleotide sequence encoding hedgehog kinase polypeptide as a hybridization probe for, *i.e.,* diagnosis of the presence of a tumor in a mammal.

DNA comprising the coding sequence of full-length or mature hedgehog kinase polypeptide as disclosed herein can also be employed as a probe to screen for homologous DNAs (such as those encoding naturally-occurring variants of hedgehog kinase) in human tissue cDNA libraries or human tissue genomic libraries.

Hybridization and washing of filters containing either library DNAs is performed under the following high stringency conditions. Hybridization of radiolabeled hedgehog kinase-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1 x SSC and 0.1 % SDS at 42°C.

DNAs having a desired sequence identity with the DNA encoding full-length native sequence hedgehog kinase polypeptide can then be identified using standard techniques known in the art.

EXAMPLE 10: Expression of hedgehog kinase in *E. coli*

This example illustrates preparation of an unglycosylated form of hedgehog kinase by recombinant expression in *E. coli.*

The DNA sequence encoding the preceding hedgehog kinase polypeptide sequences is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli;* see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the hedgehog kinase coding region, lambda transcriptional terminator, and an argU gene.

The ligation mixture is then used to transform a selected *E. coli* strain using the methods described in Sambrook *et al.,* supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized hedgehog kinase polypeptide can then be purified using a metal chelating column under conditions that allow tight binding of the protein.

The preceding hedgehog kinase polypeptide sequences may be expressed in *E. coli* in a poly-His tagged form, using the following procedure. The DNA encoding hedgehog kinase is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an *E. coli* host based on strain 52 (W3110 fuhA (tonA) lon galE rpoHts(htpRts) clpP(lacIq). Transfonnants are first grown in LB containing 50 mg/ml carbenicillin at 30°C with shaking until an O.D.600 of 3-5 is reached. Cultures are then diluted 50-100 fold into CRAP media (prepared by mixing 3.57 g $(NH_4)_2SO_4$, 0.71 g sodium citrate•2H2O, 1.07 g KCl, 5.36 g Difco yeast extract, 5.36 g Sheffield hycase SF in 500 mL water, as well as 110 mM MPOS, pH 7.3, 0.55% (w/v) glucose and 7 mM $MgSO_4$) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to I L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1 M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and I mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1% TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded fonns of proteins from the desired form, the reversed phase step also removes endotoxin from the samples.

Fractions containing the desired folded protein are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Phannacia) resins equilibrated in the formulation buffer and sterile filtered.

EXAMPLE 11: Expression of hedgehog kinase polypeptide in mammalian cells

This example illustrates preparation of a potentially glycosylated form of hedgehog kinase polypeptide by recombinant expression in mammalian cells.

The vector, pRK5 (see EP 307,247, published March 15, 1989), is employed as the expression vector. Optionally, DNA encoding the hedgehog kinase polypeptides described herein is ligated into pRK5 with selected restriction enzymes to allow insertion of such DNA using ligation methods such as described in Sambrook *et al.,* supra. The resulting vector is called HK-DNA.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 μg pRK5-HK DNA is mixed with about 1 μg DNA encoding the VA RNA gene [Thimmappaya et al., Cell, 31:543 (1982)] and dissolved in 500 μl of 1 mM Tris-HCl, 0.1 mM EDTA, 0.227

M $CaCl_2$. To this mixture is added, dropwise, 500 μl of 50 mM HEPES (pH 7.35), 280 mM NaCl, 1.5 mM $NaPO_4$, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells arc then washed with serum free medium, fresh medium is added and the cells are incubated for about 5 days.

Approximately 24 hours after the transfections, the culture medium is removed and replaced with culture medium (alone) or culture medium containing 200 μCi/ml $^{35}$S-cysteine and 200 μCi/ml $^{35}$S-methionine. After a 12 hour incubation, the conditioned medium is collected, concentrated on a spin filter, and loaded onto a 15% SDS gel. The processed gel may be dried and exposed to film for a selected period of time to reveal the presence of the hedgehog kinase polypeptides. The cultures containing transfected cells may undergo further incubation (in serum free medium) and the medium is tested in selected bioassays.

In an alternative technique, DNA encoding the hedgehog kinase polyeptides may be introduced into 293 cells transiently using the dextran sulfate method described by Somparyrac et al., Proc. Natl. Acad. Sci., 12:7575 (1981). 293 cells are grown to maximal density in a spinner flask and 700 μg pRK5-HK DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 μg/ml bovine insulin and 0.1 μg/ml bovine transferrin. After about four days, the conditioned media is centrifuged and filtered to remove cells and debris. The sample containing expressed hedgehog kinase can then be concentrated and purified by any selected method, such as dialysis and/or column chromatography.

In another embodiment, the hedgehog kinase polypeptide can be expressed in CHO cells. The pRK5-HK can be transfected into CHO cells using known reagents such as $CaPO_4$ or DEAE-dextran. As described above, the cell cultures can be incubated, and the medium replaced with culture medium (alone) or medium containing a radiolabel such as $^{35}$S-methionine. After determining the presence of the hedgehog kinase, the culture medium may be replaced with serum free medium. Preferably, the cultures are incubated for about 6 days, and then the conditioned medium is harvested. The medium containing the expressed hedgehog kinase polypeptide can then be concentrated and purified by any selected method.

Epitope-tagged hedgehog kinase polypeptide may also be expressed in host CHO cells. The sequence encoding the hedgehog kinase portion may be subcloned out of the pRK5 vector. The subclone insert can undergo PCR to fuse in frame with a selected epitope tag such as a poly-his tag into a Baculovirus expression vector. This poly-his tagged hedgehog kinase insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged hedgehog kinase can then be concentrated and purified by any selected method, such as by $Ni^{2+}$-chelate affinity chromatography.

Hedgehog kinase polypeptide may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (*e.g.* extracellular domains) of the respective proteins are fused to an IgGl constant region sequence containing the hinge, CH2 and CH2 domains and/or is a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents SUPERFECT® (Quiagen), DOSPER® or FUGENE® (Boehringer. Mannheim). The cells are grown as described in Lucas *et al.*, supra. Approximately 3 x $10^7$ cells are frozen in an ampule for further growth and production as described below.

The ampules containing the plasmid DNA are thawed by placement into water bath and mixed by vortexing. The contents are pipetted into a centrifuge tube containing 10 mLs of media and centrifuged at 1000 rpm for 5 minutes. The supernatant is aspirated and the cells are resuspended in 10 mL of selective media (0.2 μm filtered PS20 with 5% 0.2 μm diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with 3 x $10^5$ cells/mL.

The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x $10^6$ cells/mL. On day 0, the cell number pH ie determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35% polydimethylsiloxane emulsion, Dow Corning 365 Medical Grade Emulsion) taken. Throughout the production, the pH is adjusted as necessary to keep it at around 7.2. After 10 days, or until the viability dropped below 70%, the cell culture is harvested by centrifugation and filtering through a 0.22 μm filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 μL of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

EXAMPLE 12: Expression of Hedgehog Kinase in Yeast

The following method describes recombinant expression of hedgehog kinase polypeptide in yeast.

First, yeast expression vectors are constructed for intracellular production or secretion of the preceding hedgehog kinase sequences from the ADH2/GAPDH promoter. DNA encoding such hedgehog kinase sequences and the promoter is inserted into suitable restriction enzyme sites in the selected plasmid to direct intracellular expression of hedgehog kinase. For secretion, DNA encoding such hedgehog kinase sequences can be cloned into the selected plasmid, together with DNA encoding the ADH2/GAPDH promoter, a native hedgehog kinase signal peptide or other mammalian signal peptide, or, for example, a yeast alpha-factor or invertase secretory signal/leader sequence, and linker sequences (if needed) for expression of hedgehog kinase.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant hedgehog kinase can subsequently be isolated and purified by removing the yeast cells from the fermentation medium by centrifugation and then concentrating the medium using selected cartridge filters. The concentrate containing hedgehog kinase may further be purified using selected column chromatography resins.

EXAMPLE 13: Expression of Hedgehog Kinase in Baculovirus-Infected Insect Cells

The following method describes recombinant expression of hedgehog kinase polypeptide in Baculovirus-infected insect cells.

The sequence coding for the preceding hedgehog kinase sequence is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding the preceding hedgehog kinase sequence or the desired portion of the coding sequence of such, *e.g.* the sequence encoding an extracellular domain of a transmembrane protein or the sequence encoding the mature protein if the protein is extracellular, is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BACULOGOLD™ virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged hedgehog kinase polypeptide can then be purified, for example, by $Ni^{2+}$-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 mL Hepes, pH 7.9; 12.5 mM $MaCl_2$; 0.1 mM EDTA; 10% glycerol; 0.1% NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 $\mu$m filter. A $Ni^{2+}$-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline $A_{280}$ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching $A_{280}$ baseline again, the column is developed with a 0 to 500 mM Imidazole gradient in the secondary wash buffer. One mL fractions are collected and analyzed by SDS-PAGE and silver staining or Western blot with $Ni^{2+}$-NTA-conjugated to alkaline phosphatase, (Qiagen). Fractions containing the eluted $His_{10}$-tagged hedgehog kinase polypeptide are pooled and dialyzed against loading buffer.

Alternatively, purification of the IgG tagged (or Fc tagged) hedgehog kinase polypeptide can be performed using known chromatography techniques, including for instance, Protein A or protein G column chromatography.

EXAMPLE 14: Purification of Hedgehog Kinase polypeptide Using Specific Antibodies

Native or recombinant Hedgehog kinase polypeptides may be purified by a variety of standard techniques in the art of protein purification. For example, pro-, mature or pre-polypeptide variants of the preceding hedgehog kinase sequences are purified by immunoaffinity chromatography using antibodies specific for such sequences. In general, an immunoaffinity column is constructed by covalently coupling the anti-hedgehog kinase antibody to an activated chromatographic resin.

Polyclonal immunoglobulins are prepared from immune sera either by precipitation with ammonium sulfate or by purification on immobilized Protein A (Phannacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE™ (Phannacia LKB Biotechnology). The antibody is coupled to the resin, the resin is blocked, and the derivative resin is washed according to the manufacturer's instructions.

Such an immunoaffinity column is utilized in the purification of the preceding hedgehog kinase sequences by preparing a fraction from cells containing such sequences in a soluble form. This preparation is derived by solubilization of the whole cell or of a subcellular fraction obtained via differential centrifugation by the addition of detergent or by other methods well known in the art. Alternatively, soluble hedgehog kinase polypeptide containing a signal sequence may be secreted in useful quantity into the medium in which the cells are grown.

A soluble hedgehog kinase polypeptide-containing preparation is passed over the immunoaffinity column, and the column is washed under conditions that allow the preferential absorbance of such sequences (*e.g.*, high ionic strength buffers in the presence of detergent). Then, the column is eluted under conditions that disrupt the binding between the antibody/substrate (*e.g.*, a low pH buffer such as approximately pH 2-3, or a high concentration of a chaotrope such as urea or thiocyanate ion), and hedgehog kinase polypeptide, respectively, is collected.

## Claims

1. The use of a CSNK1A1, GYK or PRKRA hedgehog kinase antagonist for the manufacture of a medicament for treating or preventing a cell proliferative disorder, a tumour in a mammal and/or cancer, wherein the CSNK1A1, GYK or PRKRA hedgehog kinase antagonist is an RNAi molecule.

2. The use of claim 1, wherein the hedgehog kinase antagonist is GYK hedgehog kinase antagonist.

3. The use of claim 2, wherein the hedgehog kinase antagonist is PRKRA hedgehog kinase antagonist.

4. The use of Claim 1, wherein the CSNK1A1, GYK or PRKRA polypeptide which the CSNK1A1, GYK or PRKRA antagonist, respectively, inhibits comprises the sequence of Figure 1B, 1C or 1G, respectively.

5. The use of Claim 1, wherein the CSNK1A1, GYK or PRKRA hedgehog kinase antagonist is conjugated to a growth inhibitory agent or cytotoxic agent.

6. An *in vitro* method for diagnosing the presence of a cell proliferative disorder comprising comparing the level of expression of a gene encoding a CSNK1A1, GYK or PRKRA hedgehog kinase polypeptide: (a) in a test sample of tissue or cells obtained from said mammal, and (b) in a control sample of known normal noncancerous tissue or cells of the same tissue origin or type; wherein a higher expression level of the CSNK1A1, GYK or PRKRA polypeptide in the test sample, as compared to the control sample, is indicative of the presence of a cell proliferative disorder in the mammal from which the test sample was obtained.

7. An *in vitro* method of modulating the proliferation, differentiation or survival of uncommitted stem cells in culture comprising contacting such cells with an effective amount of a CSNK1A1, GYK, PRKRA hedgehog kinase antagonist.

8. The use of claim 5, wherein the growth inhibitory agent or cytotoxic agent is selected from the group consisting of: a maytansinoid, a calicheamicin, an antibiotic, a radioactive isotope, nucleolytic enzyme.

## Patentansprüche

1. Verwendung eines CSNK1A1-, GYK- oder PRKRA-Hedgehog-Kinase-Antagonisten für die Herstellung eines Medikaments zur Behandlung oder Prävention einer zellproliferativen Erkrankung, eines Tumors in einem Säugetier und/oder Krebs, wobei der CSNK1A1-, GYK- oder PRKRA-Hedgehog-Kinase-Antagonist ein RNAi-Molekül ist.

2. Verwendung nach Anspruch 1, wobei der Hedgehog-Kinase-Antagonist der GYK-Hedgehog-Kinase-Antagonist ist.

3. Verwendung nach Anspruch 2, wobei der Hedgehog-Kinase-Antagonist der PRKRA-Hedgehog-Kinase-Antagonist ist.

4. Verwendung nach Anspruch 1, wobei das CSNK1A1-, GYK- oder PRKRA-Polypeptid, das den CSNK1A1-, GYK-, beziehungsweise PRKRA-Antagonisten inhibiert, die Sequenz aus Figur 1B, 1C, beziehungswiese 1G umfasst.

5. Verwendung nach Anspruch 1, wobei der CSNK1A1-, GYK- oder PRKRA-Hedgehog-Kinase-Antagonist mit einem wachstumsinhibitorischen Mittel oder zytotoxischen Mittel konjugiert ist.

6. *In vitro*-Verfahren für die Diagnose des Vorliegens einer zellproliferativen Erkrankung, umfassend den Vergleich des Expressionslevels eines Gens, das ein CSNK1A1-, GYK- oder PRKRA-Hedgehog-Kinase-Polypeptid kodiert: (a) in einer zu untersuchenden Probe von Gewebe oder Zellen, die von dem Säugetier erhalten wurde, und (b) in einer Kontrollprobe von einem bekannten normalen nicht kanzerösen Gewebe oder Zellen des gleichen Gewebeursprungs oder -typs; wobei ein höherer Expressionslevel des CSNK1A1-, GYK- oder PRKRA-Polypeptids in der zu untersuchenden Probe, verglichen mit der Kontrollprobe, das Vorliegen einer zellpfoliferativen Erkrankung in dem Säugetier, aus dem die zu untersuchende Probe erhalten wurde, anzeigt.

7. *In vitro*-Verfahren für die Modulation der Proliferation, Differenzierung oder des Überlebens von nicht festgelegten Stammzellen in Kultur, umfassend das Inkontaktbringen solcher Zellen mit einer wirksamen Menge eines CSNK1A1-, GYK- oder PRKRA-Hedgehog-Kinase-Antagonisten.

8. Verwendung nach Anspruch 5, wobei das wachstumsinhibitorische Mittel oder das zytotoxische Mittel aus der Gruppe bestehend aus:

   einem Maytansinoid, einem Calicheamicin, einem Antibiotikum, einem radioaktiven Isotop, nukleolytischen Enzym ausgewählt ist.

## Revendications

1. Utilisation d'un antagoniste de la kinase Hedgehog CSNK1A1, GYK ou PRKRA pour la fabrication d'un médicament destiné au traitement ou à la prévention d'un trouble de prolifération cellulaire, d'une tumeur chez un mammifère et/ou d'un cancer, où l'antagoniste de la kinase Hedgehog CSNK1A1, GYK ou PRKRA est une molécule d'ARNi.

2. Utilisation selon la revendication 1, où l'antagoniste de la kinase Hedgehog est un antagoniste de la kinase Hedgehog GYK.

**3.** Utilisation selon la revendication 2, où l'antagoniste de la kinase Hedgehog est un antagoniste de la kinase Hedgehog PRKRA.

**4.** Utilisation selon la revendication 1, où le polypeptide de la CSNK1A1, GYK ou PRKRA qui est inhibé par l'antagoniste de la CSNK1A1, GYK ou PRKRA respectivement, comprend la séquence de la figure 1B, 1C ou 1G, respectivement.

**5.** Utilisation selon la revendication 1, où l'antagoniste de la kinase Hedgehog CSNK1A1, GYK ou PRKRA est conjugué à un agent inhibiteur de croissance ou un agent cytotoxique.

**6.** Procédé *in vitro* pour diagnostiquer la présence d'un trouble de prolifération cellulaire comprenant la comparaison du taux d'expression d'un gène codant pour un polypeptide de la kinase Hedgehog CSNK1A1, GYK ou PRKRA : (a) dans un échantillon de tissu ou de cellules à tester obtenu chez ledit mammifère, et (b) dans un échantillon contrôle de tissu ou de cellules non cancéreux normal de la même origine ou du même type tissulaire ; dans lequel un taux d'expression supérieur du polypeptide de CSNK1A1, GYK ou PRKRA dans l'échantillon à tester, comparativement à l'échantillon contrôle, indique la présence d'un trouble de prolifération cellulaire chez le mammifère à partir duquel l'échantillon à tester a été obtenu.

**7.** Procédé *in vitro* de modulation de la prolifération, de la différenciation ou de la survie des cellules souches non engagées en culture comprenant la mise en contact de telles cellules avec une quantité efficace d'un antagoniste de la kinase Hedgehog CSNK1A1, GYK ou PRKRA.

**8.** Utilisation selon la revendication 5, où l'agent inhibiteur de croissance ou l'agent cytotoxique est choisi dans le groupe constitué : d'un maytansinoïde, d'une calichéamicine, d'un antibiotique, d'un isotope radioactif, d'une enzyme nucléolytique.

**Mouse CDC2L1 Protein:**

MGDEKDSWKVKTLDEILQEKKRRKEQEEKAEIKRLKNSDDRDSKRDSLEEGELRDHRMEITIRN
SPYRREDSMEDRGEEDDSLAIKPPQQMSRKEKAHHRKDEKRKEKRRHRSHSAEGGKHARVKEKE
REHERRKRHREEQDKARREWERQKRREMAREHSRRERDRLEQLERKRERERKLREQQKEQREQK
ERERRAEERRKEREARREVSAHHRTMREEYSDKGKVGHWSRSPLRPPRERFEMGDNRKPVKEEK
VEERDLLSDLQDISDSERKTSSAESSSAESGSGSEEEEEEEEEEEEEEGSTSEESEEEEEEEEE
EEEEETGSNSEEASEQSAEEVSDEEMSEDEDRENENHILVVPESRFDRDSGDSEEGEEEVGEGT
PQSSAPTEGDYVPDSPALSPIELKQELPKYLPALQGCRSVEEFQCLNRIEEGTYGVVYRAKDKK
TDEIVALKRLKMEKEKEGFPITSLREINTILKAQHPNIVTVREIVVGSNMDKIYIVMNYVEHDL
KSLMETMKQPFLPGEVKTLMIQLLSGVKHLHDNWILHRDLKTSNLLLSHAGILKVGDFGLAREY
GSPLKAYTPVVVTLWYRAPELLLGAKEYSTAVDMWSVGCIFGELLTQKPLFPGKSDIDQINKIF
KDLGTPSEKIWPGYNDLPAVKKMTFSEYPYNNLRKRFGALLSDQGFDLMNKFLTYYPGRRINAE
DGLKHEYFRETPLPIDPSMFPTWPAKSEQQRVKRGTSPRPPEGGLGYSQLGDDDLKETGFHLTT
TNQGASAAGPGFSLKF

*FIG. 1A*

**Mouse CSNK1a1 Protein:**

MASSSGSKAEFIVGGKYKLVRKIGSGSFGDIYLAINITNGEEVAVKLESQKARHPQLLYESKLY
KILQGGVGIPHIRWYGQEKDYNVLVMDLLGPSLEDLFNFCSRRFTMKTVLMLADQMISRIEYVH
TKNFIHRDIKPDNFLMGIGRHCNKLFLIDFGLAKKYRDNRTRQHIPYREDKNLTGTARYASINA
HLGIEQSRRDDMESLGYVLMYFNRTSLPWQGLKAATKKQKYEKISEKKMSTPVEVLCKGFPAEF
AMYLNYCRGLRFEEAPDYMYLRQLFRILFRTLNHQYDYTFDWTMLKQKAAQQAASSSGQGQQAQTPTGF

*FIG. 1B*

**Mouse GYK Protein:**

MAAAKKAVLGPLVGAVDQGTSSTRFLVFNSKTAELLSHHQVEIKQEFPREGWVEQDPKEILQSV
YECIEKTCEKLGQLNIDISNIKAIGVSNQRETTVVWDKVTGEPLYNAVVWLDLRTQSTVENLSK
RIPGNNNFVKSKTGLPLSTYFSAVKLRWLLDNVKKVQEAVEENRALFGTIDSWLIWSLTGGIHG
GVHCTDVTNASRTMLFNIHSLEWDKELCEFFGIPMEILPNVRSSSEIYGLMKAGALEGVPISGC
LGDQSAALVGQMCFQDGQAKNTYGTGCFLLCNTGHKCVFSEHGLLTTVAYKLGRDKPVYYALEG
SVAIAGAVIRWLRDNLGIIKSSEEIEKLAKEVGTSYGCYFVPAFSGLYAPYWEPSARGIICGLT
QFTNKCHIAFAALEAVCFQTREILDAMNRDCGIPLSHLQVDGGMTSNKILMQLQADILYIPVVK
PSMPETTALGAAMAAGAAEGVGVWSLEPEDLSAVTMERFEPQINAEESEIRYSTWKKAVMKSIG
WVTTQSPESGIP

*FIG. 1C*

## Mouse NEK1 Protein:

MEKYVRLQKIGEGSFGKAVLVKSTEDGRHYVIKEINISRMSDKERQESRREVAVLANMKHPNIV
QYKESFEENGSLYIVMDYCEGGDLFKRINAQKGALFQEDQILDWFVQICLALKHVHDRKILHRD
IKSQNIFLTKDGTVQLGDFGIARVLNSTVELARTCIGTPYYLSPEICENKPYNNKSDIWALGCV
LYELCTLKHAFEAGNMKNLVLKIISGSFPPVSPHYSYDLRSLLSQLFKRNPRDRPSVNSILEKG
FIAKRIEKFLSPQLIAEEFCLKTLSKFGPQPLPGKRPASGQGVSSFVPAQKITKPAAKYGVPLT
YKKYGDKKLLEKKPPPKHKQAHQIPVKKMNSGEERKKMSEEAAKKRRLEFIEKEKKQKDQIRFL
KAEQMKRQEKQRLERINRAREQGWRNVLRAGGSGEVKASFFGIGGAVSPSPCSPRGQYEHYHAI
FDQMQRLRAEDNEARWKGGIYGRWLPERQKGHLAVERANQVEEFLQRKREAMQNKARAEGHVGL
LQNLASLYGGRPSSSRGGKPRNNEEEVYLARLRQIRLQNFNERQQIKAKLRGENKEADGTKGQE
ATEETDMRLKKMESLKAQTNARAAVLKEQLERKRKEAYEREKKVWEEHLVARVKSSDVPLPLEL
LETGGSPSKQQVKPVISVTSALKEVGLDGSLTDTQEEEMEKSNSAISSKREILRRLNENLKAQE
DEKEKQHHSGSCETVGHKDEREYETENAISSDRKKWEMGGQLVIPLDAVTLDTSFSATEKHTVG
EVIKLDSNGSPRKVWGKNPTDSVLKILGEAELQLQTELLENTSFKSEVYAEEENYKPLLTEEEN
LQCISKEINPSATVDSTETKSPKFTEVSPQMSEGNVEEPDDLETEVLQEPSSTHTDGSLPPVLN
DVWTREKEAAKETELEDKVAVQQSEVCEDRIPGNVDQSCKDQRDPAVDDSPQSGCDVEKSVQPE
SIFQKVVHSKDLNLVQAVHCSPEEPIPIRSHSDSPPKTKSKNSLLIGLSTGLFDANNPKMLRTC
SLPDLSKLFRTLMDVPTVGDVHQDSLEIDELEDEPIKEGPSDSEDTVFEETDTDLQELQASMEQ
LLREQPGDEYSEEEESVLKSSDVEQTARGTDAPDEEDNPSSESALNEEWHSDNSDAETTSECEY
DSVFNHLEELRLHLEQEMGFEKFFEVYEKVKAIHEDEDENIEICSTIVENILGNEHQHLYAKIL
HLVMADGAYQEDNDE

***FIG. 1D***

## Mouse PLK1 Protein:

MNAVAKAGKLARAPTDLGKGGVLGDAAPSAPVAAPLAKEILEVLVDPRSRRQYIRGRFLGKGGF
AKCFEISDADTKEVFAGKIVPKSLLLKPHQKEKMSMEISIHRSLAHQHVVGFHDFFEDSDFVFV
VLELCRRRSLLELHKRRKALTEPEARYYLRQIVLGCQYLHRNQVIHRDLKLGNLFLNEDLEVKI
GDFGLATKVEYEGERKKTLCGTPNYIAPEVLSKKGHSFEVDVWSIGCIMYTLLVGKPPFETSCL
KETYLRIKKNEYSIPKHINPVAASLIQKMLQTDPTARPTIHELLNDEFFTSGYIPARLPITCLT
IPPRFSIAPSSLDPSSRKPLKVLNKGVENPLPDRPREKEEPVVRETNEAIECHLSDLLQQLTSV
NASKPSERGLVRQEEAEDPACIPIFWVSKWVDYSDKYGLGYQLCDNSVGVLFNDSTRLILYNDG
DSLQYIERDGTESYLTVSSHPNSLMKKITLLNYFRNYMSEHLLKAGRNITPREGDELARLPYLR
TWFRTRSAIILHLSNGTVQINFFQDHTKLILCPLMAAVTYINEKRDFQTYRLSLLEEYGCCKEL
ASRLRYARTMVDKLLSSRSASNRLKAS

***FIG. 1E***

## Mouse PRKAR1A Protein:

MASGSMATSEEERSLRECELYVQKHNIQALLKDSIVQLCTTRPERPMAFLREYFERLEKEEARQ
IQCLQKTGIRTDSREDEISPPPPNPVVKGRRRRGAISAEVYTEEDAASYVRKVIPKDYKTMAAL
AKAIEKNVLFSHLDDNERSDIFDAMFPVSFIAGETVIQQGDEGDNFYVIDQGEMDVYVNNEWAT
SVGEGGSFGELALIYGTPRAATVKAKTNVKLWGIDRDSYRRILMGSTLRKRKMYEEFLSKVSIL
ESLDKWERLTVADALEPVQFEDGQKIVVQGEPGDEFFIILEGTAAVLQRRSENEEFVEVGRLGP
SDYFGEIALLMNRPRAATVVARGPLKCVKLDRPRFERVLGPCSDILKRNIQQYNSFVSLSV

***FIG. 1F***

**Mouse PRKRA Protein:**

MSHSRHRAEAPPLQREDSGTFSLGKMITAKPGKTPIQVLHEYGMKTKNIPVYECERSDVQVHVP
TFTFRVTVGDITCTGEGTSKKLAKHRAAEAAINILKANASICFAVPDPLMPDPSKQPKNQLNPI
GSLQELAIHHGWRLPEYTLSQEGGPAHKREYTTICRLESFMETGKGASKKQAKRNAAEKFLAKF
SNISPENHISLTNVVGHSLGCTWHSLRNSPGEKINLLKRSLLSLPNTDYIQLLSEIASEQGFNI
TYLDIEELSANGQYQCLAELSTSPITVCHGSGISCGNAQSDAAHNALQYLKIIAERK

***FIG. 1G***

**Mouse TTBK2 Protein:**

MGLANRRHRLGTCSPISTRGGENCKTGKIKCLLKETHSWLRREIGNRLNGIHPWELDACVAVLP
HQGFAMSGGGEQPDILSVGILVKERWKVLRKIGGGGFGEIYDALDMLTRENVALKVESAQQPKQ
VLKMEVAVLKKLQGKDHVCRFIGCGRNDRFNYVVMQLQGRNLADLRRSQSRGTFTISTTLRLGK
QILESIESIHSVGFLHRDIKPSNFAMGRFPSTCRKCFMLDFGLARQFTNSCGDVRPPRAVAGFR
GTVRYASINAHRNREMGRHDDLWSLFYMLVEFVVGQLPWRKIKDKEQVGSIKERYDHRLMLKHL
PPEFSTFLDHISSLDYFTKPDYQLLTSVFDNSIKTFGVIESDPFDWEKSGTDGSLTTTTTSATP
QLHTRLTPAAIGIANATPIPGDLLRENTDEVFPDEQLSDGENGIPVGVSPDKLPGSLGHPRPQE
KDVWEEMDINKNKIKLGICKAATEEENSHGQVNGILNAPSLGSPIRVRSEITQPDRDVPLVRKL
RSIHSFELEKRLTLEPKPDTDKFLETCMEKMQKDSSAGKEPVPPALPHKPCVPVVTHTDHIWHY
DDEYLPDASKPASANTPEQADGGGSNGFIAVNLSSCKQEVDSKEWVIVDKEQDLQDFRTNEVLG
HKTTGSPSDEEPEVLQVLEGSPQDEKIQVGPWTDNHHLKKESSGVVLALSAECPATAASELYTD
RLDLQAGAASQFITVTPTSPMEAQAEGPLTAITIPRPSVASTQSTSGSFHYGPQPEKKDLQPLE
PTVELYSPRENFSGLVVTEGELASGGSRVDLGLQIDHTGHDMLPNMRDGDTSQDLGPKDPPDHN
RLAVKEFEHLPGETEERSLLLGSENEDERLSKGQHCIEVSSPGELVTAERAQLAATEPLHVSET
QNCSVLPNQDKTHEIMKLLAVGTSEISPQAIDPHAEGQIGQMAAMQKNKLFKDDGIQSESLPRQ
QGDLSAFLHQEGKREKVVPRNGELYHCVSENEHGPPTRKDMLRSSFVTRHSRIPVLAQEIDSTF
ESSSAISAKEKLLQKKAYQPEIVKLLVEKRQFKSFLGDLSSASDKLIEEKLAAVPVPFSEEEVF
APFSRLAADSHLSRSVEDSFLSPIISQARKSKIPRPVSWVSTDQINGSASPQFLPRPPPGKPPV
RPGVEARLRRYKVLGSSNSDSDLFSRLAQILQNGSQKSRSTTQCKSPGSPHNPKTPPKSPVVPR
RSPSASPRSSSLPRTSSSSPSRAGRPHHDQRSSSPHLGRSKSPPSHSGSSSSRRSCQQEHCKPS
KNGPKGSGSLHHHSTSSKTPPGKSKPASKLSR

***FIG. 1H***

**Mouse TTK Protein:**

MEAEELIGSSVTIDSIMSKMRDIKNKINEDCTDELSLSKICADHTETVNQIMRVGNTPENWLNF
LLKLEKNSSPLNDDLLNKLIGRYSQAIEVLPPDKYGQNESFARIQVRLAELKAIQEPDDARDYF
QMARENCKKFAFVHVSFAQFELSQGNLKKSEQLLHKAVETGAVPLQMLETAMRNLHLQKKQLLP
EEDKKSVSASTVLSAQEPFSSSLGNVQNRSISCESRGQAGAARVLYGENLPPQDAEVRHQNPFK
QTHAAKRSCPFGRVPVNLLNSPDFYVKTDSSAVTQLTTRLALSSVPLPYVTCLLHLQLLALAGL
AKGSGPDRDAILPGSRPRGSDSYELRGLKPIQTIYLKDSLVSNEKSSELMSDLIALKSKTDSSL
TKLEETKPEIAERRPMQWQSTRKPECVFQNPAAFAPLRHVPDVTPKADKESPPISVPKWLDPKS
ACETPSSSSLDDYMKCFKTPVVKNDFPPACPSSTPYSQLARLQQQQQQGLSTPLQSLQISGSSS
INECISVNGRIYSILKQIGSGGSSKVFQVLNEKKQINAIKYVNLEDADSQTIESYRNEIAFLNK
LQQHSDKIIRLYDYEITEQYIYMVMECGNIDLNSWLKKKKSINPWERKSYWKNMLEAVHIIHQH
GIVHSDLKPANFVIVDGMLKLIDFGIANQMQPDTTSIVKDSQVGTVNYMAPEAIRDMSSSRENS
KIRTKVSPRSDVWSLGCILYYMTYGRTPFQHIINQVSKLHAIINPAHEIEFPEISEKDLRDVLK
CCLVRNPKERISIPELLTHPYVQIQPHPGSQMARGATDEMKYVLGQLVGLNSPNSILKTAKTLY
ERYNCGEGQDSSSSKTFDKKRERK

***FIG. 1I***

## Mouse CDC2L1 DNA:

```
1     atgggtgatg aaaaggactc ttggaaagtg aaaacgttag atgaaattct ccaggaaaag
61    aaacgaagga aagaacaaga ggagaaagca gagataaaac gcttaaaaaa ttctgatgac
121   cgcgattcca aaagggattc ccttgaggag ggggagctga gagatcaccg aatggagatc
181   acaatcagga actcaccata tagaagagag gattctatgg aagacagagg agaggaggat
241   gattctctgg ccatcaaacc accccagcaa atgtctcgga aagaaaaggc tcatcacaga
301   aaagacgaga aaagaaaaga gaaacgtcga catcgtagcc attcagcaga gggagggaaa
361   catgccagag tgaaagagaa agaaagggag cacgaacgcc ggaaacgcca ccgagaagaa
421   caagataaag ctcgaaggga gtgggaaaga cagaagagga gggaaatggc gagagaacat
481   tccagaagag agagggaccg cctggagcag ttagaaagga agagggagcg ggagcgcaag
541   ctgagggagc agcagaagga gcagcgggag cagaaggagc gggaacggag ggcagaggag
601   cgccgcaaag agagagaagc gcgtagggaa gtctctgcac atcaccgtac catgagggag
661   gagtacagtg ataaggggaa ggttggccac tggagccgca gccctctgag gccaccaaga
721   gagcgctttg agatgggaga caaccggaag ccagtaaaag aagagaaggt ggaagagaga
781   gacttgttgt cagacctcca agacatcagt gacagcgaga ggaaaaccag ctcagctgag
841   tcttcatcag cagaatcagg ctcaggttct gaagaggagg aggaggaaga agaagaggaa
901   gaagaagaag aagggagcac cagtgaagaa tcagaggaag aagaggaaga agaagaggag
961   gaggaagaag aggagactgg gagcaactct gaggaggcct ctgaacagtc cgcagaagaa
1021  gtcagtgatg aggaaatgag tgaagatgaa gacagagaaa acgagaacca catcttggtt
1081  gttccagagt cacgatttga ccgagattct ggggacagtg aagaagggga ggaagaagtt
1141  ggtgaaggga ctccacagag cagtgccccc accgaaggag actatgttcc tgactctcca
1201  gcactgtcac ctattgagct aaaacaagaa ctgcccaaat acctgccagc cctgcaggga
1261  tgcaggagtg tagaggagtt tcagtgtctg aacaggattg aagaaggcac ctatggggtg
1321  gtctacagag caaaggacaa gaaaacagat gaaattgtgg ctctgaagcg gttaaagatg
1381  gagaaggaga aggaaggctt cccaatcacg tcgctgaggg aaatcaacac catcctcaag
1441  gcccagcacc ccaacatcgt caccgtcagg gaaattgttg tgggaagtaa catggacaag
1501  atctacattg tgatgaacta cgtggaacat gacctcaaga gcctaatgga gaccatgaag
1561  cagcccttcc tgccagggga ggtgaagacc ctgatgattc agctgctgag tggggtaaag
1621  cacctccatg acaattggat cctacaccgt gacctgaaga cctctaacct cctgctgagc
1681  catgctggca ttctcaaggt gggcgacttt gggctggctc gggagtatgg ttcaccccta
1741  aaggcctaca ctccagttgt tgtaaccctg tggtatcgtg ccccagaact actgcttggt
1801  gctaaggaat actccacagc tgtggacatg tggtcggtag gctgcatatt tggagaactg
1861  ctgacacaga aacctctgtt ccctgggaag tcagatattg atcagattaa caagatttc
1921  aaggacctgg gtactcctag tgagaaaatc tggcctggct ataatgacct cccagccgtc
1981  aagaagatga ccttcagcga gtatccctat aacaacctcc gcaagagatt tggggctttg
2041  ttatcagatc aaggctttga tctcatgaac aagttcctga catactaccc tggcaggagg
2101  atcaacgcag aagatggcct caagcacgaa tatttccgag agactcccct ccccatcgac
2161  ccatccatgt tccccacgtg gcctgccaag agtgagcagc agagagtgaa gcgaggcacg
2221  agtccacggc ctcctgaggg cggcctgggc tacagccagc tgggtgatga tgacctgaag
2281  gagacgggct tccacctcac caccaccaac cagggagcct cagctgcagg ccctggcttc
2341  agcctcaagt tctga
```

*FIG. 2A*

**Mouse CSNK1A1 DNA:**

1 atggcgagca gcagcggctc caaggccgaa tttatcgtcg gtggaaaata caaactggtg
61 cggaagatcg gatctggctc tttcggggac atttatctag cgatcaacat caccaatggc
121 gaggaagtgg cagtgaaact agaatcccag aaggccaggc atccccagtt gctgtacgag
181 agcaaactgt ataagattct tcaaggtggg gttggcatcc ctcacatacg gtggtatggg
241 caagaaaaag actataatgt actagtcatg gatctcctgg gacccagcct tgaagacctc
301 ttcaacttct gttcaaggag gttcactatg aagaccgtac ttatgttagc tgaccagatg
361 atcagtagaa ttgaatatgt gcatacaaag aattttatac acagagacat taaaccagat
421 aacttcctaa tgggtattgg gcgtcactgt aataagttat tccttattga ttttggtttg
481 gccaaaaagt acagagacaa caggacaagg caacacatac catacagaga agataaaaac
541 ctcactggca ctgcccgata cgccagcatc aatgcacatc ttggtattga gcagagtcgc
601 cgagatgaca tggaatcttt aggatatgtt ttgatgtatt ttaatagaac cagtctgccg
661 tggcaaggac tgaaggctgc aacaaagaaa caaaaatatg aaaagattag tgaaaagaag
721 atgtccactc ctgttgaggt gttgtgtaag ggtttcctg cagaatttgc catgtactta
781 aattactgtc gtgggctgcg ctttgaggaa gctccggatt acatgtatct gaggcagcta
841 ttccgcatcc ttttcaggac cctgaaccac caatatgact acacatttga ttggacgatg
901 ttaaagcaga aagcagccca gcaggcagcc tcttccagtg ggcagggtca gcaggcccaa
961 accccccacag gtttctaa

***FIG. 2B***

**Mouse GYK DNA:**

1 atggcagccg cgaagaaagc agttctgggg ccattggtgg gagcagtgga ccagggtacc
61 agctcgacac gttttttggt tttcaattca aaaacagctg aacttcttag tcatcatcaa
121 gtagaaataa aacaggaatt cccaagagaa ggatgggtag aacaagaccc gaaggaaatt
181 ctgcagtctg tttatgagtg tatagagaaa acgtgtgaga aacttggaca gctcaatatt
241 gatatttcca acatcaaagc cattggtgtc agcaaccaga gggaaaccac agtagtctgg
301 gacaaggtca ccggagagcc tctctataat gccgtggtgt ggcttgacct aagaacccag
361 tctactgttg agaaccttag taaaagaatt ccaggaaata ataactttgt caagtccaag
421 acaggccttc cacttagcac gtatttcagt gcagtgaaac ttcgttggct ccttgacaac
481 gtgaaaaagg tccaagaggc tgttgaagaa aatagagctc tttttgggac cattgattca
541 tggcttattt ggagtttaac aggaggaatc catgggggtg tccactgtac agatgtaaca
601 aatgcaagca ggacgatgct tttaacatt cattctttgg aatgggataa agagctctgc
661 gaattttttg gaattccaat ggaaattctt cccaacgttc ggagttcttc tgagatctat
721 ggcctaatga aagctggggc cttggaaggt gtaccaatat ctgggtgttt gggggaccag
781 tctgctgctt tggtgggaca aatgtgcttc caggatggac aggccaaaaa cacgtatgga
841 acagggtgct tcttattgtg caacacgggc cataagtgtg tatttctga acatggcctc
901 ctgacaaccg tagcatataa acttggcaga gacaaacctg tgtattatgc gttggaaggt
961 tccgtggcta tagctggtgc tgtaatccgc tggctaagag acaaccttgg aattattaag
1021 tcctctgagg aaattgaaaa acttgctaag gaagtaggta cttcttatgg ctgctacttc
1081 gttccagcat tttcagggtt atatgcgcct tattgggagc ccagtgcaag agggatcatc
1141 tgtggactca ctcagttcac caataaatgt catatcgctt ttgctgcact agaagctgtt
1201 tgtttccaaa cccgagagat tttggatgcc atgaatcgcg actgtggaat tccactcagt
1261 catttacagg tagatggagg aatgaccagc aataaaattc ttatgcagct acaagcagac
1321 attctgtata ttccagtagt gaaaccctcc atgcctgaaa caactgcact aggcgctgcc
1381 atggcagctg gggctgcaga gggggttggt gtgtggagtc ttgaacctga ggatttgtca
1441 gctgtcacaa tggagcggtt tgaacctcag atcaatgctg aagaaagcga aatccgttac
1501 tccacatgga agaaagctgt gatgaagtca attggttggg ttacaactca gtctccagaa
1561 agtggtatcc cataa

***FIG. 2C***

## Mouse NEK1 DNA:

1 atggagaagt atgtgagact gcagaagatt ggagaaggtt catttggaaa agctgttctt
61 gttaaatcga cagaggatgg cagacattat gtcatcaagg aaattaacat ctcaagaatg
121 tctgataaag aaaggcaaga atcaaggaga gaagttgctg tattggcaaa catgaagcat
181 ccaaatattg tccaatataa agaatcattt gaagaaaatg gctctctcta catagtaatg
241 gattactgtg aaggaggtga tttgtttaaa cgaataaatg ctcagaaagg cgctctgttt
301 caagaagacc agattttgga ctggtttgtg cagatatgtt tggctctgaa gcatgtacat
361 gatagaaaaa ttcttcaccg agacataaag tcacagaaca tatttctaac caaagatggg
421 acagtgcagc ttggagattt tggaattgct cgagttctta atagtactgt agagctggct
481 cgaacttgca taggcactcc atactacttg tcacctgaaa tctgtgaaaa caagccttat
541 aacaataaaa gtgacatttg ggctttgggc tgtgtccttt atgagttgtg tacacttaaa
601 catgcatttg aagctggaaa catgaaaaac ctggtactga agataatctc cggatccttt
661 cctccagtgt ctccacatta ctcctatgat ctccgcagct tgctgtctca gttatttaaa
721 agaaatccta gggatagacc atcagtcaac tccatattgg agaaaggttt tatagctaaa
781 cgaatcgaaa agtttctctc ccctcagctt attgcagaag aattttgtct aaaaacactt
841 tcaaagtttg gaccacagcc tctcccaggt aaaagaccag catcaggaca aggtgtcagt
901 tcttttgtcc ctgctcagaa aatcacaaag cctgctgcta aatacggagt gcctttaaca
961 tataagaagt atggagataa aaagttactt gagaaaaaac cacccccaaa acataaacag
1021 gcccatcaaa ttcccgtgaa gaaaatgaat tctggagaag aaaggaagaa aatgtctgag
1081 gaagcagcaa aaaaaagaag gttggaattt attgagaaag aaaagaagca aaaggatcag
1141 attaggttcc tgaaggctga gcagatgaag cggcaagaga agcagcggtt ggagaggata
1201 aatagggcca gggaacaagg atggaggaat gttttaaggg ctggtggaag cggtgaagta
1261 aaggcttcct tttttggcat tggaggggct gtctctccat caccgtgttc tcctcgaggc
1321 cagtatgaac attaccatgc catttttgac caaatgcagc ggctaagagc agaagataat
1381 gaagcaagat ggaagggggg aatctatggt cgatggctcc cagaaaggca aaaaggacac
1441 ttagctgtag agagagccaa ccaagtggaa gaattcctac agcgtaaacg agaagctatg
1501 cagaataaag cccgagccga aggacacgtg ggactcctgc aaaacctggc atctctgtat
1561 ggaggcaggc ccagctcttc aagaggaggg aagccaagga acaatgagga agaggtttat
1621 ttggcaagac tgaggcaaat aagactacaa aattttaatg agcgccaaca gattaaagcc
1681 aaacttcgtg gtgagaataa agaagctgat ggtaccaaag gacaagaagc aactgaagag
1741 actgacatga ggctcaaaaa gatggagtca cttaaggcgc aaacaaatgc acgtgctgct
1801 gtactaaaag aacagctgga gcgaaaaaga aaggaagctt atgaaagaga aaagaaagta
1861 tgggaagaac atttggtggc gagggtaaaa agctcagatg ttcctctgcc tttggaactt
1921 cttgaaacag gtggttctcc atcaaagcag caggtgaagc ctgtcatttc tgtgacttca
1981 gctttgaaag aagtgggcct ggatggaagt ttaactgata cccaggaaga agaaatggaa
2041 aagagtaaca gtgctatttc aagtaagcga gaaatcctgc gtaggctaaa tgaaaatctt
2101 aaagctcaag aggatgaaaa ggaaaagcag catcactcag gttcttgtga gaccgttggt
2161 cacaaagatg agagagagta tgagacagaa aatgccattt cctctgatcg caagaagtgg
2221 gagatgggag gtcagcttgt gattcctctc gatgcagtga cactggatac atccttctct
2281 gcaaccgaaa aacatactgt gggagaggtt attaaattag attctaatgg ctctccaaga
2341 aaagtctggg ggaaaaaccc tacagattct gtgctgaaga tacttggaga agctgaatta
2401 cagctacaga cagaactact agaaaacaca tcttttaaaa gtgaggttta tgctgaagag
2461 gagaactaca aaccccttact tactgaagaa gagaatctgc agtgcatttc aaaagaaata
2521 aatccatcag ctactgttga ttctactgaa acgaaaagtc caaagtttac tgaggtgtct
2581 ccacaaatgt cagaaggaaa tgtggaagaa cctgatgatt tggaaacaga agttctacaa
2641 gagccaagta gcacacacac agatgggagt ttgccacctg ttcttaatga tgtgtggact
2701 agagagaagg aagcagctaa ggaaactgag ttggaagata aggttgctgt gcagcagagt
2761 gaagtttgtg aagatagaat tccagggaac gtggaccaat cctgtaagga tcagagagat
2821 cctgcagtag acgattctcc gcagtctggc tgtgatgtag agaagtcagt acagccagaa

# *FIG. 2D-1*

```
2881 tcgattttcc agaaagtggt tcattctaag gacttgaact tagttcaggc agttcattgc
2941 tcaccagaag aaccaattcc aattcgatct cactctgatt ctccaccaaa aactaagagc
3001 aagaattcct tactgattgg actttcaact ggtctgtttg atgcaaacaa tccaaagatg
3061 ctgaggacct gctcacttcc agatctttcc aagctgttca gaaccctaat ggacgttccc
3121 actgtggggg acgttcatca agacagtctt gaaatcgatg agctggaaga tgaaccaatt
3181 aaagaagggc cttctgattc cgaagacact gtatttgaag aaactgacac agatttacaa
3241 gagcttcagg cctcaatgga gcagctgctt agggagcaac caggtgacga atacagtgag
3301 gaggaagagt ctgttttaaa aagcagcgat gtggagcaga cagcaagagg gacagatgcc
3361 ccagacgagg aggacaaccc cagcagcgaa agcgccctga acgaggaatg gcactcagat
3421 aatagtgacg ctgagaccac tagtgaatgt gaatatgaca gtgtctttaa ccatttagag
3481 gaactaagac ttcacttgga gcaagaaatg ggctttgaaa agttctttga ggtttatgag
3541 aaagtaaagg ctattcatga ggatgaagat gaaaatattg aaatttgttc aacaatagtt
3601 gagaatattt tgggcaatga gcaccagcat ctctatgcca agattctgca tttagtcatg
3661 gcagatggag cctatcagga agataatgat gaataa
```

***FIG. 2D-2***

**Mouse PLK1 DNA:**

```
1    atgaatgcag tggccaaagc tggaaagctg gctcgagcac caaccgacct cgggaaaggt
61   ggggtcctgg gagatgcagc tcccagtgcc ccagtggctg ccccactggc gaaagaaatt
121  ctggaggtcc tagtggaccc acgcagccgg cggcagtata tacggggccg ctttctgggt
181  aaaggaggct tcgccaaatg cttcgagatc tcagacgcag acacaaaaga ggtgttcgca
241  ggcaagatcg tgcctaagtc tttgctgctc aagccccacc agaaggagaa gatgtctatg
301  gagatctcaa ttcaccgcag cctagcacac caacacgtcg taggcttcca tgactttttt
361  gaggacagcg actttgtatt tgtagttttg gagctctgtc gcaggaggtc cctcctggag
421  ctgcacaaga ggaggaaggc actgaccgag cctgaggccc gctactacct gcgacagata
481  gtcctgggct gccagtacct gcaccgcaat caggtcattc acagggacct caagctgggc
541  aacctcttcc tgaacgagga tctggaggtg aaaatagggg attttggctt ggcaaccaaa
601  gtggaatatg aaggggaacg aaagaagacc ttgtgtggca ctcctaacta catagctcct
661  gaggtgctga gcaagaaggg acacagtttt gaggtggatg tgtggtccat tgggtgcatc
721  atgtatacct tgctagtggg caagcctccc tttgagacct cgtgcctaaa agagacctac
781  ctccggatca agaaaaatga atacagtatt cccaagcaca tcaacccagt ggccgcctcc
841  ctcatccaga agatgcttca gacagacccc actgcccgcc ccaccattca cgagttgctc
901  aatgacgagt tcttcacttc tggctacatc cccgcccgtc tccctattac ctgcctcacc
961  atcccaccaa ggttttcaat cgctcccagc agcctggacc ccagcagcag gaaacctctc
1021 aaagtcctca ataaaggtgt ggagaacccc ctgcctgacc gtccccggga gaaagaggaa
1081 ccggtggtcc gggagacaaa tgaggccatt gagtgccacc ttagtgactt gctacagcag
1141 ctgaccagtg tcaacgcctc caagccctcg gagcgcgggc tggtgcggca agaggaggct
1201 gaggatcctg cctgcatccc catcttctgg gtcagcaagt gggtggacta ttcggacaag
1261 tatggccttg ggtatcagct gtgtgacaac agtgtggggg tgctttttaa tgactcaaca
1321 cgcctgattc tctacaatga cggggacagc ctgcagtaca tagagcgtga tggcacggag
1381 tcctatctca ctgtgagctc ccatcccaat tccttgatga agaagatcac tctcctcaac
1441 tatttccgca attacatgag tgagcacctg ctgaaggcag gacgcaacat cacaccccgg
1501 gaaggcgacg agctggcccg gctgccctac ctacgaacgt ggttccgcac acgcagcgcc
1561 atcatcctgc acctcagcaa cggcaccgtg cagattaact tcttccagga ccacaccaaa
1621 cttatcctgt gcccccctgat ggcagcggtg acctacatca acgagaagag ggacttccaa
1681 acgtaccgcc tgagcctcct ggaggagtat ggctgctgca aggagctggc cagccgcctc
1741 cgctacgccc gcaccatggt agacaagctg ctgagctcgc gctccgccag caaccgcctc
1801 aaggcctcct ag
```

***FIG. 2E***

## Mouse PRKAR1A DNA:

```
1     atggcgtctg gcagtatggc aaccagtgag gaagagcgga gtctccggga atgcgagctc
61    tatgtgcaga agcacaatat ccaggccctg ctgaaggact ccatcgtgca gctgtgcact
121   acgcggcccg agaggcccat ggcattcctt cgggaatact ttgagaggtt ggagaaggag
181   gaggcaagac agattcagtg tctacagaaa accggcatcc gtactgactc gagggaggac
241   gagatctctc ctccaccccc caatccagtg gtgaagggcc gacggcgccg aggtgctatc
301   agtgctgaag tttacactga ggaggatgct gcctcctacg ttagaaaggt tattccaaaa
361   gattataaga caatggctgc tttagccaag gccatcgaaa agaatgtgct gttttcacac
421   cttgatgata acgagagaag tgacattttt gatgctatgt ttccagtctc ctttattgct
481   ggagagacgg ttattcagca aggtgatgaa ggggataact tctatgtgat tgatcaagga
541   gaaatggatg tctatgtcaa taatgaatgg gcaaccagtg ttggggaagg agggagcttt
601   ggagagctgg ctttgattta tggaacaccc agagcagcca ctgtcaaagc aaagacaaac
661   gtgaaactgt ggggcatcga ccgagacagc taccgaagaa tcctcatggg aagcactctg
721   cgaaagagga agatgtatga agaattcctt agtaaagtgt ctattttaga gtctctggac
781   aagtgggagc gtctcacagt agccgatgca ttggagcctg tccagtttga agatggacag
841   aagatcgtgg tgcaaggaga gccgggggat gagttcttca tcattttaga gggcacagct
901   gctgtgctgc agcgtcggtc agaaaacgaa gaatttgttg aagtgggacg actggggcct
961   tctgattatt ttggtgaaat tgccctgctg atgaatcgtc ctcgggctgc cactgtggtt
1021  gcccggggcc ctttgaagtg cgttaagttg gaccggcctc ggtttgaacg cgtccttggc
1081  ccgtgctcag acatcctcaa gcggaacatc cagcagtaca acagcttcgt gtccctgtcc
1141  gtctga
```

*FIG. 2F*

## Mouse PRKRA DNA:

```
1     atgtcccata gcaggcatcg tgccgaggcc ccgccgctgc agcgcgagga cagcgggacc
61    ttcagtttgg gcaagatgat aacagctaag cctgggaaaa caccgattca ggtattgcac
121   gagtacggca tgaagaccaa gaacattcca gtttatgaat gtgaaagatc cgatgtgcaa
181   gtacacgtgc ccactttcac cttcagagta accgttggtg acataacttg cacaggtgaa
241   ggtacgagta agaagctggc gaagcacaga gctgcggagg ccgccataaa catttgaaa
301   gccaatgcaa gtatttgctt tgcagttcct gacccettaa tgcctgatcc atccaaacag
361   ccgaagaacc agctgaatcc aattggctca ttacaggaat tagcaattca ccatggctgg
421   cgacttcctg aatataccct ttcccaggaa ggaggaccgg ctcataagag ggaatacacc
481   acgatctgca ggcttgaatc attcatggaa actggaaagg gggcatcaaa aaaacaagcc
541   aagagaaatg ctgctgagaa atttctcgcc aagtttagta atatttctcc agagaaccac
601   atttctctaa cgaacgtggt tggacattcc ctaggatgca cttggcactc cttgaggaat
661   tcccctggtg agaagatcaa cctcctgaaa aggagcctcc tcagtctccc gaacacagac
721   tacatccagc tgcttagtga gattgccagc gagcaaggct ttaacataac gtatttggat
781   atagaggagc tgagtgccaa cggacagtat cagtgtctcg cagagctgtc caccagtcct
841   atcaccgtgt gtcacggctc aggcatctcc tgtggcaatg cacagagtga cgctgctcac
901   aatgctctgc agtatttaaa gataatagca gaaagaaagt ag
```

*FIG. 2G*

## Mouse TTBK2 DNA:

```
1     atgggactag ccaaccgacg ccacaggctg ggaacgtgct ctccaatcag cacccgcggc
61    ggagagaatt gtaaaaccgg taagatcaag tgcctattga aagagactca ctcttggcta
121   agaagagaaa taggaaatag gttaaatgga atccaccctt gggagctaga tgcctgtgta
181   gctgttttac ctcatcaggg ttttgcaatg agtggaggag gagagcagcc agatatcctc
241   agtgttggaa tcctggtcaa agaaagatgg aaagtgttaa gaaagattgg aggtggggc
301   tttggagaaa tttacgatgc cttggacatg ctcaccaggg agaatgtggc gctgaaggtg
361   gagtcagctc agcagccaaa gcaggttctg aagatggagg ttgctgtgtt gaagaaactg
421   caagggaaag accatgtttg tagatttatt ggctgtggga gaaatgatcg tttcaactac
481   gtggtcatgc aattgcaggg acggaatctg gcagatctcc gccgtagcca atcccggggc
541   acattcacta ttagcactac ccttcgtctt gggaaacaga ttctggagtc tattgaaagc
601   atacattctg tgggattcct tcacagagac atcaaaccgt caaacttcgc catgggacgt
661   ttccccagta catgtaggaa atgtttcatg cttgattttg gcttggctcg acaatttact
721   aattcctgtg gtgacgtcag accacctcgt gctgtggcag gctttcgagg gacagttcgt
781   tatgcatcaa tcaatgctca tcggaacagg gaaatgggaa gacatgatga cctttggtct
841   ttattctaca tgttggtgga gtttgtggtt ggccaactgc cttggagaaa aataaaggac
901   aaggagcaag taggctccat taaggagaga tatgaccaca ggctcatgtt aaaacacctc
961   cctccagaat tcagcacctt tcttgaccat atttcctctt tggattattt tacaaaaccg
1021  gactaccagc ttctaacatc cgtgtttgac aatagcatca agacctttgg agtaattgag
1081  agtgacccgt ttgactggga gaagagtgga actgatggct ccctgacaac caccaccacc
1141  tctgccaccc ctcagctgca cacccgcctg accccctgctg ctatcggaat tgcaaatgcc
1201  accccccatcc caggagactt gcttcgagaa aatacagatg aagtgtttcc agatgaacag
1261  cttagtgatg ggagaacgg aatccctgtt ggtgtatcac cagataaatt gcctggatct
1321  ctggggcacc cacgccctca ggaaaaggat gtctgggaag agatggatat caacaagaac
1381  aagataaagc tgggaatttg caaagcagct actgaagaag aaaatagcca tggtcaagta
1441  aatggcatac tcaatgctcc aagccttggt tcaccaattc gtgtccgatc agagattact
1501  cagccagaca gagatgttcc gttagtaagg aagctacgtt ctattcacag ctttgagctg
1561  gaaaaacgtt tgacacttga gccaaagcca gatactgaca agtttctgga gacctgcatg
1621  gagaaaatgc agaaagattc cagtgcagga aaagaacctg tccccctgc tctgcctcat
1681  aagccgtgtg tccccgtcgt gacccacact gaccacatct ggcactatga tgacgaatat
1741  cttcctgatg cctccaagcc tgcctctgcc aacaccccgg agcaggcaga tggtggtggc
1801  agcaacggat ttatagctgt taacttaagc tcttgcaaac aggaggttga ttccaaagaa
1861  tgggtgattg tggacaagga gcaagacctt caggacttta ggacaaatga ggtgttaggc
1921  cataagacaa ctggaagccc ttcagatgag gagcctgaag tgcttcaggt ccttgaggga
1981  tcacctcaag atgaaaagat ccaagtaggt ccttggactg acaaccatca cttaaagaag
2041  gaaagctcag gtgtggtttt agcactttct gctgaatgcc ctgctactgc tgcttcagaa
2101  ctgtacacag ataggctaga cctccaggct ggagctgcaa gtcagttcat cacagtgact
2161  cccacaagtc ccatggaggc acaagcagaa ggacccctga ctgcgattac aattcctaga
2221  ccttctgtgg catcaacaca gtcaacttca ggaagctttc actatggccc acaaccagag
2281  aagaaagatc ttcagccctt ggagcccact gtagaactgt actctccaag ggagaacttc
2341  tccggcttgg ttgtgacaga gggtgaacta gctagcggag gaagcagagt ggatttggga
2401  cttcagatag atcacactgg tcatgacatg ttacccaaca tgagagatgg tgacacatct
2461  caagacttgg gaccaaaaga ccctcctgac cataatagat tagctgtgaa agaatttgaa
2521  catctccctg gagaaacaga agagagaagc cttcttctgg gctcagagaa tgaagatgag
2581  aggttaagta aagggcagca ctgtattgaa gtctcttccc caggagagtt agtgactgca
2641  gagagggctc agttagctgc cacagaacct ctgcatgtat cagagacaca gaactgtagt
2701  gtgctaccaa atcaggacaa aacccatgag ataatgaagc ttttggcagt tggaacttca
2761  gaaatttctc ctcaagccat tgacccacat gctgaagggc agataggcca gatggcagca
2821  atgcagaaaa ataagctatt taaagatgat ggtattcaga gtgaaagctt gccaaggcag
2881  cagggagacc tctctgcttt tttgcaccaa gagggtaaga gagagaaagt tgtccctaga
```

**FIG. 2H-1**

2941 aatggagagc tctatcattg tgtctcagag aatgagcatg gtcctcctac tcggaaggac
3001 atgctccggt catcctttgt gaccaggcac agccggatcc ctgttttagc acaagaaata
3061 gactcaactt ttgaatcatc ctctgctatt tctgcaaaag aaaagcttct acagaagaaa
3121 gcctatcagc cagaaatagt caaacttctt gtagaaaaaa ggcagttcaa gtctttcctg
3181 ggagacctct caagtgcctc tgataagctg atagaggaga aactagccgc tgttccagtc
3241 ccttttctg aggaggaagt cttcgctccc ttttctagac tggcagcaga ttcccacctg
3301 agtagatcag ttgaagatag ctttctgtca cccatcatct cccaggctag aaagagcaag
3361 attccaaggc cagtatcctg ggtcagcaca gatcaaatta atggctccgc ttcacctcag
3421 ttcttgcctc ggccaccacc aggaaagcca ccagtcaggc ctggagtaga agccaggcta
3481 cgcagatata aagttctagg gagtagtaac tctgactcag acctttctc tcgcctggcc
3541 caaattcttc aaaatggatc tcagaaatcc cggagtacta cccaatgcaa gagcccagga
3601 tctcctcaca atccaaaaac accacccaag agtccagttg tacctcgaag gagtcccagt
3661 gcctctcctc gaagctcatc cttgcctcga acatctagtt cctcaccatc tagggctgga
3721 cggccccacc atgaccagag gagttcttcc ccacatctgg ggagaagcaa gtcacccccct
3781 agccactcag gatcatcgtc ctccaggagg tcctgccaac aggagcattg caaacccagc
3841 aagaatggcc caaaaggatc tggcagcctc caccaccact caaccagctc taaaactccc
3901 ccagggaaga gtaagccagc cagtaaactc agcagatag

## FIG. 2H-2

### Mouse TTK DNA:

1 atggaggctg aagagttaat tggcagcagt gtgacgattg attccatcat gagcaaaatg
61 agagatatta aaaataagat aaatgaagac tgtactgatg agctaagctt gtctaaaatc
121 tgtgccgatc acaccgaaac tgttaaccaa attatgaggg ttgggaacac cccagagaac
181 tggttgaatt tcttgctgaa actagagaaa aacagctcac ctctaaatga cgatctttta
241 aataagctga ttggtcggta tagtcaagcg attgaagtac ttcctccaga taaatacggc
301 cagaatgaga gctttgctcg aatacaagtg agacttgctg aactaaaagc tattcaagag
361 cctgatgatg cccgtgacta cttccagatg gccagggaaa actgcaagaa gtttgctttt
421 gtgcacgtat cttttgcaca gtttgaactg tctcaaggca atcttaaaaa aagtgagcag
481 cttcttcata aagctgtaga gactggggcg gtgccgctgc agatgctgga gacggccatg
541 cgtaacttac acctccagaa aaagcagctg cttccggagg aggacaagaa gagtgtgtca
601 gcatcgacag tactaagtgc ccaagagccg ttctccagct cacttggaaa tgtacagaat
661 aggagcatca gctgtgagtc cagaggacag gctggggcag ccagggtttt atatggagag
721 aacttgcctc cacaagatgc cgaagtgagg catcaaaacc ccttcaagca gactcacgca
781 gctaaacggt catgcccctt tggaagagtc ccagtcaatc ttctaaacag cccagatttc
841 tatgtgaaga cagatagctc agctgtgaca cagttaacaa caaggctagc cttaagctct
901 gtacccttgc cgtacgtaac ctgcctcctg cacttacagc tgctggcgct cgcaggcttg
961 gcaaaggggt caggaccaga ccgagacgcg attctgcccg gctccagacc acgtggcagt
1021 gattcctatg aactgagagg tttaaagccc attcaaacta tctatttgaa agactctttg
1081 gtgtccaatg aaaagagttc tgaacttatg tctgatttaa tagccttgaa gagtaaaaca
1141 gattcaagtc taacaaaatt ggaagaaact aagccagaga ttgcagaaag aaggcccatg
1201 cagtggcagt ctaccagaaa gcccgagtgt gtgttccaga accctgctgc ctttgcaccc
1261 ctgcggcacg ttccagatgt caccccgaag gctgacaaag agtcaccacc aatatcagtt
1321 cctaaatggc ttgatccaaa gtctgcttgt gagacaccta gtagcagctc cttggatgat
1381 tacatgaaat gttttaagac tccagttgta aagaatgact ttccacctgc ctgtccatca
1441 tcaacacctt acagccagct tgcccgcctc cagcagcaac agcagcaggg actcagcact

## FIG. 2I-1

1501 cctcttcaaa gcttgcagat ttcaggttct tcatcaataa atgaatgcat ttcagttaac
1561 ggaagaattt attccatatt aaagcagata ggcagtggag gttccagtaa ggtgtttcag
1621 gtattgaatg agaaaaaaca gataaacgct atcaaatatg tgaacctaga agacgccgat
1681 agccaaacta ttgagagcta ccgcaacgag atagcgtttt tgaacaaact acagcaacac
1741 agtgataaga tcatccgcct ctatgattat gaaatcaccg agcagtacat ctacatggta
1801 atggaatgtg gaaacattga cctaaatagt tggcttaaaa agaaaaaatc catcaatcca
1861 tgggaacgca agagctactg gaaaaacatg ttggaggcag tacacataat ccatcagcat
1921 ggtattgttc atagtgatct gaagcctgct aactttgtga tagtggatgg aatgctaaag
1981 ctaattgatt ttgggattgc aaaccaaatg cagccagaca caacaagcat tgttaaagat
2041 tctcaggttg gcacagttaa ctatatggcc ccagaagcaa tcagagacat gtcttcttca
2101 agagaaaatt cgaaaatcag gaccaaggta agtcccagaa gtgatgtctg gtccttgggg
2161 tgcattttgt actacatgac ttatgggagg acgccatttc agcacatcat caatcaggtc
2221 tctaaactgc acgccataat caaccctgct catgagattg aatttcccga gatttcggaa
2281 aaagatcttc gagacgtgtt aaagtgctgt ttagtgagga accctaaaga gaggatatct
2341 atccctgagc ttctcacaca tccgtatgtt caaattcagc ccatccagg cagccaaatg
2401 gctaggggag ccactgatga aatgaaatat gtgttgggtc aacttgttgg tctgaattct
2461 cctaactcca tcttgaaaac tgcaaaaact ttgtatgaac gttataattg tggtgaaggt
2521 caagattctt cgtcatccaa gacttttgac aaaaagagag aaagaaagtg a

FIG. 2I-2

**Human CDC2L1 Protein:**

MGDEKDSWKVKTLDEILQEKKRRKEQEEKAEIKRLKNSDDRDSKRDSLEEGELRDHCMEITIRN
SPYRREDSMEDRGEEDDSLAIKPPQQMSWKEKVHHRKDEKRKEKCRHHSHSAEGGKHARVKERE
HERRKRHREEQDKARREWERQKRREMAREHSRRERDRLEQLERKRERERKMREQQKEQREQKER
ERRAEERRKEREARREVSAHHRTMREDYSDKVKASHWSRSPPRPPRERFELGDGRKPVKEEKME
ERDLLSDLQDISDSERKTSSAESSSAESGSGSEEEEEEEEEEEGSTSEESEEEEEEEEEEEETG
SNSEEASEQSAEEVSEEEMSEDEERENENHLLVVPESRFDRDSGESEEAEEEVGEGTPQSSALT
EGDYVPDSPALLPIELKQELPKYLPALQGCRSVEEFQCLNRIEEGTYGVVYRAKDKKTDEIVAL
KRLKMEKEKEGFPITSLREINTILKAQHPNIVTVREIVVGSNMDKIYIVMNYVEHDLKSLMETM
KQPFLPGEVKTLMIQLLRGVKHLHDNWILHRDLKTSNLLLSHAGILKVGDFGLAREYGSPLKAY
TPVVVTQWYRAPELLLGAKEYSTAVDMWSVGCIFGELLTQKPLFPGNSEIDQINKVFKELGTPS
EKIWPGYSELPVVKKMTFSEHPYNNLRKRFGALLSDQGFDLMNKFLTYFPGRRISAEDGLKHEY
FRETPLPIDPSMFPTWPAKSEQQRVKRGTSPRPPEGGLGYSQLGDDDLKETGFHLTTTNQGASA
AGPGFSLKF

FIG. 3A

**Human CSNK1A1 Protein:**

MASSSGSKAEFIVGGKYKLVRKIGSGSFGDIYLAINITNGEEVAVKLESQKARHPQLLYESKLY
KILQGGVGIPHIRWYGQEKDYNVLVMDLLGPSLEDLFNFCSRRFTMKTVLMLADQMISRIEYVH
TKNFIHRDIKPDNFLMGIGRHCNKLFLIDFGLAKKYRDNRTRQHIPYREDKNLTGTARYASINA
HLGIEQSRRDDMESLGYVLMYFNRTSLPWQGLKAATKKQKYEKISEKKMSTPVEVLCKGFPAEF
AMYLNYCRGLRFEEAPDYMYLRQLFRILFRTLNHQYDYTFDWTMLKQKAAQQAASSSGQGQQAQ
TPTGKQTDKTKSNMKGF

FIG. 3B

**Human GYK Protein:**

MAASKKAVLGPLVGAVDQGTSSTRFLVFNSKTAELLSHHQVEIKQEFPREGWVEQDPKEILHSV
YECIEKTCEKLGQLNIDISNIKAIGVSNQRETTVVWDKITGEPLYNAVVWLDLRTQSTVESLSK
RIPGNNNFVKSKTGLPLSTYFSAVKLRWLLDNVRKVQKAVEEKRALFGTIDSWLIWSLTGGVNG
GVHCTDVTNASRTMLFNIHSLEWDKQLCEFFGIPMEILPNVRSSSEIYGLMKISHSVKAGALEG
VPISGCLGDQSAALVGQMCFQIGQAKNTYGTGCFLLCNTGHKCVFSDHGLLTTVAYKLGRDKPV
YYALEGSVAIAGAVIRWLRDNLGIIKTSEEIEKLAKEVGTSYGCYFVPAFSGLYAPYWEPSARG
IICGLTQFTNKCHIAFAALEAVCFQTREILDAMNRDCGIPLSHLQVDGGMTSNKILMQLQADIL
YIPVVKPSMPETTALGAAMAAGAAEGVGVWSLEPEDLSAVTMERFEPQINAEESEIRYSTWKKA
VMKSMGWVTTQSPESGIP

***FIG. 3C***

**Human NEK1 Protein:**

MEKYVRLQKIGEGSFGKAILVKSTEDGRQYVIKEINISRMSSKEREESRREVAVLANMKHPNIV
QYRESFEENGSLYIVMDYCEGGDLFKRINAQKGVLFQEDQILDWFVQICLALKHVHDRKILHRD
IKSQNIFLTKDGTVQLGDFGIARVLNSTVELARTCIGTPYYLSPEICENKPYNNKSDIWALGCV
LYELCTLKHAFEAGSMKNLVLKIISGSFPPVSLHYSYDLRSLVSQLFKRNPRDRPSVNSILEKG
FIAKRIEKFLSPQLIAEEFCLKTFSKFGSQPIPAKRPASGQNSISVMPAQKITKPAAKYGIPLA
YKKYGDKKLHEKKPLQKHKQAHQTPEKRVNTGEERRKISEEAARKRRLEFIEKEKKQKDQIISL
MKAEQMKRQEKERLERINRAREQGWRNVLSAGGSGEVKAPFLGSGGTIAPSSFSSRGQYEHYHA
IFDQMQQQRAEDNEAKWKREIYGRGLPERGILPGVRPGFPYGAAGHHHFPDADDIRKTLKRLKA
VSKQANANRQKGQLAVERAKQVEEFLQRKREAMQNKARAEGHMVYLARLRQIRLQNFNERQQIK
AKLRGEKKEANHSEGQEGSEEADMRRKKIESLKAHANARAAVLKEQLERKRKEAYEREKKVWEE
HLVAKGVKSSDVSPPLGQHETGGSPSKQQMRSVISVTSALKEVGVDSSLTDTRETSEEMQKTNN
AISSKREILRRLNENLKAQEDEKGKQNLSDTFEINVHEDAKEHEKEKSVSSDRKKWEAGGQLVI
PLDELTLDTSFSTTERHTVGEVIKLGPNGSPRRAWGKSPTDSVLKILGEAELQLQTELLENTTI
RSEISPEGEKYKPLITGEKKVQCISHEINPSAIVDSPVETKSPEFSEASPQMSLKLEGNLEEPD
DLETEILQEPSGTNKDESLPCTITDVWISEEKETKETQSADRITIQENEVSEDGVSSTVDQLSD
IHIEPGTNDSQHSKCDVDKSVQPEPFFHKVVHSEHLNLVPQVQSVQCSPEESFAFRSHSHLPPK
NKNKNSLLIGLSTGLFDANNPKMLRTCSLPDLSKLFRTLMDVPTVGDVRQDNLEIDEIEDENIK
EGPSDSEDIVFEETDTDLQELQASMEQLLREQPGEEYSEEEESVLKNSDVEPTANGTDVADEDD
NPSSESALNEEWHSDNSDGEIASECECDSVFNHLEELRLHLEQEMGFEKFFEVYEKIKAIHEDE
DENIEICSKIVQNILGNEHQHLYAKILHLVMADGAYQEDNDE

***FIG. 3D***

**Human PLK1 Protein:**

MSAAVTAGKLARAPADPGKAGVPGVAAPGAPAAAPPAKEIPEVLVDPRSRRRYVRGRFLGKGGF
AKCFEISDADTKEVFAGKIVPKSLLLKPHQREKMSMEISIHRSLAHQHVVGFHGFFEDNDFVFV
VLELCRRRSLLELHKRRKALTEPEARYYLRQIVLGCQYLHRNRVIHRDLKLGNLFLNEDLEVKI
GDFGLATKVEYDGERKKTLCGTPNYIAPEVLSKKGHSFEVDVWSIGCIMYTLLVGKPPFETSCL
KETYLRIKKNEYSIPKHINPVAASLIQKMLQTDPTARPTINELLNDEFFTSGYIPARLPITCLT
IPPRFSIAPSSLDPSNRKPLTVLNKGLENPLPERPREKEEPVVRETGEVVDCHLSDMLQQLHSV
NASKPSERGLVRQEEAEDPACIPIFWVSKWVDYSDKYGLGYQLCDNSVGVLFNDSTRLILYNDG
DSLQYIERDGTESYLTVSSHPNSLMKKITLLKYFRNYMSEHLLKAGANITPREGDELARLPYLR
TWFRTRSAIILHLSNGSVQINFFQDHTKLILCPLMAAVTYIDEKRDFRTYRLSLLEEYGCCKEL
ASRLRYARTMVDKLLSSRSASNRLKAS

***FIG. 3E***

### Human PRKAR1A Protein:

MESGSTAASEEARSLRECELYVQKHNIQALLKDSIVQLCTARPERPMAFLREYFERLEKEEAKQ
IQNLQKAGTRTDSREDEISPPPPNPVVKGRRRRGAISAEVYTEEDAASYVRKVIPKDYKTMAAL
AKAIEKNVLFSHLDDNERSDIFDAMFSVSFIAGETVIQQGDEGDNFYVIDQGETDVYVNNEWAT
SVGEGGSFGELALIYGTPRAATVKAKTNVKLWGIDRDSYRRILMGSTLRKRKMYEEFLSKVSIL
ESLDKWERLTVADALEPVQFEDGQKIVVQGEPGDEFFIILEGSAAVLQRRSENEEFVEVGRLGP
SDYFGEIALLMNRPRAATVVARGPLKCVKLDRPRFERVLGPCSDILKRNIQQYNSFVSLSV

*FIG. 3F*

### Human PRKRA Protein:

MSQSRHRAEAPPLEREDSGTFSLGKMITAKPGKTPIQVLHEYGMKTKNIPVYECERSDVQIHVP
TFTFRVTVGDITCTGEGTSKKLAKHRAAEAAINILKANASICFAVPDPLMPDPSKQPKNQLNPI
GSLQELAIHHGWRLPEYTLSQEGGPAHKREYTTICRLESFMETGKGASKKQAKRNAAEKFLAKF
SNISPENHISLTNVVGHSLGCTWHSLRNSPGEKINLLKRSLLSIPNTDYIQLLSEIAKEQGFNI
TYLDIDELSANGQYQCLAELSTSPITVCHGSGISCGNAQSDAAHNALQYLKIIAERK

*FIG. 3G*

### Human TTBK2 Protein:

MQCLAAALKDETNMSGGGEQADILPANYVVKDRWKVLKKIGGGGFGEIYEAMDLLTRENVALKV
ESAQQPKQVLKMEVAVLKKLQGKDHVCRFIGCGRNEKFNYVVMQLQGRNLADLRRSQPRGTFTL
STTLRLGKQILESIEAIHSVGFLHRDIKPSNFAMGRLPSTYRKCYMLDFGLARQYTNTTGDVRP
PRNVAGFRGTVRYASVNAHKNREMGRHDDLWSLFYMLVEFAVGQLPWRKIKDKEQVGMIKEKYE
HRMLLKHMPSEFHLFLDHIASLDYFTKPDYQLIMSVFENSMKERGIAENEAFDWEKAGTDALLS
TSTSTPPQQNTRQTAAMFGVVNVTPVPGDLLRENTEDVLQGEHLSDQENAPPILPGRPSEGLGP
SPHLVPHPGGPEAEVWEETDVNRNKLRINIGKSPCVEEEQSRGMGVPSSPVRAPPDSPTTPVRS
LRYRRVNSPESERLSTADGRVELPERRSRMDLPGSPSRQACSSQPAQMLSVDTGHADRQASGRM
DVSASVEQEALSNAFRSVPLAEEEDFDSKEWVIIDKETELKDFPPGAEPSTSGTTDEEPEELRP
LPEEGEERRRLGAEPTVRPRGRSMQALAEEDLQHLPPQPLPPQLSQGDGRSETSQPPTPGSPSH
SPLHSGPRPRRRESDPTGPQRQVFSVAPPFEVNGLPRAVPLSLPYQDFKRDLSDYRERARLLNR
VRRVGFSHMLLTTPQVPLAPVQPQANGKEEEEEEEEDEEEEEEDEEEEEEEEEEEEEEEEEEE
EEEAAAAVALGEVLGPRSGSSSEGSERSTDRSQEGAPSTLLADDQKESRGRASMADGDLEPEEG
SKTLVLVSPGDMKKSPVTAELAPDPDLGTLAALTPQHERPQPTGSQLDVSEPGTLSSVLKSEPK
PPGPGAGLGAGTVTTGVGGVAVTSSPFTKVERTFVHIAEKTHLNVMSSGGQALRSEEFSAGGEL
GLELASDGGAVEEGARAPLENGLALSGLNGAEIEGSALSGAPRETPSEMATNSLPNGPALADGP
APVSPLEPSPEKVATISPRRHAMPGSRPRSRIPVLLSEEDTGSEPSGSLSAKERWSKRARPQQD
LARLVMEKRQGRLLLRLASGASSSSSEEQRRASETLSGTGSEEDTPASEPAAALPRKSGRAAAT
RSRIPRPIGLRMPMPVAAQQPASRSHGAAPALDTAITSRLQLQTPPGSATAADLRPKQPPGRGL
GPGRAQAGARPPAPRSPRLPASTSAARNASASPRSQSLSRRESPSPSHQARPGVPPPRGVPPAR
AQPDGTPSPGGSKKGPRGKLQAQRATTKGRAGGAEGRAGAR

*FIG. 3H*

**Human TTK Protein:**

MESEDLSGRELTIDSIMNKVRDIKNKFKNEDLTDELSLNKISADTTDNSGTVNQIMMMANNPED
WLSLLLKLEKNSVPLSDALLNKLIGRYSQAIEALPPDKYGQNESFARIQVRFAELKAIQEPDDA
RDYFQMARANCKKFAFVHISFAQFELSQGNVKKSKQLLQKAVERGAVPLEMLEIALRNLNLQKK
QLLSEEEKKNLSASTVLTAQESFSGSLGHLQNRNNSCDSRGQTTKARFLYGENMPPQDAEIGYR
NSLRQTNKTKQSCPFGRVPVNLLNSPDCDVKTDDSVVPCFMKRQTSRSECRDLVVPGSKPSGND
SCELRNLKSVQNSHFKEPLVSDEKSSELIITDSITLKNKTESSLLAKLEETKEYQEPEVPESNQ
KQWQSKRKSECINQNPAASSNHWQIPELARKVNTEQKHTTFEQPVFSVSKQSPPISTSKWFDPK
SICKTPSSNTLDDYMSCFRTPVVKNDFPPACQLSTPYGQPACFQQQQHQILATPLQNLQVLASS
SANECISVKGRIYSILKQIGSGGSSKVFQVLNEKKQIYAIKYVNLEEADNQTLDSYRNEIAYLN
KLQQHSDKIIRLYDYEITDQYIYMVMECGNIDLNSWLKKKKSIDPWERKSYWKNMLEAVHTIHQ
HGIVHSDLKPANFLIVDGMLKLIDFGIANQMQPDTTSVVKDSQVGTVNYMPPEAIKDMSSSREN
GKSKSKISPKSDVWSLGCILYYMTYGKTPFQQIINQISKLHAIIDPNHEIEFPDIPEKDLQDVL
KCCLKRDPKQRISIPELLAHPYVQIQTHPVNQMAKGTTEEMKYVLGQLVGLNSPNSILKAAKTL
YEHYSGGESHNSSSSKTFEKKRGKK

*FIG. 3I*

## Human CDC2L1 DNA:

```
1     atgggtgatg aaaaggactc ttggaaagtg aaaactttag atgaaattct tcaggaaaag
61    aaacgaagga aggaacaaga ggagaaagca gagataaaac gcttaaaaaa ttctgatgac
121   cgggattcca agcgggattc ccttgaggag ggggagctga gagatcactg catggagatc
181   acaataagga actccccgta tagaagagaa gactcaatgg aagacagagg agaagaagat
241   gattctttgg ccatcaaacc accccagcaa atgtcttgga aagaaaaagt tcatcacaga
301   aaagatgaaa agaggaaaga aaaatgtagg catcatagcc attcagcaga agggggggaag
361   catgctagag tgaaagaaag agagcacgaa cgtcggaaac gacatcgaga agaacaggat
421   aaagctcgcc gggaatggga aagacagaag agaagggaaa tggcaaggga gcattccagg
481   agagaaaggg accgcttgga gcagttagaa aggaagcggg agcgggagcg caagatgcgg
541   gagcagcaga aggagcagcg ggagcagaag gagcgcgagc ggcgggcgga ggagcggcgc
601   aaggagcggg aggcccgcag ggaagtgtct gcacatcacc gaacgatgag agaggactac
661   agcgacaaag tgaaagccag ccactggagt cgcagcccgc ctcggccgcc gcgggagcgg
721   ttcgagttgg gagacggccg gaagccagta aaagaagaga aaatggaaga aagggacctg
781   ctgtccgact tacaggacat cagcgacagc gagaggaaga ccagctcggc cgagtcctcg
841   tcagcagaat caggctcagg ttctgaggaa gaagaggagg aggaggaaga ggaggaggag
901   gaagggagca ccagtgaaga atcagaggag gaagaggaag aggaggagga ggagaccggc
961   agcaactctg aggaggcatc agagcagtct gccgaagaag taagtgagga agaaatgagt
1021  gaagatgaag aacgagaaaa tgaaaaccac ctcttggttg ttccagagtc acggttcgac
1081  cgagattccg gggagagtga agaagcagag gaagaagtgg gtgagggaac gccgcagagc
1141  agcgccctga cagagggcga ctatgtgccc gactcccctg ccctgttgcc catcgagctc
1201  aagcaggagc tgcccaagta cctgccggcc ctgcagggct gccggagcgt cgaggagttc
1261  cagtgcctga acaggatcga ggagggcacc tatggagtgg tctacagagc aaaagacaag
1321  aaaacagatg aaattgtggc tctaaagcgg ctgaagatgg agaaggagaa ggagggcttc
1381  ccgatcacgt ccctgaggga gatcaacacc atcctcaagg cccagcatcc caacattgtc
1441  accgttagag agattgtggt gggcagcaac atggacaaga tctacatcgt gatgaactat
1501  gtggagcacg acctcaagag cctgatggag accatgaaac agcccttcct gccaggggag
1561  gtgaagaccc tgatgatcca gctgctgcgt ggggtgaaac acctgcacga caactggatc
1621  ctgcaccgtg acctcaagac gtccaacctg ctgctgagcc acgccggcat cctcaaggtg
1681  ggtgattttg ggctggcgcg ggagtacgga tcccctctga aggcctacac cccggtcgtg
1741  gtgacccagt ggtaccgcgc cccagagctg ctgcttggtg ccaaggaata ctccacggcc
1801  gtggacatgt ggtcagtggg ctgcatcttc ggggagctgc tgactcagaa gcctctgttc
1861  cccgggaatt cggaaatcga tcagatcaac aaagtgttca aggagctggg gacccccagt
1921  gagaaaatct ggcccggcta cagtgagctc ccagtagtca agaagatgac cttcagcgag
1981  caccccctaca acaacctccg caagcgcttc ggggctctgc tctcagacca gggcttcgac
2041  ctcatgaaca agttcctgac ctacttcccc gggaggagga tcagcgctga ggacggcctc
2101  aagcatgagt atttccgcga gacccccctc cccatcgacc cctccatgtt ccccacgtgg
2161  cccgccaaga gcgagcagca gcgtgtgaag cggggcacca gcccgaggcc ccctgaggga
2221  ggcctgggct acagccagct gggtgacgac gacctgaagg agacgggctt ccaccttacc
2281  accacgaacc agggggcctc tgccgcgggc cccggcttca gcctcaagtt ctga
```

*FIG. 4A*

**Human CSNK1A1 DNA Sequence:**

```
1    atggcgagta gcagcggctc caaggctgaa ttcattgtcg gagggaaata taaactggta
61   cggaagatcg ggtctggctc cttcggggac atctatttgg cgatcaacat caccaacggc
121  gaggaagtgg cagtgaagct agaatctcag aaggccaggc atccccagtt gctgtacgag
181  agcaagctct ataagattct tcaaggtggg gttggcatcc cccacatacg gtggtatggt
241  caggaaaaag actacaatgt actagtcatg gatcttctgg gacctagcct cgaagacctc
301  ttcaatttct gttcaagaag gttcacaatg aaaactgtac ttatgttagc tgaccagatg
361  atcagtagaa ttgaatatgt gcatacaaag aattttatac acagagacat taaaccagat
421  aacttcctaa tgggtattgg gcgtcactgt aataagttat tccttattga ttttggtttg
481  gccaaaaagt acagagacaa caggacaagg caacacatac catacagaga agataaaaac
541  ctcactggca ctgcccgata tgctagcatc aatgcacatc ttggtattga gcagagtcgc
601  cgagatgaca tggaatcatt aggatatgtt ttgatgtatt ttaatagaac cagcctgcca
661  tggcaagggc taaaggctgc aacaaagaaa caaaaatatg aaaagattag tgaaaagaag
721  atgtccacgc ctgttgaagt tttatgtaag ggtttcctg cagaatttgc gatgtactta
781  aactattgtc gtgggctacg ctttgaggaa gccccagatt acatgtatct gaggcagcta
841  ttccgcattc ttttcaggac cctgaaccat caatatgact acacatttga ttggacaatg
901  ttaaagcaga aagcagcaca gcaggcagcc tcttccagtg ggcagggtca gcaggcccaa
961  acccccacag gcaagcaaac tgacaaaacc aagagtaaca tgaaaggttt ctaa
```

*FIG. 4B*

**Human GYK DNA:**

```
1    atggcagcct caaagaaggc agttttgggg ccattggtgg gggcggtgga ccagggcacc
61   agttcgacgc gcttttttggt tttcaattca aaaacagctg aactacttag tcatcatcaa
121  gtagaaataa aacaagagtt cccaagagaa ggatgggtgg aacaggaccc taaggaaatt
181  ctacattctg tctatgagtg tatagagaaa acatgtgaga aacttggaca gctcaatatt
241  gatatttcca acataaaagc tattggtgtc agcaaccaga gggaaaccac tgtagtctgg
301  gacaagataa ctggagagcc tctctacaat gctgtggtgt ggcttgatct aagaacccag
361  tctaccgttg agagtcttag taaaagaatt ccaggaaata ataactttgt caagtccaag
421  acaggccttc cacttagcac ttacttcagt gcagtgaaac ttcgttggct ccttgacaat
481  gtgagaaaag ttcaaaaggc cgttgaagaa aaacgagctc tttttgggac tattgattca
541  tggcttattt ggagtttgac aggaggagtc aatggaggtg tccactgtac agatgtaaca
601  aatgcaagta ggactatgct tttcaacatt cattctttgg aatgggataa acaactctgc
661  gaatttttttg gaattccaat ggaaattctt ccaaatgtcc ggagttcttc tgagatctat
721  ggcctaatga aaatctctca tagcgtgaaa gctggggcct tggaaggtgt gccaatatct
781  gggtgtttag gggaccagtc tgctgcattg gtgggacaaa tgtgcttcca gattggacaa
841  gccaaaaata cgtatggaac aggatgtttc ttactatgta atacaggcca taagtgtgta
901  ttttctgatc atggccttct caccacagtg gcttacaaac ttggcagaga caaaccagta
961  tattatgctt tggaaggttc tgtagctata gctggtgctg ttattcgctg gctaagagac
1021 aatcttggaa ttataaagac ctcagaagaa attgaaaaac ttgctaaaga agtaggtact
1081 tcttatggct gctacttcgt cccagcattt tcggggttat atgcacctta ttgggagccc
1141 agcgcaagag ggataatctg tggactcact cagttcacca ataaatgcca tattgctttt
1201 gctgcattag aagctgtttg tttccaaact cgagagattt tggatgccat gaatcgagac
1261 tgtggaattc cactcagtca tttgcaggta gatggaggaa tgaccagcaa caaaattctt
1321 atgcagctac aagcagacat tctgtatata ccagtagtga agccctcaat gcccgaaacc
1381 actgcactgg gtgcggctat ggcggcaggg gctgcagaag gagtcggcgt atggagtctc
1441 gaacccgagg atttgtctgc cgtcacgatg gagcggtttg aacctcagat taatgcggag
1501 gaaagtgaaa ttcgttattc tacatggaag aaagctgtga tgaagtcaat gggttgggtt
1561 acaactcaat ctccagaaag tggtattcca taa
```

*FIG. 4C*

## Human NEK1 DNA:

```
1     atggagaagt atgttagact acagaagatt ggagaaggtt catttggaaa agccattctt
61    gttaaatcta cagaagatgg cagacagtat gttatcaagg aaattaacat ctcaagaatg
121   tccagtaaag aaagagaaga atcaaggaga gaagttgcag tattggcaaa catgaagcat
181   ccaaatattg tccagtatag agaatcattt gaagaaaatg gctctctcta catagtaatg
241   gattactgtg agggagggga tctgtttaag cgaataaatg ctcagaaagg cgtttttgttt
301   caagaggatc agatttgga ctggtttgta cagatatgtt tggccctgaa acatgtacat
361   gatagaaaaa ttcttcatcg agacattaaa tctcagaaca tatttttaac taaagatgga
421   acagtacaac ttggagattt tggaattgct agagttctta atagtactgt agagctggct
481   cgaacttgca tagggacccc atactacttg tcacctgaaa tctgtgaaaa caaaccttac
541   aataataaaa gtgacatttg ggctctgggg tgtgtccttt atgagctgtg tacacttaaa
601   catgcttttg aagctggcag tatgaaaaac ctggtactga agataatatc tggatctttt
661   ccacctgtgt ctttgcatta ttcctatgat ctccgcagtt tggtgtctca gttatttaaa
721   agaaatccta gggatagacc atcagtcaac tccatattgg agaaaggttt tatagccaaa
781   cgcattgaaa agtttctctc tcctcagctt attgcagaag aattttgtct aaaaacattt
841   tcgaagtttg gatcacagcc tataccagct aaaagaccag cttcaggaca aaactcgatt
901   tctgttatgc ctgctcagaa aattacaaag cctgccgcta aatatggaat acctttagca
961   tataagaaat atggagataa aaaattacac gaaaagaaac cactgcaaaa acataaacag
1021  gcccatcaaa ctccagagaa gagagtgaat actggagaag aaaggaggaa aatatctgag
1081  gaagcagcaa gaaagagaag gctggaattt attgaaaaag aaaagaaaca aaaggatcag
1141  attattagtt taatgaaggc tgaacaaatg aaaaggcaag aaaaggaaag gttggaaaga
1201  ataaataggg ccagggaaca aggatggaga aatgtgctaa gtgctggtgg aagtggtgaa
1261  gtaaaggctc cttttctggg cagtggaggg actatagctc catcatcttt ttcttctcga
1321  ggacagtatg aacattacca tgccattttt gaccaaatgc agcaacaaag agcagaagat
1381  aatgaagcta aatggaaaag agaaatatat ggtcgaggtc ttccagaaag aggaattctg
1441  cctggagttc gtccaggatt tccttatggg gctgcaggtc atcaccattt tcctgatgct
1501  gatgatatta gaaaaacttt gaaaagattg aaggcggtgt ctaaacaagc caatgcaaac
1561  aggcaaaaag ggcagctagc tgtagaaaga gctaaacaag tagaagagtt cctgcagcga
1621  aaacgggaag ctatgcagaa taaagctcga gccgaaggac atatggttta tctggcaaga
1681  ctgaggcaaa taagactaca gaatttcaat gagcgccaac agattaaagc caaacttcgt
1741  ggtgaaaaga aagaagctaa tcattctgaa ggacaagaag gaagtgaaga ggctgacatg
1801  aggcgcaaaa aaatcgaatc actgaaggcc catgcaaatg cacgtgctgc tgtactaaaa
1861  gaacaactag aacgaaagag aaaggaggct tatgagagag aaaaaaaagt gtgggaagag
1921  catttggtgg ctaaaggagt taagagttct gatgtttctc caccttttggg acagcatgaa
1981  acaggtggct ctccatcaaa gcaacagatg agatctgtta tttctgtaac ttcagctttg
2041  aaagaagttg gcgtggacag tagtttaact gatacccggg aaacttcaga agagatgcaa
2101  aagaccaaca atgctatttc aagtaagcga gaaatacttc gtagattaaa tgaaaatctt
2161  aaagctcaag aagatgaaaa aggaaagcag aatctctctg atactttga gataaatgtt
2221  catgaagatg ccaaagagca tgaaaaagaa aaatcagttt catctgatcg caagaagtgg
2281  gaggcaggag gtcaacttgt gattcctctg gatgagttaa cactagatac atccttctct
2341  acaactgaaa gacatacagt gggagaagtt attaaattag gtcctaatgg atctccaaga
2401  agagcctggg ggaaaagtcc gacagattct gttctaaaga tacttggaga agctgaacta
2461  caacttcaga cagaactatt agaaaataca actattagaa gtgagatttc tcccgaaggg
2521  gaaaagtaca aacccttaat tactggagaa aaaaaagtac aatgtatttc acatgaaata
2581  aacccatcag ctattgttga ttctcctgtt gagacaaaaa gtcccgagtt cagtgaggca
2641  tctccacaga tgtcattgaa actggaagga aatttagaag aacctgatga tttggaaaca
2701  gaaattctac aagagccaag tggaacaaac aaagatgaga gcttgccatg cactattact
2761  gatgtgtgga ttagtgagga aaaagaaaca aaggaaactc agtcggcaga taggatcacc
2821  attcaggaaa atgaagtttc tgaagatgga gtctcgagta ctgtggacca acttagtgac
```

*FIG. 4D-1*

2881 attcatatag agcctggaac caatgattct cagcactcta aatgtgatgt agataagtct
2941 gtgcaaccgg aaccattttt ccataaggtg gttcattctg aacacttgaa cttagtccct
3001 caagttcaat cagttcagtg ttcaccagaa gaatcctttg catttcgatc tcactcgcat
3061 ttaccaccaa aaaataaaaa caagaattcc ttgctgattg gactttcaac tggtctgttt
3121 gatgcaaaca acccaaagat gttaaggaca tgttcacttc cagatctctc aaagctgttc
3181 agaaccctta tggatgttcc caccgtagga gatgttcgtc aagacaatct tgaaatagat
3241 gaaattgaag atgaaaacat taaagaagga ccttctgatt ctgaagacat tgtgtttgaa
3301 gaaactgaca cagatttaca agagctgcag gcctcgatgg aacagttact tagggaacaa
3361 cctggtgaag aatacagtga agaagaagag tcagtcttga agaacagtga tgtggagcca
3421 actgcaaatg ggacagatgt ggcagatgaa gatgacaatc ccagcagtga aagtgccctg
3481 aacgaagaat ggcactcaga taacagtgat ggtgaaattg ctagtgaatg tgaatgcgat
3541 agtgtcttta accatttaga ggaactgaga cttcatctgg agcaggaaat gggctttgaa
3601 aaattctttg aggtttatga gaaaataaag gctattcatg aagatgaaga tgaaaatatt
3661 gaaatttgtt caaaaatagt tcaaaatatt ttgggaaatg aacatcagca tctttatgcc
3721 aagattcttc atttagtcat ggcagatgga gcctaccaag aagataatga tgaataa

FIG. 4D-2

## Human PLK1 DNA:

1 atgagtgctg cagtgactgc agggaagctg gcacgggcac cggccgaccc tgggaaagcc
61 ggggtccccg gagttgcagc tcccggagct ccggcggcgg ctccaccggc gaaagagatc
121 ccggaggtcc tagtggaccc acgcagccgg cggcgctatg tgcgggggcg ctttttgggc
181 aagggcggct ttgccaagtg cttcgagatc tcggacgcgg acaccaagga ggtgttcgcg
241 ggcaagattg tgcctaagtc tctgctgctc aagccgcacc agagggagaa gatgtccatg
301 gaaatatcca ttcaccgcag cctcgcccac cagcacgtcg taggattcca cggctttttc
361 gaggacaacg acttcgtgtt cgtggtgttg gagctctgcc gccggaggtc tctcctggag
421 ctgcacaaga ggaggaaagc cctgactgag cctgaggccc gatactacct acggcaaatt
481 gtgcttggct gccagtacct gcaccgaaac cgagttattc atcgagacct caagctgggc
541 aacctttttcc tgaatgaaga tctggaggtg aaaataggga attttggact ggcaaccaaa
601 gtcgaatatg acggggagag gaagaagacc ctgtgtggga ctcctaatta catagctccc
661 gaggtgctga gcaagaaagg gcacagtttc gaggtggatg tgtggtccat tgggtgtatc
721 atgtatacct tgttagtggg caaaccacct tttgagactt cttgcctaaa agagacctac
781 ctccggatca agaagaatga atacagtatt cccaagcaca tcaaccccgt ggccgcctcc
841 ctcatccaga agatgcttca gacagatccc actgcccgcc caaccattaa cgagctgctt
901 aatgacgagt tctttacttc tggctatatc cctgcccgtc tccccatcac ctgcctgacc
961 attccaccaa ggttttcgat tgctcccagc agcctggacc ccagcaaccg gaagcccctc
1021 acagtcctca ataaaggctt ggagaacccc ctgcctgagc gtccccggga aaaagaagaa
1081 ccagtggttc gagagacagg tgaggtggtc gactgccacc tcagtgacat gctgcagcag
1141 ctgcacagtg tcaatgcctc caagccctcg gagcgtgggc tggtcaggca agaggaggct
1201 gaggatcctg cctgcatccc catcttctgg gtcagcaagt gggtggacta ttcggacaag
1261 tacggccttg ggtatcagct ctgtgataac agcgtggggg tgctcttcaa tgactcaaca
1321 cgcctcatcc tctacaatga tggtgacagc ctgcagtaca tagagcgtga cggcactgag
1381 tcctacctca ccgtgagttc ccatcccaac tccttgatga agaagatcac cctccttaaa
1441 tatttccgca attacatgag cgagcacttg ctgaaggcag gtgccaacat cacgccgcgc
1501 gaaggtgatg agctcgcccg gctgccctac ctacggacct ggttccgcac ccgcagcgcc
1561 atcatcctgc acctcagcaa cggcagcgtg cagatcaact tcttccagga tcacaccaag
1621 ctcatcttgt gcccactgat ggcagccgtg acctacatcg acgagaagcg ggacttccgc
1681 acataccgcc tgagtctcct ggaggagtac ggctgctgca aggagctggc cagccggctc
1741 cgctacgccc gcactatggt ggacaagctg ctgagctcac gctcggccag caaccgtctc
1801 aaggcctcct aa

FIG. 4E

## Human PRKAR1A DNA:

```
1     atggagtctg gcagtaccgc cgccagtgag gaggcacgca gccttcgaga atgtgagctc
61    tacgtccaga agcataacat tcaagcgctg ctcaaagatt ctattgtgca gttgtgcact
121   gctcgacctg agagacccat ggcattcctc agggaatact ttgagaggtt ggagaaggag
181   gaggcaaaac agattcagaa tctgcagaaa gcaggcactc gtacagactc aagggaggat
241   gagatttctc ctcctccacc caacccagtg gttaaaggta ggaggcgacg aggtgctatc
301   agcgctgagg tctacacgga ggaagatgcg gcatcctatg ttagaaaggt tataccaaaa
361   gattacaaga caatggccgc tttagccaaa gccattgaaa agaatgtgct gttttcacat
421   cttgatgata atgagagaag tgatattttt gatgccatgt tttcggtctc ctttatcgca
481   ggagagactg tgattcagca aggtgatgaa ggggataact tctatgtgat tgatcaagga
541   gagacggatg tctatgttaa caatgaatgg gcaaccagtg ttggggaagg agggagcttt
601   ggagaacttg ctttgattta tggaacaccg agagcagcca ctgtcaaagc aaagacaaat
661   gtgaaattgt ggggcatcga ccgagacagc tatagaagaa tcctcatggg aagcacactg
721   agaaagcgga agatgtatga ggaattcctt agtaaagtct ctattttaga gtctctggac
781   aagtgggaac gtcttacggt agctgatgca ttggaaccag tgcagtttga agatgggcag
841   aagattgtgg tgcagggaga accaggggat gagttcttca ttattttaga ggggtcagct
901   gctgtgctac aacgtcggtc agaaaatgaa gagtttgttg aagtgggaag attggggcct
961   tctgattatt ttggtgaaat tgcactactg atgaatcgtc ctcgtgctgc cacagttgtt
1021  gctcgtggcc ccttgaagtg cgttaagctg gaccgaccta gatttgaacg tgttcttggc
1081  ccatgctcag acatcctcaa acgaaacatc cagcagtaca acagttttgt gtcactgtct
1141  gtctga
```

FIG. 4F

## Human PRKRA DNA:

```
1     atgtcccaga gcaggcaccg cgccgaggcc ccgccgctgg agcgcgagga cagtgggacc
61    ttcagtttgg ggaagatgat aacagctaag ccagggaaaa caccgattca ggtattacac
121   gaatacggca tgaagaccaa gaacatccca gtttatgaat gtgaaagatc tgatgtgcaa
181   atacacgtgc ccactttcac cttcagagta accgttggtg acataacctg cacaggtgaa
241   ggtacaagta agaagctggc gaaacataga gctgcagagg ctgccataaa cattttgaaa
301   gccaatgcaa gtatttgctt tgcagttcct gacccettaa tgcctgaccc ttccaagcaa
361   ccaaagaacc agcttaatcc tattggttca ttacaggaat tggctattca tcatggctgg
421   agacttcctg aatataccct ttcccaggag ggaggacctg ctcataagag agaatatact
481   acaatttgca ggctagagtc atttatggaa actggaaagg gggcatcaaa aaagcaagcc
541   aaaaggaatg ctgctgagaa atttcttgcc aaatttagta atatttctcc agagaaccac
601   atttctttaa caaatgtagt aggacattct ttaggatgta cttggcattc cttgaggaat
661   tctcctggtg aaaagatcaa cttactgaaa agaagcctcc ttagtattcc aaatacagat
721   tacatccagc tgcttagtga aattgccaag gaacaaggtt ttaatataac atatttggat
781   atagatgaac tgagcgccaa tggacaatat caatgtcttg ctgaactgtc caccagcccc
841   atcacagtct gtcatggctc cggtatctcc tgtggcaatg cacaaagtga tgcagctcac
901   aatgctttgc agtatttaaa gataatagca gaaagaaagt aa
```

FIG. 4G

**Human TTBK2 DNA:**

```
1     atgcagtgcc tagcggccgc ccttaaggac gaaaccaaca tgagtggggg aggggagcag
61    gccgacatcc tgccggccaa ctacgtggtc aaggatcgct ggaaggtgct gaaaaagatc
121   gggggcgggg gctttggtga gatctacgag gccatggacc tgctgaccag ggagaatgtg
181   gccctcaagg tggagtcagc ccagcagccc aagcaggtcc tcaagatgga ggtggccgtg
241   ctcaagaagt tgcaagggaa ggaccatgtg tgcaggttca ttggctgtgg caggaacgag
301   aagtttaact atgtagtgat gcagctccag ggccggaacc tggccgacct gcgccgtagc
361   cagccgcgag gcaccttcac gctgagcacc acattgcggc tgggcaagca gatcttggag
421   tccatcgagg ccatccactc tgtgggcttc ctgcaccgtg acatcaagcc ttcaaacttt
481   gccatgggca ggctgccctc cacctacagg aagtgctata tgctggactt cgggctggcc
541   cggcagtaca ccaacaccac gggggatgtg cggccccctc ggaatgtggc cgggtttcga
601   ggaacggttc gctatgcctc agtcaatgcc cacaagaacc gggagatggg ccgccacgac
661   gacctgtggt ccctcttcta catgctggtg gagtttgcag tgggccagct gccctggagg
721   aagatcaagg acaaggaaca ggtagggatg atcaaggaga agtatgagca ccggatgctg
781   ctgaagcaca tgccgtcaga gttccacctc ttcctggacc acattgccag cctcgactac
841   ttcaccaagc ccgactacca gttgatcatg tcagtgtttg agaacagcat gaaggagagg
901   ggcattgccg agaatgaggc ctttgactgg gagaaggcag gcaccgatgc cctcctgtcc
961   acgagcacct ctaccccgcc ccagcagaac acccggcaga cggcagccat gtttggggtg
1021  gtcaatgtga cgccagtgcc tggggacctg ctccgggaga acaccgagga tgtgctacag
1081  ggagagcacc tgagtgacca ggagaatgca cccccaattc tgcccgggag gccctctgag
1141  gggctgggcc ccagtcccca ccttgtcccc caccccgggg gtcctgaggc tgaagtctgg
1201  gaggagacag atgtcaaccg gaacaaactc cggatcaaca tcggcaaaag cccctgtgtg
1261  gaggaggaac agagccgagg catgggggtc cccagctccc cagtgcgtgc ccccccagac
1321  tcccccacaa ccccagtccg ttctctgcgc taccggaggg tgaacagccc tgagtcagaa
1381  aggctgtcca cggcggacgg gcgagtggag ctacctgaga ggaggtcacg gatggatctg
1441  cctggctcgc cctcgcgcca ggcctgctcc tctcagccag cccagatgct gtcagtggac
1501  acaggccacg ctgaccgaca ggccagtggc cgcatggacg tgtcagcctc tgtggagcag
1561  gaggccctga gcaacgcctt ccgctcggtg ccgctggctg aggaggagga tttcgacagc
1621  aaagagtggg tcatcatcga caaggagacg gagctcaagg acttccctcc aggggctgag
1681  cccagcacat cgggcaccac ggatgaggag cccgaggagc tgcggccact gcccgaggag
1741  ggcgaagagc ggcggcggct gggggcagag cccaccgtcc ggcccggggg acgcagcatg
1801  caggcgctgg cggaggagga cctgcagcat ttgccgcccc agcccctgcc accccagctg
1861  agccagggcg atggccgttc cgagacgtca cagcccccca cgcctggcag cccttcccac
1921  tcacccctgc actcgggacc ccgccctcga cggagagagt cggaccccac aggcccacag
1981  agacaggtgt tctccgtggc gcccccattt gaggtgaatg gcctcccacg agctgtgcct
2041  ctgagtctgc cctaccagga cttcaaaaga gacctctccg attaccgaga acgggcgcgg
2101  ttgctcaaca gggtccggag ggtgggcttc tcgcacatgc tgctcaccac cccccaggtc
2161  ccactggctc ctgttcagcc tcaggctaat gggaaggagg aagaggagga ggaggaggaa
2221  gatgaggaag aggaagaaga ggatgaggaa gaagaagagg aggaagagga agaggaggag
2281  gaagaagagg aggaggagga agaggaggag gaggctgcag cggcagttgc cttgggggag
2341  gtgctggggc ctcgtagtgg ctccagcagt gaggggagtg agaggagcac tgaccggagc
2401  caggagggtg ccccgtccac gctgctggca gacgatcaga aggagtccag ggccgggcc
2461  tccatggccg atggggacct ggagcctgag gagggctcca aaacgctggt gcttgtctct
2521  cctggcgaca tgaagaagtc gcccgtcact gccgaactgg cccccgaccc cgacctgggc
2581  accctggctg ccctcactcc tcagcatgag cggccccagc ccacgggcag ccagctggac
2641  gtatctgagc caggcaccct gtcctctgtc ctcaagtctg agcccaagcc cccgggcct
2701  ggggcagggc tgggggccgg gacagtgacc acaggggtcg gggcgtggc agtcacctcc
2761  tcacccttca ccaaagttga gaggaccttt gtgcacattg cggagaaaac ccacctcaac
```

*FIG. 4H-1*

2821 gtcatgtctt ccggtggaca agccttgcgg tctgaggagt tcagcgctgg gggcgagctg
2881 ggtctggagc tggcctctga tgggggcgct gtggaggagg gggcccgagc gcccctggag
2941 aacggcctcg ccctgtcagg gctgaatggg gctgagatag agggctctgc cctgtctggg
3001 gccccccggg aaacccccctc agagatggcc acaaactcac tgcccaatgg cccggccctt
3061 gcagacgggc cagccccggt gtccccgctg gagccaagcc ctgagaaagt ggccaccatc
3121 tcccccagac gccatgctat gccaggctct cgccccagga gccgtatccc tgtcctgctc
3181 tctgaggagg acacgggctc ggagccctca ggctcactgt cggccaaaga gcggtggagc
3241 aagcgggctc ggccgcagca ggacctggcg cggctggtga tggagaagag gcagggccgc
3301 ctgctgttgc ggctggcctc aggggcctcg tcctcctcca gtgaggagca gcgccgtgcc
3361 tctgagaccc tctcaggcac gggctctgag gaggacacgc ccgcctctga gccggcagcg
3421 gccttgccca ggaagagcgg gagggcagcc gccaccagga gccggattcc ccgccccatt
3481 ggcctccgca tgcccatgcc tgttgcagcc cagcagcccg ccagcagatc ccatggcgcg
3541 gccccagcat tggacacagc catcaccagc aggctccagc tgcagacgcc cccagggtcg
3601 gccactgctg ctgacctccg ccccaaacaa cctcctggcc gcggcctggg cccagggcga
3661 gcccaagccg gagccaggcc cccagcgccg cgcagcccgc gcctccccgc gtccacatcc
3721 gccgcgcgca atgccagcgc gtccccccgg agccagtccc tgtcccgcag agagagcccc
3781 tccccctcgc accaggcccg gcccggggtc cccccgcccc ggggcgtccc gccggcccgg
3841 gcccagcctg atggcacccc ctcccccggg ggctccaaga aaggacccag agggaaactc
3901 caggctcagc gcgcaacaac caaaggccgg gcaggaggcg cggagggccg ggctggggcc
3961 agataa

*FIG. 4H-2*

## Human TTK DNA:

1 atggaatccg aggatttaag tggcagagaa ttgacaattg attccataat gaacaaagtg
61 agagacatta aaaataagtt taaaaatgaa gaccttactg atgaactaag cttgaataaa
121 atttctgctg atactacaga taactcggga actgttaacc aaattatgat gatggcaaac
181 aacccagagg actggttgag tttgttgctc aaactagaga aaaacagtgt tccgctaagt
241 gatgctcttt taaataaatt gattggtcgt tacagtcaag caattgaagc gcttcccca
301 gataaatatg gccaaaatga gagttttgct agaattcaag tgagatttgc tgaattaaaa
361 gctattcaag agccagatga tgcacgtgac tactttcaaa tggccagagc aaactgcaag
421 aaatttgctt ttgttcatat atcttttgca caatttgaac tgtcacaagg taatgtcaaa
481 aaaagtaaac aacttcttca aaaagctgta gaacgtggag cagtaccact agaaatgctg
541 gaaattgccc tgcggaattt aaacctccaa aaaaagcagc tgctttcaga ggaggaaaag
601 aagaatttat cagcatctac ggtattaact gcccaagaat cattttccgg ttcacttggg
661 catttacaga ataggaacaa cagttgtgat tccagaggac agactactaa agccaggttt
721 ttatatggag agaacatgcc accacaagat gcagaaatag gttaccggaa ttcattgaga
781 caaactaaca aaactaaaca gtcatgccca tttggaagag tcccagttaa ccttctaaat
841 agcccagatt gtgatgtgaa gacagatgat tcagttgtac cttgttttat gaaaagacaa
901 acctctagat cagaatgccg agatttggtt gtgcctggat ctaaaccaag tggaaatgat
961 tcctgtgaat taagaaattt aaagtctgtt caaaatagtc atttcaagga acctctggtg
1021 tcagatgaaa agagttctga acttattatt actgattcaa taaccctgaa gaataaaacg
1081 gaatcaagtc ttctagctaa attagaagaa actaaagagt atcaagaacc agaggttcca
1141 gagagtaacc agaaacagtg gcaatctaag agaaagtcag agtgtattaa ccagaatcct
1201 gctgcatctt caaatcactg gcagattccg gagttagccc gaaaagttaa tacagagcag
1261 aaacatacca cttttgagca acctgtcttt tcagtttcaa aacagtcacc accaatatca
1321 acatctaaat ggtttgaccc aaaatctatt tgtaagacac caagcagcaa taccttggat
1381 gattacatga gctgttttag aactccagtt gtaaagaatg actttccacc tgcttgtcag
1441 ttgtcaacac cttatggcca acctgcctgt ttccagcagc aacagcatca aatacttgcc
1501 actccacttc aaaatttaca ggttttagca tcttcttcag caaatgaatg catttcggtt
1561 aaaggaagaa tttattccat tttaaagcag ataggaagtg gaggttcaag caaggtattt
1621 caggtgttaa atgaaaagaa acagatatat gctataaaat atgtgaactt agaagaagca
1681 gataaccaaa ctcttgatag ttaccggaac gaaatagctt atttgaataa actacaacaa
1741 cacagtgata agatcatccg actttatgat tatgaaatca cggaccagta catctacatg
1801 gtaatggagt gtggaaatat tgatcttaat agttggctta aaaagaaaaa atccattgat
1861 ccatgggaac gcaagagtta ctggaaaaat atgttagagg cagttcacac aatccatcaa
1921 catggcattg ttcacagtga tcttaaacca gctaactttc tgatagttga tggaatgcta
1981 aagctaattg attttgggat tgcaaaccaa atgcaaccag atacaacaag tgttgttaaa
2041 gattctcagg ttggcacagt taattatatg ccaccagaag caatcaaaga tatgtcttcc
2101 tccagagaga atgggaaatc taagtcaaag ataagcccca aaagtgatgt ttggtcctta
2161 ggatgtattt tgtactatat gacttacggg aaaacaccat ttcagcagat aattaatcag
2221 atttctaaat tacatgccat aattgatcct aatcatgaaa ttgaatttcc cgatattcca
2281 gagaaagatc ttcaagatgt gttaaagtgt tgtttaaaaa gggacccaaa acagaggata
2341 tccattcctg agctcctggc tcatccctat gttcaaattc aaactcatcc agttaaccaa
2401 atggccaagg gaaccactga agaaatgaaa tatgttctgg gccaacttgt tggtctgaat
2461 tctcctaact ccattttgaa agctgctaaa actttatatg aacactatag tggtggtgaa
2521 agtcataatt cttcatcctc caagactttt gaaaaaaaaa ggggaaaaaa atga

***FIG. 4I***

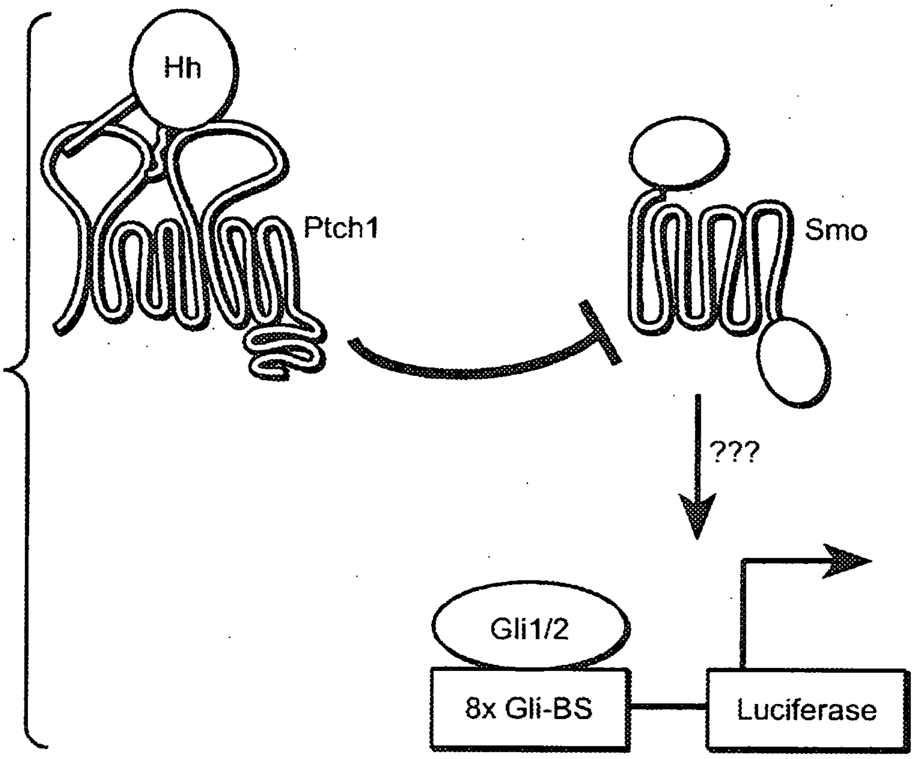
Hh
Ptch1
Smo
???
Gli1/2
8x Gli-BS
Luciferase

FIG. 5A

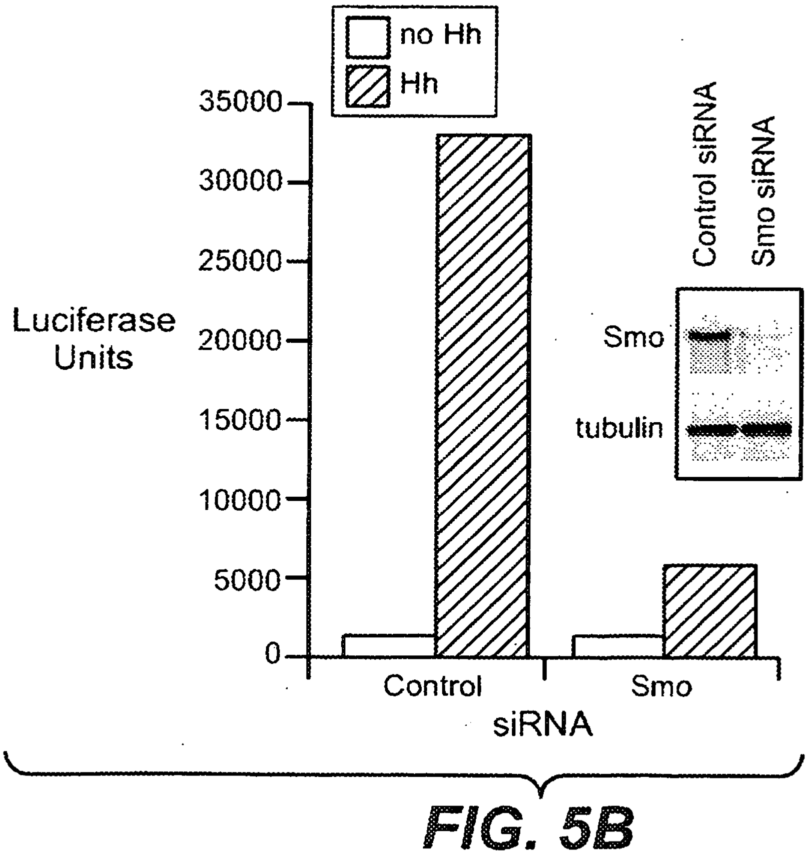
no Hh
Hh
Luciferase Units
35000
30000
25000
20000
15000
10000
5000
0
Control
Smo
siRNA
Control siRNA
Smo siRNA
Smo
tubulin

FIG. 5B

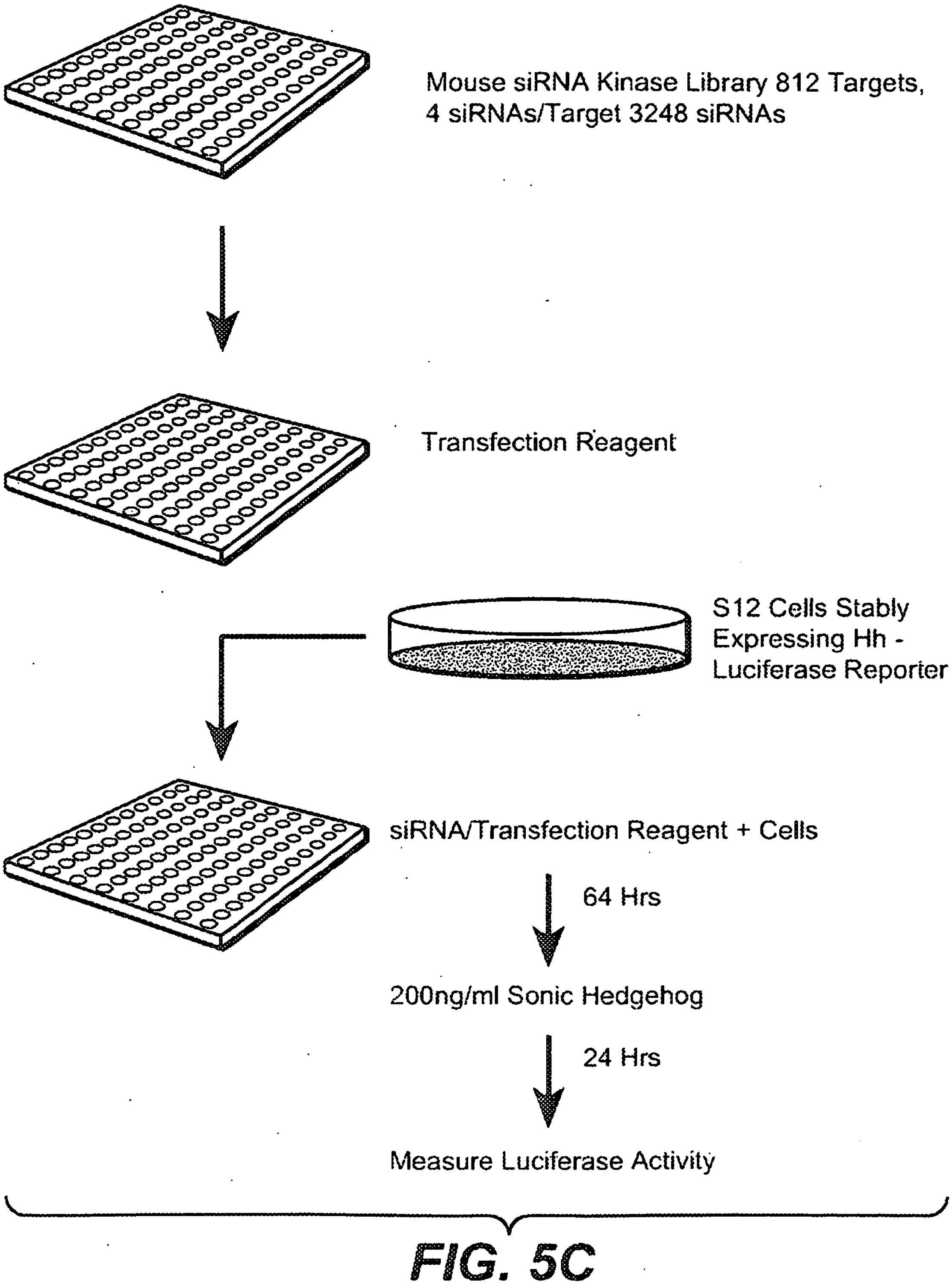
Mouse siRNA Kinase Library 812 Targets,
4 siRNAs/Target 3248 siRNAs
Transfection Reagent
S12 Cells Stably
Expressing Hh -
Luciferase Reporter
siRNA/Transfection Reagent + Cells
64 Hrs
200ng/ml Sonic Hedgehog
24 Hrs
Measure Luciferase Activity

FIG. 5C

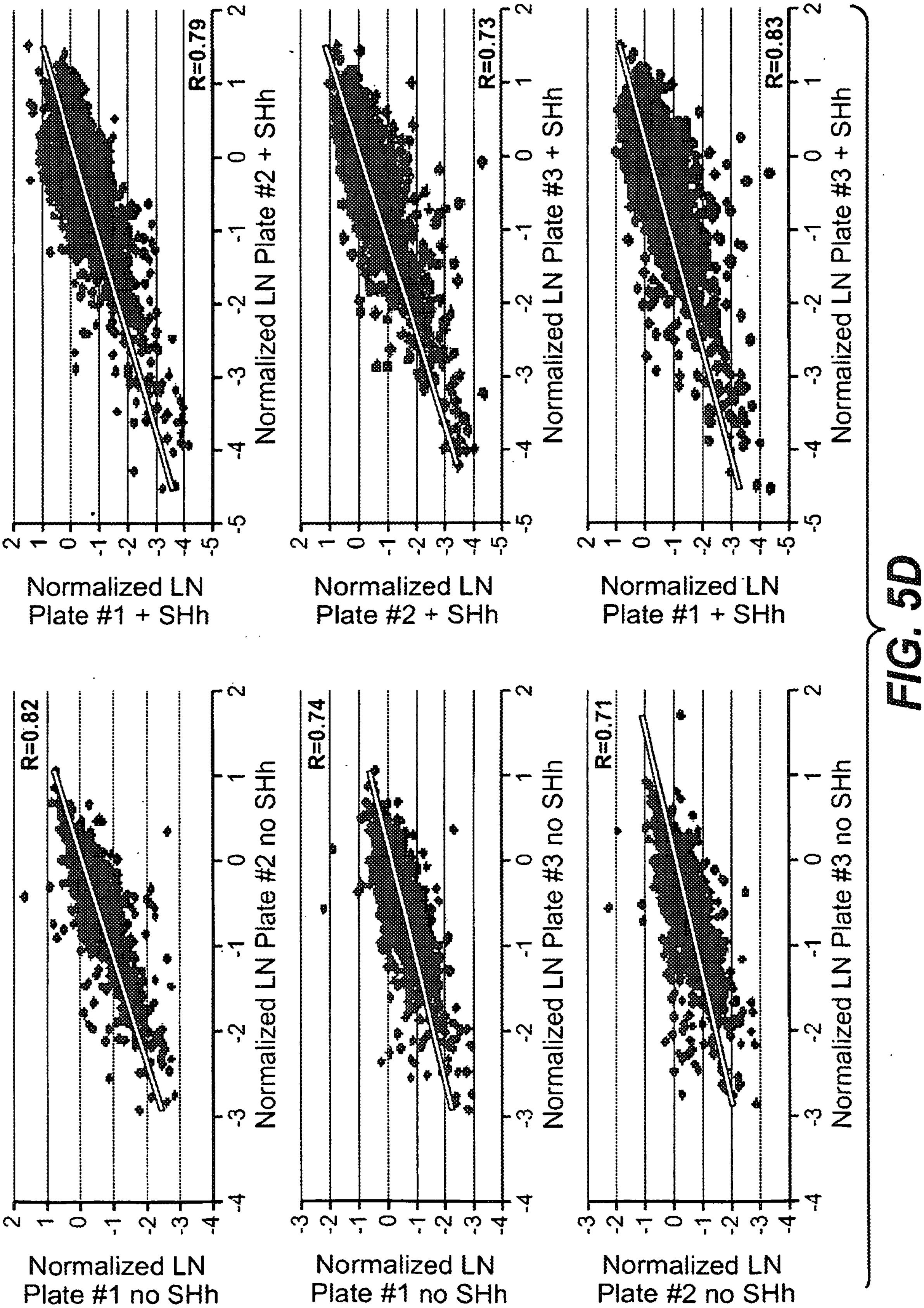
Normalized LN Plate #1 + SHh
Normalized LN Plate #2 + SHh
R=0.79
Normalized LN Plate #2 + SHh
Normalized LN Plate #3 + SHh
R=0.73
Normalized LN Plate #1 + SHh
Normalized LN Plate #3 + SHh
R=0.83
Normalized LN Plate #1 no SHh
Normalized LN Plate #2 no SHh
R=0.82
Normalized LN Plate #1 no SHh
Normalized LN Plate #3 no SHh
R=0.74
Normalized LN Plate #2 no SHh
Normalized LN Plate #3 no SHh
R=0.71

FIG. 5D

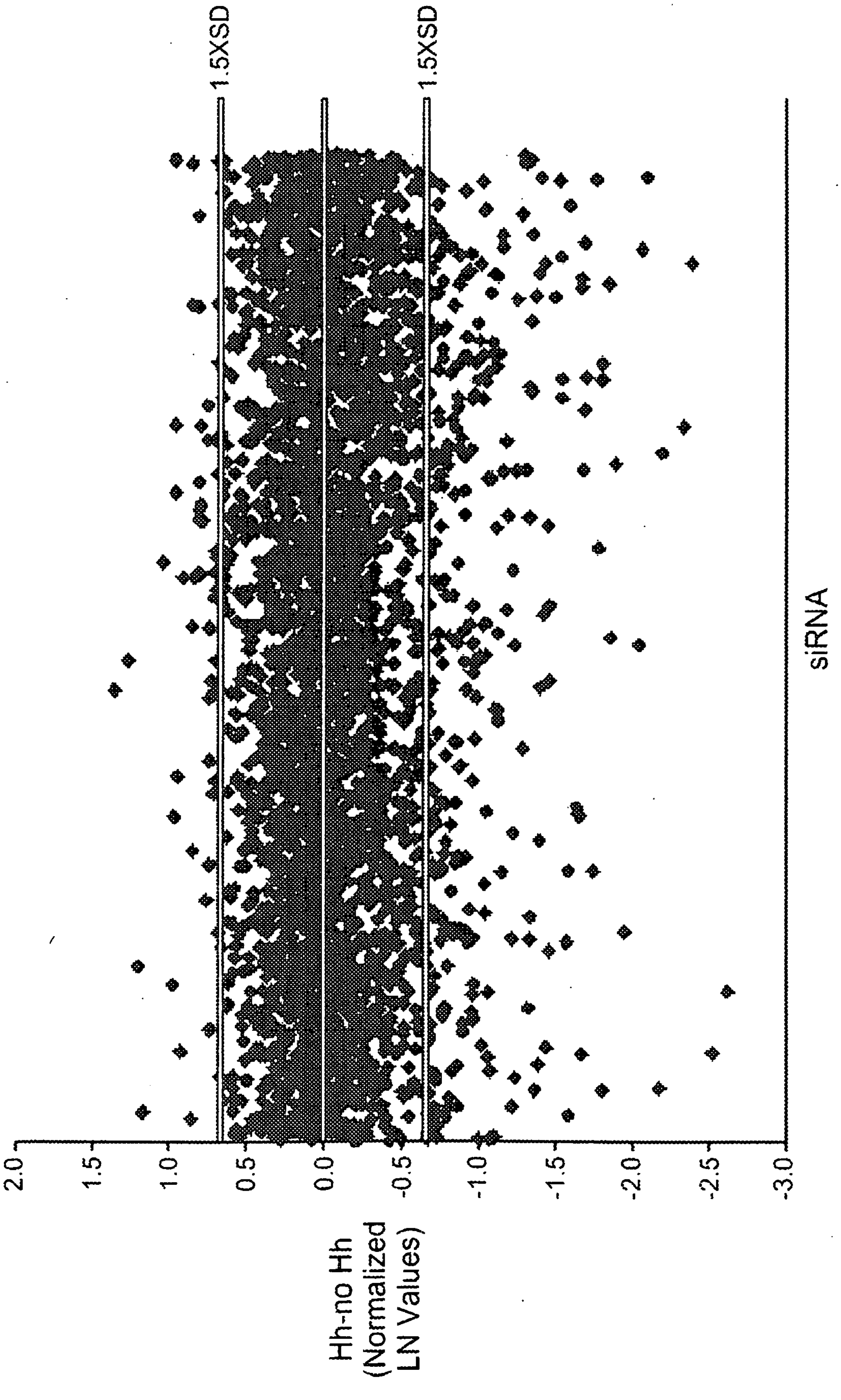
1.5XSD
1.5XSD
2.0
1.5
1.0
0.5
0.0
-0.5
-1.0
-1.5
-2.0
-2.5
-3.0
Hh-no Hh (Normalized LN Values)
siRNA

FIG. 5E

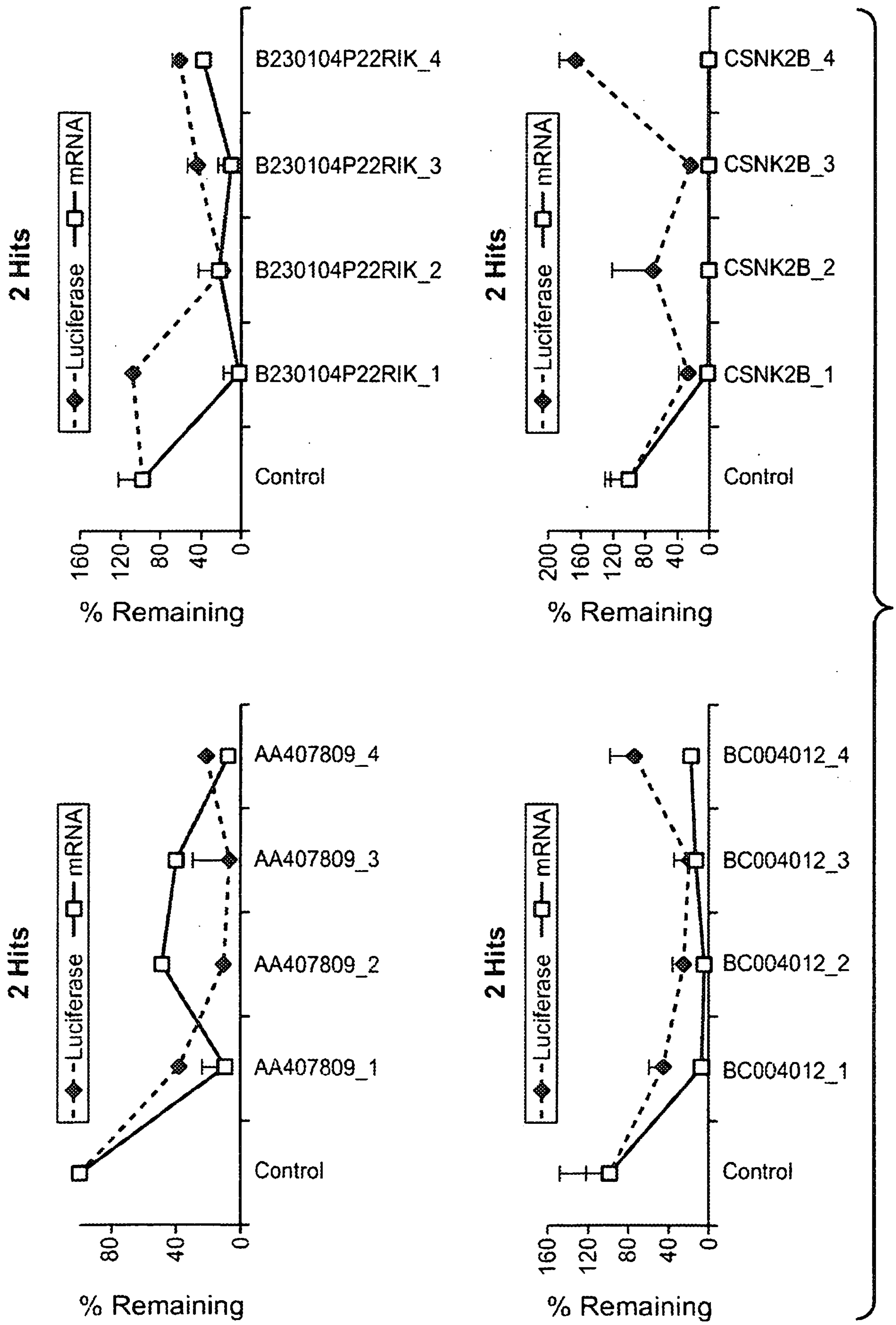
2 Hits
Luciferase
mRNA
% Remaining
160
120
80
40
0
Control
B230104P22RIK_1
B230104P22RIK_2
B230104P22RIK_3
B230104P22RIK_4
2 Hits
Luciferase
mRNA
% Remaining
200
160
120
80
40
0
Control
CSNK2B_1
CSNK2B_2
CSNK2B_3
CSNK2B_4
2 Hits
Luciferase
mRNA
% Remaining
80
40
0
Control
AA407809_1
AA407809_2
AA407809_3
AA407809_4
2 Hits
Luciferase
mRNA
% Remaining
160
120
80
40
0
Control
BC004012_1
BC004012_2
BC004012_3
BC004012_4

FIG. 6A-1

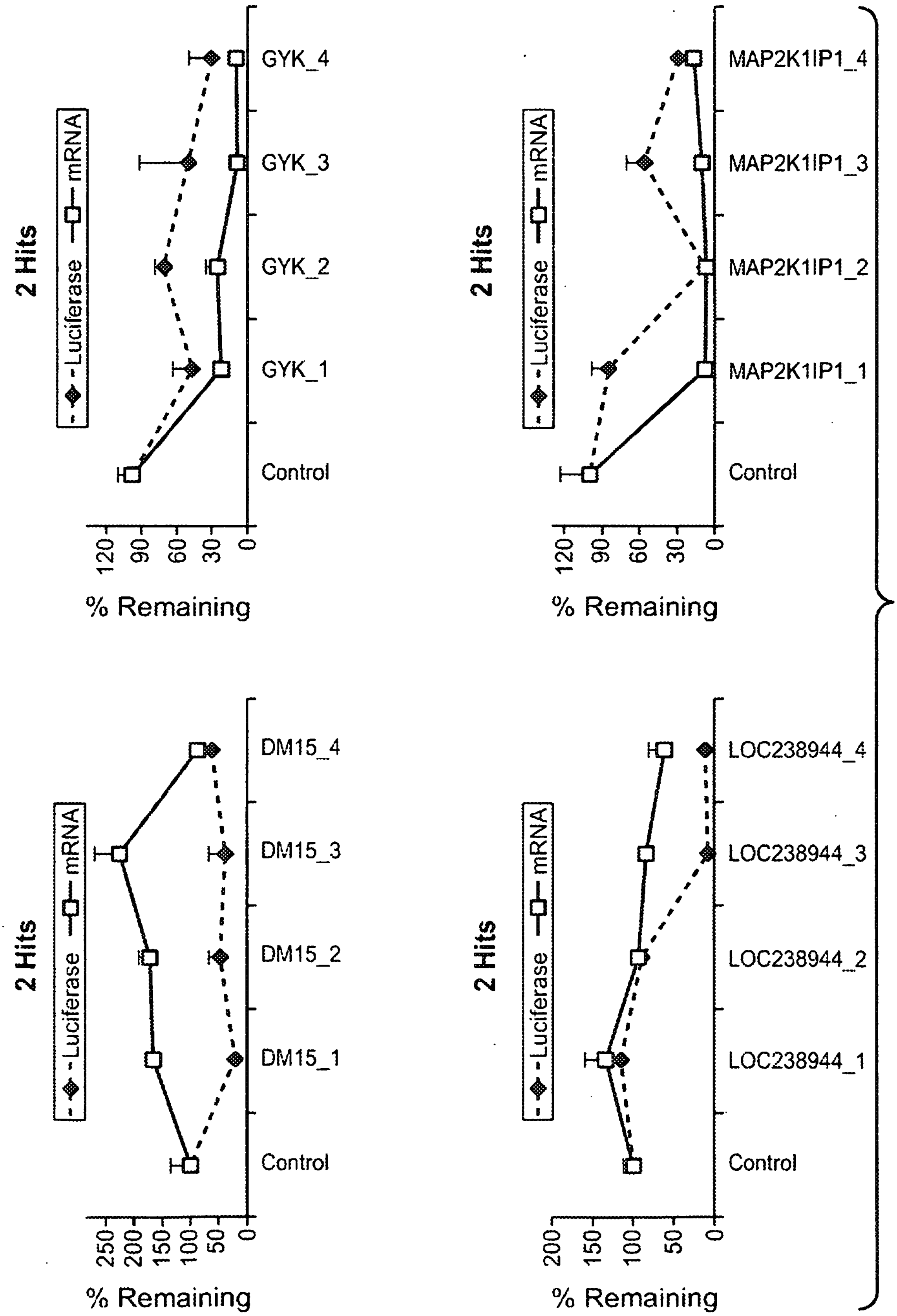
2 Hits
Luciferase
mRNA
% Remaining
GYK_4
GYK_3
GYK_2
GYK_1
Control
DM15_4
DM15_3
DM15_2
DM15_1
MAP2K1IP1_4
MAP2K1IP1_3
MAP2K1IP1_2
MAP2K1IP1_1
LOC238944_4
LOC238944_3
LOC238944_2
LOC238944_1

FIG. 6A-2

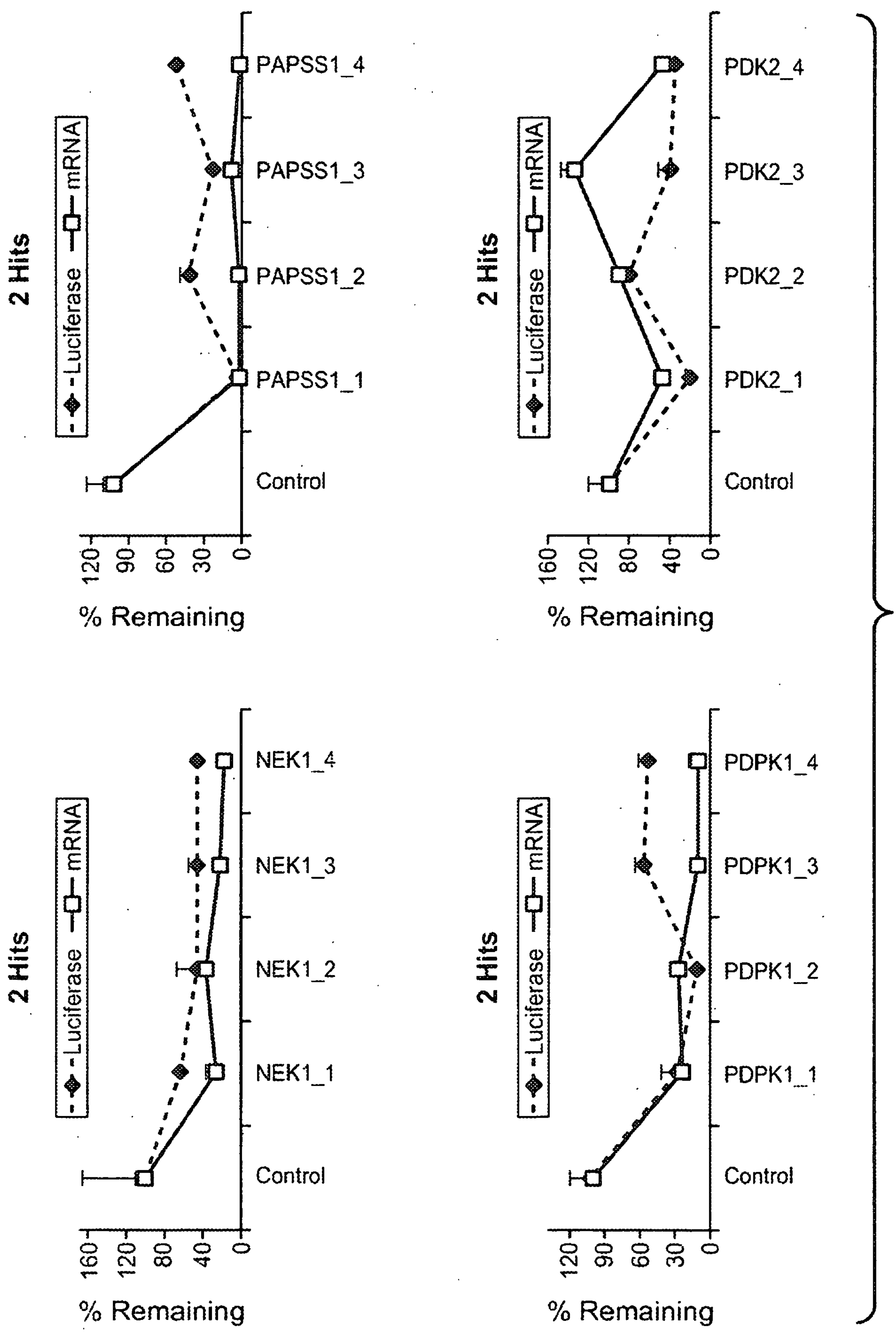
2 Hits
Luciferase
mRNA
% Remaining
120
90
60
30
0
Control
PAPSS1_1
PAPSS1_2
PAPSS1_3
PAPSS1_4
160
120
80
40
0
Control
PDK2_1
PDK2_2
PDK2_3
PDK2_4
160
120
80
40
0
Control
NEK1_1
NEK1_2
NEK1_3
NEK1_4
120
90
60
30
0
Control
PDPK1_1
PDPK1_2
PDPK1_3
PDPK1_4

*FIG. 6A-3*

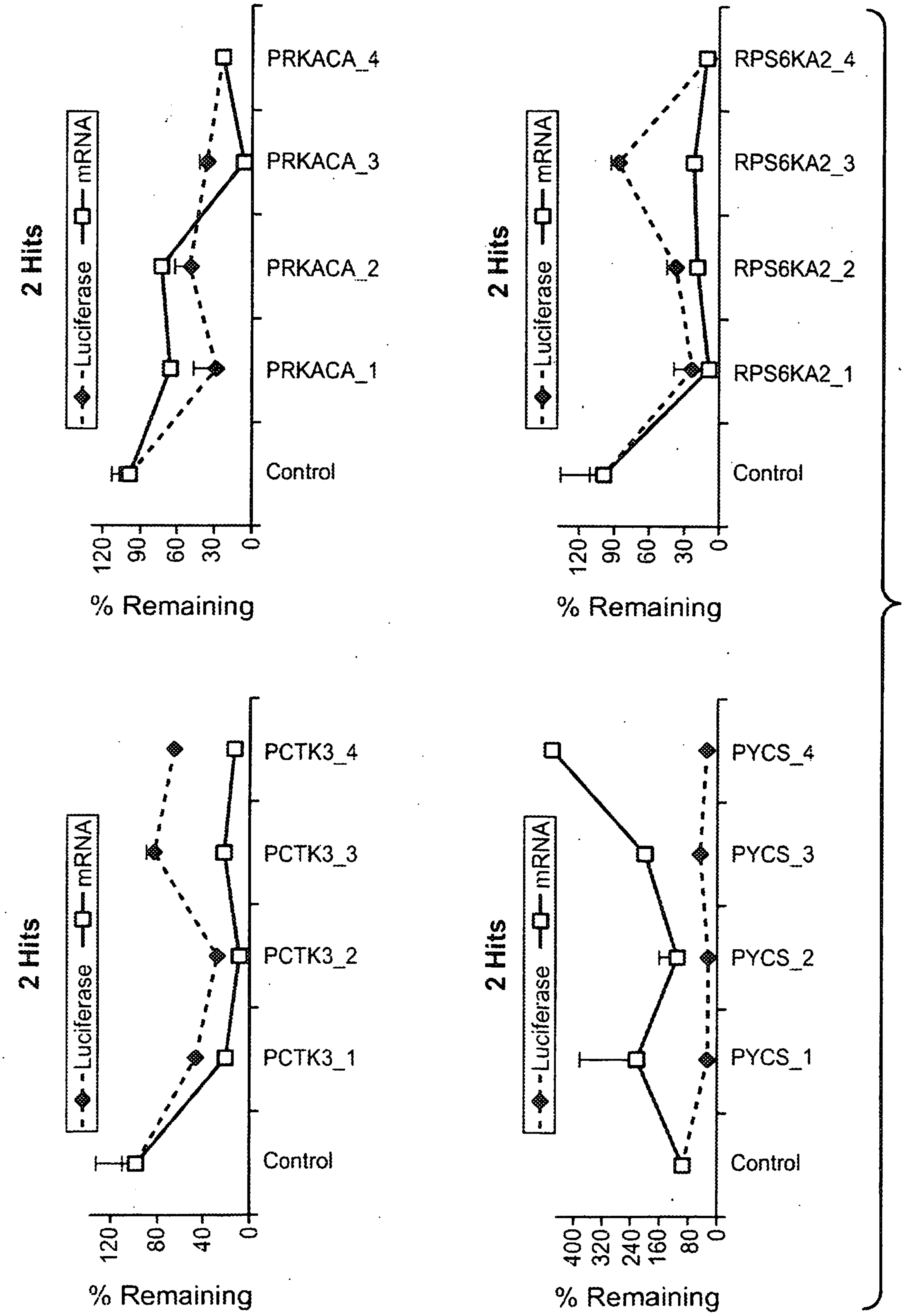
2 Hits
Luciferase
mRNA
% Remaining
120
90
60
30
0
Control
PRKACA_1
PRKACA_2
PRKACA_3
PRKACA_4
2 Hits
Luciferase
mRNA
% Remaining
120
90
60
30
0
Control
RPS6KA2_1
RPS6KA2_2
RPS6KA2_3
RPS6KA2_4
2 Hits
Luciferase
mRNA
% Remaining
120
80
40
0
Control
PCTK3_1
PCTK3_2
PCTK3_3
PCTK3_4
2 Hits
Luciferase
mRNA
% Remaining
400
320
240
160
80
0
Control
PYCS_1
PYCS_2
PYCS_3
PYCS_4

FIG. 6A-4

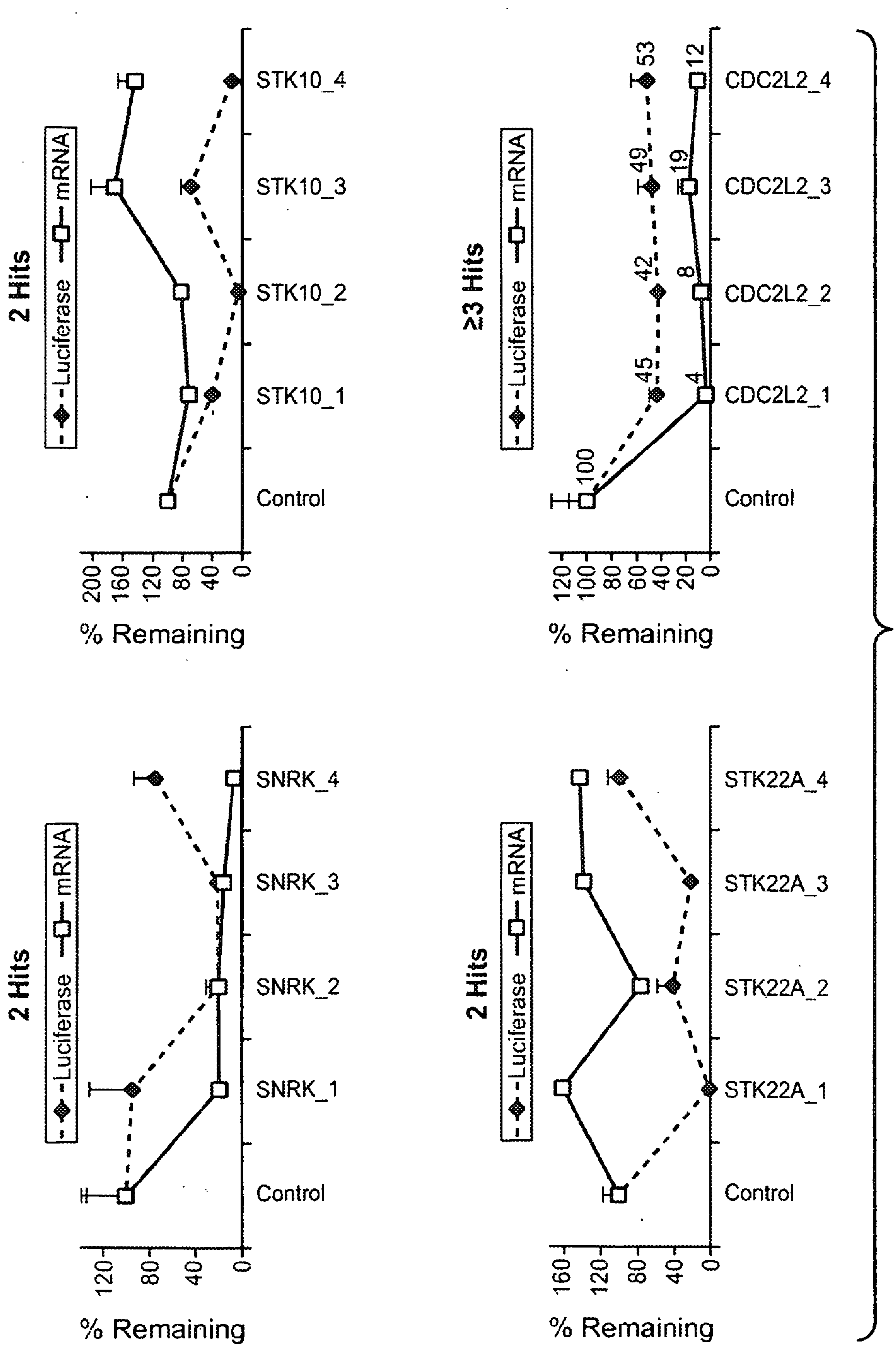
2 Hits
Luciferase
mRNA
% Remaining
STK10_4
STK10_3
STK10_2
STK10_1
Control
≥3 Hits
CDC2L2_4
CDC2L2_3
CDC2L2_2
CDC2L2_1
SNRK_4
SNRK_3
SNRK_2
SNRK_1
STK22A_4
STK22A_3
STK22A_2
STK22A_1

FIG. 6A-5

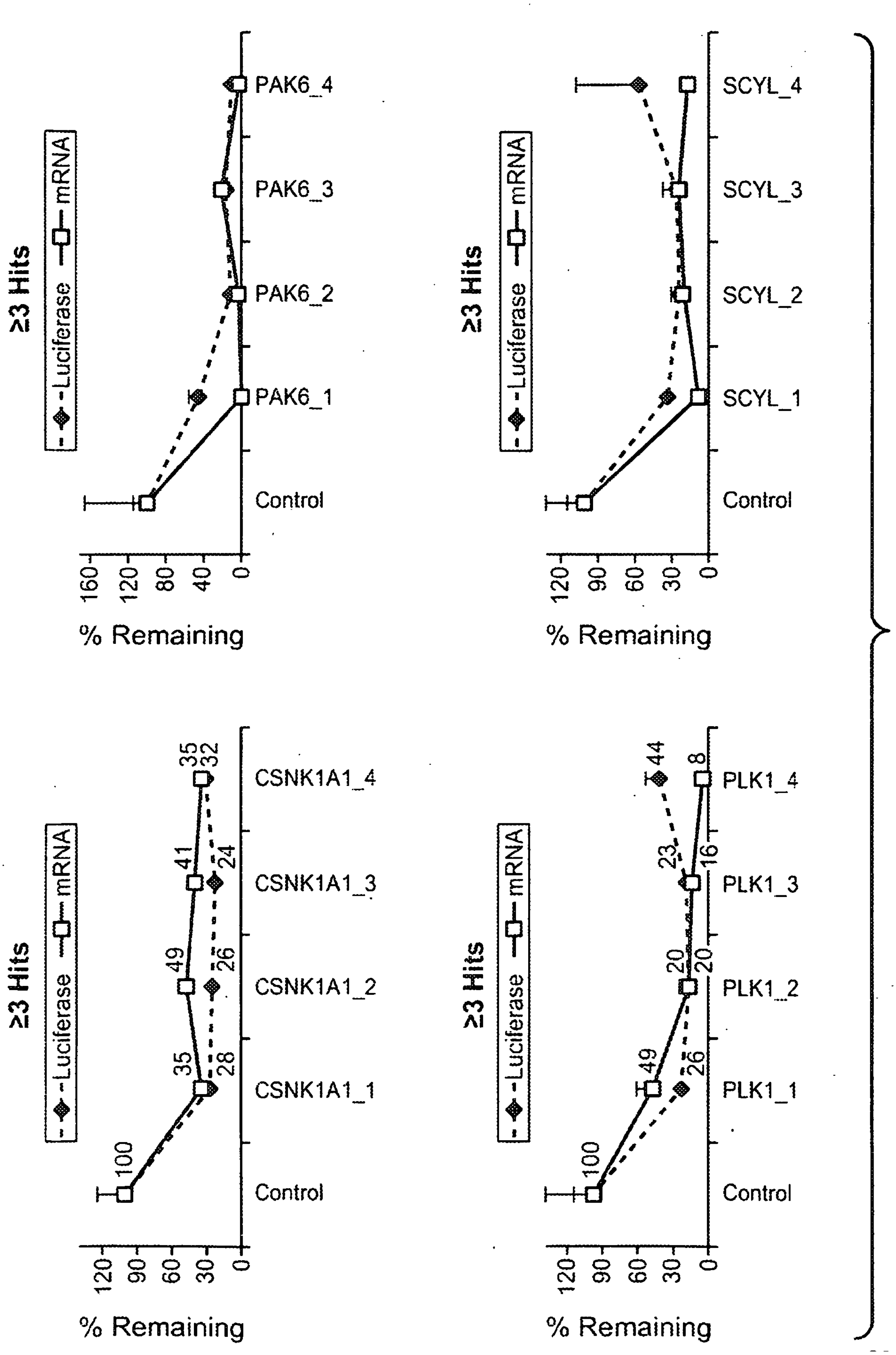
≥3 Hits
- ◆ -Luciferase —□— mRNA
% Remaining
PAK6_1
PAK6_2
PAK6_3
PAK6_4
Control
SCYL_1
SCYL_2
SCYL_3
SCYL_4
CSNK1A1_1
CSNK1A1_2
CSNK1A1_3
CSNK1A1_4
PLK1_1
PLK1_2
PLK1_3
PLK1_4


FIG. 6A-6

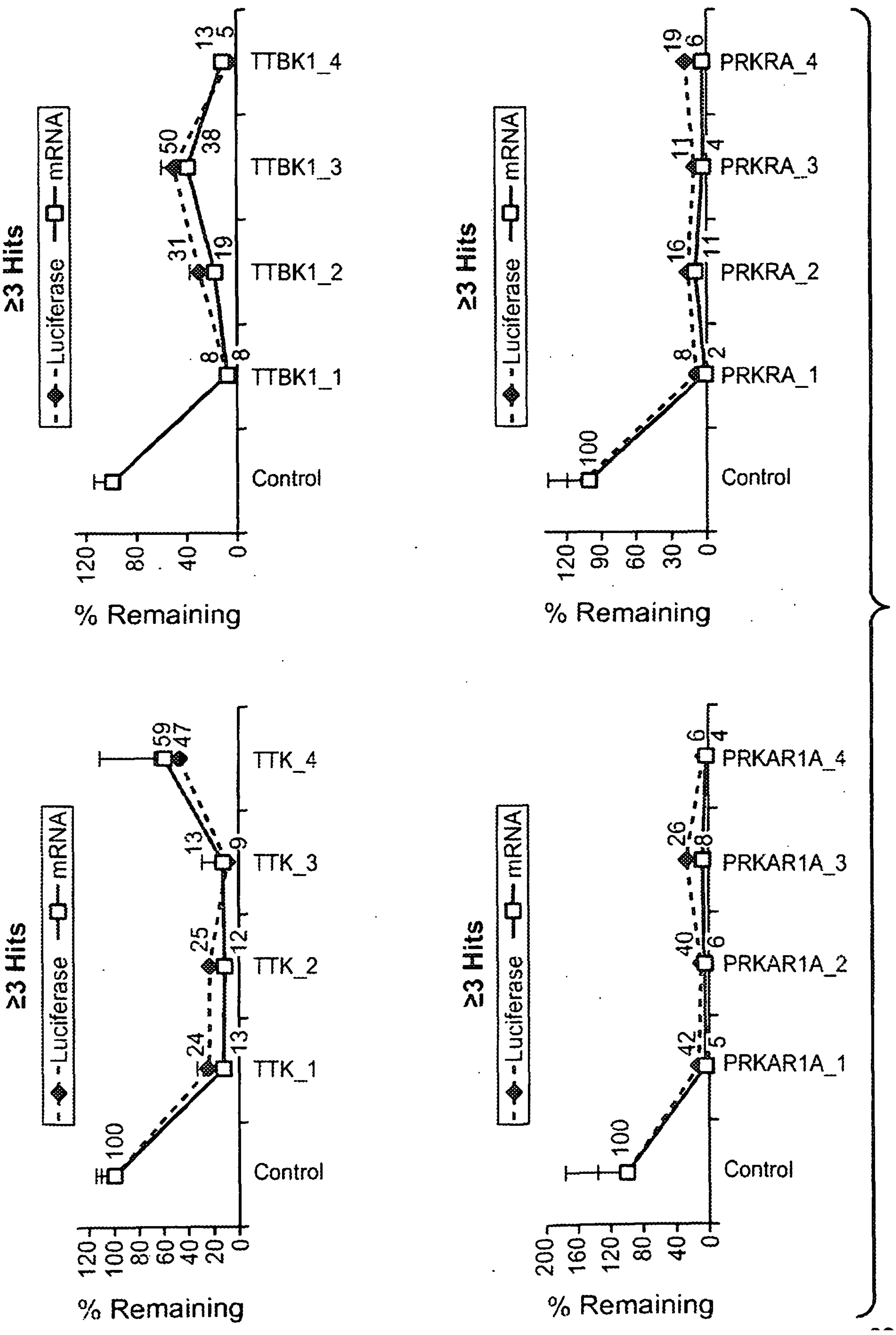
≥3 Hits
Luciferase
mRNA
% Remaining
Control
TTBK1_1
TTBK1_2
TTBK1_3
TTBK1_4
TTK_1
TTK_2
TTK_3
TTK_4
PRKRA_1
PRKRA_2
PRKRA_3
PRKRA_4
PRKAR1A_1
PRKAR1A_2
PRKAR1A_3
PRKAR1A_4

FIG. 6A-7

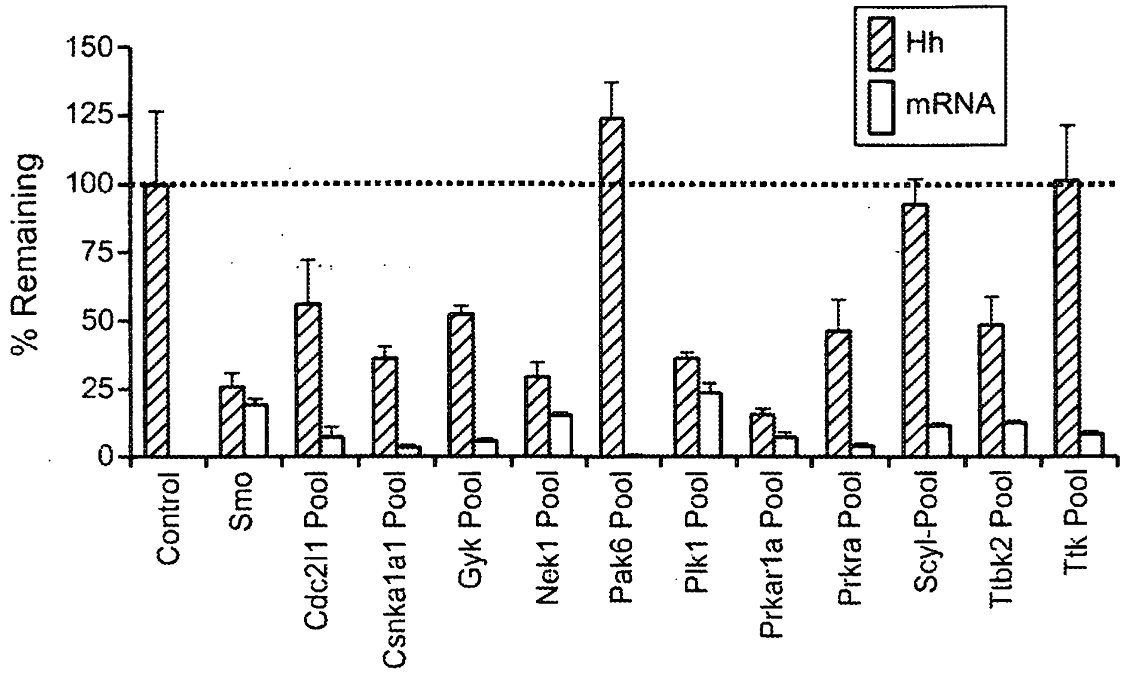
Hh
mRNA
150
125
100
75
50
25
0
% Remaining
Control
Smo
Cdc2l1 Pool
Csnka1a1 Pool
Gyk Pool
Nek1 Pool
Pak6 Pool
Plk1 Pool
Prkar1a Pool
Prkra Pool
Scyl-Pool
Ttbk2 Pool
Ttk Pool

FIG. 6B

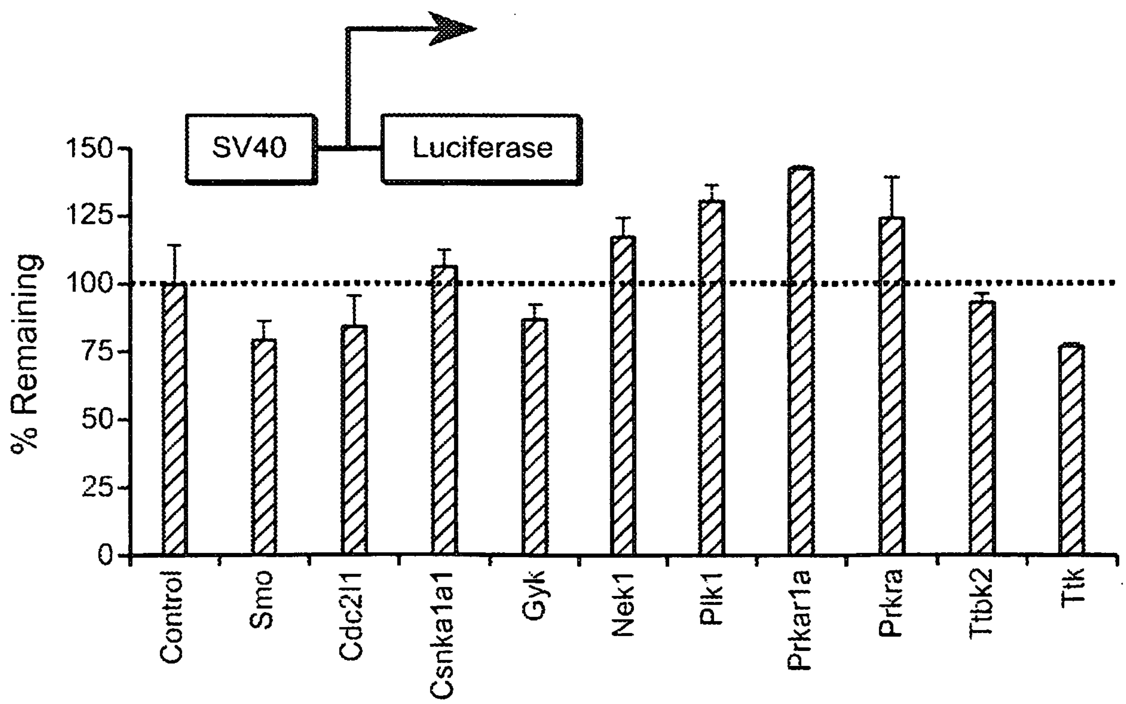
SV40
Luciferase
150
125
100
75
50
25
0
% Remaining
Control
Smo
Cdc2l1
Csnka1a1
Gyk
Nek1
Plk1
Prkar1a
Prkra
Ttbk2
Ttk

FIG. 6C

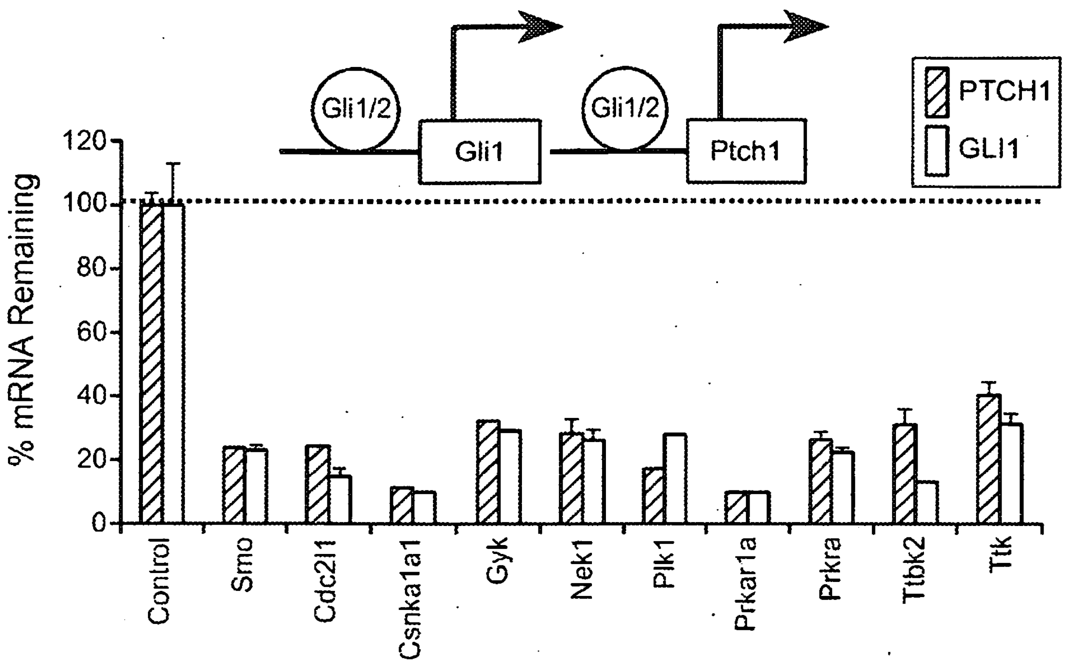

Gli1/2
Gli1
Gli1/2
Ptch1
PTCH1
GLI1
120
100
80
60
40
20
0
% mRNA Remaining
Control
Smo
Cdc2l1
Csnka1a1
Gyk
Nek1
Plk1
Prkar1a
Prkra
Ttbk2
Ttk


FIG. 6D

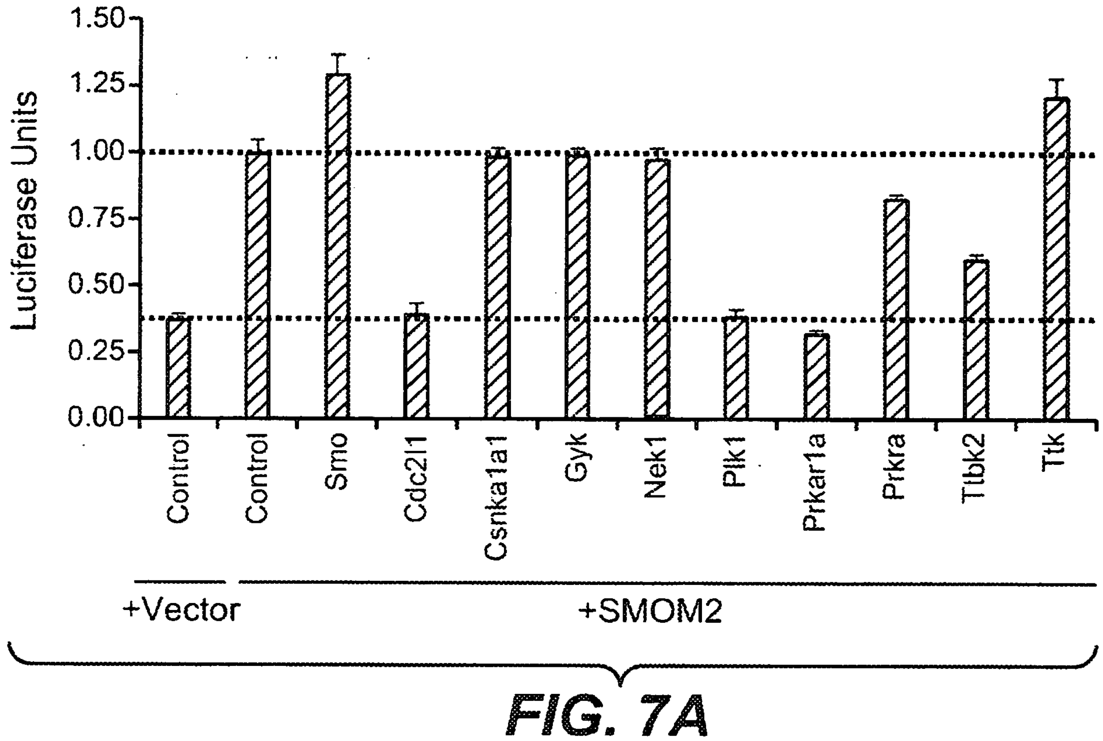

Luciferase Units
1.50
1.25
1.00
0.75
0.50
0.25
0.00
Control
Control
Smo
Cdc2l1
Csnka1a1
Gyk
Nek1
Plk1
Prkar1a
Prkra
Ttbk2
Ttk
+Vector
+SMOM2


FIG. 7A

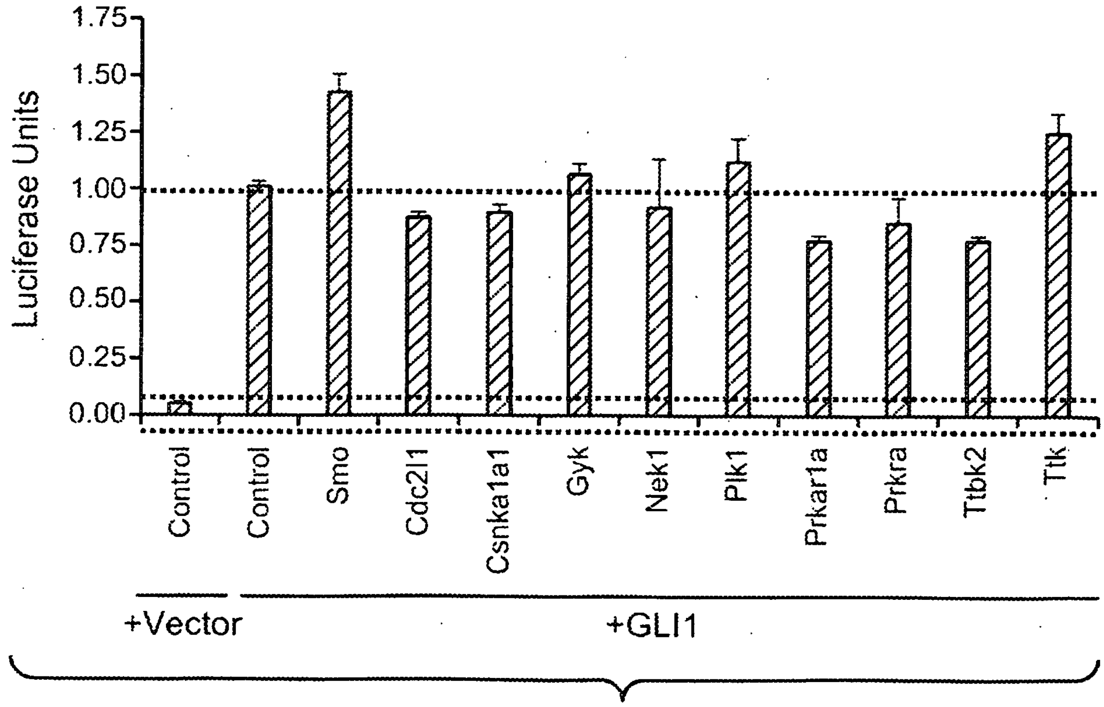

Luciferase Units
1.75
1.50
1.25
1.00
0.75
0.50
0.25
0.00
Control
Control
Smo
Cdc2l1
Csnka1a1
Gyk
Nek1
Plk1
Prkar1a
Prkra
Ttbk2
Ttk
+Vector
+GLI1


FIG. 7B

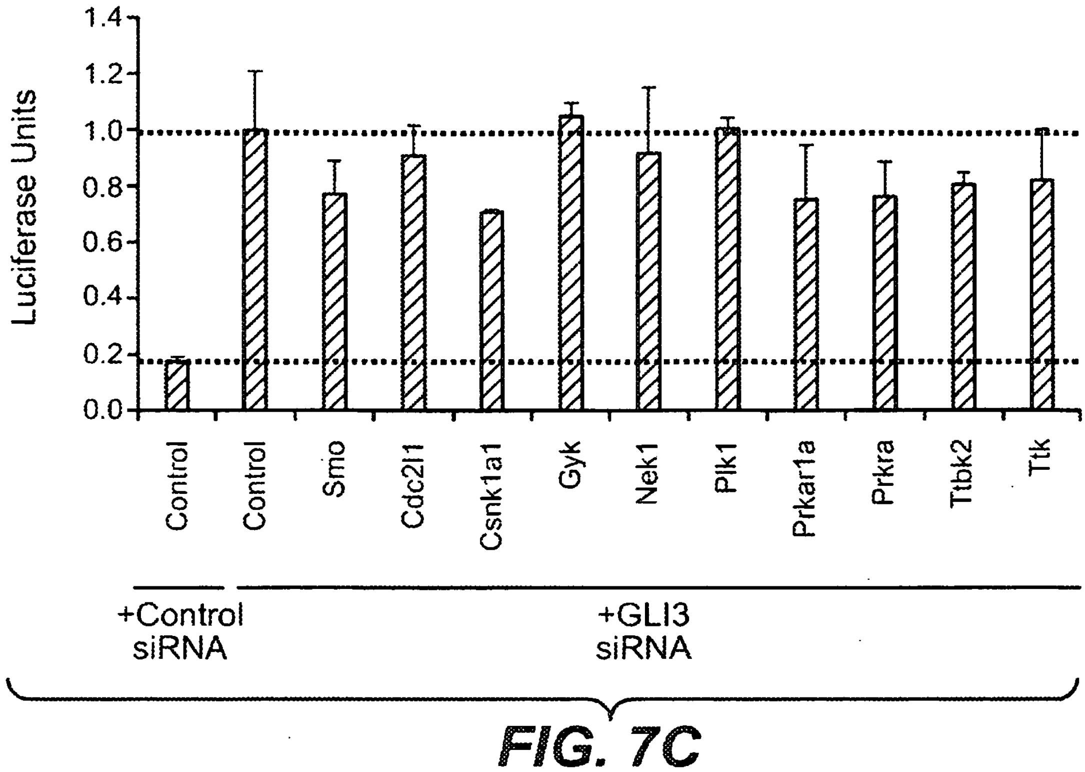
1.4
1.2
1.0
0.8
0.6
0.4
0.2
0.0
Luciferase Units
Control
Control
Smo
Cdc2l1
Csnk1a1
Gyk
Nek1
Plk1
Prkar1a
Prkra
Ttbk2
Ttk
+Control siRNA
+GLI3 siRNA

FIG. 7C

FIG. 7D

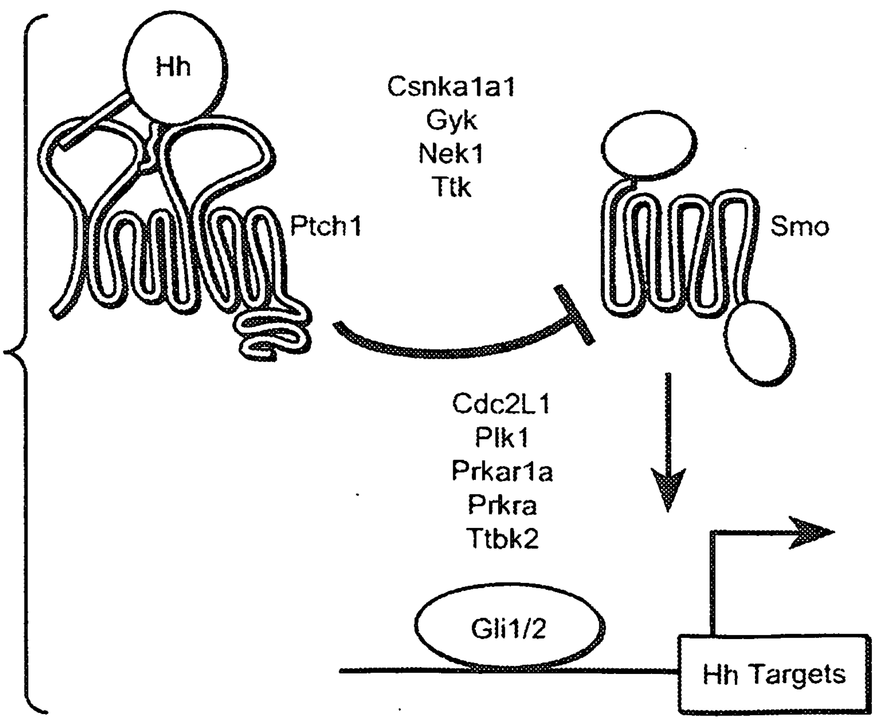
Hh
Csnka1a1
Gyk
Nek1
Ttk
Ptch1
Smo
Cdc2L1
Plk1
Prkar1a
Prkra
Ttbk2
Gli1/2
Hh Targets

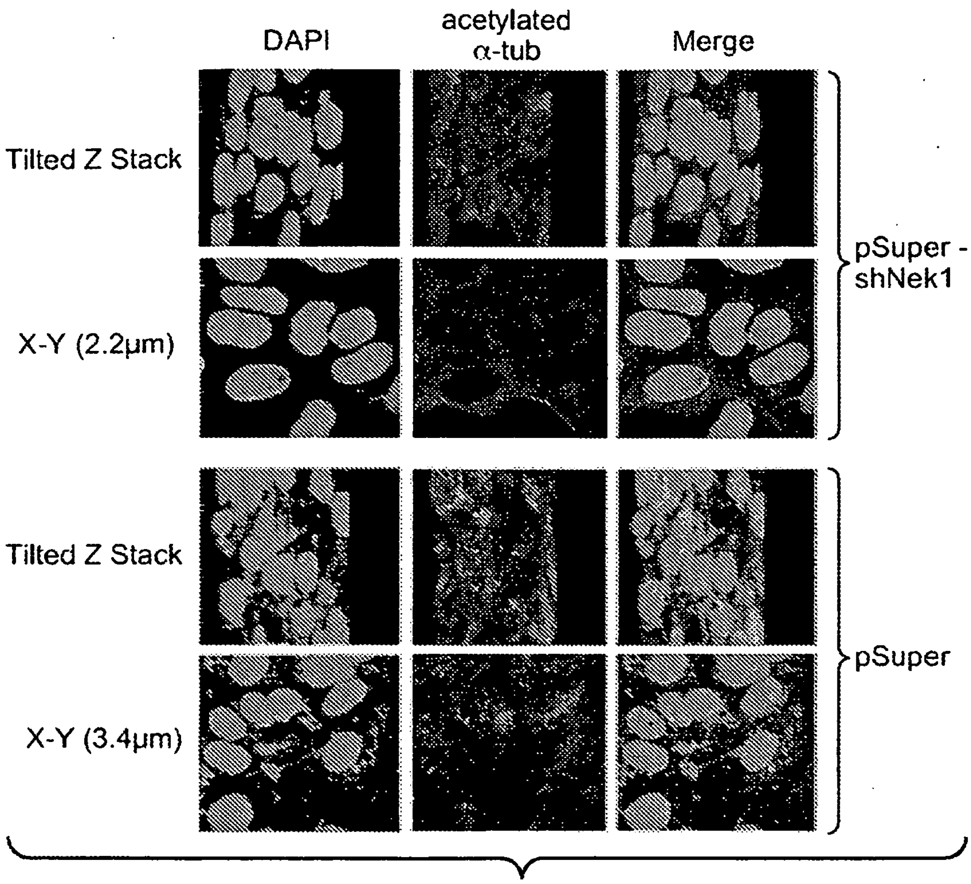
DAPI
acetylated α-tub
Merge
Tilted Z Stack
X-Y (2.2μm)
Tilted Z Stack
X-Y (3.4μm)
pSuper - shNek1
pSuper

*FIG. 7E*

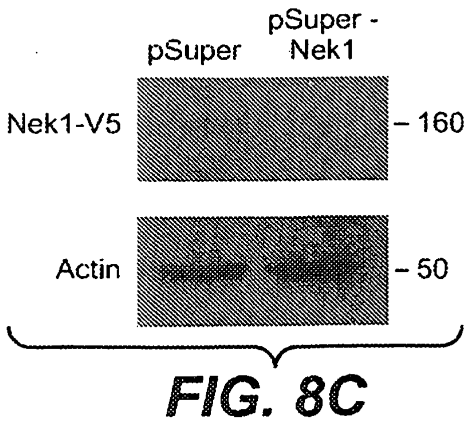
pSuper
pSuper - Nek1
Nek1-V5
Actin
– 160
– 50

*FIG. 8C*

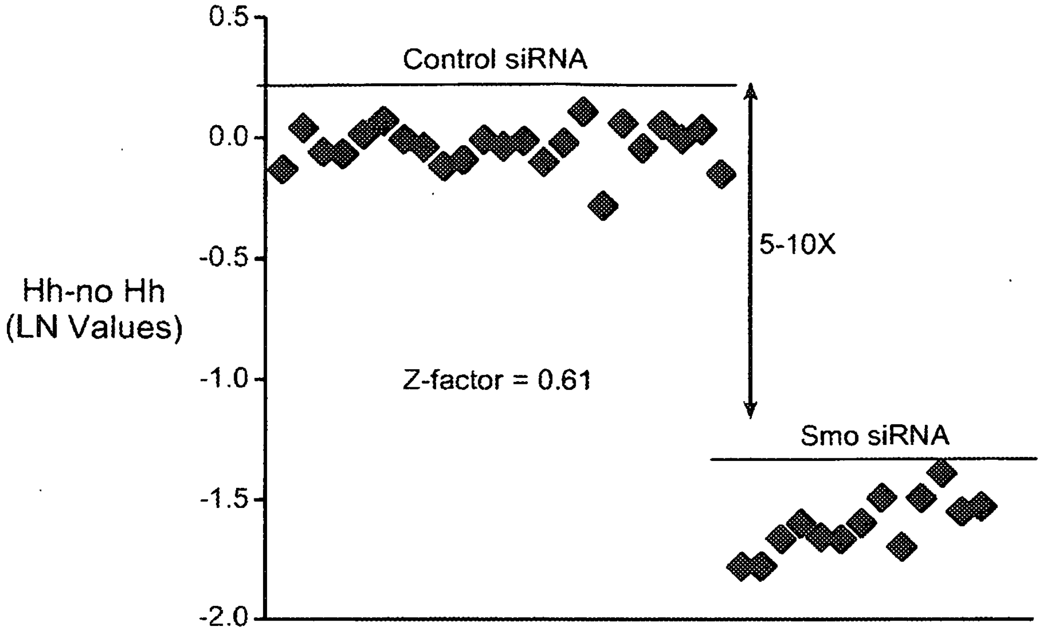
0.5
0.0
-0.5
-1.0
-1.5
-2.0
Hh-no Hh
(LN Values)
Control siRNA
5-10X
Z-factor = 0.61
Smo siRNA

*FIG. 8A*

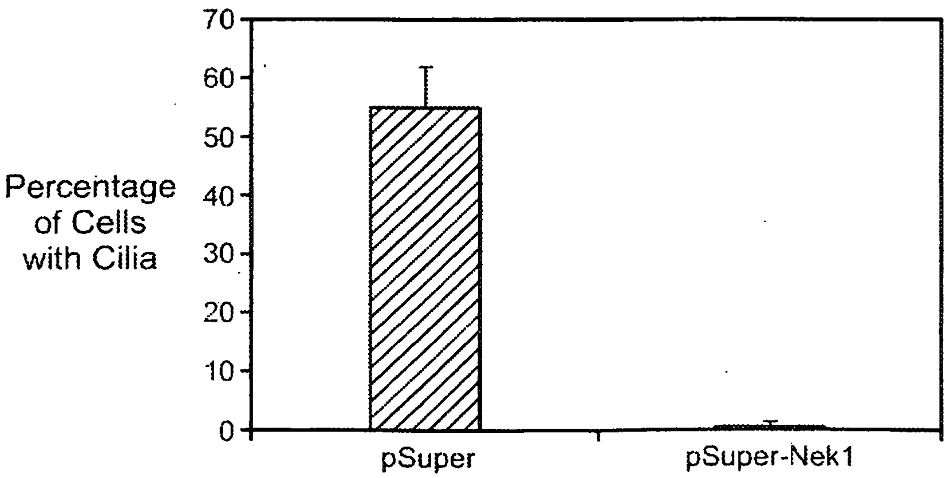
70
60
50
40
30
20
10
0
Percentage
of Cells
with Cilia
pSuper
pSuper-Nek1

*FIG. 8B*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9518856 A
- WO 9617924 A
- WO 2004054657 A
- WO 03000873 A
- US 4275149 A
- US 4816567 A
- US 5641870 A
- US 5556762 A
- US 5750373 A
- US 4708871 A
- US 4833092 A
- US 5223409 A
- US 5403484 A
- US 5571689 A
- US 5663143 A
- WO 8403506 A
- WO 8403564 A
- WO 9845479 A
- US 4933294 A
- WO 9105264 A
- US 5401638 A
- US 5092871 A
- US 4955892 A
- US P4892538 A
- US 5106627 A
- US 4391909 A
- US 4353888 A
- US P5041138 A
- US 5565332 A
- US 5573905 A
- US 5567610 A
- US 5229275 A
- US 5869046 A
- WO 9316185 A
- US 5571894 A
- US 5587458 A
- WO 9411026 A
- US 3896111 A
- US 4151042 A
- US 4137230 A
- US 4248870 A
- US 4256746 A
- US 4260608 A
- US 4265814 A
- US 4294757 A
- US 4307016 A
- US 4308268 A
- US 4308269 A
- US 4309428 A
- US 4313946 A
- US 4315929 A
- US 4317821 A
- US 4322348 A
- US 4331598 A
- US 4361650 A
- US 4364866 A
- US 4424219 A
- US 4450254 A
- US 4362663 A
- US 4371533 A
- US 5208020 A
- US 5416064 A
- EP 0425235 B1
- US 5712374 A
- US 5714586 A
- US 5739116 A
- US 5767285 A
- US 5770701 A
- US 5770710 A
- US 5773001 A
- US 5877296 A
- US 5053394 A
- WO 9321232 A
- WO 9534683 A
- US 5627024 A
- US 5766905 A
- WO 9814277 A
- WO 9820169 A
- WO 9820159 A
- WO 9820036 A
- WO 9735196 A
- WO 9746251 A
- WO 9747314 A
- WO 9709446 A
- US 5498538 A
- US 5432018 A
- WO 9815833 A
- US 5723286 A
- US 5580717 A
- US 5427908 A
- US 5498530 A
- US 5770434 A
- US 5734018 A
- US 5698426 A
- US 5763192 A
- US 5723323 A
- US 5364934 A
- WO 8905859 A

- US 4399216 A
- DD 266710
- US 4946783 A
- US 5648237 A, Carter
- US 5789199 A, Joly
- US 5840523 A, Simmons
- EP 139383 A
- US 4943529 A
- EP 402226 A
- EP 183070 A
- EP 244234 A
- EP 394538 A
- WO 9100357 A
- US 5010182 A
- EP 362179 A
- WO 9013646 A
- EP 36776 A
- EP 73657 A
- GB 2211504 A
- EP 117060 A
- EP 117058 A
- US 4767704 A
- US 4657866 A
- US 4927762 A
- US 4560655 A
- US 5122469 A
- WO 9003430 A
- WO 8700195 A
- US RE30985 E
- US 3773919 A
- EP 0600517 A
- WO 9607321 A
- US 4892538 A
- US 5283187 A
- WO 9325673 A
- WO 9106629 A
- US 5108921 A
- US 5354844 A
- US 5416016 A
- US 5459127 A
- US 5521291 A
- US 5543158 A
- US 5547932 A
- US 5583020 A
- US 5591721 A
- US 4426330 A
- US 4534899 A
- US 5013556 A
- US 5213804 A
- US 5227170 A
- US 5264221 A
- US 5356633 A
- US 5395619 A
- US 5417978 A
- US 5462854 A
- US 5469854 A
- US 5512295 A
- US 5527528 A
- US 5534259 A
- US 5543152 A
- US 5556948 A
- US 5580575 A
- US 5595756 A
- WO 9010048 A
- WO 9013641 A
- WO 9104753 A
- WO 9010448 A
- US 4987071 A
- US 5116742 A, Cech
- WO 9402610 A, Marasco
- WO 9503832 A, Duan
- WO 9733551 A
- US 0110482 W
- US 4975278 A
- EP 1391213 A
- US 6248564 B
- EP 307247 A

**Non-patent literature cited in the description**


- **Echelard et al.** *Cell,* 1993, vol. 75, 1417-30
- **Ericson et al.** *Cell,* 1995, vol. 81, 747-56
- **Marti et al.** *Nature,* 1995, vol. 375, 322-25
- **Hynes et al.** *Neuron,* 1997, vol. 19, 15-26
- **Krauss et al.** *Cell,* 1993, vol. 75, 1431-44
- **Laufer et al.** *Cell,* 1994, vol. 79, 1165-73
- **Fan and Tessier-Lavigne.** *Cell,* 1994, vol. 79, 1175-86
- **Johnson et al.** *Cell,* 1994, vol. 79, 1165-73
- **Bellusci et al.** *Devel.,* 1997, vol. 124, 53-63
- **Oro et al.** *Science,* 1997, vol. 276, 817-21
- **Apelqvist et al.** *Curr. Biol.,* 1997, vol. 7, 801-804
- **Bellusci et al.** *Dev. Suppl.,* 1997, vol. 124, 53-63
- **Bitgood et al.** *Curr. Biol.,* 1996, vol. 6, 298-304
- **Roberts et al.** *Development,* 1995, vol. 121, 3163-74
- **Vortkamp et al.** *Science,* 1996, vol. 273, 613-22
- **Motoyama et al.** *Nature Genetics,* 1998, vol. 18, 104-106
- **Carpenter et al.** *P.N.A.S. (U.S.A.),* 1998, vol. 95 (23), 13630-40
- **Gli. Ho et al.** *Curr. Opin. Neurobiol.,* 2002, vol. 12, 57-63
- **Nybakken et al.** *Curr. Opin. Genet. Dev.,* 2002, vol. 12, 503-511

- **i Altaba et al.** *Nat. Rev. Neurosci.,* 2002, vol. 3, 24-33
- **Stecca et al.** *J. Biol.,* 2002, vol. 1 (2), 9
- **Boring et al.** *CA Cancel J. Clin.,* 1993, vol. 43, 7
- **Klagsbrun ; D'Amore.** *Annu. Rev. Physiol.,* 1991, vol. 53, 217-39
- **Folkman ; Shing.** *J. Biol. Chem.,* 1992, vol. 267 (16), 10931-4
- **Beck ; D'Amore.** *FASEB J.,* 1997, vol. 11 (5), 365-73
- **Yancopoulos et al.** *Cell,* 1998, vol. 93 (5), 661-4
- **Buschman ; Scaper.** *J. Pathol.,* 2000, vol. 190 (3), 338-42
- **Cherrington et al.** *Adv. Cancer Res.,* 2000, vol. 79, 1-38
- **Ausubel et al.** Current Protocols in Molecular Biology. Wiley Interscience Publishers, 1995
- **Sambrook et al.** Molecular Cloning: A Laboratory Manual. Cold Spring Harbor Press, 1989
- **Morrison et al.** *Proc. Natl. Acad. Sci. USA,* 1984, vol. 81, 6851-6855
- **Jones et al.** *Nature,* 1986, vol. 321, 522-525
- **Reichmann et al.** *Nature,* 1988, vol. 332, 323-329
- **Presta.** *Curr. Op. Struct. Biol.,* 1992, vol. 2, 593-596
- **Zapata et al.** *Protein Eng.,* 1995, vol. 8 (10), 1057-1062
- **Pluckthun.** The Pharmacology of Monoclonal Antibodies. Springer-Verlag, 1994, vol. 113, 269-315
- **P.D. Zamore.** *Science,* 2002, vol. 296, 1265
- **Hannan ; Rossi.** *Nature,* 2004, vol. 431, 371-378
- **Sandy et al.** *BioTechniques,* 2005, vol. 39, 215-224
- **Paddison et al.** *Proc Natl Acad Sci USA,* 2002, vol. 99 (3), 1443-8
- **Geysen et al.** *Proc. Natl. Acad. Sci. U.S.A,* 1984, vol. 81, 3998-4002
- **Geysen et al.** *Proc. Natl. Acad. Sci. U.S.A.,* 1985, vol. 82, 178-182
- **Geysen et al.** *Synthetic Peptides as Antigens,* 1986, 130-149
- **Geysen et al.** *J. Immunol. Meth.,* 1987, vol. 102, 259-274
- **Schoofs et al.** *J. Immunol.,* 1988, vol. 140, 611-616
- **Cwirla, S. E. et al.** *Proc. Natl. Acad. Sci. USA,* 1990, vol. 87, 6378
- **Lowman, H.B. et al.** *Biochemistry,* 1991, vol. 30, 10832
- **Clackson, T. et al.** *Nature,* 1991, vol. 352, 624
- **Marks, J. D. et al.** *J. Mol. Biol.,* 1991, vol. 222, 581
- **Kang, A.S. et al.** *Proc. Natl. Acad. Sci. USA,* 1991, vol. 88, 8363
- **Smith, G. P.** *Current Opin. Biotechnol.,* 1991, vol. 2, 668
- **Moore et al.** *Cytotechnology,* 1995, vol. 17, 1-11
- **Sias et al.** *J. Immunol. Methods,* 1990, vol. 132, 73-80
- **Agnew.** *Chem Intl. Ed. Engl.,* 1994, vol. 33, 183-186
- Cell cycle regulation, oncogenes, and antineoplastic drugs. **Murakami et al.** The Molecular Basis of Cancer. WB Saunders, 1995, 13
- **Folkman et al.** *Cancer Res.,* 1985, vol. 43, 175-203
- **Ogden et al.** *Biochem Pharmacol.,* 2004, vol. 67, 805
- **Huangfu et al.** *Development,* 2006, vol. 133, 3
- **Jia et al.** *Cell. Mol. Life Sci.,* 2006, vol. 63 (11), 1249-1265
- **Stone et al.** *Nature,* 1996, vol. 384, 129-133
- **Kasper et al.** *Eur. J. Cancer,* 2006, vol. 42, 437
- **Chen et al.** *Proc. Nat. Acad. Sci. USA,* 2006, vol. 95, 2349
- **Price et al.** *Cell,* 2002, vol. 108, 823
- **Price et al.** *Development,* 1999, vol. 126, 4331
- **Ji et al.** *Nature,* 2002, vol. 416, 548
- **Cheng et al.** *Proc. Nat. Acad. Sci. USA,* 2002, vol. 99, 5442
- **Kogerman et al.** *Nat. Cell Biol.,* 1999, vol. 1, 312
- **Ding et al.** *Curr. Biol.,* 1999, vol. 9, 1119
- **Pearse et al.** *Dev. Biol.,* 1999, vol. 212, 323
- **Lum et al.** *Mol. Cell,* 2003, vol. 12, 1261
- **Jia et al.** *Genes Dev.,* 2003, vol. 17, 2709
- **Ruel et al.** *Nat. Cell Biol.,* 2003, vol. 5, 907
- **Ogden et al.** *Curr. Biol.,* 2006, vol. 13, 1998
- **Merchant et al.** *Mol. Cell. Biol.,* 2005, vol. 25, 7054
- **Chen et al.** *Mol. Cell Biol.,* 2005, vol. 25, 7042
- **Varjosalo et al.** *Dev. Cell,* 2006, vol. 10, 177
- **Huangfu et al.** *Nature,* 2006, vol. 426, 83
- **Huangfu et al.** *Proc. Nat. Acad. Sci. USA,* 2005, vol. 102, 11325
- **May et al.** *Dev. Biol.,* 2005, vol. 287, 378
- **Corbit et al.** *Nature,* 2005, vol. 437, 1018
- **Haycraft et al.** *PLoS Genet,* 2005, vol. 1, e53
- **Liu et al.** *Development,* 2003, vol. 132, 1679
- **Avidor-Reiss et al.** *Cell,* 2004, vol. 117, 527
- **Han et al.** *Curr. Biol.,* 2003, vol. 13, 1679
- **Frank-Kamenetsky et al.** *J. Biol.,* 2002, vol. 1, 10
- *J Mol. Med,* 1999, vol. 77 (6), 459-68

- *Cell,* 2000, vol. 100 (4), 423-34
- *Development,* 2000, vol. 127 (19), 4923-4301
- **Dunnett et al.** *J. Exp. Biol.,* 1987, vol. 123, 265-289
- **Apelqvist et al.** *Curr. Biol.,* 1997, vol. 7, 801-4
- **Bellusci et al.** *Development,* 1997, vol. 124, 53
- **Fujita et al.** *Biochem. Biophys. Res.Commun.,* 1997, vol. 238, 658
- **Stone et al.** *Clin. Orthop. Relat. Red.,* 1990, vol. 252, 129
- **Grande et al.** *J. Orthop. Res.,* 1989, vol. 7, 208
- **Takigawa et al.** *Bone Miner,* 1987, vol. 2, 449
- **Walitani et al.** *J. Bone Jt. Surg.,* 1989, vol. 71B, 74
- **Vacanti et al.** *Plast. Resconstr. Surg.,* 1991, vol. 88, 753
- **von Schroeder et al.** *J. Biomed Mater. Res.,* 1991, vol. 25, 329
- **Freed et al.** *J. Biomed. Mater. Res.,* 1993, vol. 27, 11
- **Bitgood et al.** *Cur. Biol,* 1996, vol. 6 (3), 298-304
- **Levine et al.** *J. Neurosci.,* 1997, vol. 17, 6277
- **Likewise, Jensen et al.** *Development,* 1997, vol. 124, 363
- **Sato et al.** *J. Clin. Invest.,* 1999, vol. 104, 855-864
- **Jarov et al.** *Dev. Biol.,* 2003, vol. 261 (2), 520-536
- **Kohler et al.** *Nature,* 1975, vol. 256, 495
- **Goding.** Monoclonal Antibodies: Principles and Practice. Academic Press, 1986, 59-103
- **Kozbor.** *J. Immunol.,* 1984, vol. 133, 3001
- **Brodeur et al.** Monoclonal Antibody Production Techniques and Applications. Marcel Dekker, Inc, 1987, 51-63
- **Riechmann et al.** *Nature,* 1988, vol. 332, 323-329
- **Riechmann et al.** *Nature,* 1988, vol. 332, 323-327
- **Verhoeyen et al.** *Science,* 1988, vol. 239, 1534-1536
- **Sims et al.** *J. Immunol.,* 1993, vol. 151, 2296
- **Chothia et al.** *J. Mol. Biol.,* 1987, vol. 196, 901
- **Carter et al.** *Proc. Natl. Acad. Sci. USA,* 1992, vol. 89, 4285
- **Presta et al.** *J. Immunol.,* 1993, vol. 151, 2623
- **McCafferty et al.** *Nature,* 1990, vol. 348, 552-553
- **Johnson, Kevin S. ; Chiswell, David J.** *Current Opinion in Structural Biology,* 1993, vol. 3, 564-571
- **Clackson et al.** *Nature,* 1991, vol. 352, 624-628
- **Marks et al.** *J. Mol. Biol.,* 1991, vol. 222, 581-597
- **Griffith et al.** *EMBO J.,* 1993, vol. 12, 725-734
- **Morimoto et al.** *J ournal of Biochemical and Bio-physical Methods,* 1992, vol. 24, 107-117
- **Brennan et al.** *Science,* 1985, vol. 229, 81
- **Carter et al.** *Bio/Technology,* 1992, vol. 10, 163-167
- **Vitetta et al.** *Science,* 1987, vol. 238, 1098
- **Liu et al.** *Proc. Natl. Acad. Sci. USA,* 1996, vol. 93, 8618-8623
- **Chari et al.** *Cancer Research,* 1992, vol. 52, 127-131
- **Carlsson et al.** *Biochem. J.,* 1978, vol. 173, 723-737
- **Hinman et al.** *Cancer Research,* 1993, vol. 53, 3336-3342
- **Lode et al.** *Cancer Research,* 1998, vol. 58, 2925-2928
- **Fraker et al.** *Biochem. Biophys. Res. Commun.,* 1978, vol. 80, 49-57
- Monoclonal Antibodies in Immunoscintiography. CRC Press, 1989
- **Geysen et al.** *Proc. Natl. Acad. Sci. U.S.A.,* 1984, vol. 81, 3998-4002
- **Cwirla, S. E. et al.** *Proc. Natl. Acad. Sc i. USA,* 1990, vol. 87, 6378
- **Scott, J.K. ; Smith, G. P.** *Science,* 1990, vol. 249, 386
- **Ren et al.** *Gene,* 1998, vol. 215, 439
- **Zhu et al.** *Cancer Research,* 1998, vol. 58 (15), 3209-3214
- **Jiang et al.** *Infection & Immunity,* 1997, vol. 65 (11), 4770-4777
- **Ren et al.** *Gene,* 1997, vol. 195 (2), 303-311
- **Ren.** *Protein Sci.,* 1996, vol. 5, 1833
- **Efimov et al.** *Virus Genes,* 1995, vol. 10, 173
- **Smith ; Scott.** *Methods in Enzymology,* 1993, vol. 217, 228-257
- **Li et al.** *Mol Biotech.,* 1998, vol. 9, 187
- Antibodies: A Laboratory Manual. Cold Spring Harbor Laboratory, 1988
- **Carter et al.** *Nucl. Acids Res.,* 1986, vol. 13, 4331
- **Zoller et al.** *Nucl. Acids Res.,* 1987, vol. 10, 6487
- **Wells et al.** *Gene,* 1985, vol. 34, 315
- **Wells et al.** *Philos. Trans. R. Soc. London SerA,* 1986, vol. 317, 415
- **Cunningham ; Wells.** *Science,* 1989, vol. 244, 1081-1085
- **Creighton.** The Proteins. W.H. Freeman & Co,
- **Chothia.** *J. Mol. Biol.,* 1976, vol. 150, 1
- **Stewart et al.** Solid-Phase Peptide Synthesis. W.H. Freeman Co, 1969
- **Merrifield.** *J. Am. Chem. Soc.,* 1963, vol. 85, 2149-2154

- **Sambrook et al.** Molecular Cloning: A Laboratory Manual. Cold Spring Harbor Laboratory Press, 1989
- **Dieffenbach et al.** PCR Primer; A Laboratory Manual. Cold Spring Harbor Laboratory Press, 1995
- Mammalian Cell Biotechnology: A Practical Approach. IRL Press, 1991
- **Shaw et al.** *Gene,* 1983, vol. 23, 315
- **Graham ; van der Eb.** *Virology,* 1978, vol. 52, 456-457
- **Van Solingen et al.** *J. Bact,* 1977, vol. 130, 946
- **Hsiao et al.** *Proc. Natl. Acad. Sci. (USA),* 1979, vol. 76, 3829
- **Keown et al.** *Methods in Enzymology,* 1990, vol. 185, 527-537
- **Mansour et al.** *Nature,* 1988, vol. 336, 348-352
- **Beach ; Nurse.** *Nature,* 1981, vol. 290, 140
- **Fleer et al.** *Bio/Technology,* 1991, vol. 9, 968-975
- **Louvencourt et al.** *J. Bacteriol.,* 1983, vol. 154 (2), 737-742
- **Van den Berg et al.** *Bio/Technology,* 1990, vol. 8, 135
- **Sreekrishna et al.** *J. Basic Microbiol.,* 1988, vol. 28, 265-278
- **Case et al.** *Proc. Natl, Acad. Sci. USA,* 1979, vol. 76, 5259-5263
- **Ballance et al.** *Biochem. Biophys. Res. Commun.,* 1983, vol. 112, 284-289
- **Tilbum et al.** *Gene,* 1983, vol. 26, 205-221
- **Yelton et al.** *Proc. Natl. Acad. Sci. USA,* 1984, vol. 81, 1470-1474
- **Kelly ; Hynes.** *EMBO J.,* 1985, vol. 4, 475-479
- **C. Anthony.** *The Biochemistry of Methylotrophs,* 1982, 269
- **Graham et al.** *J. Gen Virol.,* 1977, vol. 36, 59
- **Urlaub et al.** *Proc. Natl. Acad. Sci. USA,* 1980, vol. 77, 4216
- **Mather.** *Biol. Reprod.,* 1980, vol. 23, 243-251
- **Mather et al.** *Annals N.Y. Acad. Sci.,* 1982, vol. 383, 44-68
- **Stinchcomb et al.** *Nature,* 1979, vol. 282, 39
- **Kingsman et al.** *Gene,* 1979, vol. 7, 141
- **Tschemper et al.** *Gene,* 1980, vol. 10, 157
- **Jones.** *Genetics,* 1977, vol. 85, 12
- **Chang et al.** *Nature,* 1978, vol. 275, 615
- **Goeddel et al.** *Nature,* 1979, vol. 281, 544
- **Goeddel.** *Nucleic Acids Res.,* 1980, vol. 8, 4057
- **deBoer et al.** *Proc. Natl. Acad. Sci. USA,* 1983, vol. 80, 21-25
- **Hitzeman et al.** *J. Biol. Chem.,* 1980, vol. 255, 2073
- **Hess et al.** *J. Adv. Enzyme Reg.,* 1968, vol. 7, 149
- **Holland.** *Biochemistry,* 1978, vol. 17, 4900
- **Gething et al.** *Nature,* 1981, vol. 293, 620-625
- **Mantei et al.** *Nature,* 1979, vol. 281, 40-46
- **Ham et al.** *Meth. Enz.,* 1979, vol. 58, 44
- **Barnes et al.** *Anal. Biochem.,* 1980, vol. 102, 255
- **Thomas.** *Proc. Natl. Acad. Sci. USA,* 1980, vol. 77, 5201-5205
- **Deutscher.** *Methods in Enzymology,* 1990, 182
- **Scopes.** Protein Purification: Principles and Practice. Springer-Verlag, 1982
- **Lindmark et al.** *J. Immunol. Meth.,* 1983, vol. 62, 1-13
- **Guss et al.** *EMBO J.,* 1986, vol. 5, 15671575
- Remington: The Science of Practice of Pharmacy. Philadelphia College of Pharmacy and Science, 2000
- Chemotherapy Service. Williams & Wilkins, 1992
- **Anderson et al.** *Science,* 1992, vol. 256, 808-813
- **Stein ; Cohen.** *Cancer Res.,* 1988, vol. 48, 2659
- **van der Krol et al.** *BioTechniques,* 1988, vol. 6, 958
- **Ohkawa. J. et al.** *J. Biochem,* 1995, vol. 118, 251-258
- **Sigurdsson, S. T. ; Eckstein. F.** *Trends Biotechnol,* 1995, vol. 13, 286-289
- **Rossi, J. J.** *Trends Biotechnol.,* 1995, vol. 13, 301-306
- **Kiehntopf, M. et al.** *J. Mol. Med.,* 1995, vol. 73, 65-71
- **Bartel, D. ; Szostak. J. W.** *Science,* 1993, vol. 261, 1411-1418
- **Carlson, J. R.** *Mol. Cell. Biol.,* 1988, vol. 8, 2638-2646
- **Biocca, S. et al.** *EMBO J.,* 1990, vol. 9, 101-108
- **Werge, T. M. et al.** *FEBS Letters,* 1990, vol. 274, 193-198
- **Carlson, J. R.** *Proc. Natl. Acad. Sci. USA,* 1993, vol. 90, 7427-7428
- **Marasco, W. A. et al.** *Proc. Natl. Acad. Sci. USA,* 1993, vol. 90, 7889-7893
- **Biocca, S. et al.** *Bio/Technology,* 1994, vol. 12, 396-399
- **Chen, S-Y. et al.** *Human Gene Therapy,* 1994, vol. 5, 595-601
- **Duan, L et al.** *Proc. Natl. Acad. Sci. USA,* 1994, vol. 91, 5075-5079
- **Chen, S-Y. et al.** *Proc. Natl. Acad. Sci. USA,* 1994, vol. 91, 5932-5936
- **Beerli, R. R. et al.** *J. Biol Chem.,* 1994, vol. 269, 23931-23936

- **Beerli. R. R. et al.** *Biochem. Biophys. Res. Commun.,* 1994, vol. 204, 666-672
- **Mhashilkar, A. M. et al.** *EMBO J,* 1995, vol. 14, 1542-1551
- **Richardson, J. H. et al.** *Proc. Natl. Acad Sci. USA,* 1995, vol. 92, 3137-3141
- **Kabat, E. A. et al.** Sequences of Proteins of Immunological Interest. U.S. Department of Health and Human Services, 1991
- **Fields ; Song.** *Nature (London),* 1989, vol. 340, 245-246
- **Chien et al.** *Proc. Natl. Acad. Sci. USA,* 1991, vol. 88, 9578-9582
- **Chevray ; Nathans.** *Proc. Natl. Acad. Sci. USA,* 1991, vol. 89, 5789-5793
- **Coligan et al.** Current Protocols in Immun. 1991, vol. 1
- **Rossi.** *Current Biology,* 1994, vol. 4, 469-471
- **Peraldi et al.** *J. Biochem.,* 1992, vol. 285, 71-78
- **Schraag et al.** *Anal. Biochem.,* 1993, vol. 211, 233-239
- **Cleavland.** *Anal. Biochem.,* 1990, vol. 190, 249-253
- **Farley.** *Anal. Biochem.,* 1992, vol. 203, 151-157
- **Lozaro.** *Anal. Biochem.,* 1991, vol. 192, 257-261
- **Weernink ; Kijken.** *J. Biochem. Biophs. Methods,* 1996, vol. 31, 49
- **Braunwalder et al.** *Anal. Biochem.,* 1996, vol. 234, 23
- **Kentrup et al.** *J. Biol. Chem.,* 1996, vol. 271, 3488
- **Rusken et al.** *Meth. Enzymol.,* 1991, vol. 200, 98
- **Hanke et al.** *J. Biol. Chem.,* 1996, vol. 271, 695
- **Tiganis et al.** *Arch. Biochem. Biophys.,* 1996, vol. 325, 289
- **Morawetz et al.** *Mol. Gen. Genet.,* 1996, vol. 250, 17
- **Budde et al.** *Int J. Pharmacognosy,* 1995, vol. 33, 27
- **Casnellie.** *Meth. Enz.,* 1991, vol. 200, 115
- **Frank-Kamenetsky et al.** *J Biol.,* 2002, vol. 1, 10
- **Lin et al.** *Nucleic Acid Res.,* 2005, vol. 33, 4527
- **Jackson et al.** *RNA,* 2006, vol. 12, 1197
- **Birmingham et al.** *Nat. Methods,* 2006, vol. 3, 199
- **Fedorov et al.** *RNA,* 2006, vol. 12, 1188
- **Xie et al.** *Nature,* 1998, vol. 391, 90
- **Murone et al.** *Curr. Biol.,* 1999, vol. 9, 76
- **Mahjoub et al.** *Mol. Biol. Cell,* 2004, vol. 15, 5172
- **Wloga et al.** *Mol. Biol. Cell,* 2006, vol. 17, 2799
- **Upadhya et al.** *Proc. Natl. Acad. Sci. USA,* 2000, vol. 97, 217
- **Mahjoub et al.** *J Am. Soc. Nephrol.,* 2005, vol. 16, 3485
- **Surpili et al.** *Biochemistry,* 2003, vol. 42, 15369
- **Echeverri et al.** *Nat. Methods,* 2006, vol. 3, 777
- **Ma et al.** *Nature,* 2006, vol. 443, 359
- **Wang et al.** *Genes Dev.,* 1999, vol. 13, 2838
- **Huang et al.** *J. Biol. Chem,* 2002, vol. 277, 19889
- **Jia et al.** *Dev Cell.,* 2005, vol. 9, 819
- **Lum et al.** *Science,* 2003, vol. 299, 2039
- **Smo. Jia et al.** *Nature,* 2004, vol. 432, 1045
- **Zhang et al.** *Proc. Natl. Acad. Sci. USA,* 2004, vol. 101, 17900
- **Apionishev et al.** *Nat. Cell Biol.,* 2005, vol. 7, 86
- **Nybakken et al.** *Nat. Genet.,* 2005, vol. 37, 1323
- **Soung et al.** *Mol. Cell Biol.,* 2006, vol. 26, 8316-8335
- **Ishikawa et al.** *Nat. Cell Biol.,* 2005, vol. 7, 517
- **Higuchi et al.** *Biotechnology,* 1992, vol. 10, 413-417
- **Livak et al.** *PCR Methods Appl.,* 1995, vol. 4, 357-362
- **Heid et al.** *Genome Res.,* 1996, vol. 6, 986-994
- **Pennica et al.** *Proc. Natl. Acad. Sci. USA,* 1998, vol. 95 (25), 14717-14722
- **Pitti et al.** *Nature,* 1998, vol. 396 (6712), 699-703
- **Bieche et al.** *Int. J. Cancer,* 1998, vol. 78, 661-666
- **Lu ; Gillett.** *Cell Vision,* 1994, vol. 1, 169-176
- **Payne.** *Cancer Cell,* 2003, vol. 3, 207-212
- **Syrigos ; Epenetos.** *Anticancer Research,* 1999, vol. 19, 605-614
- **Niculescu-Duvaz ; Springer.** *Adv. Drug Del. Rev.,* 1997, vol. 26, 151-172
- **Baldwin et al.** *Lancet,* 15 March 1986, 603-05
- Antibody Carriers Of Cytotoxic Agents In Cancer Therapy: A Review. **Thorpe et al.** Monoclonal Antibodies '84: Biological And Clinical Applications. 1985, 475-506
- **Rowland et al.** *Cancer Immunol. Immunother.,* 1986, vol. 21, 183-87
- **Mandler et al.** *J. of the Nat. Cancer Inst.,* 2000, vol. 92 (19), 1573-1581
- **Mandler et al.** *Bioorganic & Med. Chem. Letters,* 2000, vol. 10, 1025-1028
- **Mandler et al.** *Bioconjugate Chem.,* 2002, vol. 13, 786-791

- **Lode et al.** *Cancer Res.,* 1998, vol. 58, 2928
- **Hinman et al.** *Cancer Res.,* 1993, vol. 53, 3336-3342
- **Better et al.** *J. Biol. Chem.,* 1994, vol. 13, 9644-9650
- **Bernhard et al.** *Bioconjugate Chem.,* 1994, vol. 5, 126-132
- **Greenwood et al.** *Therapeutic Immunology,* 1994, vol. 1, 247-255
- **Tu et al.** *Proc. Natl. Acad. Sci USA,* 1999, vol. 96, 4862-4867
- **Kanno et al.** *J. of Biotechnology,* 2000, vol. 76, 207-214
- **Chmura et al.** *Proc. Nat. Acad. Sci. USA,* 2001, vol. 98 (15), 8480-8484
- **Mendoza et al.** *Cancer Res.,* 2002, vol. 62, 5485-5488
- **Bolivar et al.** *Gene,* 1977, vol. 2, 95
- **Thimmappaya et al.** *Cell,* 1982, vol. 31, 543
- **Somparyrac et al.** *Proc. Natl. Acad. Sci.,* 1981, vol. 12, 7575
- **Ausubel et al.** Current Protocols of Molecular Biology. John Wiley and Sons, 1997
- **Lucas et al.** *Nucl. Acids Res.,* 1996, vol. 24 (9), 1774-1779
- **O'Reilley et al.** Baculovirus expression vectors: A Laboratory Manual. Oxford University Press, 1994
- **Rupert et al.** *Nature,* 1993, vol. 362, 175-179